(19) Europäisches Patentamt
European Patent Office
Office européen des brevets

(11) **EP 1 751 276 B1**

(12) **EUROPEAN PATENT SPECIFICATION**

(45) Date of publication and mention of the grant of the patent:
**14.01.2009 Bulletin 2009/03**

(21) Application number: **05741681.0**

(22) Date of filing: **18.05.2005**

(51) Int Cl.:
***C12N 7/04*** *(2006.01)* ***A61K 39/00*** *(2006.01)*
***A61K 39/187*** *(2006.01)*

(86) International application number:
**PCT/EP2005/005377**

(87) International publication number:
**WO 2005/111201 (24.11.2005 Gazette 2005/47)**

# (54) VACCINE COMPRISING AN ATTENUATED PESTIVIRUS

IMPFSTOFF DER EINEN ATTENUIERTEN PESTIVIRUS ENTHÄLT

VACCIN COMPRENANT UN PESTIVIRUS ATTENUE

(84) Designated Contracting States:
**AT BE BG CH CY CZ DE DK EE ES FI FR GB GR HU IE IS IT LI LT LU MC NL PL PT RO SE SI SK TR**
Designated Extension States:
**BA YU**

(30) Priority: **19.05.2004 DE 102004025452**

(43) Date of publication of application:
**14.02.2007 Bulletin 2007/07**

(73) Proprietor: **Boehringer Ingelheim Vetmedica GmbH**
**55216 Ingelheim am Rhein (DE)**

(72) Inventors:
- **MEYERS, Gregor**
  **72141 Walddorfhaeslach (DE)**
- **EGE, Andreas**
  **72076 Tübingen (DE)**
- **MEYER, Christiane**
  **48163 Münster (DE)**
- **VON FREYBURG, Martina**
  **74076 Heilbronn (DE)**

(74) Representative: **Hammann, Heinz**
**Boehringer Ingelheim GmbH**
**CD-Patents**
**Binger Strasse 173**
**55216 Ingelheim am Rhein (DE)**

(56) References cited:
**EP-A- 1 013 757 WO-A-03/023041**
**CA-A1- 2 363 493**

- **MAYER D ET AL: "Attenuation of classical swine fever virus by deletion of the viral N<pro> gene" VACCINE, BUTTERWORTH SCIENTIFIC. GUILDFORD, GB, vol. 22, no. 3-4, 2 January 2004 (2004-01-02), pages 317-328, XP004479351 ISSN: 0264-410X**
- **VAN GENNIP H G P ET AL: "Experimental non-transmissible marker vaccines for classical swine fever (CSF) by trans-complementation of E<rns> or E2 of CSFV" VACCINE, BUTTERWORTH SCIENTIFIC. GUILDFORD, GB, vol. 20, no. 11-12, 22 February 2002 (2002-02-22), pages 1544-1556, XP004340375 ISSN: 0264-410X**
- **BOLIN S R: "CONTROL OF BOVINE VIRAL DIARRHEA INFECTION BY USE OF VACCINATION" VETERINARY CLINICS OF NORTH AMERICA. FOOD ANIMAL PRACTICE, SAUNDERS, PHILADELPHIA, PA, US, vol. 11, no. 3, November 1995 (1995-11), pages 615-625, XP009049066 ISSN: 0749-0720**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 751 276 B1

## Description

## BACKGROUND OF THE INVENTION

### *TECHNICAL FIELD*

The present invention relates to the field of animal health and in particular to attenuated pestiviruses such as bovine viral diarrhea virus (BVDV).

### *BACKGROUND INFORMATION*

Pestiviruses are causative agents of economically important diseases of animals in many countries worldwide. Presently known virus isolates have been grouped into four different species which together form one genus within the family *Flaviviridae.*

I/II Bovine viral diarrhea virus (BVDV) type 1 (BVDV-1) and type 2 (BVDV-2) cause bovine viral diarrhea (BVD) and mucosal disease (MD) in cattle (Baker, 1987; Moennig and Plagemann, 1992; Thiel et al., 1996). The division of BVDV into 2 species is based on significant differences at the level of genomic sequences (summarized in Heinz et al., 2000) which are also obvious from limited cross neutralizing antibody reactions (Ridpath et al. 1994).

III Classical swine fever virus (CSFV), formerly named hog cholera virus, is responsible for classical swine fever (CSF) or hog cholera (HC) (Moennig and Plagemann, 1992; Thiel et al., 1996).

IV Border disease virus (BDV) is typically found in sheep and causes border disease (BD). After intrauterine infection of lambs with BDV persistently infected lambs can be born that are weak and show different abnormalities among which the 'hairy shaker' syndrome is best known (Moennig and Plagemann, 1992; Thiel et al., 1996).

Pestiviruses are small enveloped viruses with a single stranded RNA genome of positive polarity lacking both 5' cap and 3' poly(A) sequences. The viral genome codes for a polyprotein of about 4000 amino acids giving rise to final cleavage products by co- and posttranslational processing involving cellular and viral proteases. The viral proteins are arranged in the polyprotein in the order $NH_2$-$N^{pro}$-C-$E^{rns}$-E1-E2-p7-NS2-NS3-NS4A-NS4B-NSSA-NSSB-COOH (Lindenbach and Rice, 2001). Protein C (= core- or capsidprotein) and the glycoproteins $E^{rns}$, E1 and E2 represent structural components of the pestivirus virion as demonstrated for CSFV (Thiel et al., 1991). This also holds true for BVDV. E2 and to a lesser extent $E^{rns}$ were found to be targets for antibody neutralization (Donis et al., 1988; Paton et al., 1992; van Rijn et al., 1993; Weiland et al., 1990,1992). $E^{rns}$ lacks a typical membrane anchor and is secreted in considerable amounts from the infected cells; this protein has been reported to exhibit RNase activity (Hulst et al., 1994; Schneider et al., 1993; Windisch et al., 1996). The function of this enzymatic activity for the viral life cycle is presently unknown. The enzymatic activity depends on the presence of two stretches of amino acids conserved between the pestivirus $E^{rns}$ and different known RNases of plant and fungal origin. Both of these conserved sequences contain a histidine residue (Schneider et al., 1993). Exchange of each of these residues against lysine in the $E^{rns}$ protein of a CSFV vaccine strain resulted in the destruction of RNase activity (Hulst et al., 1998). Introduction of these mutations into the genome of the CSFV vaccine strain did not influence viral viability or growth properties but led to a virus exhibiting a cytopathogenic phenotype (Hulst et al., 1998). Similarly, Meyers et al. showed that an RNase negative variant of the virulent CSFV strain Alfort/Tübingen was fully viable. However, the respective virus mutant showed no cytopathogenic phenotype (Meyers et al., 1999).

$N^{pro}$ represents the first protein encoded by the long open reading frame in the pestivirus RNA. $N^{pro}$ represents a nonstructural protein that has protease activity and cleaves itself of the nascent polyprotein (Stark et al., 1993; Wiskerchen et al., 1991) presumably already during translation. $N^{pro}$ is a cysteine protease (Rümenapf et al., 1998) that is not essential for virus replication (Tratschin et al., 1998). Recently, it was shown that $N^{pro}$ somehow interferes with the cellular antiviral defense so that it can be hypothesized to modulate the immune system within an infected host (Rüggli et al., 2003). Mayer and coworkers presented indications for an attenuation of CSFV in consequence of a deletion of the' $N^{pro}$ gene (Mayer et al., 2004).

Present BVDV vaccines for the prevention and treatment of BVDV infections still have drawbacks (Oirschot et al. 1999). Vaccines against the classical BVDV-1 provide only partial protection from BVDV-2 infection, and vaccinated dams may produce calves that are persistently infected with virulent BVDV-2 (Bolin et al., 1991, Ridpath et al., 1994). This problem is probably due to the great antigenic diversity between type 1 and type 2 strains which is most pronounced in the glycoprotein E2, the major antigen for virus neutralization (Tijssen et al., 1996). Most monoclonal antibodies against type 1 strains fail to bind to type 2 viruses (Ridpath et al., 1994).

Vaccines comprising attenuated or killed viruses or viral proteins expressed in heterologous expression systems have been generated for CSFV and BVDV and are presently used. Killed vaccines (inactivated whole virus) or subunit vaccines (conventionally purified or heterologously expressed viral proteins) are most often inferior to live vaccines in their efficacy to produce a full protective immune response even in the presence of adjuvants.

The structural basis of the attenuation of BVDV used as life vaccines is not known. These vaccines, although attenuated, are most often associated with safety problems. The vaccine viruses may cross the placenta of pregnant animals, e.g. cows and lead to clinical manifestations in the fetus and/or the induction of persistently infected calves. Therefore, they cannot be applied to breeding herds that contain pregnant cows. Pregnant cows have to be kept separate from vaccinated cattle to protect fetuses and must not be vaccinated themselves. Furthermore, revertants of attenuated live BVDV pose a serious threat to animals. For conventionally derived attenuated viruses wherein the attenuation is achieved by conventional multiple passaging, the molecular origin as well as the genetic stability of the attenuation remains unknown and reversion to the virulent wild-type is unpredictable.

Because of the importance of an effective and safe as well as detectable prophylaxis and treatment of pestiviral infections, there is a strong need for improved attenuated pestiviruses, such as BVDV, with a high potential for induction of immunity as well as a defined basis of attenuation which can also be distinguished from pathogenic pestiviruses, such as BVDV, as well as compositions and vaccines comprising said attenuated pesitiviruses, such as BVDV.

Therefore, the technical problem underlying the present invention is to provide improved attenuated pestivirus, referably an attenuated BVDV for use as live attenuated vaccines. Such improved attenuated pestivirus, preferably BVDV, should especially (i) not cross the placenta themselves and (ii) induce an immunity that prevents viral transmission across the placenta and thereby prevents pregnancy problems like abortion of the fetus or birth of persistently infected host such calves in the case of BVDV infection.

## BRIEF DESCRIPTION OF THE DRAWINGS

Fig. 1 Serum neutralisation against NY93/C (BVDV type II)
Fig. 2 Serum neutralisation assay against KE9 (BVDV type I)
Fig. 3 Serum neutralisation assay against NY93/C (BVDV type II)

All subsequent Sequences are the show the deleted regions indicated with dashes (-), which are also numbered, whereas the sequences in the sequence listing attached hereto are continuously numbered without the deleted regions or amino acid codons.

| | |
|---|---|
| SEQ ID NO:1 | XIKE-A-cDNA sequence |
| SEQ ID NO:2 | XIKE-A-NdN-cDNA sequence |
| SEQ ID NO:3 | XIKE-B-cDNA sequence |
| SEQ ID NO:4 | XIKE-B-NdN-cDNA |
| SEQ ID NO:5 | XIKE-A amino acid sequence |
| SEQ ID NO:6 | XIKE-A-NdN amino acid sequence |
| SEQ ID NO:7 | XIKE-B amino acid sequence |
| SEQ ID NO:8 | XIKE-B-NdN amino acid sequence |
| SEQ ID NO:9 | XIKE-C-NdN amino acid sequence |
| SEQ ID NO:10 | XIKE-C-NdN-cDNA sequence |
| SEQ ID NO:11 | XIKE-C-cDNA sequence |
| SEQ ID NO:12 | XIKE-C amino acid sequence |

## BRIEF SUMMARY OF THE INVENTION

The present invention relates to attenuated pestivivirus, preferably to attenuated BVDV, wherein at least one mutation is in the coding sequence for glycoprotein $E^{rns}$ and at least another mutation in the coding sequence for $N^{pro}$ which preferably leads to combined inactivation of the RNase activity residing in glycoprotein $E^{rns}$ in addition to the inactivation of the (hypothesized) immunemodulating activity residing in $N^{pro}$. The invention also relates to methods for attenuating pestivirus in such that the attenuation results in an attenuated pestivirus, preferably in an attenuated BVDV, as described above. The present invention furthermore relates to nucleic acids molecules encoding said attenuated pestiviruses, preferably encoding attenuated BVDV, compositions and vaccines comprising the attenuated pestivirus, preferably BVDV as disclosed herein.

## DETAILED DESCRIPTION OF THE INVENTION

### *DEFINITIONS OF TERMS USED IN THE DESCRIPTION:*

Before the embodiments of the present invention it must be noted that as used herein and in the appended claims, the singular forms "a", "an", and "the" include plural reference unless the context clearly dictates otherwise. Thus, for example, reference to "a BVDV" includes a plurality of such BVDV, reference to the "cell" is a reference to one or more cells and equivalents thereof known to those skilled in the art, and so forth. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies as reported in the publications which might be used in connection with the invention. Nothing herein is to be construed as an admission that the invention is not entitled to antedate such disclosure by virtue of prior invention.

The term "pestivirus" as used herein refers to all members of the genus *Pestivirus,* including BVDV, CSFV and BDV, within the family *Flaviviridae.*

The term "CSFV" as used herein refers to all viruses belonging to species of classical swine fever virus (CSFV) in the genus *Pestivirus* within the family *Flaviviridae.*

The term "BVDV" as used herein refers to all viruses belonging to species bovine viral diarrhea virus (BVDV) type 1 (BVDV-1) and BVDV type 2 (BVDV-2) in the genus *Pestivirus* within the family *Flaviviridae* (Heinz et al., 2000). The more classical BVDV type 1 strains' and the more recently recognized BVDV type 2 strains display some limited but distinctive differences in nucleotide and amino acid sequences.

The term "$N^{pro}$" as understood herein relates to the first protein encoded by the viral open reading frame and cleaves itself from the rest of the synthesized polyprotein (Stark, et. al., J. Virol. 67:7088-7093 (1993); Wiskerchen, et al., Virol. 65:4508-4514 (1991)). Said term, depending on-the context, may also relate to the remaining "$N^{pro}$" amino acids after mutation of the encoding nucleotide sequence or to the coding nucleotide sequence for said protein itself. "Protease activity residing in $N^{pro}$" relates to the polypeptide cleavage activity of said "$N^{pro}$".

"$E^{rns}$" as used herein relates to the glycoprotein $E^{rns}$ which represents a structural component of the pestivirus virion (Thiel et al., 1991). $E^{rns}$ lacks a typical membrane anchor and is secreted in considerable amounts from the infected cells; this protein has been reported to exhibit RNase activity (Hulst et al., 1994; Schneider et al., 1993; Windisch et al., 1996). It should be noted that the term glycoprotein E0 is often used synonymously to glycoprotein $E^{rns}$ in publications. Said term, depending on the context, may also relate to the mutated "$E^{rns}$" protein after mutation of the encoding nucleotide sequence or to the coding nucleotide sequence for said protein itself. "RNase activity residing in glycoprotein $E^{rns}$" relates to the RNA cleavage activity of said glycoprotein, i.e. the ability of the glycoprotein $E^{rns}$ to hydrolyze RNA. The term "inactivation of the RNase activity residing in said glycoprotein" refers to the inability or reduced capability of a modified glycoprotein $E^{rns}$ to hydrolyze RNA as compared to the unmodified wild type of said glycoprotein $E^{rns}$.

Attenuation: "An attenuated pestivirus or BVDV particle" as used herein means that there is a statistically significant difference between the virulence of attenuated pestivirus or BVDV particles of the present invention, wherein said attenuated viral particles being attenuated by a method described herein, and wild-type pestivirus or BVDV isolates from which said attenuated pestivirus or BVDV particles have been derived, for the predominant clinical parameters, in case of BVDV for diarrhea, pyrexia and lethality in animals infected with the same dose, preferably $6x10^6 TCID_{50}$. Thus, said attenuated BVDV particles do not cause diarrhea, pyrexia and lethality and thus may be used in a vaccine.

Inactivation of $E^{rns}$ as used herein means RNase activity not significantly above the level measured for noninfected control cells in an RNase assay as described in Meyers et al., 1999. "Not significantly above the level measured for noninfected control cells in an RNase assay as described in Meyers et al., 1999, means for example, that the RNase activity is less than 150% compared to the noninfected control cells.

Inactivation of $N^{pro}$ as used herein means the prevention or considerable reduction of the probable immunemodulating activity of $N^{pro}$ by mutation. In a preferred embodiment this mutation prevents or considerably reduces the interference of $N^{pro}$ with the induction of an interferon response by the infected cells as described by Rüggli et al., (2003). In this case, the inactivation of $N^{pro}$ would allow the cell to mount a normal interferon response.

"Processing signal" as used herein relates to a substance that ensures the generation of a functional N-terminal of the C protein of the pestivirus, preferably of BVDV, in particular a substance selected from the group of ubiquitin, LC3, SUMO-1, NEDD8, GATE-16 and GABA(A)RAP. Also proteases selected from the group of Intein, picornavirus 3C, caridovirus 2A and p15 of rabbit hemorrhagic disease virus are understood as "processing signals" as used herein. Any other similar processing signal known to the skilled person that ensures the generation of a functional N-terminal of the C protein shall also be comprised in the term "processing signal".

"Protein C" or "C protein" or "C-protein" as used herein relates to a structural component of the pestivirus virion (Thiel et al., 1991). "Protein C" is the capsid or core protein of pestiviruses. Said term, depending on the context, may also relate to the "Protein C" with one or several amino acids exchanges resulting from mutation of the encoding nucleotide sequence.

A "fragment" according to the invention is any subunit of a polynucleotide molecule according to the invention, i.e. any subset. For DNA, said fragment is characterized in that it is shorter than the DNA covering the full length viral genome.

A "functional variant" of the nucleotide molecule according to the invention is a nucleotide molecule which possesses a biological activity (either functional or structural) that is substantially similar to the nucleotide molecule according to the invention. The term "functional variant" also includes "a fragment", "a functional variant", "variant based on the degenerative nucleic acid code" or "chemical derivative". Such a "functional variant" e.g. may carry one or several nucleotide exchanges, deletions or insertions. Said functional variant at least partially retains its biological activity, e.g. function as an infectious clone or a vaccine strain, or even exhibits improved biological activity. "Possess a biological activity that is substantially similar" means with respect to the pestiviruses provided herewith, for example, that said pestivirus is attenuated in a manner described herein and result in an non-pathogenic virus suitable for the production of live attenuated virus, which loss ability to pass the placenta but mediates an immune response after vaccination.

A "variant based on the degenerative nature of the genetic code" is a variant resulting from the fact that a certain amino acid may be encoded by several different nucleotide triplets. Said variant at least partially retains its biological activity, or even exhibits improved biological activity.

A molecule is "substantially similar" to another molecule if both molecules have substantially similar nucleotide sequences or biological activity. Thus, provided that two molecules possess a similar activity, they are considered variants as that term is used herein if the nucleotide sequence is not identical, and two molecules which have a similar nucleotide sequence are considered variants as that term is used herein even if their biological activity is not identical.

A mutation as used herein relates to modifications in the nucleic acid molecules encoding the proteins / amino acids according to the invention. Said mutations relate to, but are not limited to, substitutions (replacement of one or several nucleotides/base pairs), deletions (removal of one or several nucleotides/base pairs), and/or insertions (addition of one or several nucleotides/base pairs). As used herein, mutation may be a single mutation or several mutations, therefore, often the term "mutation(s)" is used and relates to both a single mutation and several mutations. Said mutations include, but are not limited to point mutations (single nucleotide mutations) or larger mutations wherein e.g. parts of the encoding nucleic acid molecules are deleted, substituted and/or additional coding nucleic acid is inserted. Said mutations may result in a modified expressed polypeptide due to the change in the coding sequence. Such modified polypeptides are desired, as set out in the disclosure of the invention as set out below.

The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete virus particles in either their original form or as attenuated particles in a so called modified live vaccine (MLV) or particles inactivated by appropriate methods in a so called killed vaccine (KV). In another form the immunologically active component of a vaccine may comprise appropriate elements of said organisms (subunit vaccines) whereby these elements are generated either by destroying the whole particle or the growth cultures containing such particles and optionally subsequent purification steps yielding the desired structure(s), or by synthetic processes including an appropriate manipulation by use of a suitable system based on, for example, bacteria, insects, mammalian or other species plus optionally subsequent isolation and purification procedures, or by induction of said synthetic processes in the animal needing a vaccine by direct incorporation of genetic material using suitable pharmaceutical compositions (polynucleotide vaccination). A vaccine may comprise one or simultaneously more than one of the elements described above. The term "vaccine" as understood herein is a vaccine for veterinary use comprising antigenic substances and is administered for the purpose of inducing a specific and active immunity against a disease provoked by a pestivirus infection, preferably by a BVDV infection. The attenuated pestivirus, in particular the attenuated BVDV as described herein, confer active immunity that may be transferred passively via maternal antibodies against the immunogens it contains and sometimes also against antigenically related organisms. A vaccine of the invention refers to a vaccine as defined above, wherein one immunologically active component is a BVDV or of pestiviral origin or derived from a nucleotide sequence that is more than 70% homologous to any known pestivirus sequence (sense or antisense).

The term "live vaccine" refers to a vaccine comprising a living, in particular, a living viral active component.

Additional components to enhance the immune response are constituents commonly referred to as "adjuvants", like e.g. aluminiumhydroxide, mineral or other oils or ancillary molecules added to the vaccine or generated by the body after the respective induction by such additional components, like but not restricted to interferons, interleukins or growth factors.

A "pharmaceutical composition" essentially consists of one or more ingredients capable of modifying physiological e.g. immunological functions of the organism it is administered to, or of organisms living in or on the organism.

The term includes, but is not restricted to, antibiotics or antiparasitics, as well as other constituents commonly used to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, controlled release properties. One non-limiting example of such a pharmaceutical composition, solely given for demonstration purposes, could be prepared as follows: Cell culture supernatant of an infected cell culture is mixed with a stabilizer (e.g. spermidine and/or BSA (bovine serum albumin)) and the mixture is subsequently lyophilized or dehydrated by other methods. Prior to vaccination, said mixture is then rehydrated in aqueous (e.g. saline, PBS (phosphate buffered saline)) or nonaqueous solutions (e.g. oil emulsion, aluminum-based adjuvant).

***DISCLOSURE OF THE INVENTION***

The solution to the above technical problem is achieved by the description and the embodiments characterized in the claims.

It has surprisingly been found that pestiviruses, in particular BVDV can be more effectively attenuated by introducing at least one mutation in the coding sequence for glycoprotein $E^{rns}$ and at least another mutation in the coding sequence for $N^{pro}$ which preferably leads to combined inactivation of the RNase activity residing in glycoprotein $E^{rns}$ in addition to the inactivation of the immunomodulating activity residing in $N^{pro}$. An immunomodulating effect in one aspect is indicated but not limited to the indicated function for one pestivirus in an exemplary manner by Rüggli et al. (2003)..

A pestivirus, in particular BVDV attenuated in accordance with the present invention may be advantageously used in vaccines. Said attenuated pestivirus, in particular said attenuated BVDV now provide live vaccines of high immunogenicity. Surprisingly, the pestivirus, in particular the BVDV according to the invention furthermore are safe for use in pregnant animals as they do not cross the placenta. This is exemplified in a non-limiting manner for BVDV in example 3.

Furthermore, live vaccines with defined mutations as a basis for attenuation will allow to avoid the disadvantages of the present generation of vaccines, e.g. the risk of reversion to an mor pathogenic strain. A further advantage of said attenuating mutations lies in their molecular uniqueness which allows to use them as distinctive labels for an attenuated pestivirus, in particular BVDV and to distinguish them from pestivirus, in particular BVDV from the field. Therefore, in one aspect the present invention provides an attenuated pestivirus, in particular an attenuated BVDV having at least one mutation in the coding sequence for glycoprotein $E^{rns}$ and at least another mutation in the coding sequence for $N^{pro}$. Preferably, in such attenuated pestivirus, preferably in such attenuated BVDV said mutation in the coding sequence for glycoprotein $E^{rns}$ leads to inactivation of the RNase activity residing in $E^{rns}$ and/or said mutation in the coding sequence for $N^{pro}$ leads to inactivation of said $N^{pro}$. Said inactivation may take place by any mutation known to the person skilled in the art of the $E^{rns}$- and the $N^{pro}$-coding sequence, wherein the mutations are any mutation as defined in the "definitions" section, such as deletions, insertion mutations and/or substitution mutations. Most preferably, the mutation(s) are deletions, as the likelihood for revertation to the wild type is the lowest for deletions.

It has been shown that the glycoprotein $E^{rns}$ forms a disulfide-bonded homodimer of about 97 kD, wherein each monomer consists of 227 amino acids corresponding to the amino acids 268 to 494 of the CSFV polyprotein as described by Rümenapf et al. (1993). The genome sequence of the Alfort/Tübingen strain of CSFV is available in the GenBank/EMBL data library under accession number J04358; alternatively, the amino acid sequence for the BVDV strain CP7 can be accessed in the GenBank/EMBL data library (accession number U63479); in the BVDV CP7 polyprotein, the $E^{rns}$ protein corresponds to residues 271 to 497. Two regions of amino acids are highly conserved in glycoprotein $E^{rns}$ as well as in some plant and fungal RNase-active proteins (Schneider et al., 1993). These two regions are of particular importance to the RNase enzymatic activity. The first region consists of the region at the amino acids at position 295 to 307 (298 to 310 for BVDV strain cp7) and the second region consists of the amino acids at position 338 to 357 (341 to 360 for BVDV strain cp7) of said viral polyprotein as exemplified for the Alfort strain of CSFV in Meyers et al., 1999 (numbering according to the published deduced amino acid sequence of CSFV strain Alfort/Tübingen (Meyers et al., 1989). The amino acids of particular importance to the RNase activity as mentioned above are by no means limited to the exact position as defined for the Alfort/Tübingen strain of CSFV but are simply used in an exemplary manner to point out the preferred amino acids being at that position or corresponding to that position in other strains such as found in BVDV, BDV and pestiviruses in general since they are highly conserved. For pestiviruses other than the CSFV Alfort/Tübingen strain the numbering of the positions of the preferred amino acids can be different but an expert in the field of the molecular biology of pestiviruses will easily identify these preferred amino acids by the high degree of conservation of this amino acid sequence and the position of these motifs in the sequence context. In one particular non-limiting example, the position of CSFV Alfort/Tübingen 346 is identical to position 349 of BVDV strain cp7.

As a consequence, the present invention preferably relates to a BVDV according to the invention, wherein said mutation(s) in the coding sequence for glycoprotein $E^{rns}$ are located in the encoding nucleotide sequence corresponding to amino acids at position 298 to 310 and/or position 341 to 360. Preferably, such mutations are (amino acids are given in the one letter symbols; the amino acid before the position number indicates the amino acid to be substituted, the amino acid after the position number the substituting amino acid (del indicates deletion): for example, H300L means histidine 300 was substituted by leucine:

Suitable modification of the glycoprotein $E^{rns}$ are for example, the single substitutions/deletions: S298G, H300K, H300L, H300R, H300del, W303G, P304del, E305A, C308G, R343G, E345del, W346G, K348A, H349K, H349L, H349del, H349Q, H349SV (mutation H349S and insertion of V), K348R, W351P, W351G, W351L, W351K, W351H; the double substitutions/deletions: H300L/H349L, K348del/H349del, H349del/G350del, E345del/H349del, W303G/E305A, H300K/H349K, H300K/H349L and the triple deletions: L299del/H300del/G300del, K348del/H349del/G350del. Numbering is according to the published amino acid sequence of BVDV CP7 for all the mutants listed above (the given numbers minus 3 would correspond to the equivalent residues of the CSFV Alfort/Tübingen amino acid sequence). All the above-listed mutants were at least tested as respective CSFV or BVDV mutants without mutations in the $N^{pro}$region. Suitable mutants of the pestiviral glycoprotein $E^{rns}$ are provided, for example, by WO 99/64604, which is incorporated herein at its whole. It should be noted, however, that according to the present invention, at least one additional mutation in the $N^{pro}$ region, as disclosed in further detail below, must be present.

It was particularly found that deletion or substitution of the histidine residue at position 346 (CSFV) or 349 (BVDV) leads to effective inactivation of $E^{rns}$ and therefore leads to particularly useful pestiviral live vaccines. The present invention demonstrates that pestiviruses are viable and code for an $E^{rns}$ protein without RNase activity when the histidine residue at position 346 of the viral polyprotein (numbering according to the published sequence of CSFV Alfort/Tübingen (Meyers et al., 1989)), or at position 349 (numbering according to the published sequence of BVDV CP7 (Meyers et al., 1996b)) if said pestivirus is BVDV, which represents one of the conserved putative active site residues of the $E^{rns}$ RNase, is deleted. Thus, preferably, the invention also relates to a BVDV according to the invention, wherein said mutation in the coding sequence for glycoprotein $E^{rns}$ is a deletion or substitution of the histidine residue at position 349. Even more specifically, the putative active site of the RNase is represented by the conserved $E^{rns}$ sequences SLHGIWPEKICTG and/or LQRHEWNKHGWCNWFHIEPW (sequence of the BVDV-2 New York'93 protein given here in an exemplary manner; minor changes can possibly be found in other pestivirus sequences but the identity of the motif will always be obvious for an expert in the field. As an example, the corresponding amino acid sequences of BVDV-1- CP7 would be SLHGIWPEKICTG and/or LQRHEWNKHGWCNWYNIEPW and that of CSFV Alfort/Tübingen SLHGIWPEKICKG and/or LQRHEWNKHGWCNWYNIDPW). Thus, preferably, the invention further relates to a BVDV according to the invention, wherein said mutation(s) in the coding sequence for glycoprotein $E^{rns}$ are located in the nucleotide sequence coding for the conserved $E^{rns}$ sequence SLHGRWPEKICTG and/or LQRHEWNKHGWCNWFHIEPW. These sequences are representing the putative active site of the RNase. The sequences SLHGIWPEKIC and RHEWNKHGWCNW of the putative $E^{rns}$ active site are even more conserved across pestiviruses. Thus, preferably, the invention also relates to a pestivirus, in particular to BVDV having at least one mutation in the coding sequence of the $N^{rns}$ protein and the glycoprotein $E^{rns}$, wherein said mutation(s) in the coding sequence for glycoprotein $E^{rns}$ are located in the nucleotide sequence coding for the conserved $E^{rns}$ sequence SLHGIWPEKIC and/or RHEWNKHGWCNW. Preferably, the mutation is located in only one of said sequences. Thus the invention also relates to a pestivirus, in particular to BVDV having at least one mutation in the coding sequence of the $N^{pro}$ protein and the glycoprotein $E^{rns}$, wherein said mutation(s) in the coding sequence for glycoprotein $E^{rns}$ are located in the nucleotide sequence coding for the conserved $E^{rns}$ sequence SLHGIWPEKIC or RHEWNKHGWCNW. Preferably, such mutations concern two different amino acids, i.e. are double mutations. Thus, said mutations may be 1 to 3 nucleotide mutations in two different tripletts encoding two amino acids. Thus, the invention also relates to a pestivirus, in particular to BVDV having at least one mutation in the coding sequence of the $N^{pro}$ protein and the glycoprotein $E^{rns}$, wherein said mutation(s) in the coding sequence for glycoprotein $E^{rns}$ are two mutations located in the nucleotide sequence coding for the conserved $E^{rns}$ sequence SLHGIWPEKIC and/or RHEWNKHGWCNW. Preferably, such mutations concern a single amino acid. Thus, said mutation may be 1 to 3 nucleotide mutations in one triplett encoding one amino acid. Thus, the invention also relates to a pestivirus, in particular to BVDV having at least one mutation in the coding sequence of the $N^{pro}$ protein and the glycoprotein $E^{rns}$, wherein a single mutation is located in the conserved $E^{rns}$ sequence SLHGIWPEKIC or RHEWNKHGWCNW.

As mentioned above, the attenuated pestiviruses provided by the present invention, having at least on mutation in the coding sequence of the glycoprotein $E^{rns}$ and in the coding sequence of the the $N^{Pro}$ protein, wherein said mutation preferably result in inactivation of the RNase activity residing in the glycoprotein $E^{RNS}$ and of the immunomodulating activity residing in $N^{pro}$. Inactivation of the $N^{pro}$ is achieved in pestiviruses, in particular BVDV of the specified formula described more in detail below, wherein between 0 and all amino acids of $N^{pro}$ are present; ubiquitin or LC3 or another sequence serving as processing signal (e.g. SUMO-1, NEDD8, GATE-16,GABA(A)RAP, or proteases like e.g. Intein, picornavirus 3C, caridovirus 2A, or p15 of rabbit hemorrhagic disease virus) is present or absent. In case a processing signal is present, the coding sequence of the processing signal is inserted at or close to the C-terminal end of the (remaining part of the) $N^{pro}$-protein. Only in the case that a processing signal is present, any number of amino acids coding for $N^{pro}$ (=$N^{pro}$ amino acids) may be present. In case no processing signal sequence is inserted, a maximum of 12 amino acids, preferably aminoterminal amino acids, of $N^{pro}$ may be present, the remaining amino acids have to be deleted. Furthermore, other than the $E^{rns}$ mutations as disclosed above (at least one of which has to be present in the pestivirus, in particular in BVDV according to the invention), the remaining sequences of the pestivirus, in particular BVDV may remain unchanged, i.e. are not mutated, or may also have mutations close to the N-terminal end of the C-protein. A number of more specific embodiments as disclosed below exemplify this.

Thus, the invention relates to a pestivirus, in particular to BVDV according to the invention, wherein said mutation (s) in the coding sequence for $N^{pro}$ lead to an encoded polyprotein as characterized by the following formula:

$$[N^{pro}]_x\text{-}[PS]_y\text{-}[C\text{-term}]$$

and wherein:

[$N^{pro}$] relates to the $N^{pro}$ portion of said polyprotein, wherein "x" represents the number of amino acids of the $N^{pro}$present in the polyprotein;

[PS] relates to a processing signal selected from: ubiquitin, LC3, SUMO-1, NEDD8, GATE-16 or GABA(A)RAP) or proteases like e.g. Intein, picornavirus 3C, caridovirus 2A, or p15 of rabbit hemorrhagic disease virus or any processing signal known to the skilled person that ensures the generation of a functional N-terminal of the C-protein. "Y" may be = 0, which means that no processing signal is present (= PS is absent), or "Y" may be = 1, which means that a processing signal is present (= PS present).

**[C-term]** relates to the complete pestivirus, in particular the complete BVDV polyprotein except for $N^{pro}$, but including the capsid (C)-protein and any other protein present in the pestivirus polyprotein, in particular in the BVDV polyprotein including the carboxyterminal NS5B. Preferably, the glycoprotein $E^{rns}$ in said [C-term] is mutated, in such that the RNase activity residing in the glycoprotein $E^{rns}$ is inactivated. The term "any other protein present in the pestivirus polyprotein /BVDV polyprotein" relates to $E^{rns}$, E1, E2, p7, NS2, NS3, NS4A, NS4B and NS5A, wherin glycoprotein $E^{rns}$ is mutated, preferably as disclosed herein (see above), in such that the RNase activity residing in the glycoprotein $E^{rns}$ is inactivated. Preferably, the pestivirus, in particular the BVDV according to the invention has a C-protein which is not mutated except for the amino acid at position 2 which is changed from D to N. Therefore, [C-term*] is the same as [C-term] but with a mutation at position 2 of the C-protein (N instead of D);

if "y" is = 0 (means no [PS] present) then"x" is 0 to 12, (means no $N^{pro}$ specific amino acid or 1 to 12 amino acids of $N^{pro}$, preferably of the N-terminus of $N^{pro}$, are present);

if "y" is = 1 (means [PS] is present) then "x" is 0 to 168; (means no $N^{pro}$ specific amino acid or 1 to all 168 amino acids of $N^{pro}$, preferably of the N-terminus of $N^{pro}$, are present).

Also more preferably, the invention relates to a pestivirus, in particular to BVDV according to the invention, wherein said mutation(s) in the coding sequence for $N^{pro}$ lead to an encoded polyprotein as characterized by the following formula:

$$[N^{pro}]_1\text{-}[PS]_0\text{-}[C\text{-term}]$$

and wherein the definitions are as defined above.

A specific example thereof is disclosed below, wherein the N-terminal methionine is followed by the C-protein and any other protein present in the polyprotein including the carboxyterminal NS5B Hence, most preferably, the invention relates to a pestivirus, in particular BVDV according to the invention, wherein said mutation(s) in the coding sequence for $N^{pro}$ lead to an encoded polyprotein as characterized by the following formula:

$$M[C\text{-term}].$$

and wherein the definitions are as defined above.

Also more preferably, the invention relates to a pestivirus, in particular to BVDV according to the invention, wherein said mutation(s) in the coding sequence for $N^{pro}$ lead to an encoded polyprotein as characterized by the following formula:

$$[N^{pro}]_3\text{-}[PS]_0\text{-}[C\text{-term}]$$

and wherein the definitions are as defined above.

A specific example of BVDV is disclosed below, wherein the N-terminal methionine is followed by the $N^{pro}$ sequence EL and the C-protein and any other protein present in the polyprotein including the carboxyterminal NS5B. Hence, most preferably, the invention relates to a BVDV according to the invention, wherein said mutation(s) in the coding sequence for $N^{pro}$ lead to an encoded polyprotein as characterized by the following formula:

MEL-[C-term]

and wherein the definitions are as defined above.

Also more preferably, the invention relates to a pestivirus, in particular to BVDV according to the invention, wherein said mutation(s) in the coding sequence for $N^{pro}$ lead to an encoded polyprotein as characterized by the following formula:

$$[N^{pro}]_4\text{-}[PS]_0\text{-}[C\text{-term}]$$

and wherein the definitions are as defined above.

A specific example of BVDV is disclosed below, wherein the N-terminal methionine is followed by the $N^{pro}$ sequence ELF and the C-protein and any other protein present in the polyprotein including the carboxyterminal NS5B. Hence, most preferably, the invention relates to a BVDV according to the invention, wherein said mutation(s) in the coding sequence for $N^{pro}$ lead to an encoded polyprotein as characterized by the following formula:

MELF-[C-term].

and wherein the definitions are as defined above.

Also more preferably, the invention relates to pestivirus, in particular to BVDV according to the invention, wherein said mutation(s) in the coding sequence for $N^{pro}$ lead to an encoded polyprotein as characterized by the following formula:

$$[N^{pro}]_6\text{-}[PS]_0\text{-}[C\text{-term}]$$

and wherein the definitions are as defined above.

A specific example of BVDV is disclosed below, wherein the N-terminal methionine is followed by the $N^{pro}$ sequence ELFSN and the C-protein and any other protein present in the polyprotein including the carboxyterminal NS5B. Hence, most preferably, the invention relates to a BVDV according to the invention, wherein said mutation(s) in the coding sequence for $N^{pro}$ lead to an encoded polyprotein as characterized by the following formula:

MELFSN-[C-term].

and wherein the definitions are as defined above.

Also more preferably, the invention relates to a pestivirus, in particular to BVDV according to the invention, wherein said mutation(s) in the coding sequence for $N^{pro}$ lead to an encoded polyprotein as characterized by the following formula:

$$[N^{pro}]_4\text{-}[PS]_0\text{-}[C\text{-term*}]$$

and wherein the definitions are as defined above except for the fact that the aminoterminal part of the C-protein is changed.

A specific example of BVDV is disclosed below, wherein the N-terminal methionine is followed by the $N^{pro}$ sequence ELF and in the C-protein sequence, the amino acid at position 2 is changed from D to N. Therefore, the aminoterminal C-protein sequence is SNEGSK... instead of SDEGSK. Hence, most preferably, the invention relates to a BVDV according to the invention, wherein said mutation(s) in the coding sequence for $N^{pro}$ lead to an encoded polyprotein as characterized by the following formula:

MELF-[C-term*],

wherein in the C-protein the amino acid at position 2 is changed from D to N, and wherein the definitions are as defined above.

Also more preferably, the invention relates to a pestivirus, in particular BVDV according to the invention, wherein said mutation(s) in the coding sequence for $N^{pro}$ lead to an encoded polyprotein as characterized by the following formula:

$$[N^{pro}]_x\text{-}[PS]_1\text{-}[C\text{-term}],$$

wherein the definitions are as defined as above,
and wherein PS is any of the PS disclosed above, preferably selected from the group of ubiquitin or LC3.

A specific example of BVDV is disclosed below, wherein the N-terminal methionine is followed by any 21 or 28 $N^{pro}$ amino acids, ubiquitin or LC3 and the C-protein. Hence most preferably, the invention relates to a BVDV according to the invention, wherein said mutation(s) in the coding sequence for $N^{pro}$ lead to an encoded polyprotein as characterized by the following formula:

$[N^{pro}]_{22}\text{-}[PS]_1\text{-}[C\text{-term}]$, wherein preferably, the PS is ubiquitin or LC3

or

$[N^{pro}]_{29}\text{-}[PS]_1\text{-}[C\text{-term}]$, wherein preferably, the PS is ubiquitin or LC3.

Ubiquitin is a well known highly conserved cellular protein of 76 amino acids. Among other functions, ubiquitin is a key player in protein catabolism since conjugation with ubiquitin can mark a protein for degradation via the proteasome. Ubiquitin conjugated with or fused to other proteins via the carboxyterminal glycin can be cleaved off by cellular ubiquitin-specific proteases. Thus, fusion of a protein to the carboxyterminus of ubiquitin will usually result in defined proteolytic cleavage of the fusion protein into its components when expressed within a cell.

LC3 (light chain 3 of microtubule associated proteins) represents a cellular protein of 125 amino acids that serves a variety of functions (length given for bovine LC3). Recently, a fundamental role of the protein in autophagy has been defined. During this process, LC3 is activated by carboxyterminal cleavage. Thereby, a new carboxyterminus is generated that consists of glycine. LC3 is then conjugated via the carboxyterminal glycine to phosphatidylethanolamine present in the membranes of autophagic vesicles. Because of this process, a protein fused to the carboxyterminus of LC3 will be cleaved off by a cellular protease at a defined position.

Also more preferably, the invention relates to a pestivirus, preferably to BVDV according to the invention, wherein said mutation(s) in the coding sequence for $N^{pro}$ lead to an encoded polyprotein as characterized by the following formula selected from the group of :

$[N^{pro}]_2\text{-}[PS]_y\text{-}[C\text{-term}]$ and preferably ME-$[PS]_y$-[C-term];

$[N^{pro}]_5$-$[PS]_y$-[C-term] and preferably MELFS-$[PS]_y$-[C-term];

$[N^{pro}]_7$-$[PS]_y$-[C-term] and preferably MELFSNE-$[PS]_y$-[C-term];

$[N^{pro}]_8$-$[PS]_y$-[C-term] and preferably MELFSNEL-$[PS]_y$-[C-term];

$[N^{pro}]_9$-$[PS]_y$-[C-term] and preferably MELFSNELL-$[PS]_y$-[C-term];

$[N^{pro}]_{10}$-$[PS]_y$-[C-term] and preferably MELFSNELLY-$[PS]_y$-[C-term];

$[N^{pro}]_{11}$-$[PS]_y$-[C-term] and preferably MELFSNELLYK-$[PS]_y$-[C-term];

and

$[N^{pro}]_{12}$-$[PS]_y$-[C-term] and preferably MELFSNELLYKT-$[PS]_y$-[C-term]

and wherein the definitions are as defined as above. The preferably disclosed embodiments refers to BVDV. Most preferably, y is 0 (no PS present).

Also more preferably, said BVDV according to the invention as described *supra* is a BVDV type 1 BVDV. Most preferably, said BVDV according to the invention as described *supra* is a BVDV type 2 BVDV. BVDV-1 and BVDV-2 are differentiated according to features of their genomic sequences (Heinz et al., 2000 and references therein). BVDV-1 as disclosed herein may be used in the manufacture of a composition for use in the prevention and/or treatment of BVDV type 1 infections in breeding stocks of cattle, in pregnant cows and in the induction of fetal protection against BVDV type 1 infection is pregnant cows. Surprisingly, a BVDV-2 as disclosed herein may be used in the manufacture of a composition for use in the prevention and/or treatment of BVDV type 1 infections in breeding stocks of cattle. In particular, the invention relates to the use of a BVDV type 2 according to the invention in the manufacture of a composition for use in the prevention and/or treatment of BVDV type 1 infections in pregnant cows. Preferably, the BVDV type 2 according to the invention may be used in the manufacture of a composition for use in the induction of fetal protection against BVDV type 1 infections in pregnant cows. Surprisingly also, a BVDV-1 as disclosed herein may be used in the manufacture of a composition for use in the prevention and/or treatment of BVDV type 2 infections in breeding stocks of cattle. In particular, the invention relates to the use of a BVDV type 1 according to the invention in the manufacture of a composition for use in the prevention and/or treatment of BVDV type 2 infections in pregnant cows. Preferably, the BVDV type 1 according to the invention may be used in the manufacture of a composition for use in the induction of fetal protection against BVDV type 2 infections in pregnant cows. Most preferred is the use of BVDV type 1 and type 2 in combination for the manufacture of a composition for use in the prevention and/or treatment of BVDV type 1 and or type 2 infections in breeding stocks of cattle, in pregnant cows and in the induction of fetal protection against BVDV type 1 and/or type 2 infections is pregnant cows.

Most preferably, the wild type BVDV according to the invention which is to be mutated as disclosed herein corresponds to amino acid sequence SEQ ID No. 5 (termed XIKE A) or is a functional variant thereof. Most preferably also, the BVDV according to the invention has a $N^{pro}$ mutation according to the invention and corresponds to amino acid sequence SEQ ID No. 6 (termed XIKE-A-NdN) or is a functional variant thereof. Preferably, such a functional variant is at least 65% homologous to the amino acid sequence disclosed herein. On the amino acid level, homologies are very roughly: BVDV-1/-BVDV-1: 93%; BVDV-1/-BVDV-2: 84%; BVDV-2/-BVDV-2: 98%. Therefore, more preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant.is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

Most preferably also, the BVDV according to the invention has a $E^{rns}$ mutation according to the invention which has a deletion of the codon coding for histidine 349, and corresponds to amino acid sequence SEQ ID No. 7 (termed XIKE-B) or is a functional variant thereof. Most preferably also, the BVDV according to the invention has both a $E^{rns}$ mutation and a $N^{pro}$ mutation according to the invention, wherein the codon coding for histidine 349 of $E^{rns}$ is deleted and also the complete $N^{pro}$ coding region is deleted, except for codons 1 to 4, thus amino acids MELF of $N^{pro}$ remain. Said mutant corresponds to amino acid sequence SEQ ID No. 8 (termed XIKE-B-NdN) or is a functional variant thereof. Preferably, such a functional variant is at least 65% homologous to the amino acid sequence disclosed herein. More preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

Most preferably also, the BVDV according to the invention has a $E^{rns}$ mutation according to the invention which has a substitution of the codon coding for histidine 300 by the codon coding for leucine and corresponds to amino acid sequence SEQ ID No. 9 (termed XIKE-C) or is a functional variant thereof. Most preferably also, the BVDV according to the invention has both a $E^{rns}$ mutation and a $N^{pro}$ mutation according to the invention, wherein the codon coding for histidine 300 is substituted by the codon coding for leucine and also the complete $N^{pro}$ coding region is deleted, except for codons 1 to 4, thus amino acids MELF of $N^{pro}$ remain. Said mutant corresponds to amino acid sequence SEQ ID No. 10 (termed XIKE-C NdN) or is a functional variant thereof. Preferably, such a functional variant is at least 65% homologous to the amino acid sequence disclosed herein. More preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

Another important embodiment of the invention described herein is a composition comprising a pestivirus, in particular a BVDV according to the invention and a solution. The skilled person knows additional components which may be comprised in said composition (see also Remington's Pharmaceutical Sciences. (1990). 18th ed. Mack Publ., Easton). The expert may use known injectable, physiologically acceptable sterile solutions. For preparing a ready-to-use solution for parenteral injection or infusion, aqueous isotonic solutions, such as e.g. saline or corresponding plasma protein solutions are readily available. The pharmaceutical compositions may be present as lyophylisates or dry preparations, which can be reconstituted with a known injectable solution directly before use under sterile conditions, e.g. as a kit of parts.

The final preparation of the compositions of the present invention are prepared for e.g. injection by mixing said pestivirus, preferably BVDV according to the invention with a sterile physiologically acceptable solution, that may be supplemented with known carrier substances or/and additives (e.g. serum albumin, dextrose, sodium bisulfite, EDTA). Said solution may be based on a physiologically acceptable solvent, e.g. an aqueous solution between pH 7 and 8. The pH may be stabilised by a pharmaceutically acceptable buffer. The solution may also contain further stabilising agents like a detergent like Tween 20, serum albumin such as BSA (bovine serum albumin), ascorbic acid, and/or spermidine. The composition may also comprise adjuvants, e.g. aluminiumhydroxide, mineral or other oils or ancillary molecules added to the vaccine or generated by the body after the respective induction by such additional components, like but not restricted to interferons, interleukins or growth factors.

For example, in a composition according to the invention, the pestivirus, in particular BVDV may be solved in:

| | |
|---|---|
| *Pestivirus (preferably BVDV)* | *$10^2$ - $10^8$ $TCID_{50}$* |
| *SGS** | *25 % v/v* |
| *Cell culture medium* | *qsp 1 dose* |

| **SGS:* | *Composition per 2 ml* |
|---|---|
| *Sucrose* | *75 mg* |
| *Gelatine* | *20 mg* |
| *Potassium hydroxide* | *0.274 mg* |
| *L- glutamic acid* | *0.72 mg* |
| *Potassium dihydrogen phosphate* | *0.516 mg* |
| *Dipotassium phosphate* | *1.254 mg* |

(continued)

| | |
|---|---|
| *Water for injection* | *qsp 2 ml* |

If the composition is first lyophilized or dehydrated by other methods, then, prior to vaccination, said composition is rehydrated in aqueous (e.g. saline, PBS (phosphate buffered saline)) or nonaqueous solutions (e.g. oil emulsion (mineral oil, or vegetable/metabolizable oil based/single or double emulsion based), aluminum-based, carbomer based adjuvant).

Preferably, the composition according to the invention induces an immunological response in an animal. More preferred, the composition according to the invention is a vaccine. A vaccine as understood herein comprises a pestivirus, in particular BVDV according to the invention and is defined above (section "definitions")

Most preferred, the composition according to the invention further comprises a pharmaceutically acceptable carrier or excipient. Several carriers or excipients are disclosed above. The composition may comprise, if aimed at injections or infusion, substances for preparing isotonic solutions, preservatives such as p-hydroxybenzoates, stabilizers, such as alkalisalts of ethylendiamintetracetic acid, possibly also containing emulsifying and/ or dispersing.

The composition according to the invention may be applied intradermally, intratracheally, or intravaginally. The composition preferably may be applied intramuscularly or intranasally. In an animal body, it can prove advantageous to apply the pharmaceutical compositions as described above via an intravenous or by direct injection into target tissues. For systemic application, the intravenous, intravascular, intramuscular, intranasal, intraarterial, intraperitoneal, oral, or intrathecal routes are preferred. A more local application can be effected subcutaneously, intradermally, intracutaneously, intracardially, intralobally, intramedullarly, intrapulmonarily or directly in or near the tissue to be treated (connective-, bone-, muscle-, nerve-, epithelial tissue). Depending on the desired duration and effectiveness of the treatment, the compositions according to the invention may be administered once or several times, also intermittently, for instance on a daily basis for several days, weeks or months and in different dosages.

The invention also relates to the use of a pestivirus, in particular BVDV according to the invention in the manufacture of a vaccine for the prophylaxis and treatment of pestiviral infections, in particular of BVDV infections.

Another important part of the invention is a polynucleotide molecule comprsing the nucleic acid coding for a pestivirus, in particular for a BVDV according to the invention, or a fragment, functional variant, variant based on the degenerative nucleic acid code, fusion molecule or a chemical derivative thereof. Preferably, said polynucleotide molecule is DNA. Also preferably, said polynucleotide molecule is RNA. In a more preferred embodiment, said polynucleotide molecule also comprises the nucleotide sequence of a functional 5'- and/or 3'-non-translated region of a pestivirus, in particular of BVDV.

There are several nucleotide sequences known in the art, which represents the basis for the production of a polynucleotide molecule coding for a pestivirus attenuated according to the present invention, having at least one mutation in the coding sequence of $N^{pro}$ and at least one in the coding sequence of glycoprotein $E^{rns}$, wherein said mutations result in an combined inactivation of the RNase activity residing in glycoprotein $E^{rns}$ and in the inactivation of the immunomodulating activity residing in $N^{pro}$. Examples of nuclecic acid sequences of wild-type sequences of several members of pestiviruses are listed below:

| *Border disease virus* | |
|---|---|
| Strain BD31 | NCBI GenBank Accession No. [U70263] |
| Strain X818 | NCBI GenBank Accession No. [AF037405] |
| *Bovine viral diarrhea virus 1* | |
| Strain NADL | NCBI GenBank Accession No. [M31182] |
| Strain Osloss | NCBI GenBank Accession No. [M96687] |
| Strain SD-1 | NCBI GenBank Accession No. [M96751] |
| Strain CP7 | NCBI GenBank Accession No. [U63479] |
| *Bovine viral diarrhea virus 2* | |
| Strain 890 | NCBI GenBank Accession No. [U18059] |
| Strain C413 | NCBI GenBank Accession No. [AF002227] |

(continued)

| *Classical swine fever virus* | |
|---|---|
| Strain Alfort/187 | NCBI GenBank Accession No. [X87939] |
| Strain Alfort-Tübingen | NCBI GenBank Accession No. [J04358] |
| Strain Brescia | NCBI GenBank Accession No. [M31768] |
| Strain C strain | NCBI GenBank Accession No. [Z46258] |

The mutations/modifications according to the invention relating to the coding sequence of $N^{pro}$ and $E^{rns}$ are described above more in detail. Having this information, a person skilled in the art is able to realize the manufacture of any polynucleotide/polynucleic acid coding for a pestivirus according to the present invention. Furthermore, this person is able to manufacture an attenuated pestivirus according to the invention. Molecular method for introducing a mutation into a polynucleotide sequence, cloning and amplification of said mutated polynucleotide are for example provided by Sambrook et 1989 or Ausubel et al. 1994.

Most preferably, the wild type BVDV according to the invention which is to be mutated as disclosed herein is encoded by the nucleic acid sequence SEQ ID No. 1 (termed XIKE A) or a functional variant thereof. Most preferably also, the BVDV according to the invention has a $N^{pro}$ mutation according to the invention and is encoded by nucleic acid sequence SEQ ID No. 2 (termed XIKE-A-NdN) or a functional variant thereof. Preferably, such a functional variant is at least 65% homologous to the nucleic acid sequence disclosed herein. On the nucleic acid level, homologies are very roughly: BVDV-1/-BVDV-1: 80%; BVDV-1/-BVDV-2: 70%; BVDV-2/- BVDV-2: 96%. Therefore, more preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the nucleic acid sequence disclosed herein. More preferably also, such functional variant is at least 91%, 92%, 93%, 94%, 95%, 96%, 97% or 98% homologous to the nucleic acid sequence disclosed herein. Most preferably, such functional variant is at least 99% or 99.9% homologous to the nucleic acid sequence disclosed herein.

Most preferably also, the BVDV according to the invention has a $E^{rns}$ mutation according to the invention which has a deletion of codon H349 and is encoded by nucleic acid sequence SEQ ID No. 7 (termed XIKE-B) or by a functional variant thereof. Most preferably also, the BVDV according to the invention has both a $E^{rns}$ mutation and a $N^{pro}$ mutation according to the invention, wherein the codon coding for histidine 349 of $E^{rns}$ is deleted and also the complete $N^{pro}$ coding region is deleted, except for codons 1 to 4, thus amino acids MELF of $N^{pro}$ remain. Said mutant is encoded by nucleic acid sequence SEQ ID No. 8 (termed XIKE-B-NdN) or by a functional variant thereof. Preferably, such a functional variant is at least 65% homologous to the nucleic acid sequence disclosed herein. More preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the nucleic acid sequence disclosed herein. More preferably also, such functional variant is at least 91%, 92%, 93%, 94%, 95%, 96%, 97% or 98% homologous to the nucleic acid sequence disclosed herein. Most preferably, such functional variant is at least 99% or 99.9% homologous to the nucleic acid sequence disclosed herein.

Most preferably also, the BVDV according to the invention has a $E^{rns}$ mutation according to the invention which is a substitution of codon "H300" by a leucine codon, and is encoded by nucleic acid sequence SEQ ID No. 11 (termed XIKE-C) or a functional variant thereof. Most preferably also, the BVDV according to the invention has both a $E^{rns}$ mutation and a $N^{pro}$ mutation according to the invention, wherein the codon coding for histidine 300 is substituted by the codon coding for leucine and also the complete $N^{pro}$ coding region is deleted, except for codons 1 to 4, thus amino acids MELF of $N^{pro}$ remain. Said mutant is encoded by nucleic acid sequence SEQ ID No. 12 (termed XIKE-C-NdN) or by a functional variant thereof. Preferably, such a functional variant is at least 65% homologous to the nucleic acid sequence disclosed herein. More preferable, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the nucleic acid sequence disclosed herein. More preferably also, such functional variant is at least 91%, 92%, 93%, 94%, 95%, 96%, 97% or 98% homologous to the nucleic acid sequence disclosed herein. Most preferably, such functional variant is at least 99% or 99.9% homologous to the nucleic acid sequence disclosed herein.

Another important aspect of the invention is a method for attenuating a pestivirus, characterized in that at least one mutation in the coding sequence for glycoprotein $E^{rns}$ and at least another mutation in the coding sequence for $N^{pro}$ is generated in a pestivirus genome. According to a preferred embodiment, said pestivirus is BVDV.

According to a more preferred embodiment, said method comprises the steps:

a) reverse transcription of a wild-type pestivirus nucleotide sequence into a cDNA;
b) cloning said cDNA;
c) introducing mutations selected from the group of deletions, insertion mutations and/or substitution mutations into said cDNA, wherein said mutations are located in the coding sequence encoding glycoprotein $E^{rns}$ and the protease $N^{pro}$,
d) incorporating the cDNA into a plasmid or into a DNA virus capable of directing the transcription of pestivirus cDNA into RNA in vitro or upon infection of suitable cells.

Regarding the method for attenuating a BVDV according to the invention, said preferred methods comprisses the steps:

a) reverse transcription of a wild-type BVDV nucleotide sequence into a cDNA;
b) cloning said cDNA;
c) introducing mutations selected from the group of deletions, insertion mutations and/or substitution mutations into said cDNA, wherein said mutations are located in the coding sequence encoding glycoprotein $E^{rns}$ and the protease $N^{pro}$,
d) incorporating the cDNA into a plasmid or into a DNA virus capable of directing the transcription of pestivirus cDNA into RNA in vitro or upon infection of suitable cells.

Yet another important embodiment of the invention is a method for the preparation of a medicament for the treatment of disease caused by a pestivirus, wherein a pestivirus according to the invention or a composition according to the invention, wherein the said pestivirus or said composition is administered to an animal in need thereof at a suitable dosis as known to the skilled person and the reduction of symptoms of said pestivirus infection.

Yet another important embodiment of the invention is a method for the preparation of a medicament for the treatment of disease caused by BVDV, wherein a BVDV according to the invention or a composition according to the invention, wherein the said BVDV or said composition is administered to an animal in need thereof at a suitable dosis as known to the skilled person and the reduction of symptoms of BVDV infection such as viremia and leukopenia and/or pyrexia and/or diarrhea is monitored.

**EXAMPLES**

The following examples serve to further illustrate the present invention; but the same should not be construed as limiting the scope of the invention disclosed herein.

**EXAMPLE 1**

***BVDV XIKE-B: foetopathogenicity assessment in pregnant heifers***

BVDV XIKE-B, an RNase negative mutant of the highly pathogenic BVDV type 2 isolate NewYork'93/C was recovered from the infectious cDNA clone pKANE40B and showed wild type-like (wt-like) growth characteristics in tissue culture. In animal experiments the mutant virus was found to be considerably attenuated so that it represented a promising candidate for development of a live attenuated vaccine virus (Meyer et al., 2002). To test whether this attenuated virus is still able to cross the placenta and infect the fetus, pregnant heifers were infected with XIKE-B. As a control wild type BVDV recovered from cDNA clone pKANE40A was used The respective virus named XIKE-A expresses an active $E^{rns}$ RNase in the infected cell.
The study aimed to assess the safety of XIKE-A and XIKE-B in pregnant animals.

**EXPERIMENTAL DESIGN**

Ten pregnant heifers were selected from a BVDV negative herd. The following groups of 5 heifers were included in the trial:

| | No. | Inoculation | Virus |
|---|---|---|---|
| Group 1: | 5 | One i. n. administration, 3 ml in each nostril | XIKE-A |
| Group 2: | 5 | One i. n. administration, 3 ml in each nostril | XIKE-B |

Heifers were moved to the experimental facilities 8 days before inoculations. Pregnancy status was confirmed after transport into the experimental facility. Heifers were between days 60 and 90 of gestation on the day of inoculation. Inoculation took place for all animals at one point of time.

Heifers were monitored for the presence of clinical signs of BVDV infection including abortions during the observation period. Blood samples were collected from the animals for serology, antigen detection and white blood cells were counted. The experiment was terminated 9 weeks after infection. Non-aborted cows were slaughtered, the uterus examined and collected. Foetal organ samples were collected during routine necropsy and examined for BVDV infection.

The presence of fetal infection was the main evaluation parameter, composed from the number of BVDV-related cow mortality, the number of BVDV-related abortions and the number of BVDV positive fetuses at termination. In addition to the main parameter, clinical signs characteristic for BVDV infection, viraemia and white blood cell counts in cows and rectal temperature after challenge were evaluated.

**Animals**

Heifers were purchased from a farm free of BVDV.

Only animals, which met the following inclusion criteria, were used.

**Inclusion criteria**

- Free of BVD antibodies; each individual will be tested in the serum antibody test prior to transport and at the initiation of the study (at the animal test facility).
- Free of BVDV; plasma and/or buffy-coat preparation from each individual will be tested by a suitable test.
- Clinically healthy at the initiation of the study judged upon physical examination. The health examination of the animals was accomplished in accordance with the current, generally accepted veterinary practice.
- Pregnancy confirmed by physical examination before inoculation. Pregnancy was between 60 - 90 days at the time of inoculation, proven by insemination records.

**Test Strain A**

| | |
|---|---|
| Description: | XIKE A, life virus BVDV strain |
| Composition: | Experimental material comprising of cell culture supernatant of low passaged XIKE-A |
| BVD components: | BVDV type II strain: XIKE-A |
| Supplied by: | Dr. Gregor Meyers, "Bundesforschungsanstalt für Viruskrankheiten der Tiere" (BFAV), Paul-Ehrlich-Straße 28, 72076 Tübingen, Germany |
| Applied BVD virus dose: | Type 1 strain: $10^5$ $TCID_{50}$/6ml (TCID = Tissue Culture Infective Dose) |
| Applied vaccine volume: | 3 ml per nostril |
| Application route: | Intranasal |
| Preparation of dosage form: | The inoculum was sent in a pre-diluted frozen form in a 50 ml vial on dry ice and was to be stored at -70 °C before inoculation. Immediately before inoculation of Group 1 heifers, the material was thawed avoiding local temperatures above 37 °C. After no ice was visible in the fluid, material was gently stirred and immediately used for inoculation of the animals. |
| Unused inoculum: | The volume of the unused material was be measured and split on two aliquots before immediate freezing in dry ice or liquid nitrogen and stored for re-titration purposes. Virus and contaminated plastic or glassware were incubated with an appropriate volume of an 8 - 10 % formaldehyde solution for at least 24 hours at room temperature before discarding in order to inactivate viruses. |

**Test Strain B**

| | |
|---|---|
| Description: | XIKE B, life virus BVDV strain |

(continued)

| | |
|---|---|
| Composition: | Experimental material comprising of cell culture supernatant of low passaged XIKE B |
| BVD components: | BVDV type II strain: XIKE-B |
| Supplied by: | Dr. Gregor Meyers, "Bundesforschungsanstalt für Viruskrankheiten der Tiere" (BFAV), Paul-Ehrlich-StraBe 28, 72076 Tübingen, Germany |
| Applied BVD virus dose: | Type 1 strain: $10^5$ $TCID_{50}$/6ml (TCID = Tissue Culture Infective Dose) |
| Applied vaccine volume: | 3 ml per nostril |
| Application route: | Intranasal |
| Preparation of dosage form: | The inoculum was sent in a pre-diluted frozen form in a 50 ml vial on dry ice and was stored at -70 °C before inoculation. Immediately before inoculation of Group 2 heifers, the material was thawed avoiding local temperatures above 37 °C. After no ice was visible in the fluid, material was gently stirred and immediately used for inoculation of the animals. |
| Unused vaccine: | The volume of the unused material was measured and split on two aliquots before immediate freezing in dry ice or liquid nitrogen and stored for re-titration purposes. Virus and contaminated plastic or glassware was incubated with an appropriate volume of an 8 - 10 % formaldehyde solution for at least 24 hours at room temperature before discarding in order to inactivate viruses. |

**Pregnancy Control**

Pregnancy was confirmed immediately before inoculation.

**Inoculation of Heifers**

The inoculation is Day 0 of the experiment.

In each nostril, 3 ml of the test material was administered intranasally by syringe without needle. Each time a new sterile syringe was taken. Administration was performed during the aspiration phase in order to minimize loss of fluid via expiration of material.

**Post-Inoculation Observations**

Collection and examination of blood samples

Blood was collected following standard, aseptic procedures (disinfecting the bleeding site). A new sterile syringe and needle was used for each animal.

**Blood collection to prepare serum**

At least 10 ml blood was collected from the heifers immediately before inoculation, then weekly after infection and at the termination of the study. Serum was stored at -20 °C until required.

**Blood collection for leukocyte counts and buffy coat preparations**

For leukocyte counting, 3 ml blood was transferred immediately after collection to suitable sterile vessels (Venoject, Terumo Europe N.V., Leuven, Belgium), pre-filled with 0.06 ml EDTA (0.235 MOL/L).

For buffy coat preparations, at least 15 ml blood was transferred immediately after collection to suitable sterile vessels, pre-filled with 0.1 ml Heparin solution (Na-heparin for inj., 5 000 IU/ml lot. A7B163A exp. date: 11/2000: Gedeon Richter RT, Budapest, Hungary) yielding at least 20 IU Heparin per ml blood in the blood sample. The content was carefully mixed thereafter.

For preparation of buffy coats and leukocyte counting, blood was collected from the heifers

- on every day, between Day 0 and Day 14 after infection;

- on every second day, between Day 15 and Day 40, or until all animals were negative for virus isolation for three consecutive sampling time points.

**Preparation of serum**

Blood was allowed to clot at room temperature, and separated by centrifugation. Each serum sample was divided into two aliquots of at least 2 ml each. One set of aliquots was assayed for BVDV specific antibodies by ELISA. The rest of the sera was frozen and stored at -20 °C until required.

**Leukocyte counts**

Leukocyte counts was determined with a coulter-counter semi-automated electronic device (Diatron Minicell-16, Messtechnik GmbH, Wien, Austria) with a claimed accuracy of 0.1 x $10^9$ / 1, 100 / µl. The instrument was used (calibration and leukocyte-counts) according to the manufacturer's recommendations.

**Preparation of buffy coats**

Heparin blood samples was transported to the laboratory as soon as possible. Buffy coat preparation procedure, following a standard laboratory procedure was performed under aseptic conditions (sterile pipettes, handling, clean bench etc.).

The obtained buffy coats were re-suspended in a small volume (2 ml) of RPMI 1640 and frozen at -70 °C in two aliquots of 0,5 ml. The residual 1ml buffy coats was immediately used for determination of blood cell associated BVDV by co-cultivation in a permissive cell culture.

**BVD serum antibody ELISA-test**

Each serum sample was tested for the presence of BVDV-antibodies using a suitable and validated ELISA test (Svanovir™ BVDV antibody test Cat# 10-2200-10). Test was validated and performed according to the manufacturer's recommendations. Positive samples were diluted according to the $\log_2$ scale to determine BVDV antibody titers.

**BVD antigen assay(s)**

Each buffy coat sample was assayed for the presence of BVDV by co-cultivation of the freshly prepared buffy-coats with susceptible cells or a cell-line. No freezing was allowed before co-cultivation.

Plasma was collected and provided to Man-Gene from each sample.

**Clinical observations**

**Observation of heifers**

Animals were examined daily from Day 0 - 42 post inoculation for the presence of clinical symptoms by a sufficiently trained veterinarian.

All clinical signs were recorded and described by its nature, consistence/touch, severity (mild, medium or severe) location, size of the area affected, and they will be scored according to agreed and standard definitions. Special attention was paid to respiratory signs (respiration, its rate; nasal or ocular discharge; conjunctivitis, sneezing, coughing, etc.) and diarrhea.

**Rectal temperatures**

Rectal temperatures were measured daily in each heifer, at the same hour of the day (preferably in the morning) from 5 days prior to the inoculation till 21 days post infection.

Daily measurement of rectal temperature was continued until each animal had rectal temperatures below or equal to 39 °C for at least 3 consecutive days.

**Detection of interrupted pregnancy**

Pregnancy was confirmed and suspicion for abortion or resorption of the fetus was established by rectal examination. A trained veterinarian examined all animals at inoculation, 1 and 2 months post-inoculation. The examination was carried out according to the generally accepted veterinary practice.

Heifers were examined daily for any sign of abortion until termination of the study (8 - 12 weeks post-challenge).

### Termination of the Study

The study was terminated by slaughtering the heifers and extracting the fetuses. Fetuses and fetal material were transferred into closed transport containers marked with the number of the cow and the date/time. Containers were transported to a selected necropsy room.

Necropsy of the heifers was not required. Necropsy will be performed on fetuses, findings recorded and a panel of samples collected as described below.

### Post-Mortem Examination

A detailed necropsy of the experimental animals was done in each case of death.

Post-mortem examinations were carried out by an experienced veterinary surgeon and the data were recorded on appropriate data sheets. Further laboratory tests were performed according to the clinical signs and lesions observed. If the diagnosis of the necropsy referred to a diseases caused by microbial agent the diagnosis was verified by an appropriate test, specific for the agent.

Each tissue sample was collected in at least 2 separate, labeled containers and snap-frozen in liquid nitrogen. Samples were stored at -70 °C until required.

### Aborted fetuses and study termination

At least the following tissue samples were collected from the fetuses:

- exudate from the peritoneal cavity or thorax, if present,
- mesenteric lymph nodes,
- spleen,
- thymus,
- cerebellum,
- kidney,
- bone marrow from the sternum,
- sample from the placenta, if available.

### Dead or sacrificed heifers

At least the following tissue samples were collected:

- blood for buffy coat, if available,
- blood for serum, if available,
- Peyer's patches,
- mesenteric lymph nodes,
- spleen,
- kidney,
- uterus, including a sample from the placenta, if available.

### Storage and Transport of Samples

| Samples: | Storage: |
|---|---|
| Serum | -20 °C |
| Buffy coat | -70 °C |
| Virus | -70 °C |
| Tissue from heifers | -70 °C |

(continued)

| Samples: | Storage: |
|---|---|
| Tissue from fetuses | -70 °C |

Samples were sent for laboratory analysis as required by the sponsor. The choice of samples and the timing of transport were agreed with the study monitor or the project manager. As a matter of general principle, samples coming from aborted material or from new-born calves were investigated as soon as possible.

## RESULTS

Mortality

Heifer No. 626 (Group 1) died on Day 13 PI (post inoculation). The following table summarises the observed clinical signs and lesions revealed during necropsy:

| Heifer | In-life observations | Post mortem findings |
|---|---|---|
| No. 626 | • signs of disease from 7 DPI<br>• lachrymation, nasal discharge on 7 - 12 DPI<br>• loss of appetite from 8 - 12 DPI<br>• diarrhoea on 11 - 12 DPI<br>• elevated respiratory rate on 9 - 10 and 12 DPI<br>• coughing on 9 DPI<br>• abnormal breathing on 12 DPI | • dehydration<br>• haemorrhages on the serous membranes<br>• hyperaemia of the Peyer's patches<br>• oedema of the lung |

These clinical and gross-pathological findings are consistent with BVDV induced lesions, therefore it may be concluded that the reason of death was the BVDV infection.

### Abortions after Infection

One heifer had clinical abortion in each group. Heifer No. 615 (Group 1) aborted on Day 38 PI, Heifer No. 469 (Group 2) aborted on Day 39 PI. Both foetuses showed the signs of autolysis, and they were estimated to die at least 3 - 7 days before the abortion (around 32 - 35 DPI). In Group 1, no foetus was found in Heifer No. 526 during the slaughter examination at termination. Gross-pathology of the uterus revealed the followings:

- the right uterine horn was slightly enlarged,
- the remains of placenta with progressed autolysis was retained in the lumen.

The findings on the uterus of Heifer No. 526 is consistent with a "silent" abortion, most likely due to the BVD infection.

### Clinical Observation of Heifers

A summary of the clinical observation data and duration of clinical signs in the groups are presented below.

Clinical signs and the days post inoculation (DPI) when they were observed

| Group 1 (XIKE-A) | | | | | |
|---|---|---|---|---|---|
| Clinical sign | Animal ID | | | | |
| | 526 | 598 | 615 | 618 | 626* |
| | DPI | | | | |
| Loss of appetite | 8 - 13 | 8 - 18 | 8 - 18 | 8 - 16 | 8 - 12 |

(continued)

| Group 1 (XIKE-A) | | | | | |
|---|---|---|---|---|---|
| Clinical sign | Animal ID | | | | |
| | 526 | 598 | 615 | 618 | 626* |
| Lachrymation | 7 - 10 | 7- 8, 10-12 | 8 - 10 | 8 - 10 | 7 - 8 |
| Conjunctivitis | 9 - 10 | 9 - 12 | 9 - 11 | 9-11 | - |
| Nasal discharge | 7-13 | 7-9, 11-12 | 8 -13 | 8 - 12 | 7-12 |
| Oral erosion | - | - | - | - | - |
| Oral haemorrhage | - | - | - | - | - |
| Diarrhoea | - | 11-16 | 10-15 | 11 - 15 | 11-12 |
| Coughing | 9 | 10, 15 | 9 | 8 - 9, 13 | 9 |
| Abnormal breathing | - | 11 - 14 | 12 - 14 | 12 - 14 | 12 |
| Elevated respiratory rate | - | 10 - 13 | 9 - 13 | 8 - 13 | 9 - 10, 12 |
| Hoof erosion | - | - | - | - | - |
| Group 2 (XIKE-B) | | | | | |
| Clinical sign | Animal ID | | | | |
| | 469 | 588 | 565 | 608 | 619 |
| | | | | | |
| Loss of appetite | - | - | - | - | - |
| Lachrymation | - | - | - | - | - |
| Conjunctivitis | - | - | - | - | - |
| Nasal discharge | - | - | - | - | - |
| Oral erosion | - | - | - | - | - |
| Oral haemorrhage | - | - | - | - | - |
| Diarrhoea | - | - | - | - | - |
| Coughing | - | - | - | - | - |
| Abnormal breathing | - | - | - | - | - |
| Elevated respiratory rate | - | - | - | - | - |
| Hoof erosion | - | - | - | - | - |
| *Heifer No. 626 died on Day 13 PI | | | | | |

All Group 1 animals infected with XIKE-A exhibited a broad spectrum of clinical signs. Respiratory signs appeared first accompanied by loss of appetite, and a few days later heifers developed diarrhoea with the exception of Heifer No. 526. One heifer died and another one aborted (see before) after infection. All these signs are consistent with the symptoms expected after infection with a virulent BVDV strain.

All Group 2 animals infected with XIKE-B were free of clinical signs. At the same time, one heifer had abortion during the observation period.

**Rectal Temperatures**

No abnormal temperature changes were detected before the infection of the animals.
In Group 2, all temperature values remained within the physiological range from Day 0 to Day 21 after infection.
All Group 1 animals showed elevated rectal temperature after infection that were detected between Days 7 - 11 PI.

## Findings at Study Termination

At study termination, foetuses were examined at slaughter.
No foetus was recovered from Heifer No. 526 (see section 10.2 "Abortions after Infection").
The following findings were observed at the necropsy of the foetuses:

| Animal No. | Findings | Conclusion |
|---|---|---|
| Group 1 | | |
| 598 | Ascites, general oedema, autolysis | Died at least 2 weeks earlier |
| 618 | Ascites, general oedema, autolysis | Died at least 3 weeks earlier |
| Group 2 | | |
| 565 | Ascites, general oedema, liver degeneration | Foetus considered non-viable |
| 588 | Normal | - |
| 608 | Normal, perirenal oedema | - |
| 619 | General autolysis | Died 3 - 6 weeks earlier |

The findings suggest that 2 Group 1 animals (Heifers No. 598 and No. 618) and one Group 2 animal (Heifer No. 619) died several weeks before extraction, and so they can be considered abortions.

## Abortions Modified by Post-Mortem Findings

After the post-mortem examination it was not clear why some of the heifers had not had abortion. Dead foetuses should be considered as abortions, therefore the clinical picture was modified after the termination of the study as follows:

Group 1

| Animal No. | Conclusion |
|---|---|
| 526 | BVD abortion (uterus with placenta post-mortem) |
| 598 | BVD abortion (foetus post-mortem) |
| 615 | Clinical BVD abortion |
| 618 | BVD abortion (foetus post-mortem) |
| 626 | Died due to BVD |

Group 2

| Animal No. | Conclusion |
|---|---|
| 469 | Clinical BVD abortion |
| 565 | Expected BVD abortion; non-viable foetus |
| 588 | Normal |
| 608 | Normal |
| 619 | BVD abortion (foetus post-mortem) |

## Examination of Blood Samples

## Leukocyte count

WBC counting was interrupted on Day 26 PI, as all animals became negative for virus isolation for this time point.

0 DPI values were considered as individual baseline for comparison.
In Group 2, the leukocyte counts never went to 40 % or more below the baseline value until the end of the observation period (26 DPI).
In Group 1, one animal (Heifer No. 598) had WBC count below the 40 % baseline for one day.

**Serology**

None of the selected animals had BVDV specific antibody in their sera before the infection.
After infection, all surviving Group 1 heifers developed BVDV specific antibodies detected from 3 weeks PI and lasted until the end of the observation period in all study animals. In Group 2, 4 out of the 5 heifers had BVDV specific antibodies detected from 4 weeks PI. Measurable antibody response lasted only in 3 animals until the end of the observation period. Titres were lower in Group 2 than in Group 1.

**Virus Detection by Co-Cultivation**

**Buffy coats**

BVDV was detected in both groups. The duration of virus detection is summarised below. All samples were co-cultivated immediately after collection, i. e. without freezing.

| Animal No. | DPI when BVDV was detected |
|---|---|
| Group 1 | |
| 615 | 5 - 12 |
| 526 | 5 - 9 |
| 626 | 5-12 |
| 618 | 5 - 11, 14 |
| 598 | 5-11,13 |
| Group 2 | |
| 565 | 7 - 9 |
| 588 | 8 |
| 608 | 6-9 |
| 469 | 8 |
| 619 | 5 - 11 |

**Tissue samples**

The presence of BVD virus in the dead heifer and the foetuses is summarised below:

Heifer:

| Animal No. | | BVDV in tissue samples |
|---|---|---|
| Group 1 | | |
| 626 | | Present# |

Foetuses:

| Animal No. | BVDV in tissue samples |
|---|---|
| Group 1 | |
| 615 | Not present# |
| 526 | NT |
| 626 | Present# |
| 618 | Not present |
| 598 | Present |
| Group 2 | |
| 565 | Present |
| 588 | Not present |
| 608 | Present |
| 469 | Not present# |
| 619 | Not present |
| NT = Not tested | |

Samples were co-cultivated immediately after collection (i. e. without freezing), except "#" marked ones, from which only frozen samples were available.

**Summary of BVD related clinical and laboratory data**

Group 1

| Animal No. | Conclusion | BVD |
|---|---|---|
| 526 | BVD abortion (uterus with placenta post-mortem) | NT (no sample found) |
| 598 | BVD abortion (foetus post-mortem) | + (foetus)* |
| 615 | Clinical BVD abortion | - (foetus)* |
| 618 | BVD abortion (foetus post-mortem) | - (foetus)* |
| 626 | Died due to BVD | + (foetus) / + (heifer) |
| NT: not tested | | |

Group 2

| Animal No. | Conclusion | BVD |
|---|---|---|
| 469 | Clinical BVD abortion | - (foetus)* |
| 565 | Expected BVD abortion; non-viable foetus | + (foetus) |
| 588 | Normal | - (foetus) |
| 608 | Normal | + (foetus) |
| 619 | BVD abortion (foetus post-mortem) | - (foetus)* |
| * Foetuses were autolysed at the time of sampling | | |

**Conclusion:**

The study aimed to assess the safety of XIKE-A and XIKE-B in pregnant animals.
Ten pregnant heifers were selected from a BVDV negative herd. Two groups of 5 heifers were included in the trial: one was inoculated with XIKE-A the other with XIKE-B virus strain. Heifers were between days 60 and 90 of gestation on the day of inoculation.

Heifers were monitored for the presence of clinical signs of BVDV infection including abortions during the observation period. Blood samples were collected from the animals for serology, antigen detection and white blood cells were counted. The experiment was terminated 9 weeks after infection. Non-aborted cows were slaughtered, the uterus examined and collected. Foetal organ samples were collected during routine necropsy and examined for BVDV infection.

The presence of fetal infection was the main evaluation parameter, composed from the number of BVDV-related cow mortality, the number of BVDV-related abortions and the number of BVD positive fetuses at termination. In addition to the main parameter, clinical signs characteristic for BVDV infection, viraemia and white blood cell count in cows and rectal temperature after challenge were evaluated.

The XIKE-B virus proved to be less pathogenic than XIKE-A, nevertheless BVD related abortion and infection of the foetus was observed in the XIKE-B group, too. Therfore it can be concluded that the inactivation of the $E^{rns}$ RNase does not prevent fetal infection.

**EXAMPLE 2**

***BVDV XIKE-A-NdN: foetopathogenicity assessment in pregnant heifers***

The $N^{pro}$ gene has been shown to be nonessential for growth of CSFV in tissue culture (Tratschin et al., 1998). Even though a proof for BVDV attenuation in consequence of $N^{pro}$ deletion is still missing, a role of this protein in the interaction between virus and host seemed to be possible and was actually indicated by recent experiments for CSFV (Mayer et al., 2004; Rüggli et al., 2003). We therefore wanted to investigate, whether the deletion of the major part of the $N^{pro}$ coding sequence leads to a virus that no longer infects the fetus in pregnant heifers. The $N^{pro}$ gene except for the 5' terminal 4 codons was deleted from the full length cDNA clone pKANE40A according to standard procedures. The resulting mutant full length clone was used as template for in vitro transcription and the resulting cRNA was transfected into MDBK cells as described (Meyer et al., 2002). The recovered virus was amplified in tissue culture and then used in the animal experiment described below. BVDV XIKE-B served as a control since it was shown before that it is able to cross the placenta (EXAMPLE 1).

**OBJECTIVE(S)/PURPOSE OF THE STUDY**

The study aims to assess the safety of a live attenuated BVDV with a genomic deletion of most of the $N^{pro}$ coding region in pregnant animals.

Material and Methods applied are described in Example 1

**STUDY DESIGN**

Eight pregnant heifers were assigned at random to two groups. They were treated and observed according to the following schedule:

| | Group 1 | Group 2 |
|---|---|---|
| N | 5 | 3 |
| Treatments | XIKE-A-NdN | XIKE-B / control |
| Route | Intramuscular | |
| Vaccination time | between days 60 and 90 of pregnancy (day 0 of the study) | |
| Observations Post-vaccination (in life) | • Clinical signs<br>• Serum at days 0, 14, 28, 42 and at termination<br>• WBC at day 0 and then daily for 14 days<br>• Buffy coat at day 0 and then daily for 14 days | |
| Post-mortem (day 60) | • Gross-pathology<br>• Organ panel for virus isolation | |

| | |
|---|---|
| Type of study: | open controlled clinical study |
| Experimental unit: | Individual animal |

(continued)

| | Group 1 | Group 2 |
|---|---|---|
| Method of blinding: | Partial blinding. No detailed procedures for blinding and access to treatment schedule were applied. The observing veterinarian at the study location and the pathologist were not be aware of the treatment; they only received a protocol extract relevant to their tasks. Vaccination was performed by the investigator or his assignee. Samples for virus isolation were coded by the investigator until all results are available. | |

**RESULTS**

All heifers were healthy and pregnant at study start. All animals proved to be free of BVDV and BVDV antibodies before the initiation of the.

**Preparation and Control of the Virus used for the Infection**

Samples were collected throughout the dilution steps and assayed on the day of preparation, i.e. without freezing by co-cultivation on suitable tissue culture. The results of virus titration are shown in the following table.

| Sample ID | Virus strain | Dilution / description | $Log_{10}$ titre/ml |
|---|---|---|---|
| VT1a | XIKE-A/NdN (S) | 1:2 (at 4 °C) | 4.4 |
| VT1b | | #2a on ice without opening | 4.0 |
| VT1c | | Return of #2b | 2.8 |
| VT2a | XIKE-B | 1:2.2 (at 4 °C) | 2.3 |
| VT2b | | #3a on ice without opening | 2.8 |
| VT2c | | Return of #3b | Negative |

**Clinical Symptoms of BVDV Infection**

The table below gives a summary about the animals that had clinical signs during the observation period.

Clinical signs and the days post inoculation (DPI) when they were observed

| Group 1 (XIKE-A NdN) | | | Group 2 (XIKE-B) | |
|---|---|---|---|---|
| Clinical sign | Animal ID | | Animal ID | |
| | 1583 | | 1438 | 1585 |
| | | | | |
| Loss of appetite | 8 | | - | 10 |
| Lachrymation | - | | - | - |
| Conjunctivitis | - | | - | - |
| Nasal discharge | - | | - | - |
| Oral erosion | - | | - | - |
| Oral haemorrhage | - | | - | - |
| Diarrhoea | - | | - | - |
| Coughing | - | | 12 | 10-13 |
| Abnormal breathing | - | | - | - |
| Elevated respiratory rate | - | | - | - |
| Hoof erosion | - | | - | - |

Only mild and transient clinical signs were observed in some of the animals in each group. In Group 1, one out of the 5 heifers had loss of appetite on day 8 PI. In Group 2, two out of the 3 animals had clinical signs. Both heifers experienced coughing around day 21 PI that was accompanied with loss of appetite in one of the animals.

**Rectal Temperatures**

No abnormal temperature changes were detected before the inoculation of the animals. The few cases of elevated temperatures measured after the inoculation are summarised in the table below.

| Group | Animal ID | Temperature (°C) | PI day |
|---|---|---|---|
| 1 | 1583 | 39.9 | 8 |
| | 1621 | 39.0 | 5 |
| 2 | 1438 | 39.0 | 2 |
| | 1585 | 40.8 | 9 |

One animal had slightly elevated temperature in each group, and also one animal had fever in each group. Fever was detected on day 8 or 9 PI. Temperature values always returned to normal value on the following day.

**Leukocyte Counts**

Some leukopenia was observed in all groups between PI days 3 - 8. The number of animals with at least 40 % reduction in white blood cell count was the following:

| Group | Number of animals having leukopenia / total |
|---|---|
| 1 | 3/5 (60%) |
| 2 | 1/3 (33%) |

**Serology (BVDV antibodies)**

In compliance with the study protocol, all heifers were free of BVDV antibodies before vaccination. In Group 1 (inoculated with XIKE-A NdN) and Group 2 (inoculated with XIKE-B), complete seroconversion was detected only at study termination (2 months after inoculation).

**BVD virus isolation from buffy coats**

No viremia was detected

**BVD virus isolation from foetal tissue samples**

| | Group 1 | Group 2 |
|---|---|---|
| N | 5 | 3 |
| Treatments | XIKE-A-NdN | XIKE-B / control |
| Route | Intramuscular | Intramuscular |
| Number of fetuses in which fetal transmission was detected: | 4 out of 5 foetuses infected | 2 out of 3 foetuses infected |
| Conclusion of the virus used for treatment has the potential to be transmitted over the placenta: | Fetal transmission for XIKE-A-NdN observed | Fetal transmission for XIKE - B observed |

**Conclusion**

The $N^{pro}$ deletion resulted in a considerable attenuation of the BVDV in comparison to the parental virus XIKE-A that was shown to be highly pathogenic (Meyer et al., 2002). However, the $N^{pro}$ deletion alone is not preventing transmission of a NY93 based virus recombinant to the foetus after inoculation of pregnant cows.

**EXAMPLE 3**

***BVDV XIKE-B-NdN: foetopathogenicity assessment in pregnant heifers***

To be able to test the potential of a combination of RNase inactivation and $N^{pro}$ deletion with regard to BVDV attenuation and fetal transmission, different BVDV-2 mutants with deletions within the $N^{pro}$ coding region were established based on the infectious cDNA clone pKANE40B, the RNase negative mutant of pKANE40A with a deletion of codon 349. The recovered viruses were analyzed with regard to presence of the desired mutations, the absence of second site mutations in the regions flanking the introduced changes and their growth characteristics in tissue culture. XIKE-B-NdN (V-pK88C), a variant containing a deletion of the complete $N^{pro}$ coding region except for codons 1 to 4 in addition to the RNase inactivating deletion of codon 349 was chosen for an animal experiment since it combined the desired mutations with acceptable growth characteristics. The aim of the study was to assess the safety of a live attenuated BVDV isolate in pregnant animals.
Five BVDV-negative, pregnant heifers were inoculated intranasally with an infective dose of $10^5$ TCID50/animal XIKE-B-NdN (back titration data are depicted in Table 3.1). Clinical data were recorded daily. Blood samples were collected for white blood cell counting, for buffy-coat preparation and serology. After termination of the study foetal tissues were collected for virus isolation.

**MATERIAL AND METHODS:**

As detailed for example 1:

**RESULTS**

No clinical data were observed (data not shown). Leukocyte counts remained virtually unchanged except for a significant decrease by approx. 40% below the baseline value (day0) in heifer no- 1015 on a single day (day 6 p.i.) (data not shown).

**Analysis of buffy coat preparations:**

Approximately $10^6$ leukocytes were cultured in duplicates with MDBK-cells in 24-well tissue culture plates for 5 days. Samples were freeze-thawed twice. One hundred microliter aliquots of thawed samples were inoculated onto freshly seeded 24-well tissue culture plates and tested for virus by indirect immuno-fluorescence staining (mAb Code 4, directed against a conserved epitope in nonstructural protein NS3). No BVDV could be isolated from the buffy coat preparations of animals # 921, 1013, 1015, 1055 and 1075 (Table 3.2) whereas positive controls clearly showed the correct conduction of the test.

**b) Post-mortem examination of foetal tissues**

After termination of the study the following foetal tissues were collected for virus isolation: spleen, kidney, thymus, sternum, cerebellum, placenta, intestine and abdominal fluid. Briefly, tissue suspensions were made in a mortar using sterile sea sand and ice-cold PBS without $Ca^{2+}$ and $Mg^{2+}$. Mortars were rinsed with 1 ml ice-cold PBS without $Ca^{2+}$ and $Mg^{2+}$ and suspensions were centrifuged for 10 min. at 2000 x g (4°C). The supernatant was first passed through a disposable 0.45 μm filter holder, followed by a second filter passage (0.2 μm pore size). Virus isolation was carried out in duplicates (400 μl foetal tissue suspension or 100 μl foetal abdominal fluid) on a monolayer of MDBK-cells in a 24 wells tissue culture plate (37°C, 7% CO2). Tissue samples were controlled daily for cytopathic effects or bacterial contamination, and after an incubation time of 5 days plates were frozen and thawed twice. 100 μl of samples were passaged to freshly seeded MDBK-cells. Virus was detected by indirect immuno-fluorescence staining (mAb Code 4). No BVDV could be detected in the tissue samples or foetal abdominal fluid (Table 3.3).

**c) Serological Findings**

Serum neutralization titres were determined before inoculation, 1 month post-inoculation and at termination of the study. Sera from all animals were tested in triplicates for neutralizing antibodies against NY93/C, and the endpoint dilution was read by indirect immunofluorescence staining. Results were expressed as the endpoint dilution, which neutralized approximately 100 $TCID_{50}$ and calculated by the method of Kaerber. No definite data could be obtained for day 0, and 1 and 2 weeks post infection as the sera were toxic for MBDK-cells in dilutions up to 1:16 and no neutralization could be detected at higher dilutions. Starting with the third week post vaccination all animals developed neutralising antibodies against the homologous BVDV-2 virus NY'93/C lasting till the end of the experiment (Table 3.4 and Fig. 1).

**d) Conclusions**

The data obtained during the animal study clearly show that BVDV XIKE-B-NdN represents a highly attenuated virus. In contrast to wild type virus or the single mutants XIKE-B or XIKE-A-NdN that show foetal transmission in pregnant heifers at high rates the double mutant did not cross the placenta. BVDV XIKE-B-NdN as well as similar double mutants are extremely suitable for the use in a live attenuated vaccine.

**Study No.: B01 BIVI020 and B01 BIVI022**

**Table 3.1: Back Titration of Viruses**

| Sample ID | Virus Strain | Dilution | Titre |
|---|---|---|---|
| 1a | XIKE-B-NdN | concentrated virus | $10^{5,44}$ $TCID_{50}$/ ml |
| 1 | | 1:4 | $10^{4,86}$ $TCID_{50}$/ ml |
| 6 | | residues of infection (#1) | $10^{4,27}$ $TCID_{50}$/ ml |

**Table 3.2. Detection of Viremia**

| Animal ID No. | *Days after vaccination* | | | | | | | | | | | | | | | |
|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | |
| 0921 | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | 1. isolation<br>2. isolation |
| 1013 | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | 1. isolation<br>2. isolation |
| 1015 | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | 1. isolation<br>2. isolation |
| 1055 | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | 1. isolation<br>2. isolation |
| 1075 | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | --<br>-- | 1. isolation<br>2. isolation |
| Date | 13.12<br>2001 | 14.12<br>2001 | 15.12<br>2001 | 16.12<br>2001 | 17.12<br>2001 | 18.12<br>2001 | 19.12<br>2001 | 20.12<br>2001 | 21.12<br>2001 | 22.12<br>2001 | 23.12<br>2001 | 24.12<br>2001 | 25.12<br>2001 | 26.12<br>2001 | 27.12<br>2001 | |
| - sample negative | | | | | | | | | | | | | | | | |

**Table 3.3 Analysis of foetus tissue samples for the presence of BVDV**

| Animal No. | Abdominal fluid | Thoracic Fluid | Mesenteric lymph nodes | Spleen | Kidney | Thymus | Bone marrow (sternum) | Cerebellum | Placenta | Intestine | Date of tissue collection | Isolation |
|---|---|---|---|---|---|---|---|---|---|---|---|---|
| 0921 | --<br>-- | NC | NC | --<br>-- | --<br>-- | --<br>-- | --<br>-- | --<br>-- | --<br>-- | -<br>-- | 12.02.02 | 1. isolation<br>2. isolation |
| 1013 | --<br>-- | NC | NC | --<br>-- | --<br>-- | --<br>-- | --<br>-- | --<br>-- | --<br>-- | --<br>-- | 12.02.02 | 1. isolation<br>2. isolation |
| 1015 | --<br>-- | NC | NC | --<br>-- | --<br>-- | --<br>-- | --<br>-- | --<br>-- | --<br>-- | --<br>-- | 12.02.02 | 1. isolation<br>2. isolation |
| 1055 | --<br>-- | NC | NC | --<br>-- | --<br>-- | --<br>-- | --<br>-- | --<br>-- | --<br>-- | --<br>-- | 12.02.02 | 1. isolation<br>2. isolation |
| 1075 | --<br>-- | NC | NC | --<br>-- | --<br>-- | --<br>-- | --<br>-- | --<br>-- | --<br>-- | --<br>-- | 12.02.02 | 1. isolation<br>2. isolation |

NC = Not collected
- = sample negative

**Table 3.4: B01 BIVI022 / BVDV XIKE-B-NdN; foetal protection study Serum Neutralization Assay**

| Animal ID No. | From the heifers | | | | | | | | | |
|---|---|---|---|---|---|---|---|---|---|---|
| | at selection | during ACC | 1 wPV | 2 wPV | 3 wPV | 4 wPV | 5 wPV | 6 wPV | 7 wPV | 8 wPV |
| 0921 | | * | * | * | 1:40(2) | 1:161(1) | 1:256(1) | 1:323(1) | 1:128(1) | 1:256(1) |
| 1013 | | * | * | * | 1:3(2) | NA | 1:161(1) | 1:323(1) | 1:406(1) | 1:256(1) |
| 1015 | | * | * | * | 1:64(2) | 1:161(1) | 1:256(1) | 1:323(1) | 1:406(1) | 1:323(1) |
| 1055 | | * | * | * | 1:32(2) | 1:40(2) | 1:256(1) | 1:323(1) | 1:406(1) | 1:406(1) |
| 1075 | | * | * | * | NA | 1:128(2) | 1:102(1) | 1:203(1) | 1:161(1) | 1:406(1) |
| Date | | 06.12.2001 | 20.12.2001 | 27.12.2001 | 03.01.2002 | 10.01.2002 | 17.01.2002 | 24.01.2002 | 31.01.2002 | 07.02.2002 |

(1) SNT against 1456 Nase (= NY93/C) $10^{2,03}$ $TCID_{50}$/50µl * Serum toxic for MBDK-cells in dilutions up to 1:16 ⇒ no data available
(2) SNT against 1456 Nase (= NY93/C) $10^{1,57}$ $TCID_{50}$/50µl NA data not available

The Serum Neutralisation Assay against NY93/C is illustrated in Fig. 1.

**Efficacy and crossprotection study**

Two possible problems have to be faced with regard to vaccination with attenuated virus mutants BVDV XIKE-B or BVDV XIKE-B-NdN. First, there is a general problem concerning cross protection between BVDV-1 and BVDV-2. At least vaccination with inactivated BVDV-1 vaccines did not prevent the transmission of BVDV-2 to the foetus in pregnant animals. Since protection against foetal infection represents the major aim of anti BVDV vaccination, such vaccines cannot be regarded to induce a protective immunity on a broad range. The question therefore was, whether vaccination with live attenuated BVDV-2 can prevent virus transmission to the foetus. Second, the reduced growth rates of BVDV XIKE-B-NdN might result in only a low level of protection not able to prevent transplacental infection of the foetus in pregnant heifers. To address these problems, an animal study was started. The animals (2 groups of 10 animals each) were vaccinated either with BVDV XIKE-B or XIKE-B-NdN (intended dosage: 1ml of supernatant with $10^5$ $TCID_{50}$ of virus; backtitration is shown in Tab. 3.5). None of the animals showed significant clinical signs after the vaccination except for one animal of the nonvaccinated control group with mild coughing for one day. Rectal temperature values were below 39 °C except for one animal of the nonvaccinated control group that hat 39.1°C for one day. Buffy coat samples prepared after vaccination were analysed for the presence of virus as described above. The experiments showed that only 5 of the 20 animals contained virus in the blood for 1 or 2 days at 4 to 8 days post infection (Tab. 3.6).

**Table 3.5: Back Titration of Viruses used for vaccination**

| Sample ID | Virus Strain | Dilution | Titre |
|---|---|---|---|
| 1a | XIKE-B-NdN | concentrated virus | $10^{5,44}$ $TCID_{50}$/ ml |
| 1 | | 1:4 | $10^{4,86}$ $TCID_{50}$/ ml |
| 6 | | residues of infection (#1) | $10^{4,27}$ $TCID_{50}$/ ml |
| 3 | XIKE-B | 1:11 | $10^{5,76}$ $TCID_{50}$/ ml |
| 4 | | 1:110 | $10^{4,92}$ $TCID_{50}$/ ml |
| 5 | | residues of infection (#4) | $10^{4,27}$ $TCID_{50}$/ ml |

**Study No. / Id.: B01 BIVI020 / BVDV Tü XIKE-B/XIKE-B-NdN; foetal protection study**

**Table 3.6: Inoculation with white blood cell (buffy coat) preparations collected after vaccination**

| Animal ID No. | *Days after vaccination* | | | | | | | | | | | | | | | |
|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | |
| **1134**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1141**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1142**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1145**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1149**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1151**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1152**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1156**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1158**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1160**(2) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| Date | 13.12<br>2001 | 14.12<br>2001 | 15.12<br>2001 | 16.12<br>2001 | 17.12<br>2001 | 18.12<br>2001 | 19.12<br>2001 | 20.12<br>2001 | 21.12<br>2001 | 22.12<br>2001 | 23.12<br>2001 | 24.12<br>2001 | 25.12<br>2001 | 26.12<br>2001 | 27.12<br>2001 | |
| **1197**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |
| **1200**(1) | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | ØØ<br>ØØ | 1. isolation<br>2. isolation |

(continued)

| Animal ID No. | *Days after vaccination* | | | | | | | | | | | | | | | |
|---|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 | |
| **1206(2)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅+ | ∅+<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1210(2)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1212(1)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅+<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1214(1)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1216(2)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1217(1)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅+<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1218(1)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| **1225(2)** | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | ∅∅<br>∅∅ | 1. isolation<br>2. isolation |
| Date | 13.12<br>2001 | 14.12<br>2001 | 15.12<br>2001 | 16.12<br>2001 | 17.12<br>2001 | 18.12<br>2001 | 19.12<br>2001 | 20.12<br>2001 | 21.12<br>2001 | 2212<br>2001 | 23.12<br>2001 | 24.12<br>2001 | 25.12<br>2001 | 26.12<br>2001 | 27.12<br>2001 | |

Immunofluorescence staining: Code 4
[] sample negative
+ sample positive
B bacterial contamination in well

**Code of animal numbers:**
**(1) vaccination with BVDV XIKE-B (RNase mutant)**
**(2) vaccination with BVDV XIKE-8-NdN (RNase and $N^{pro}$ double mutant)**

Four weeks after vaccination, insemination of the animals was carried out. Challenge infections were performed 60 to 90 days later using either a BVDV-1 strain (BVDV KE-9, heterologous challenge, animals vaccinated with XIKE-B) or a heterologous BVDV-2 strain (BVDV KE-13, homologous challenge, animals vaccinated with XIKE-B-NdN) (intended dosage: $10^5$ TCID50 in 6ml; backtitration is shown in Tab. 3.7). From each group of vaccinated animals 5 pregnant heifers were randomly selected for the challenge infection. Animals vaccinated with BVDV XIKE-B were challenged with the BVDV-1 strain KE-9, whereas heifers vaccinated with BVDV XIKE-B/IVdN were challenged with BVDV-2 KE-13. In addition, 2 nonvaccinated control animals were infected with each of the challenge viruses.

**Study No. / Id.: B01 BIVI020 / BVDV Tü XIKE-B-NdN; foetal protection study**

**Table 3.7: Back titration of challenge viruses**

| Virus Strain | Sample ID | Titre ($TCID_{50}$ / ml) | Mean Titre ($TCID_{50}$ / ml) |
|---|---|---|---|
| KE 9 | 1 | $10^{4,44}$ | $10^{4,94}$ |
| | | $10^{5,10}$ | |
| | 2 | $10^{4,69}$* | $10^{4,44}$ |
| | 3 | ** | |
| KE13 | 1 | $10^{4,69}$ | $10^{4,76}$ |
| | | $10^{4,82}$ | |
| | 2 | $10^{4,57}$ | $10^{4,63}$ |
| | | $10^{4,69}$ | |
| | 3*** | $10^{3,5}$ | $10^{3,5}$ |

Sample 1: stock of inoculate
Sample2: stock of inoculate returned from the stable
Sample3: excess inoculate
* Second inoculation of KE9, sample 2 wasn't interpretable because of cell death.
** KE9, sample 3 wasn't interpretable because of cell death or bacterial contamination.
*** First inoculation of KE13, sample 3 wasn't interpretable because of bacterial contamination.

The vaccinated animals did not show viremia or clinical symptoms upon challenge infection. The challenge was successful as all non-vaccinated controls were BVDV positive (Tab. 3.8). Only mild signs of disease were observed in the control groups. The white blood cell counts were nearly normal (not shown).

**Study No. / Id.: B01 BIVI020 / BVDV Tü XIKE-B/XIKE-B-NdN; foetal protection study**

**Table 3.8: Inoculation with white blood cell (buffy coat) preparations collected after challenge**

| Animal ID No. | *Days after challenge* | | | | | | | | | | | | | | | | |
|---|---|---|---|---|---|---|---|---|---|---|---|---|---|---|---|---|---|
| | 0 | 2 | 4 | 6 | 8 | 10 | 12 | 14 | 16 | 18 | 20 | 22 | 24 | 26 | 28 | 30 | Isolation |
| **1104(3)** | ∅∅ | ∅∅ | ∅∅ | ∅+ | ++ | ++ | ∅+ | ∅∅ | ∅∅ | ∅∅ | ∅∅ | ∅∅ | ∅∅ | ∅∅ | ∅∅ | ∅∅ | 1. isolation |
| | ∅∅ | ∅∅ | ∅∅ | +∅ | ++ | ∅∅ | ∅∅ | ∅∅ | ∅∅ | ∅∅ | ∅∅ | ∅∅ | ∅∅ | ∅∅ | ∅∅ | ∅∅ | 2. isolation |
| **1108(3)** | ∅∅ | ∅∅ | ∅∅ | ∅∅ | ++ | +∅ | ∅∅ | ∅∅ | ∅∅ | ∅∅ | ∅∅ | ∅∅ | ∅∅ | ∅∅ | ∅∅ | ∅∅ | 1. isolation |
| | ∅∅ | ∅∅ | ∅∅ | ∅+ | ∅∅ | ∅∅ | ∅∅ | ∅∅ | ∅∅ | ∅∅ | ∅∅ | ∅∅ | ∅∅ | ∅∅ | ∅∅ | ∅∅ | 2. isolation |
| **1126(3)** | ∅∅ | ∅∅ | ++ | +∅ | ++ | ∅+ | ∅∅ | ∅+ | ∅+ | ∅∅ | ∅∅ | ∅∅ | ∅∅ | ∅∅ | ∅∅ | ∅∅ | 1. isolation |
| | ∅∅ | | | | | | | ∅∅ | | | | | | | | | 2. isolation |
| **1145(2)** | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | 1. isolation |
| | | | | | | | ∅∅ | | | | | | | | | | 2. isolation |
| **1151(2)** | ∅∅ | ∅∅ | ∅∅ | ∅∅ | | ∅∅ | ∅∅ | ∅∅ | | ∅∅ | ∅∅ | ∅∅ | ∅∅ | ∅∅ | ∅∅ | ∅∅ | 1. isolation |
| | | | | | | | | ∅∅ | ∅∅ | | | | | | | | 2. isolation |
| | ∅∅ | ∅∅ | ∅∅ | ∅∅ | ∅∅ | ∅∅ | ∅∅ | ∅∅ | ∅∅ | ∅∅ | ∅∅ | ∅∅ | | | | | 3. isolation |
| **1152(2)** | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | ∅∅ | | 1. isolation |
| | | | | | | | | | | | | | | | | ∅∅ | 2. isolation |
| | | | | | | | | | | | | | | ∅∅ | ∅∅ | ∅∅ | 3. isolation |

(continued)

| Animal ID No. | Days after challenge | | | | | | | | | | | | | | | | |
|---|---|---|---|---|---|---|---|---|---|---|---|---|---|---|---|---|---|
| | 0 | 2 | 4 | 6 | 8 | 10 | 12 | 14 | 16 | 18 | 20 | 22 | 24 | 26 | 28 | 30 | Isolation |
| **1156**(2) | ØØ | ØØ | ØØ | ØØ | ØØ | | ØØ | ØØ | | | ØØ | ØØ | ØØ | ØØ | ØØ | ØØ | 1. isolation |
| | | | | | | | | | ØØ | | | | | | | | 2. isolation |
| | ØØ | ØØ | ØØ | ØØ | ØØ | ØØ | ØØ | ØØ | ØØ | ØØ | ØØ | ØØ | | | | | 3. isolation |
| Date | 04.22 2002 | 04.25 2002 | 04.27 2002 | 04.29 2002 | 05.01 2002 | 05.03 2002 | 05.05 2002 | 05.07 2002 | 05.09 2002 | 05.11 2002 | 05.13 2002 | 05.15 2002 | 05.17 2002 | 05.19 2002 | 05.21 2002 | 05.23 2002 | |
| **1197**(1) | ØØ | ØØ | ØØ | ØØ | | ØØ | ØØ | ØØ | ØØ | ØØ | ØØ | ØØ | ØØ | ØØ | ØØ | ØØ | 1. isolation |
| | | | | | | | | | | | | | | | | | 2. isolation |
| | ØØ | ØØ | ØØ | ØØ | ØØ | ØØ | ØØ | ØØ | ØØ | ØØ | ØØ | ØØ | | | | | 3. isolation |
| **1200**(1) | ØØ | ØØ | ØØ | ØØ | ØØ | ØØ | ØØ | ØØ | ØØ | ØØ | ØØ | ØØ | ØØ | ØØ | ØØ | ØØ | 1. isolation |
| | | | | | | | | | | | | | | | | | 2. isolation |
| **1214**(1) | ØØ | ØØ | ØØ | ØØ | ØØ | ØØ | ØØ | ØØ | ØØ | ØØ | ØØ | ØØ | ØØ | ØØ | ØØ | | 1. isolation |
| | | | | | | | | | | | | | | | | ØØ | 2. isolation |
| | | | | | | | | | | | | | | | ØØ | | 3. isolation |
| **1216**(2) | ØØ | ØØ | ØØ | ØØ | ØØ | ØØ | ØØ | ØØ | ØØ | ØØ | ØØ | ØØ | ØØ | ØØ | ØØ | ØØ | 1. isolation |
| | | | | | | | | | | | | ØØ | | | | | 2. isolation |
| | | | | | | | | | ØØ | | | ØØ | | | | | 3. isolation |

(continued)

| Animal ID No. | Days after challenge | | | | | | | | | | | | | | | | |
|---|---|---|---|---|---|---|---|---|---|---|---|---|---|---|---|---|---|
| | 0 | 2 | 4 | 6 | 8 | 10 | 12 | 14 | 16 | 18 | 20 | 22 | 24 | 26 | 28 | 30 | Isolation |
| **1217**(1) | ØØ | ØØ | ØØ | ØØ | ØØ | ØØ | ØØ | ØØ | ØØ | ØØ | ØØ | ØØ | ØØ | ØØ | ØØ<br><br>ØØ | ØØ | 1. isolation<br>2. isolation<br>3. isolation |
| **1218**(1) | ØØ | ØØ | ØØ | ØØ | ØØ | ØØ | ØØ | ØØ | ØØ | ØØ | ØØ | ØØ | ØØ | ØØ | ØØ | ØØ | 1. isolation<br>2. isolation |
| **1249**(3) | ØØ | ØØ | ØØ | ØØ | ++<br>++ | ØØ<br>+Ø | ØØ<br>ØØ | ØØ<br>ØØ | ØØ | ØØ | ØØ | ØØ | ØØ | ØØ | ØØ<br>ØØ | ØØ | 1. isolation<br>2. isolation |
| Date | 04.22 2002 | 04.25 2002 | 04.27 2002 | 04.29 2002 | 05.01 2002 | 05.03 2002 | 05.05 2002 | 05.07 2002 | 05.09 2002 | 05.11 2002 | 05.13 2002 | 05.15 2002 | 05.17 2002 | 05.19 2002 | 05.21 2002 | 05.23 2002 | |

Immunnfluorescence staining: Code 4

[]sample negative

+ sample positive

B bacterial contamination in well

**<u>Code of animal numbers:</u>**

**(1) vaccination with BVDV XIKE-B (RNase mutant)**

**(2) vaccination with BVDV XIKE-B-NdN (RNase and $N^{Pro}$ double mutant).**

**(3) nonvaccinated controls**

Serum neutralization titers were determined before inoculation, 1 month post-inoculation, before challenge, 1 month after challenge and at termination of the study: Sera from all animals were tested in triplicates for neutralizing antibodies against KE9 and NY93/C (1456Nase), and the endpoint dilution was read by indirect immunofluorescence staining. Results were expressed as the endpoint dilution, which neutralized approximately 100 $TCID_{50}$ and calculated by the method of Kaerber. At some of the higher antibody titres, the used endpoint dilution was not high enough. Against KE9, only animals vaccinated with XIKE-B developed low antibody titres starting about week 4. At challenge, all animals had antibody titres, which increased considerably starting around week 4 post challenge. XIKE-B vaccinated animals had higher antibody titres then those vaccinated with XIKE-B-NdN vaccinated. All animals developed about the same neutralization titre against NY93/C four weeks post vaccination, with marginally lower titres in XIKE-B-NdN vaccinated animals. After challenge all animals had high antibody titres. Fig. 2 shows the serum neutralization assay against KE9 (BVDV-1) and Fig. 3 shows the serum neutralization assay against NY93/C (BVDV-2).

Analysis of tissue samples obtained after termination of the study from the foetuses revealed that the material obtained from the vaccinated animals gave negative results whereas transmission had occurred in all 4 control animals (Tab. 3.9). Thus, it is clear that the established BVDV-2 mutants are well suited as efficient cross protective vaccine viruses.

**Study No. / Id.: B01 BIVI020 / BVDV Tü XIKE-B/XIKE-B-NdN; foetal protection study**

**Table 3.9: Analysis of foetus tissue samples for the presence of BVDV**

| Animal No. | Abdominal Fluid | Thoracic fluid | Mesenteric lymph nodes | Small intestine | Spleen | Thymus | Kidney | Bone marrow (stemum) | Cerebellum | Placenta | Date |
|---|---|---|---|---|---|---|---|---|---|---|---|
| **1214**(1) | NA | NA | ∅∅ | ∅∅ | ∅∅ | ∅∅ | ∅∅ | ∅∅ | ∅∅ | ∅∅ | 17.06.02 |
| **1126**(3) | ++ | *** | ++ | ++ | ++ | ++ | ++ | ++ | ++ | ++ | 17.06.02 |
| **1249**(3) | ++ | NA | ++ | ++ | ++ | ++ | ++ | ++ | ++ | ∅+ | 17.06.02 |
| **1218***(1) | NA | NA | NA | NA | NA | NA | NA | NA | NA | ∅∅** | 17.06.02 |
| **1197**(1) | ∅∅ | NA | ∅∅ | ∅∅ | ∅∅ | ∅∅ | ∅∅ | ∅∅ | ∅∅ | ∅∅ | 17.06.02 |
| **1217**(1) | ∅∅ | NA | ∅∅ | ∅∅ | ∅∅ | ∅∅ | ∅∅ | ∅∅ | ∅∅ | ∅∅ | 17.06.02 |
| **1200**(1) | ∅∅ | NA | ∅∅ | ∅∅ | ∅∅ | ∅∅ | ∅∅ | ∅∅ | ∅∅ | ∅∅ | 17.06.02 |
| **1145**(2) | ∅∅ | NA | ∅∅ | ∅∅ | ∅∅ | ∅∅ | ∅∅ | ∅∅ | ∅∅ | ∅∅ | 21.06.02 |
| **1108**(3) | +∅ | NA | ++ | ++ | ++ | ++ | ++ | ++ | ++ | ∅∅ | 21.06.02 |
| **1156**(2) | ∅∅ | NA | ∅∅ | ∅∅ | ∅∅ | ∅∅ | ∅∅ | ∅∅ | ∅∅ | ∅∅ | 21.06.02 |
| **1104**(3) | ++ | NA | ++ | ++ | ++ | ++ | ++ | ++ | ++ | ++ | 21.06.02 |
| **1216**(2) | ∅∅ | NA | ∅∅ | ∅∅ | ∅∅ | ∅∅ | ∅∅ | ∅∅ | ∅∅ | ∅∅ | 21.06.02 |
| **1151**(2) | NA | NA | ∅∅ | ∅∅ | ∅∅ | ∅∅ | ∅∅ | ∅∅ | ∅∅ | ∅∅ | 21.06.02 |
| **1152**(2) | ∅∅ | NA | ∅∅ | ∅∅ | ∅∅ | ∅∅ | ∅∅ | ∅∅ | ∅∅ | ∅∅ | 21.06.02 |

NA = not available
*No foetus was found in the uterus of heifer #1218
**Endometrium (also collected for histology)
*** Sample was not sent to BFA Tübingen **Code of animal numbers:**

**(1) vaccination with BVDV XIKW-B (RNase mutant)**
**(2) vaccination with BVDV XIKE-B-NdN (RNase and $N^{pro}$ double mutant)**
**(3) nonvaccinated controls**

### Conclusion

The challenge was successful as all non-vaccinated controls were BVDV viraemic and foetuses of all non-vaccinated controls were BVDV positive.

Both isolates gave full protection under the present test and assay conditions. Isolate XIKM-B, with the single genetic marker was shown to cross-protect against type 1 BVDV challenge in terms of BVD viraemia and transmission to the foetus after challenge. Isolate XIKE-B-NdN with the double genetic marker was able to fully protect against a heterologue type 2 BVDV challenge strain in terms of BVD viraemia and transmission to the foetus after challenge.

1. Isolate XIKE-B (type 2 isolate) was shown to cross-protect against type 1 BVDV challenge in terms of BVD viraemia and transmission to the foetus after challenge under the present test and assay conditions (n=4).
2. Isolate XIKE-B-NdN (type 2 isolate) fully protected against a heterologues type 2 BVDV challenge strain in terms of BVD viraemia and transmission to the foetus after challenge under the present test and assay conditions (n=5).

## EXAMPLE 4

### Establishment of $N^{pro}$ mutants

Further analyses of BVDV-2 mutants with $N^{pro}$ deletions. Different mutants with deletions in the $N^{pro}$-coding region of the genome were established. Initially, only true deletions or a deletion accompanied by a point mutation were introduced.

A: $[N^{pro}]_1$-[C-term];
B: $[N^{pro}]_3$-[C-term];
C: $[N^{pro}]_4$-[C-term];
D: $[N^{pro}]_6$-[C-term];
E: $[N^{Pro}]_4$-[C-term*]

In the formulas $[N^{pro}]_x$ represents the number of residues of the aminoterminus of $N^{pro}$ that are left in the mutated polyprotein amino acids, [C-term] is the complete polyprotein except for $N^{Pro}$ (starting with the C protein and ending with NS5B), and [C-term*] is the same as [C-term] but with a mutation at position 2 of the C protein (N instead of D).

The growth rates of the recovered viruses were considerably lower than those of wild type XIKE-A or the RNase negative mutant XIKE-B. There are two possible explanations for this finding: (i) dependent on the virus strain, sequences of variable length of the $N^{pro}$-coding region are necessary for efficient translation initiation (Myers et al., 2001; Tautz et al., 1999) and (ii) the fusion of additional sequences to the aminoterminus of the capsid protein interferes with capsid protein function.
To obtain better growing $N^{pro}$ deletion mutants, a second set of mutants was generated with either a bovine ubiquitin gene or a fragment of the bovine LC3-coding sequence replacing the major part of the $N^{pro}$ gene. These constructs allow efficient translation and generate a capsid protein with the correct amino terminus.

$[N^{pro}]_{22}$-[PS]- [C-term]

wherein PS is ubiquitin or LC3, C-term is the complete polyprotein except for $N^{pro}$ (starting with the C protein and ending with NS5B).

The growth rates of these mutants were more similar to what was determined for XIKE-A. It even seemed that the two RNase positive viruses according to the formula $[N^{pro}]_{22}$-[PS]- [C-term] named V-pK87F and V-pK87G showed no significant growth retardation at all, whereas the RNase negative counterpart V-pK88G once again was somewhat hampered in propagation but to a lesser extend than the formerly descirbed mutants.

Further examples of $N^{pro}$ deletion mutants may be:

*ME*SDEGSK...
*MELFS*SDEGSK...
*MELFSNE*SDEGSK...
*MELFSNEL*SDEGSK...
*MELFSNELL*SDEGSK...
*MELFSNELLY*SDEGSK...
*MELFSNELLYK*SDEGSK...

*MELFSNELLYKT*SDEGSK...
*MELFSNELLYKT* represents the aminoterminal sequence of $N^{Pro}$ of the BVDV isolate NewYork93/C.

It may also be possible to use variants of this sequence with one or several mutations. Especially the naturally occurring variations as found in other pestiviruses can be expected to be functional. Therefore, the complete list of the tested or proposed variants with the different parts of the aminoterminal end of $N^{pro}$ can be enlarged by equivalent sets with amino acid exchanges. Below, typical examples of the respective sequences are given for several pestiviruses but the possible variations are not limited to these examples.

| | |
|---|---|
| **BVDV New York93/C:** | *MELFSNELLYKT* |
| **BVDV CP13:** | *MELISNELLYKT* |
| **BVDV SD1:** | *MELITNELLYKT*_ |
| **CSFV Brescia:** | *MELNHFELLYKT* |
| **BDV X818:** | *MELNKFELLYKT* |

Thus, these variants for example may include: *MELI*-$[PS]_0$-[C-term];

*MELIS*-$[PS]_0$-[C-term];
*MELISN*-$[PS]_0$-[C-term];
*MELISNE*-$[PS]_0$-[C-term];
*MELISNEL*-$[PS]_0$-[C-term];
*MELISNELL*-$[PS]_0$-[C-term];
*MELISNELLY*-$[PS]_0$-[C-term];
*MELISNELLYK*-$[PS]_0$-[C-term];
*MELISNELLYKT*-$[PS]_0$-[C-term];
*MELIT*-$[PS]_0$-[C-term];
*MELITN*-$[PS]_0$-[C-term];
*MELITNE*-$[PS]_0$-[C-term];
*MELITNEL*-$[PS]_0$-[C-term];
*MELITNELL*-$[PS]_0$-[C-term];
*MELITNELLY*-$[PS]_0$-[C-term];
*MELITNELLYK*-$[PS]_0$-[C-term];
*MELIINELLYKT*-$[PS]_0$-[C-term];

These formulas may also have $[PS]_1$, i.e. PS may also be one of the PS as described herein. Sequences belonging to the $N^{pro}$ protein are in italics. Amino acid exchanges with regard to the sequence of BVDV NewYork93/C are in bold.

Further examples can be found e.g. by using the GenBank accession numbers given in Becher et al., 2003, Virology 311, 96-104) or by standard sequence data searches.

A further possibility could be the use of a processing signal (PS) inserted between the (residual) $N^{pro}$ sequence and the aminoterminus of the capsid protein. The PS leads to a cleavage that generates a functional capsid protein. The configuration of such constructs could be as follows:

$$[N^{pro}]_{22}\text{-PS[C-term]}$$

PS: Processing signal. Can either be a target for a protease (e.g. ubiquitin, LC3 as defined herein or a protease or an unstable peptide leading to processing at its own carboxyterminus like e.g. intein (Chong et al. 1998 and references therein) or 3C of picomaviruses, 2A of cardioviruses or aphtoviruses, p15 of rabbit hemorrhagic disease virus or the corresponding protease of other caliciviruses (Proter 1993, and references therein; Meyers et al., 2000 and references therein). When using a PS, a large number of different variants are possible since the PS ensures the generation of the correct amino terminus of the capsid protein C. Thus, when using a PS construct, all kinds of deletions or mutations of the $N^{Pro}$ sequence are expected to result in viable mutants as long as the reading frame is not shifted or translation stopped by an in frame stop codon. As an example we established a viable CSFV $N^{pro}$ deletion mutant according to the formula

$[N^{pro}]_{29}$-PS[C-term]

Especially interesting could be $N^{pro}$ mutations blocking the proteolytic activity of the protein. Rümenapf et al. (1998) have published the identification of the active site residues of the protease for CSFV Alfort Tübingen. The respective amino acids (glutamic acid at position 22, histidine at position 49 and cysteine at postion 69) are conserved for other pestiviruses. Thus, exchanges of any amino acid expect for serine or threonine for the cysteine at position 69 will result in destruction of the protease activity. Similarly, changing the glutamic acid at position 22 will most likely result in inactivation of the protease unless the new amino acid is aspartic acid. Similarly most if not all exchanges at position 49 will lead to an inactive protease).

References

Ausubel, F.M. et al., Current Protocols in molecular biology. New York: Greene Publishing Associates and Wiley-Interscience. 1994 (updated)

Baker, J.C. 1987. Bovine viral diarrhea virus: a review. J. Am. Vet. Med.Assoc. 190: 1449-1458.

Becher, P., König, M., Paton, D.J., Thiel, H.J., 1995, Further characterization of border disease virus isolates: evidence for the presence of more than three species within the genus pesivirus. Virology 209 (1), 200-206.

Chong, S., Williams, K.S., Wotkowicz, C., and Xu, M.Q.1998. Modulation of Protein Splicing of the Saccharomyces cerevisiae Vacuolar Membrane ATPase Intein. J. Biol. Chem. 273: 10567 - 10577.Donis, R.O., Corapi, W., and Dubovi, E.J. 1988. Neutralizing monoclonal antibodies to bovine viral diarrhea virus bind to the 56K to 58K glycoprotein. J. Gen. Virol. 69: 77-86.

Fuerst T.R. et al. 1986. Eukaryotic transient expression system based on recombinant vaccinia virus that synthesizes bacteriophage T7 RNA polymerase. Proc. Natl. Acad. Sci. 83: 8122-8126.

Heinz, F.X., Collett, M.S., Purcell., R.H., Cold, E.A., Howard, C.R., Houghton, M., Moormann, R.J.M., Rice, C.M., and Thiel, H.-J. 2000. Familiy Flaviviridae. PP 859-878. In: Virus Taxonomy (van Regenmortel., H.H.V., Fauquet, C.M., and Bishop, D.H.L, Eds.). Academic. Press, San Diego.

Hulst, M.M., Himes, G., Newbigin, E., Moormann, R.J.M. 1994. Glycoprotein E2 of classical swine fever virus: expression in insect cells and identification as a ribonuclease. Virology 200: 558-565.

Hulst, M.M., F.E. Panoto, A. Hooekmann, H.G.P. van Gennip., and Moormann, R.J.M. 1998. Inactivation of the RNase activity of glycoprotein Ems of classical swine fever virus results in a cytopathogenic virus. J. Virol. 72: 151-157.

Kit, M. and S. Kit. 1991. Sensitive glycoprotein gIII blocking ELISA to distinguish between pseudorabies (Aujeszky's disease) -infected and vaccinated pigs. Veterinary Microbiology 28:141-155.

Kunkel, T. A., J. D. Roberts, and R. A. Zakour. 1987. Rapid and efficient site-specific mutagenesis without phenotypic selection. Methods Enzymol. 154:367-392.

König, Matthias, 1994, Virus der klassischen Schweinepest: Untersuchungen zur Pathogenese und zur Induktion einer protektiven Immunantwort. Dissertation, Tierärztliche Hochschule Hannover, Germany.

Lindenbach, B.D., and Rice, C. M. 2001. The pestiviruses. In Fields Virology, eds. Knipe, D.M., & Howley, P.M. (Lippincott-Raven, Philadelphia), pp. 991-1042.Mayer, D., Hofmann, M.A., and Tratschin, J.D. 2004. Attenuation of classical swine fever virus by deletion of the viral N(pro) gene. Vaccine. 22:317-328.

Meyers, G., Rümenapf, T. and Thiel, H.-J. 1989. Molecular cloning and nucleotide sequence of the genome of hog cholera virus.Virology 171: 555-567.

Meyers,G., Saalmüller,A., and Büttner,M. (1999). Mutations abrogating the RNase activity in glycoprotein e(ms) of the pestivirus classical swine fever virus lead to virus attenuation. J Virol 73: 10224-10235.

Meyers, G., Tautz, N., Becher, P., Thiel, H.-J., & Kümmerer, B.M. 1996b. Recovery of cytopathogenic and noncytopathogenic bovine viral diarrhea viruses from cDNA constructs. J. Virol., 70: 8606-8613.

Meyers, G., Thiel, H.-J., and Rümenapf, T. 1996a. Classical swine fever virus: Recovery of infectious viruses from cDNA constructs and generation of recombinant cytopathogenic swine fever virus. J. Virol. 67:7088-709526.

Meyers, G., Wirblich, C., Thiel. H.-J. and Thumfart, J.O. 2000. Rabbit hemorrhagic disease Virus: genome organization and polyprotein processing of a calicivirus studied after transient expression of cDNA constructs. Virology 276:349-363.

Moennig, V. and Plagemann, J. 1992. The pestiviruses. Adv. Virus Res. 41: 53-91.

Paton, D.J., Lowings, J.P., Barrett, A.D. 1992. Epitope mapping of the gp53 envelope protein of bovine viral diarrhea virus. Virology 190: 763-772.

Pellerin, C. et. al. Identification of a new group of bovine viral diarrhea virus strains associated with severe outbreaks and high mortalities, Virology 203, 1994:260-268.

Porter, A.G. (1993). Picornavirus nonstructural proteins: emerging roles in virus replication and inhibition of host cell functions. J. Virol. 67,6917-6921.

Rüggli, N., Tratschin, J.D., Schweizer, M., McCullough, K.C., Hofmann, M.A., Summerfield, A. 2003. Classical swine fever virus interferes with cellular antiviral defense: evidence for a novel function of N(pro). J. Virol. 77:7645-7654.

Rümenapf, T., Stark, R., Heimann, M., and Thiel, H.-J. 1998. N-terminal protease of pestiviruses: identification of putative catalytic residues by site directed mutagenesis. J. Virol. 72: 2544-2547.

Rümenapf, T., Unger, G., Strauss, J.H., and Thiel, H.-J. 1993. Processing of the evelope glycoproteins of pestiviruses. J. Virol. 67: 3288-3294.Schneider, R., G. Unger, R. Stark, E. Schneider-Scherzer, and H.-J. Thiel. 1993. Identification of a structural glycoprotein of an RNA virus as a ribonuclease. Science 261: 1169-1171.

Sambrook, J., Fritsch, E.F. & Maniatis, T.,Molecular Cloning: A Laboratory Manual Cold Spring Harbor Laboratory, Cold Spring Harbor, New York, 1989

Stark, R., Meyers, G., Rümenapf, T., and Thiel, H.-J. (1993): Processing of pestivirus polyprotein: Cleavage site between autoprotease and nucleocapsid protein of classical swine fever virus. J. Virol., 67, 7088-7095.Thiel, H.-J., Plagemann, G.W., & Moennig, V. 1996. The pestiviruses. In Fields Virology, eds. Fields, B.N., Knipe, D.M., & Howley, P.M. (Lippincott-Raven, Philadelphia), pp.1059-1073.

Thiel, H.-J., Stark, R., Weiland, E., Rümenapf, T. & Meyers, G. 1991. Hog cholera virus: molecular composition of virions from a pestivirus. J. Virol. 65: 4705-4712.31.

Tratschin, J.-D., Moser, C., Ruggli, N., and Hofmann, M.A. 1998. Classical swine fever virus leader proteinase Npro is not required for viral replication in cell culture. J. Virol. 72, 7681-7684. van Rijn, P.A., van Gennip, H.G., de Meijer, EJ., Moormann, R.J. 1993. Epitope mapping of envelope glycoprotein E1 of hog cholera virus strain Brescia. J. Gen. Virol. 74: 2053-2060.

Weiland, E., Thiel, H.-J., Hess, G., and Weiland, F. (1989). Development of monoclonal neutralizing antibodies agaist bovine viral diarrhea virus after pretreatment of mice with normal bovine cells and cyclophosphamide. J. Virol. Methods 24: 237-244.

Weiland, E., Stark, R., Haas, B., Rümenapf, T., Meyers, G. and Thiel, H.-J. (1990). Pestivirus glycoprotein which induces neutralizing antibodies forms part of a disulfide-linked heterodimer. J. Virology 64, 3563-3569.

Weiland, E., Ahl, R., Stark, R., Weiland, F. and Thiel, H.-J. (1992). A second envelope glycoprotein mediates neutralization of a pestivirus, hog cholera virus. J. Virology 66, 3677-3682.

Windisch, J.M., Schneider, R., Stark, R., Weiland, E., Meyers, G., and Thiel, H.-J. 1996. RNase of classical swine fever virus: biochemical characterization and inhibition by virus-neutralizing monoclonal antibodies. J. Virol. 70: 352-358

Wiskerchen, M., Belzer, S.K., and Collett, M.S. 1991. Pestivirus gene expression: the first protein product of the bovine viral diarrhea virus large open reading frame, p20, possesses proteolytic activity J. Virol. 65:4508-4514.

## Claims

1. An attenuated pestivirus, having at least one mutation in the coding sequence for glycoprotein $E^{ms}$ and at least another mutation in the coding sequence for $N^{pro}$, wherein said mutation in the coding sequence for glycoprotein $E^{ms}$ leads to inactivation of RNase activity residing in $E^{ms}$ and said mutation in the coding sequence for $N^{pro}$ leads to inactivation of said $N^{pro}$,

2. The vitus according to claim 1, wherein said mutations are selected from the group of deletions, insertion mutations and/or substitution mutations.

3. The virus according to any one of claims 1 to 2, wherein said mutation(s) are deletions.

4. The virus according to any one of claims 1 to 3, wherein said pestivirus is a Bovine viral diarrhea virus (BVDV).

5. The virus according to claim 4, wherein said mutation(s) in the coding sequence for glycoprotein $E^{ms}$ are located in the encoding nucleotide sequence corresponding to amino acids at position 298 to 310 and/or position 341 to 360.

6. The virus according to claim 4 or 5, wherein said mutation in the coding sequence for glycoprotein $E^{ms}$ is a deletion or substitution of the histidine at position 349,

7. The virus according to anyone of claims 1 to 6, wherein said mutation(s) in the coding sequence for glycoprotein $E^{ms}$ are located in the nucleotide sequence coding for the conserved $E^{ms}$ sequence SLHGIWPEKICTG and/or LQRHEWNKHGWCNWFHIEPW.

8. The virus according to any one of claims 1 to 7, wherein said mutation(s) in the coding sequence for glycoprotein $E^{ms}$ located in the nucleotide sequence coding for the conserved $E^{ms}$ sequence SLHGIWPEKIC and/or RHEWNKHGWCNW.

9. The virus according to anyone of claims 1 to 8, wherein said mutation(s) in the coding sequence for glycoprotein $E^{ms}$ are two mutations located in the nucleotide sequence coding for the conserved $E^{ms}$ sequence SLHGIWPEKIC and/or RHEWNKHGWCNW.

10. The virus according to anyone of claims 1 to 9, wherein said mutation in the coding sequence for glycoprotein $E^{ms}$ is a single mutation located in the conserved $E^{ms}$ sequence SLHGIWPEKIC or KHEWNKHGWCNW.

11. The virus according to any one of claims 1 to 10, wherein said mutation(s) in the coding sequence for $N^{Pro}$ lead to an encoded polyprotein as **characterized by** the following formula:

$$[N^{pro}]_x\text{-}[PS]_y\text{-}[C\text{-term}]$$

and wherein:

[$N^{pro}$] relates to the $N^{pro}$ portion of said polyprotein, wherein "x" represents the number of amino acids of the $N^{pro}$ present in the polyprotein; and wherein
[PS] relates to a processing signal selected from the group consisting of: ubiquitin, LC3, SUMO-1, NEDD8, GATE-16 or GABA(A)RAP), Intein, picornavirus 3C, caridovirus 2A, or p15 of rabbit hemorrhagic disease virus; and wherein
"Y" may be = 0, which means that no processing signal is present, or "Y" may be = 1, which means that a processing signal is present; and wherein
[C-term] relates to the complete virus polyprotein except for $N^{pro}$, but including the capsid (C)-protein and any other protein present in the virus polyprotein including the carboxyterminal NS5B; and wherein
if "y" is = 0, then "x" is 0 to 12, (means no $N^{pro}$ specific amino acid or 1 to 12 amino acids of $N^{pro}$ are present); and wherein
if "y" is = 1, then "x" is 0 to 168; (means no $N^{pro}$ specific amino acid or 1 to all 168 amino acids of $N^{pro}$ are present).

12. The virus according to claim 11, wherein said mutation(s) in the coding sequence for $N^{pro}$ lead to an encoded polyprotein as **characterized by** the following formula:

$$[N^{pro}]_1\text{-}[PS]_0\text{-}[C\text{-term}]$$

13. The virus according to claim 11, wherein said mutation(s) in the coding sequence for $N^{pro}$ lead to an encoded polyprotein as **characterized by** the following formula:

$$[N^{pro}]_3\text{-}[PS]_0\text{-}[C\text{-term}]$$

and wherein the definitions are as defined in claim 11.

14. The virus according to claim 11, wherein said mutation(s) in the coding sequence for $N^{pro}$ lead to an encoded polyprotein as **characterized by** the following formula:

$$[N^{pro}]_4\text{-}[PS]_0\text{-}[C\text{-term}]$$

15. The virus according to claim 11, wherein said mutation(s) in the coding sequence for $N^{pro}$ lead to an encoded polyprotein as **characterized by** the following formula:

$$[N^{pro}]_6\text{-}[PS]_0\text{-}[C\text{-term}]$$

**16.** The virus according to to claim 11, wherein said mutation(s) in the coding sequence for $N^{pro}$ lead to an encoded polyprotein as **characterized by** the following formula:

$$[N^{pro}]_4\text{-}[PS]_0\text{-}[C\text{-term*}],$$

and wherein [C-term]* is = [C-term] wherein in the C-protein the amino acid at position 2 is changed from D to N.

**17.** The virus according to claim 11, wherein said mutation(s) in the coding sequence for $N^{pro}$ lead to an encoded polyprotein as **characterized by** the following formula:

$$[N^{pro}]_x\text{-}[PS]_1\text{-}[C\text{-term}]$$

and wherein PS is selected from the group of ubiquitin or LC3.

**18.** The virus according to claim 11, wherein said said virus is BVDV, and wherein mutation(s) in the coding sequence for $N^{pro}$ lead to an encoded polyprotein as **characterized by** a formula selected from the group consisting of:

$$M\text{-}[PS]_0\text{-}[C\text{-term}];$$

$$MEL\text{-}[PS]_0\text{-}[C\text{-term}];$$

$$MELF\text{-}[PS]_0\text{-}[C\text{-term}];$$

$$MELFS\text{-}[PS]_0\text{-}[C\text{-term}];$$

$$MELFSN\text{-}[PS]_0\text{-}[C\text{-term}];$$

$$MELFSNE\text{-}[PS]_0\text{-}[C\text{-term}];$$

$$MELFSNEL\text{-}[PS]_0\text{-}[C\text{-term}];$$

$$MELFSNELL\text{-}[PS]_0\text{-}[C\text{-term}];$$

MELFSNELLY-[PS]$_0$-[C-term];

MELFSNELLYK-[PS]$_0$-[C-term];

MELFSNELLYKT-[PS]$_0$-[C-term]

**19.** The virus according to claim 11, wherein said said virus is BVDV, and wherein said mutation(s) in the coding sequence for N$^{pro}$ lead to an encoded polyprotein as **characterized by** a formula selected from the group consisting of :

MELI-[PS]$_0$-[C-term];

MELIS-[PS]$_0$-[C-term];

MELISN-[PS]$_0$-[C-term];

MELISNE-[PS]$_0$-[C-term];

MELISNEL-[PS]$_0$-[C-term];

MELISNELL-[PS]$_0$-[C-term];

MELISNELLY-[PS]$_0$-[C-term];

MELISNELLYK-[PS]$_0$-[C-term];

MELISNELLYKT-[PS]$_0$-[C-term];

**20.** The virus according to claim 11, wherein said said virus is BVDV, and wherein said mutation(s) in the coding sequence for N$^{pro}$ lead to an encoded polyprotein as **characterized by** a formula selected from the group consisting of :

MELIT-[PS]$_0$-[C-term];

MELITN-[PS]$_0$-[C-term];

$$MELITNE-[PS]_0-[C\text{-}term];$$

$$MELITNEL-[PS]_0-[C\text{-}term];$$

$$MELITNELL-[PS]_0-[C\text{-}term];$$

$$MELITNELLY-[PS]_0-[C\text{-}term];$$

$$MELITNELLYK-[PS]_0-[C\text{-}term];$$

$$MELITNELLYKT-[PS]_0-[C\text{-}term];$$

**21.** The virus according to claim 11, wherein said said virus is BVDV, and wherein said mutation(s) in the coding sequence for $N^{pro}$ lead to an encoded polyprotein as **characterized by** the formula:

$$[N^{pro}]_x-[PS]_0-MELF-[PS]_0-[C\text{-}term^*];$$

and wherein [C-term]* is = [C-term] wherein in the C-protein the amino acid at position 2 is changed from D to N.

**22.** The virus according to claim 11, wherein said mutation(s) in the coding sequence for $N^{pro}$ lead to an encoded polyprotein as **characterized by** the formula:

$$[\,N^{pro}]_{22}-[PS]_1-[C\text{-}term],$$

and wherein PS is ubiquitin or LC3.

**23.** The virus according to any one of claims 18 to 21, wherein the $[PS]_0$ is replaced by $[PS]_1$, and wherein said PS is selected from the group of consisting of: ubiquitin, LC3, SUMO-1, NEDD8, GATE-16, GABA(A)RAP, Intein, picornavirus 3C, caridovirus 2A, and p15 of rabbit hemorrhagic disease virus.

**24.** The BVDV according to any one of claims 4 to 23, wherein said BVDV is selected from the group of BVDV type 1 or BVDV type 2.

**25.** The BVDV according to any one of claims 4 to 24, wherein the BVDV having the sequence of SEQ ID No. 8.

**26.** A composition comprising the virus according to any one of claims 1 to 25 and a solution.

**27.** The composition according to claim 26, which induces an immunological response in an animal.

**28.** The composition according to claim 26 or 27, which is a vaccine.

**29.** The composition according to any one of claims 26 to 28, wherein said composition further comprises a pharmaceutically acceptable carrier or excipient.

**30.** Use of a virus according to any one of claims 1 to 25 in the manufacture of a vaccine for the prophylaxis and treatment of a pestivirus infection.

**31.** Use of a BVDV according to any one of claims 4 to 25 in the manufacture of a vaccine for the prophylaxis and treatment of a BVDV infection.

**32.** A nucleic acid molecule comprising the nucleic acid encoding a live attenuated BVDV according to any one of claims 4 to 25.

**33.** The nucleic acid molecule according to claim 32, wherein said nucleic acid molecule is DNA.

**34.** The nucleic acid molecule according to claim 32, wherein said nucleic acid molecule is RNA.

**35.** A method for attenuating a pestivirus, **characterized in that** at least one mutation in the coding sequence for glycoprotein $E^{ms}$ and at least another mutation in the coding sequence for $N^{pro}$ is generated in a pestivirus.

**36.** The method according to claim 35, comprising the following steps:

a) reverse transcription of a wild-type pestivirus nucleotide sequence into a cDNA;
b) cloning said cDNA;
c) introducing mutations selected from the group of deletions, insertion mutations and/or substitution mutations into said cDNA, wherein said mutations are located in the coding sequence encoding glycoprotein $E^{ms}$ and the protease $N^{pro}$,
d) incorporating the cDNA into a plasmid or into a DNA virus capable of directing the transcription of pestivirus cDNA into RNA in vitro or upon infection of suitable cells.

**37.** The method according to claim 35 or 36, wherein said pestivirus is BVDV.

**38.** Use of BVDV according to any one of claims 3 to 25 or a composition according to claims 26 to 29 for the manufacture of a vaccine for the reduction of symptoms of BVDV infection such as viremia and leukopenia and/or pyrexia and/or diarrhea.

**Patentansprüche**

**1.** Attenuiertes Pestivirus mit mindestens einer Mutation in der Kodierungssequenz für Glycoprotein $E^{rns}$ und mindestens einer weiteren Mutation in der Kodierungssequenz für $N^{pro}$, wobei die Mutation in der Kodierungssequenz für Glycoprotein $E^{rns}$ zur Inaktivierung von Rnase-Aktivität, die in $E^{rns}$ gegeben ist, führt und die Mutation in der Kodierungssequenz für $N^{pro}$ zur Inaktivierung des $N^{pro}$ führt.

**2.** Virus nach Anspruch 1, wobei die Mutationen aus der Gruppe Deletionen, Insertionsmutationen und/oder Substitutionsmutationen ausgewählt sind.

**3.** Virus nach einem der Ansprüche 1 bis 2, wobei es sich bei der oder den Mutationen um Deletionen handelt.

**4.** Virus nach einem der Ansprüche 1 bis 3, wobei es sich beim Pestivirus um ein bovines Virusdiarrhoe-Virus (BVDV) handelt.

**5.** Virus nach Anspruch 4, wobei die Mutation(en) in der Kodierungssequenz für Glycoprotein $E^{rns}$ sich in der kodierenden Nucleotidsequenz entsprechend den Aminosäuren in den Positionen 298 bis 310 und/oder den Positionen 341 bis 360 befinden.

**6.** Virus nach Anspruch 4 oder 5, wobei es sich bei der Mutation in der Kodierungssequenz für Glycoprotein $E^{rns}$ um eine Deletion oder eine Substitution des Histidins in Position 349 handelt.

**7.** Virus nach einem der Ansprüche 1 bis 6, wobei die Mutation(en) in der Kodierungssequenz für Glycoprotein $E^{rns}$ sich in der Nucleotidsequenz befinden, die für die konservierte $E^{rns}$-Sequenz SLHGIWPEKICTG und/oder LQRHEWNKHGWCNWFHIEPW kodiert, befinden.

**8.** Virus nach einem der Ansprüche 1 bis 7, wobei die Mutation(en) in der Kodierungssequenz für Glycoprotein $E^{rns}$ sich in der Nucleotidsequenz, die für die konservierte $E^{rns}$-Sequenz SLHGIWPEKIC und/oder RHEWNKHGWCNW kodiert, befinden.

**9.** Virus nach einem der Ansprüche 1 bis 8, wobei es sich bei der oder den Mutationen in der Kodierungssequenz für Glycoprotein $E^{rns}$ um zwei Mutationen handelt, die sich in der Nucleotidsequenz befinden, die für die konservierte $E^{rns}$-Sequenz SLHGIWPEKIC und/oder RHEWNKHGWCNW kodiert.

**10.** Virus nach einem der Ansprüche 1 bis 9, wobei es sich bei der Mutation in der Kodierungssequenz für Glycoprotein $E^{rns}$ um eine Einzelmutation handelt, die sich in der konservierten $E^{rns}$-Sequenz SLHGIWPEKIC oder RHEWNKHGWCNW befindet.

**11.** Virus nach einem der Ansprüche 1 bis 10, wobei die Mutation(en) in der Kodierungssequenz für $N^{pro}$ zu einem kodierten Polyprotein führen, das durch die folgende Formel **gekennzeichnet** ist

$$[N^{pro}]_x\text{-}[PS]_y\text{-}[C\text{-term}]$$

wobei:

[$N^{pro}$] sich auf den $N^{pro}$-Bereich des Polyproteins bezieht, wobei "x" die Anzahl von Aminosäuren des im Polyprotein vorhandenen $N^{pro}$ darstellt; und wobei
[PS] sich auf ein Prozessierungssignal bezieht, das aus der folgenden Gruppe ausgewählt ist: Ubiquitin, LC3, SUMO-1, NEDD8, GATE-16 oder GABA(A)(RAP), Intein, Picornavirus 3C, Caridovirus 2A oder p15 von Kaninchen-Hämorrhagiekrankheit-Virus; und wobei
"y" den Wert 0 haben kann, was bedeutet, dass keine Prozessierungssignal vorhanden ist, oder "y" den Wert 1 haben kann, was bedeutet, dass ein Prozessierungssignal vorhanden ist; und wobei
[C-term] sich auf das vollständige Virus-Polyprotein mit der Ausnahme von $N^{pro}$, jedoch unter Einschluss des Capsid (C)-Proteins und auch beliebiger anderer Proteine, die im Virus-Polyprotein vorhanden sind, einschließlich des carboxyterminalen NS5B, bezieht; und wobei
wenn "y" den Wert 0 hat, "x" einen Wert von 0 bis 12 hat (was bedeutet, dass keine $N^{pro}$-spezifische Aminosäure oder 1 bis 12 Aminosäuren von $N^{pro}$ vorhanden sind); und wobei,
wenn "y" den Wert 1 hat, "x" einen Wert von 0 bis 168 hat; (was bedeutet, dass keine $N^{pro}$-spezifische Aminosäure oder 1 bis sämtliche 168 Aminosäuren von $N^{pro}$ vorhanden sind).

**12.** Virus nach Anspruch 11, wobei die Mutation(en) in der Kodierungssequenz für $N^{pro}$ zu einem kodierten Polyprotein führen, das durch die folgende Formel **gekennzeichnet** ist:

$$[N^{pro}]_1\text{-}[PS]_0\text{-}[C\text{-term}]$$

**13.** Virus nach Anspruch 11, wobei die Mutation(en) in der Kodierungssequenz für $N^{pro}$ zu einem kodierten Polyprotein führen, das durch die folgende Formel **gekennzeichnet** ist:

$$[N^{pro}]_3\text{-}[PS]_0\text{-}[C\text{-term}]$$

und wobei die Definitionen denen von Anspruch 11 entsprechen.

**14.** Virus nach Anspruch 11, wobei die Mutation(en) in der Kodierungssequenz für $N^{pro}$ zu einem kodierten Polyprotein führen, das durch die folgende Formel **gekennzeichnet** ist:

$$[N^{pro}]_4\text{-}[PS]_0\text{-}[C\text{-term}]$$

**15.** Virus nach Anspruch 11, wobei die Mutation(en) in der Kodierungssequenz für $N^{pro}$ zu einem kodierten Polyprotein führen, das durch die folgende Formel **gekennzeichnet** ist:

$[N^{pro}]_6$-$[PS]_0$-[C-term]

**16.** Virus nach Anspruch 11, wobei die Mutation(en) in der Kodierungssequenz für $N^{pro}$ zu einem kodierten Polyprotein führen, das durch die folgende Formel **gekennzeichnet** ist:

$[N^{pro}]_4$-$[PS]_0$-[C-term*]

und wobei [C-term*] die Bedeutung [C-term] hat, wobei im C-Protein die Aminosäure in Position 2 von D in N geändert ist.

**17.** Virus nach Anspruch 11, wobei die Mutation(en) in der Kodierungssequenz für $N^{pro}$ zu einem kodierten Polyprotein führen, das durch die folgende Formel **gekennzeichnet** ist:

$[N^{pro}]_x$-$[PS]_1$-[C-term]

und wobei PS aus der Gruppe Ubiquitin oder LC3 ausgewählt ist.

**18.** Virus nach Anspruch 11, wobei es sich bei dem Virus um BVDV handelt und wobei die Mutation(en) in der Kodierungssequenz für $N^{pro}$ zu einem kodierten Polyprotein führen, das durch eine Formel **gekennzeichnet** ist, die aus folgender Gruppe ausgewählt ist:

M-$[PS]_0$-[C-term]

MEL-$[PS]_0$-[C-term]

MELF-$[PS]_0$-[C-term]

MELFS-$[PS]_0$-[C-term]

MELFSN-$[PS]_0$-[C-term]

MELFSNE-$[PS]_0$-[C-term]

MELFSNEL-$[PS]_0$-[C-term]

MELFSNELL-$[PS]_0$-[C-term]

MELFSNELLY-$[PS]_0$-[C-term]

MELFSNELLYK-$[PS]_0$-[C-term]

MELFSNELLYKT-$[PS]_0$-[C-term]

**19.** Virus nach Anspruch 11, wobei es sich bei dem Virus um BVDV handelt und wobei die Mutation(en) in der Kodierungssequenz für $N^{pro}$ zu einem kodierten Polyprotein führen, das durch eine Formel **gekennzeichnet** ist, die aus folgender Gruppe ausgewählt ist:

MELI-$[PS]_0$-[C-term]

MELIS-$[PS]_0$-[C-term]

MELISN-$[PS]_0$-[C-term]

MELISNE-$[PS]_0$-[C-term]

MELISNEL-$[PS]_0$-[C-term]

MELISNELL-$[PS]_0$-[C-term]

MELISNELLY-$[PS]_0$-[C-term]

MELISNELLYK-$[PS]_0$-[C-term]

MELISNELLYKT-$[PS]_0$-[C-term]

**20.** Virus nach Anspruch 11, wobei es sich bei dem Virus um BVDV handelt und wobei die Mutation(en) in der Kodierungssequenz für $N^{pro}$ zu einem kodierten Polyprotein führen, das durch eine Formel **gekennzeichnet** ist, die aus folgender Gruppe ausgewählt ist:

MELIT-$[PS]_0$-[C-term]

MELITN-$[PS]_0$-[C-term]

MELITNE-$[PS]_0$-[C-term]

MELITNEL-$[PS]_0$-[C-term]

MELITNELL-$[PS]_0$-[C-term]

MELITNELLY-$[PS]_0$-[C-term]

MELITNELLYK-$[PS]_0$-[C-term]

MELITNELLYKT-$[PS]_0$-[C-term]

**21.** Virus nach Anspruch 11, wobei es sich bei dem Virus um BVDV handelt und wobei die Mutation(en) in der Kodierungssequenz für $N^{pro}$ zu einem kodierten Polyprotein führen, das durch die Formel **gekennzeichnet** ist:

$[N^{pro}]_x$-$[PS]_0$-MELF-$[PS]_0$-[C-term*]

und wobei [C-term*] die Bedeutung [C-term] hat, wobei im C-Protein die Aminosäure in Position 2 von D in N geändert ist.

**22.** Virus nach Anspruch 11, wobei die Mutation(en) in der Kodierungssequenz für $N^{pro}$ zu einem kodierten Polyprotein führen, das durch die Formel **gekennzeichnet** ist:

$[N^{pro}]_{22}$-$[PS]_1$-[C-term]

und wobei PS Ubiquitin oder LC3 bedeutet.

**23.** Virus nach einem der Ansprüche 18 bis 21, wobei das $[PS]_0$ durch $[PS]_1$ ersetzt ist und wobei das PS aus der Gruppe ausgewählt ist, die besteht aus: Ubiquitin, LC3, SUMO-1, NEDD8, GATE-16 oder GABA(A)(RAP), Intein, Picornavirus 3C, Caridovirus 2A oder p15 von Kaninchen-Hämorrhagiekrankheit-Virus.

**24.** BVDV nach einem der Ansprüche 4 bis 23, wobei das BVDV aus der Gruppe BVDV-Typ 1 oder BVDV-Typ 2 ausgewählt ist.

**25.** BVDBV nach einem der Ansprüche 4 bis 24, wobei das BVDV die Sequenz von SEQ ID NO:8 aufweist.

**26.** Zusammensetzung, umfassend das Virus nach einem der Ansprüche 1 bis 25 und eine Lösung.

**27.** Zusammensetzung nach Anspruch 26, die eine immunologische Reaktion bei einem Tier induziert.

**28.** Zusammensetzung nach Anspruch 26 oder 27, bei der es sich um einen Impfstoff handelt.

**29.** Zusammensetzung nach einem der Ansprüche 26 bis 28, wobei die Zusammensetzung ferner einen pharmazeutisch verträglichen Träger oder Exzipiens umfasst.

**30.** Verwendung eines Virus nach einem der Ansprüche 1 bis 25 bei der Herstellung eines Impfstoffes für die Prophylaxe und Therapie einer Pestivirus-Infektion.

**31.** Verwendung eines BVDV nach einem der Ansprüche 4 bis 25 bei der Herstellung eines Impfstoffes für die Prophylaxe und Therapie einer BVDV-Infektion.

**32.** Nucleinsäuremolekül, umfassend die Nucleinsäure, die für ein lebendes, attenuiertes BVDV nach einem der Ansprüche 4 bis 25 kodiert.

**33.** Nucleinsäuremolekül nach Anspruch 32, wobei es sich bei dem Nucleinsäuremolekül um DNA handelt.

**34.** Nucleinsäuremolekül nach Anspruch 32, wobei es sich bei dem Nucleinsäuremolekül um RNA handelt.

**35.** Verfahren zum Attenuieren eines Pestivirus, **dadurch gekennzeichnet, dass** mindestens eine Mutation in der Kodierungssequenz für Glycoprotein $E^{rns}$ und mindestens eine weitere Mutation in der Kodierungssequenz für $N^{pro}$ in einem Pestivirus erzeugt wird.

**36.** Verfahren nach Anspruch 35, umfassend die folgenden Stufen:

a) die reverse Transkription einer Wildtyp-Pestivirus-Nucleotidsequenz in eine cDNA;
b) das Klonieren dieser cDNA;
c) das Einführen von Mutationen, die aus der Gruppe Deletionen, Insertionsmutationen und/oder Substitutionsmutationen ausgewählt sind, in die cDNA, wobei sich diese Mutationen in der Kodierungssequenz, die für Glycoprotein $E^{rns}$ und die Protease $N^{pro}$ kodieren, befindet, und
d) das Einbauen der cDNA in ein Plasmid oder in ein DNA-Virus, die dazu befähigt sind, in vitro oder bei Infektion von geeigneten Zellen die Transkription von Pestivirus-cDNA in RNA zu dirigieren.

**37.** Verfahren nach Anspruch 35 oder 36, wobei es sich beim Pestivirus um BVDV handelt.

**38.** Verwendung von BVDV nach einem der Ansprüche 3 bis 25 oder einer Zusammensetzung nach den Ansprüchen 26 bis 29 zur Herstellung eines Impfstoffes zur Verringerung der Symptome einer BVDV-Infektion, wie Virämie und Leukopenie und/oder Pyrexie und/oder Diarrhoe.

## Revendications

**1.** Pestivirus atténué ayant au moins une mutation dans la séquence codante pour la glycoprotéine $E^{ms}$ et au moins une autre mutation dans la séquence codante pour $N^{pro}$, où ladite mutation dans la séquence codante pour la glycoprotéine $E^{ms}$ conduit à l'inactivation de l'activité RNase résidant dans $E^{ms}$ et ladite mutation dans la séquence codante pour $N^{pro}$ conduit à l'inactivation de ladite $N^{pro}$.

**2.** Virus selon la revendication 1 où lesdites mutations sont choisies dans le groupe des délétions, mutations par insertion et/ou mutations par substitution.

**3.** Virus selon l'une quelconque des revendications 1 à 2 où ladite ou lesdites mutations sont des délétions.

**4.** Virus selon l'une quelconque des revendications 1 à 3 où ledit pestivirus est un virus de diarrhée virale bovine (BVDV).

**5.** Virus selon la revendication 4 où ladite ou lesdites mutations dans la séquence codante pour la glycoprotéine $E^{ms}$ est ou sont situées dans la séquence nucléotidique codante correspondant aux aminoacides aux positions 298 à 310 et/ou aux positions 341 à 360.

**6.** Virus selon la revendication 4 ou 5 où ladite mutation dans la séquence codante pour la glycoprotéine $E^{ms}$ est une délétion ou substitution de l'histidine à la position 349.

**7.** Virus selon l'une quelconque des revendications 1 à 6 où ladite ou lesdites mutations dans la séquence codante pour la glycoprotéine $E^{ms}$ est ou sont situées dans la séquence nucléotidique codant la séquence de $E^{ms}$ conservée SLHGIWPEKICTG et/ou LQRHEWNKHGWCNWFHIEPW.

**8.** Virus selon l'une quelconque des revendications 1 à 6 où ladite ou lesdites mutations dans la séquence codante pour la glycoprotéine $E^{ms}$ est ou sont situées dans la séquence nucléotidique codant la séquence de $E^{ms}$ conservée SLHGIWPEKIC et/ou RHEWNKHGWCNW.

**9.** Virus selon l'une quelconque des revendications 1 à 8 où ladite ou lesdites mutations dans la séquence codante pour la glycoprotéine $E^{ms}$ sont deux mutations situées dans la séquence nucléotidique codant la séquence de $E^{ms}$ conservée SLHGIWPEKIC et/ou RHEWNKHGWCNW.

**10.** Virus selon l'une quelconque des revendications 1 à 9 où ladite mutation dans la séquence codante pour la glycoprotéine $E^{ms}$ est une mutation unique située dans la séquence de $E^{ms}$ conservée SLHGIWPEKIC ou RHEWNKHGWCNW.

**11.** Virus selon l'une quelconque des revendications 1 à 10 où ladite ou lesdites mutations dans la séquence codante pour $N^{pro}$ conduit ou conduisent à une polyprotéine codée **caractérisée par** la formule suivante :

$$[N^{pro}]_x\text{-}[PS]_y\text{-}[C\text{-}term]$$

et où :

[$N^{pro}$] concerne la portion $N^{pro}$ de ladite polyprotéine, où « x » représente le nombre d'aminoacides de la $N^{pro}$ présente dans la polyprotéine ; et où
[PS] concerne un signal de maturation choisi dans le groupe consistant en : ubiquitine, LC3, SUMO-1, NEDD8, GATE-16 ou GABA(A)RAP), intéine, 3C du picornavirus, 2A du caridovirus ou p15 du virus de la maladie hémorragique du lapin ; et où
« Y » peut être = 0, ce qui signifie qu'aucun signal de maturation n'est présent, ou « Y » peut être = 1, ce qui signifie qu'un signal de maturation est présent ; et où
[C-term] concerne la polyprotéine virale complète, excepté $N^{pro}$, mais incluant la protéine (C) de capside et toute autre protéine présente dans la polyprotéine virale incluant la NS5B carboxyterminale ; et où
si « y » est = 0, « x » est 0 à 12, (ce qui signifie pas d'aminoacide spécifique de $N^{pro}$ ou 1 à 12 aminoacides de $N^{pro}$ sont présents) ; et où
si « y » est = 1, « x » est 0 à 168, (ce qui signifie pas d'aminoacide spécifique de $N^{pro}$ ou 1 à 168 aminoacides de $N^{pro}$ sont présents).

**12.** Virus selon la revendication 11 où ladite ou lesdites mutations dans la séquence codante pour $N^{pro}$ conduit ou conduisent à une polyprotéine codée **caractérisée par** la formule suivante :

$$[N^{pro}]_1\text{-}[PS]_0\text{-}[C\text{-term}]$$

**13.** Virus selon la revendication 11 où ladite ou lesdites mutations dans la séquence codante pour $N^{pro}$ conduit ou conduisent à une polyprotéine codée **caractérisée par** la formule suivante :

$$[N^{pro}]_3\text{-}[PS]_0\text{-}[C\text{-term}]$$

et où les définitions sont définies comme dans la revendication 11.

**14.** Virus selon la revendication 11 où ladite ou lesdites mutations dans la séquence codante pour $N^{pro}$ conduit ou conduisent à une polyprotéine codée **caractérisée par** la formule suivante :

$$[N^{pro}]_4\text{-}[PS]_0\text{-}[C\text{-term}]$$

**15.** Virus selon la revendication 11 où ladite ou lesdites mutations dans la séquence codante pour $N^{pro}$ conduit ou conduisent à une polyprotéine codée **caractérisée par** la formule suivante :

$$[N^{pro}]_6\text{-}[PS]_0\text{-}[C\text{-term}]$$

**16.** Virus selon la revendication 11 où ladite ou lesdites mutations dans la séquence codante pour $N^{pro}$ conduit ou conduisent à une polyprotéine codée **caractérisée par** la formule suivante :

$$[N^{pro}]_4\text{-}[PS]_0\text{-}[C\text{-term*}]$$

et où [C-term*] est = [C-term] où dans la protéine C l'aminoacide à la position 2 est changé de D à N.

**17.** Virus selon la revendication 11 où ladite ou lesdites mutations dans la séquence codante pour $N^{pro}$ conduit ou conduisent à une polyprotéine codée **caractérisée par** la formule suivante ;

$[N^{pro}]_x$-$[PS]_1$-[C-term]

et où PS est choisi dans le groupe de l'ubiquitine ou LC3.

**18.** Virus selon la revendication 11 où ledit virus est BVDV et où ladite ou lesdites mutations dans la séquence codante pour $N^{pro}$ conduit ou conduisent à une polyprotéine codée **caractérisée par** une formule choisie dans le groupe consistant en :

M-$[PS]_0$-[C-term] ;

MEL-$[PS]_0$-[C-term] ;

MELF-$[PS]_0$-[C-term] ;

MELFS-$[PS]_0$-[C-term] ;

MELFSN-$[PS]_0$-[C-term] ;

MELFSNE-$[PS]_0$-[C-term] ;

MELFSNEL-$[PS]_0$-[C-term] ;

MELFSNELL-$[PS]_0$-[C-term] ;

MELFSNELLY-$[PS]_0$-[C-term] ;

MELFSNELLYK-$[PS]_0$-[C-term] ;

MELFSNELLYKT-$[PS]_0$-[C-term].

**19.** Virus selon la revendication 11 où ledit virus est BVDV et où ladite ou lesdites mutations dans la séquence codante pour $N^{pro}$ conduit ou conduisent à une polyprotéine codée **caractérisée par** une formule choisie dans le groupe consistant en :

MELI-$[PS]_0$-[C-term] ;

MELIS-$[PS]_0$-[C-term] ;

MELISN-$[PS]_0$-[C-term] ;

MELISNE-$[PS]_0$-[C-term] ;

MELISNEL-$[PS]_0$-[C-term] ;

MELISNELL-$[PS]_0$-[C-term] ;

MELISNELLY-$[PS]_0$-[C-term] ;

MELISNELLYK-$[PS]_0$-[C-term] ;

MELISNELLYKT-$[PS]_0$-[C-term].

**20.** Virus selon la revendication 11 où ledit virus est BVDV et où ladite ou lesdites mutations dans la séquence codante pour $N^{pro}$ conduit ou conduisent à une polyprotéine codée **caractérisée par** une formule choisie dans le groupe consistant en :

MELIT-$[PS]_0$-[C-term] ;

MELITN-$[PS]_0$-[C-term] ;

MELITNE-$[PS]_0$-[C-term] ;

MELITNEL-$[PS]_0$-[C-term] ;

MELITNELL-$[PS]_0$-[C-term] ;

MELITNELLY-$[PS]_0$-[C-term] ;

MELITNELLYK-$[PS]_0$-[C-term] ;

MELITNELLYKT-$[PS]_0$-[C-term].

**21.** Virus selon la revendication 11 où ledit virus est BVDV et où ladite ou lesdites mutations dans la séquence codante pour $N^{pro}$ conduit ou conduisent à une polyprotéine codée **caractérisée par** le formule:

$[N^{pro}]_x$-$[PS]_0$-MELF-$[PS]_0$-[C-term*] ;

et où [C-term*] est = [C-term] où dans la protéine C l'aminoacide à la position 2 est changé de D à N.

**22.** Virus selon la revendication 11 où ladite ou lesdites mutations dans la séquence codante pour $N^{pro}$ conduit ou conduisent à une polyprotéine codée **caractérisée par** le formule:

$[N^{pro}]_{22}$-$[PS]_1$-[C-term]

et où PS est l'ubiquitine ou LC3.

**23.** Virus selon l'une quelconque des revendications 18 à 21 où le $[PS]_0$ est remplacé par $[PS]_1$ et où ledit PS est choisi dans le groupe consistant en : ubiquitine, LC3, SUMO-1, NEDD8, GATE-16, GABA(A)RAP, intéine, 3C du picornavirus, 2A du caridovirus et p15 du virus de la maladie hémorragique du lapin.

**24.** BVDV selon l'une quelconque des revendications 4 à 23 où ledit BVDV est choisi dans le groupe de BVDV de type 1 et BVDV de type 2.

**25.** BVDV selon l'une quelconque des revendications 4 à 24 où le BVDV a la séquence de SEQ ID No. 8.

**26.** Composition comprenant le virus selon l'une quelconque des revendications 1 à 25 et une solution.

**27.** Composition selon la revendication 26 qui induit une réponse immunologique chez un animal.

**28.** Composition selon la revendication 26 ou 27 qui est un vaccin.

**29.** Composition selon l'une quelconque des revendications 26 à 28 où ladite composition comprend en outre un vecteur ou excipient pharmaceutiquement acceptable.

**30.** Utilisation d'un virus selon l'une quelconque des revendications 1 à 25 dans la fabrication d'un vaccin pour la prophylaxie et le traitement d'une infection à pestivirus.

**31.** Utilisation d'un BVDV selon l'une quelconque des revendications 4 à 25 dans la fabrication d'un vaccin pour la prophylaxie et le traitement d'une infection à BVDV.

**32.** Molécule d'acide nucléique comprenant l'acide nucléique codant un BVDV vivant atténué selon l'une quelconque des revendications 4 à 25.

**33.** Molécule d'acide nucléique selon la revendication 32 où ladite molécule d'acide nucléique est un ADN.

**34.** Molécule d'acide nucléique selon la revendication 32 où ladite molécule d'acide nucléique est un ARN.

**35.** Procédé pour atténuer un pestivirus **caractérisé en ce qu'**au moins une mutation dans la séquence codante pour la glycoprotéine $E^{ms}$ et au moins une autre mutation dans la séquence codante pour $N^{pro}$ sont produites dans un pestivirus.

**36.** Procédé selon la revendication 35 comprenant les étapes suivantes :

a) transcription inverse d'une séquence nucléotidique de pestivirus de type sauvage en un ADNc ;
b) clonage dudit ADNc ;
c) introduction de mutations choisies dans le groupe des délétions, des mutations par insertion et/ou des mutations par substitution dans ledit ADNc, où lesdites mutations sont situées dans la séquence codante codant la glycoprotéine $E^{ms}$ et la protéase $N^{pro}$,
d) incorporation de l'ADNc dans un plasmide ou dans un virus à ADN capable de diriger la transcription d'ADNc de pestivirus en ARN in vitro ou lors d'une infection de cellules appropriées.

**37.** Procédé selon la revendication 35 ou 36 où ledit pestivirus est BVDV.

**38.** Utilisation de BVDV selon l'une quelconque des revendications 3 à 25 ou d'une composition selon les revendications 26 à 29 pour la fabrication d'un vaccin pour la réduction des symptômes d'une infection à BVDV comme la virémie et la leucopénie et/ou la pyrexie et/ou la diarrhée.

Figur 1:

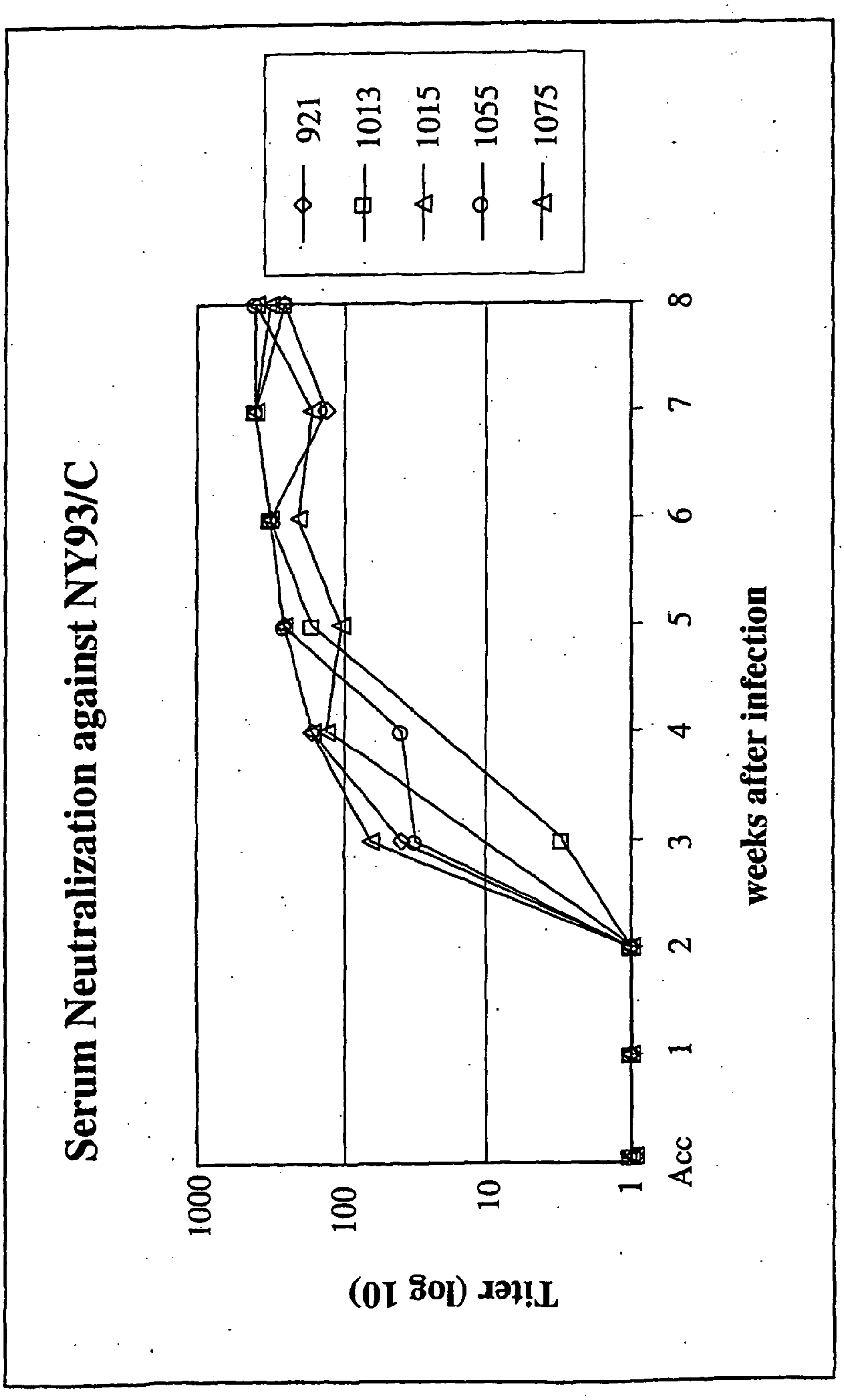
Serum Neutralization against NY93/C
Titer (log 10)
1000
100
10
1
Acc
1
2
3
4
5
6
7
8
weeks after infection
921
1013
1015
1055
1075

Figur 2:

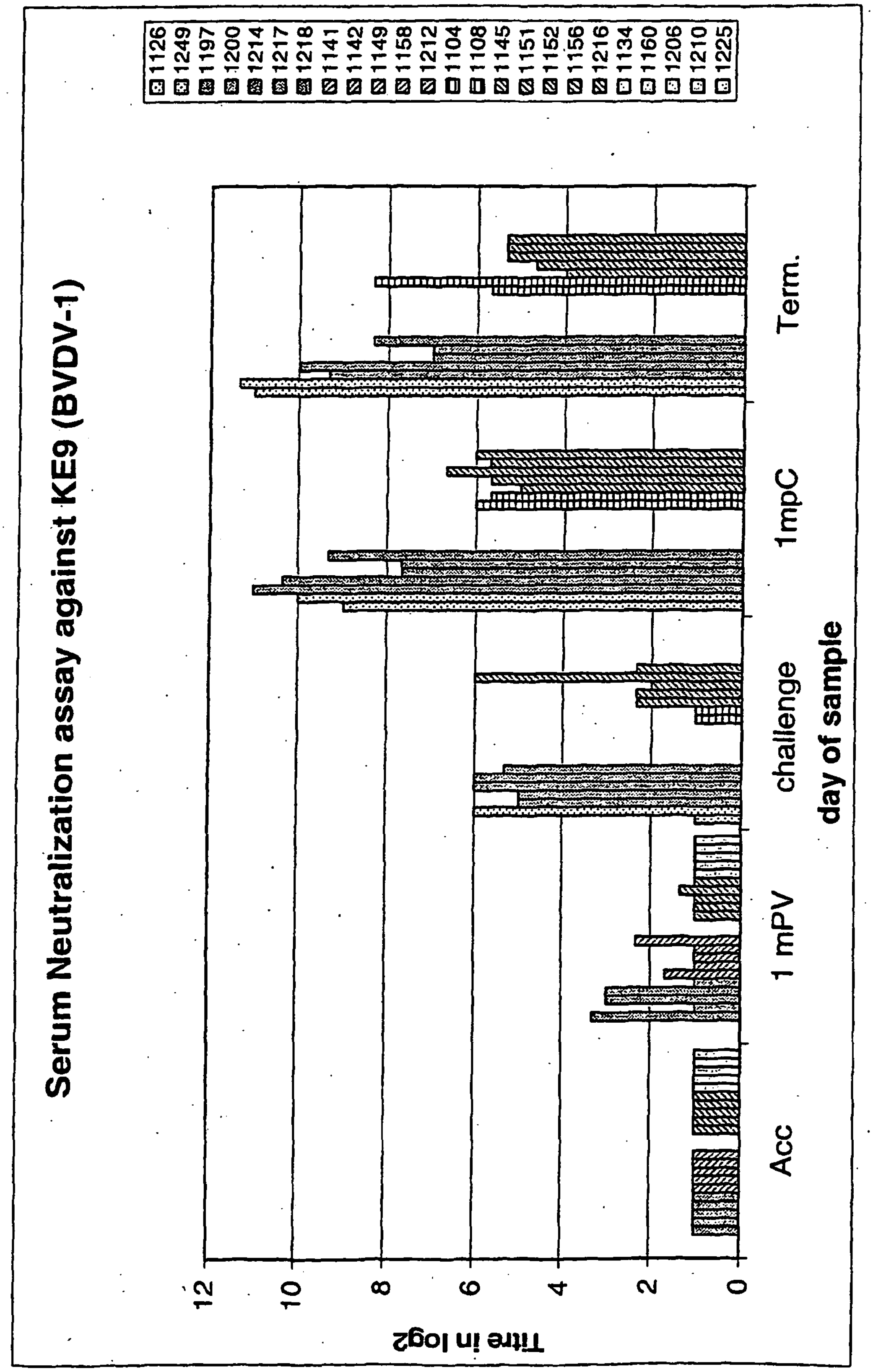
Serum Neutralization assay against KE9 (BVDV-1)
Titre in log2
0
2
4
6
8
10
12
Acc
1 mPV
challenge
1mpC
Term.
day of sample
1126
1249
1197
1200
1214
1217
1218
1141
1142
1149
1158
1212
1104
1108
1145
1151
1152
1156
1216
1134
1160
1206
1210
1225

Figur 3:

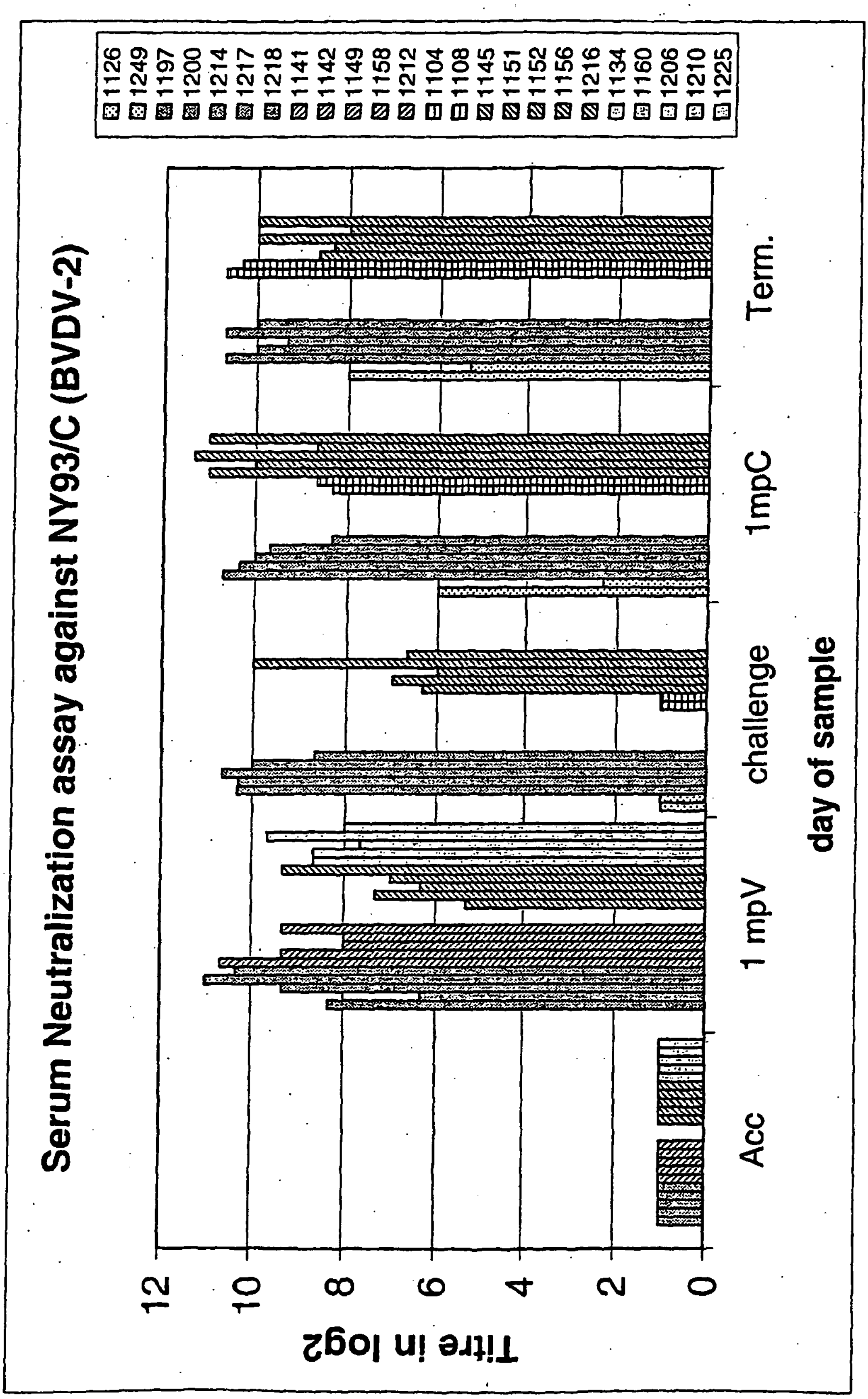
Serum Neutralization assay against NY93/C (BVDV-2)
Titre in log2
12
10
8
6
4
2
0
Acc
1 mpV
challenge
1mpC
Term.
day of sample
1126
1249
1197
1200
1214
1217
1218
1141
1142
1149
1158
1212
1104
1108
1145
1151
1152
1156
1216
1134
1160
1206
1210
1225

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9964604 A


### Non-patent literature cited in the description


- **STARK.** *J. Virol.,* 1993, vol. 67, 7088-7093
- **WISKERCHEN et al.** *Virol.,* 1991, vol. 65, 4508-4514
- **BECHER et al.** *Virology,* 2003, vol. 311, 96-104
- **AUSUBEL, F.M. et al.** Current Protocols in molecular biology. Greene Publishing Associates and Wiley-Interscience, 1994
- **BAKER, J.C.** Bovine viral diarrhea virus: a review. *J. Am. Vet. Med.Assoc.,* 1987, vol. 190, 1449-1458
- **BECHER, P. ; KÖNIG, M. ; PATON, D.J. ; THIEL, H.J.** Further characterization of border disease virus isolates: evidence for the presence of more than three species within the genus pesivirus. *Virology,* 1995, vol. 209 (1), 200-206
- **CHONG, S. ; WILLIAMS, K.S. ; WOTKOWICZ, C. ; XU, M.Q.** Modulation of Protein Splicing of the Saccharomyces cerevisiae Vacuolar Membrane ATPase Intein. *J. Biol. Chem.,* 1998, vol. 273, 10567-10577
- **DONIS, R.O. ; CORAPI, W. ; DUBOVI, E.J.** Neutralizing monoclonal antibodies to bovine viral diarrhea virus bind to the 56K to 58K glycoprotein. *J. Gen. Virol.,* 1988, vol. 69, 77-86
- **FUERST T.R. et al.** Eukaryotic transient expression system based on recombinant vaccinia virus that synthesizes bacteriophage T7 RNA polymerase. *Proc. Natl. Acad. Sci.,* 1986, vol. 83, 8122-8126
- Familiy Flaviviridae. **HEINZ, F.X. ; COLLETT, M.S. ; PURCELL., R.H. ; COLD, E.A. ; HOWARD, C.R. ; HOUGHTON, M. ; MOORMANN, R.J.M. ; RICE, C.M. ; THIEL, H.-J.** Virus Taxonomy. Academic. Press, 2000, 859-878
- **HULST, M.M. ; HIMES, G. ; NEWBIGIN, E. ; MOORMANN, R.J.M.** Glycoprotein E2 of classical swine fever virus: expression in insect cells and identification as a ribonuclease. *Virology,* 1994, vol. 200, 558-565
- **HULST, M.M. ; F.E. PANOTO ; A. HOOEKMANN ; H.G.P. VAN GENNIP. ; MOORMANN, R.J.M.** Inactivation of the RNase activity of glycoprotein Ems of classical swine fever virus results in a cytopathogenic virus. *J. Virol.,* 1998, vol. 72, 151-157
- **KIT, M. ; S. KIT.** Sensitive glycoprotein gIII blocking ELISA to distinguish between pseudorabies (Aujeszky's disease) -infected and vaccinated pigs. *Veterinary Microbiology,* 1991, vol. 28, 141-155
- **KUNKEL, T. A. ; J. D. ROBERTS ; R. A. ZAKOUR.** Rapid and efficient site-specific mutagenesis without phenotypic selection. *Methods Enzymol.,* 1987, vol. 154, 367-392
- **KÖNIG ; MATTHIAS.** Virus der klassischen Schweinepest: Untersuchungen zur Pathogenese und zur Induktion einer protektiven Immunantwort. *Dissertation,* 1994
- **LINDENBACH, B.D. ; RICE, C. M.** The pestiviruses. In Fields Virology. 2001, 991-1042
- **MAYER, D. ; HOFMANN, M.A. ; TRATSCHIN, J.D.** Attenuation of classical swine fever virus by deletion of the viral N(pro) gene. *Vaccine,* 2004, vol. 22, 317-328
- **MEYERS, G. ; RÜMENAPF, T. ; THIEL, H.-J.** Molecular cloning and nucleotide sequence of the genome of hog cholera virus. *Virology,* 1989, vol. 171, 555-567
- **MEYERS,G. ; SAALMÜLLER,A. ; BÜTTNER,M.** Mutations abrogating the RNase activity in glycoprotein e(ms) of the pestivirus classical swine fever virus lead to virus attenuation. *J Virol,* 1999, vol. 73, 10224-10235
- **MEYERS, G. ; TAUTZ, N. ; BECHER, P. ; THIEL, H.-J. ; KÜMMERER, B.M.** Recovery of cytopathogenic and noncytopathogenic bovine viral diarrhea viruses from cDNA constructs. *J. Virol.,* 1996, vol. 70, 8606-8613
- **MEYERS, G. ; THIEL, H.-J. ; RÜMENAPF, T.** Classical swine fever virus: Recovery of infectious viruses from cDNA constructs and generation of recombinant cytopathogenic swine fever virus. *J. Virol.,* 1996, vol. 67, 7088-709526
- **MEYERS, G. ; WIRBLICH, C. ; THIEL. H.-J. ; THUMFART, J.O.** Rabbit hemorrhagic disease Virus: genome organization and polyprotein processing of a calicivirus studied after transient expression of cDNA constructs. *Virology,* 2000, vol. 276, 349-363

- **MOENNIG, V. ; PLAGEMANN, J.** *The pestiviruses. Adv. Virus Res.,* 1992, vol. 41, 53-91
- **PATON, D.J. ; LOWINGS, J.P. ; BARRETT, A.D.** Epitope mapping of the gp53 envelope protein of bovine viral diarrhea virus. *Virology,* 1992, vol. 190, 763-772
- **PELLERIN, C.** Identification of a new group of bovine viral diarrhea virus strains associated with severe outbreaks and high mortalities. *Virology,* 1994, vol. 203, 260-268
- **PORTER, A.G.** Picornavirus nonstructural proteins: emerging roles in virus replication and inhibition of host cell functions. *J. Virol.,* 1993, vol. 67, 6917-6921
- **RÜGGLI, N. ; TRATSCHIN, J.D. ; SCHWEIZER, M. ; MCCULLOUGH, K.C. ; HOFMANN, M.A. ; SUMMERFIELD, A.** Classical swine fever virus interferes with cellular antiviral defense: evidence for a novel function of N(pro. *J. Virol.,* 2003, vol. 77, 7645-7654
- **RÜMENAPF, T. ; STARK, R. ; HEIMANN, M. ; THIEL, H.-J.** N-terminal protease of pestiviruses: identification of putative catalytic residues by site directed mutagenesis. *J. Virol.,* 1998, vol. 72, 2544-2547
- **RÜMENAPF, T. ; UNGER, G. ; STRAUSS, J.H. ; THIEL, H.-J.** Processing of the evelope glycoproteins of pestiviruses. *J. Virol.,* 1993, vol. 67, 3288-3294
- **SCHNEIDER, R. ; G. UNGER ; R. STARK ; E. SCHNEIDER-SCHERZER ; H.-J. THIEL.** Identification of a structural glycoprotein of an RNA virus as a ribonuclease. *Science,* 1993, vol. 261, 1169-1171
- **SAMBROOK, J. ; FRITSCH, E.F. ; MANIATIS, T.** Molecular Cloning: A Laboratory Manual. Cold Spring Harbor Laboratory, 1989
- **STARK, R. ; MEYERS, G. ; RÜMENAPF, T ; THIEL, H.-J.** Processing of pestivirus polyprotein: Cleavage site between autoprotease and nucleocapsid protein of classical swine fever virus. *J. Virol.,* 1993, vol. 67, 7088-7095
- **THIEL, H.-J. ; PLAGEMANN, G.W. ; MOENNIG, V.** The pestiviruses. In Fields Virology. Lippincott-Raven, 1996, 1059-1073
- **THIEL, H.-J. ; STARK, R. ; WEILAND, E. ; RÜMENAPF, T. ; MEYERS, G.** Hog cholera virus: molecular composition of virions from a pestivirus. *J. Virol.,* 1991, vol. 65, 4705-4712.31
- **TRATSCHIN, J.-D. ; MOSER, C. ; RUGGLI, N. ; HOFMANN, M.A.** Classical swine fever virus leader proteinase Npro is not required for viral replication in cell culture. *J. Virol.,* 1998, vol. 72, 7681-7684
- **VAN RIJN, P.A. ; VAN GENNIP, H.G. ; DE MEIJER, EJ. ; MOORMANN, R.J.** Epitope mapping of envelope glycoprotein E1 of hog cholera virus strain Brescia. *J. Gen. Virol.,* 1993, vol. 74, 2053-2060
- **WEILAND, E. ; THIEL, H.-J. ; HESS, G. ; WEILAND, F.** Development of monoclonal neutralizing antibodies agaist bovine viral diarrhea virus after pretreatment of mice with normal bovine cells and cyclophosphamide. *J. Virol. Methods,* 1989, vol. 24, 237-244
- **WEILAND, E. ; STARK, R. ; HAAS, B. ; RÜMENAPF, T. ; MEYERS, G. ; THIEL, H.-J.** Pestivirus glycoprotein which induces neutralizing antibodies forms part of a disulfide-linked heterodimer. *J. Virology,* 1990, vol. 64, 3563-3569
- **WEILAND, E. ; AHL, R. ; STARK, R. ; WEILAND, F. ; THIEL, H.-J.** A second envelope glycoprotein mediates neutralization of a pestivirus, hog cholera virus. *J. Virology,* 1992, vol. 66, 3677-3682
- **WINDISCH, J.M. ; SCHNEIDER, R. ; STARK, R. ; WEILAND, E. ; MEYERS, G. ; THIEL, H.-J.** RNase of classical swine fever virus: biochemical characterization and inhibition by virus-neutralizing monoclonal antibodies. *J. Virol.,* 1996, vol. 70, 352-358
- **WISKERCHEN, M. ; BELZER, S.K. ; COLLETT, M.S.** Pestivirus gene expression: the first protein product of the bovine viral diarrhea virus large open reading frame, p20, possesses proteolytic activity. *J. Virol.,* 1991, vol. 65, 4508-4514

**SEQUENZPROTOKOLL:**

SEQUENCE LISTING

<110> Boehringer Ingelheim Vetmedica GmbH
<120> Attenuated Bovine Viral Diarrhea Virus (BVDV)
<130> Case 1/1716
<160> 12
<170> PatentIn version 3.1

<210> 1
<211> 12332
<212> DNA

<213> Wildtyp BVDV: XIKE-A

<400> 1

gtatacgaga ttagctaaag aactcgtata tggattggac gtcaacaaat ttttaattgg 60
caacgtaggg aaccttcccc tcagcgaagg ccgaaaagag gctagccatg cccttagtag 120
gactagcaaa agtaggggac tagcggtagc agtgagttcg ttggatggcc gaacccctga 180
gtacaggggga gtcgtcaatg gttcgacact ccattagtcg aggagtctcg agatgccatg 240
tggacgaggg catgcccacg gcacatctta acccatgcgg gggttgcatg ggtgaaagcg 300
ctattcgtgg cgttatggac acagcctgat agggtgtagc agagacctgc tattccgcta 360
gtaaaaactc tgctgtacat ggcacatgga gttgttttca aatgaacttt tatacaaaac 420
atataaacaa aaaccagcag gcgtcgtgga acctgtttac gacgtcaacg ggcgcccact 480
gtttggagag agcagtgact tgcacccgca gtcaacacta aaactaccac accaacgagg 540
cagcgccaac atcctgacca atgctaggtc cctaccgcgg aaaggtgact gccggagagg 600
taatgtgtat ggaccggtga gtggcatcta tatcaaacca ggaccgatct actaccagga 660
ttatgtgggc cccgtctatc atagagcccc actggaacta tgtagggagg caagtatgtg 720
cgaaacaact aggagagttg gcagagtgac cggtagtgat gggaaattat atcatatcta 780
catctgcata gatgggtgta tcctcctgaa gagggcgact aggaaccaac cagaagtcct 840
gaaatgggta tacaacagat taaattgtcc tttatgggtc accagctgct ccgatgaagg 900
gagcaagggt gctacaagta agaagcagcc taagccagat aggatagaaa aaggtaagat 960
gaaaatagcc ccaaaagaga cagaaaaaga ttgcaaaacc agaccccccg acgcgactat 1020
agtagtagaa ggggttaagt accaggtgaa gaaaaaagga aaggtaaggg gaaaaaatac 1080
tcaagatggg ttatatcaca acaagaataa gccccctgaa tcaagaaaaa aattggaaaa 1140
ggcactgctg gcttgggcca tcttagcagc ggtcctgctt cagctggtaa caggagagaa 1200
tatcacccag tggaacttga tggacaacgg caccgaggga atacagcaag cgatgttcct 1260
aagaggggtg aacaggagtc tacatggaat ttggccagag aaaatttgca ccggagtacc 1320
aactcactta gcaacagact atgagcttaa agagatagtg gggatgatgg acgcgagtga 1380
gaagaccaac tacacgtgtt gcaggttgca aagacatgag tggaataaac atggttggtg 1440
taactggttt catatagaac cgtggatatg gttgatgaac aaaacccaaa acaacctgac 1500
agaagggcaa ccgcttaggg agtgtgctgt gacttgtagg tatgacaagg aaacagaatt 1560
gaacatcgtg acacaggcta gggacagacc tacaactctg acaggttgca agaaaggcaa 1620
gaatttctct ttcgcaggtg ttatactgga tgggccctgt aactttaaag tatcggttga 1680
agatgtgctg ttcaaggagc acgattgcgg caacatgctg caagagaccg cgatacagct 1740
actcgatggg gcaaccaaca ccattgaggg agcaagggta gggacggcca agttgacaac 1800
ctggttaggg aagcaattag ggatccttgg taagaagttg gagaacaaaa gcaaagcatg 1860
gtttggtgca catgcagcaa gtccatactg cggagtggag aggaagatcg gttacgtatg 1920
gtatacaaaa aactgcactc cagcttgcct tccaagaaac actagaataa taggccccgg 1980
gaaatttgat accaacgccg aagatggaaa aatactccat gagatggggg ggcacctctc 2040
agaatttgtc ctattgtcct tggtggttct gtctgacttt gccccggaaa ccgcgagcgt 2100
catctacttg gttctacatt ttgcgatccc gcaaagccac gttgatgtag acacatgcga 2160
caagaaccag ctgaatttaa cggtagcaac cacagtagca gaggtcatac cagggacagt 2220
gtggaaccta gggaagtatg tctgcataag accagactgg tggccatatg agacgacgac 2280
agtcttcgtc atagaggaag cagggcaagt aatcaaattg atgctaaggg ccatcagaga 2340
cttaactagg atatggaatg ctgccactac cacagctttc ttaatctttt tagtaaaagc 2400
actgagggga caactaatcc aagggctatt gtggctgatg ctaataacag gagcacaggg 2460

cttccctgaa tgcaaagagg gcttccaata tgccatatct aaagacagga aaatggggtt 2520
attggggcca gagagcttaa ctacaacatg gcacctcccc accaaaaaaa tagtggattc 2580
catggtgcat gtatggtgtg aaggaaaaga cttgaaaata ttaaaaatgt gcacaaagga 2640
agagaggtat ctagtggctg tgcacgagag agccttatca accagtgccg agtttatgca 2700
gatcagtgat gggacaatag gcccagacgt gatagatatg cctgatgact ttgagtttgg 2760
actctgccct tgtgactcaa aaccagtgat aaagggcaaa tttaatgcca gcttactgaa 2820
tggaccagct ttccagatgg tatgcccaca ggggtggact ggtacaatag aatgcacccct 2880
agcgaaccaa gacaccttgg acacaactgt cattaggaca tatagaagaa ctaccccatt 2940
tcagcggaga aaatggtgta cctatgaaaa aataataggg gaagatatct atgaatgcat 3000
tctaggtgga aactggacat gcataaccgg tgaccatagc aggttgaaag acggacctat 3060
caagaagtgt aagtggtgtg gccatgactt cgtcaactca gaggggctac cacactaccc 3120
aataggcaag tgcatgctca tcaacgagag tgggtacagg tatgtagatg acacctcttg 3180
cgataggggt ggtgtagcca tagttccatc tggcaccgta aagtgtagaa taggtaacgt 3240
cacggtgcaa gttatcgcta ctaacaatga tctgggaccc atgccttgca gcccagctga 3300
agtgatagca agtgaaggac cagtggaaaa gactgcatgc acattcaact attcaaggac 3360
tctacctaat aagtattatg agccaaggga ccggtacttc caacaataca tgttaaaagg 3420
ggagtggcaa tattggttcg acctggattc tgtagaccac cacaaagact acttctcaga 3480
gttcataatc atagcagtgg tcgccttgtt gggtggtaag tacgtactgt ggctcttgat 3540
aacatacaca atactgtctg agcagatggc tatgggtgct ggagtgaata ctgaagagat 3600
agtcatgata ggcaatttgc tgacagacag tgatattgag gttgtggttt atttccttct 3660
tctgtactta atagttaaag aggaactggc gaggaaatgg attatactgg tataccacat 3720
ccttgtagcc aaccctatga aaacaattgg ggtcgtctta ctaatgctag gggagtggt 3780
gaaggccagc agaatcaatg ctgatgacca aagtgctatg gacccatgct ttcttctcgt 3840
gacaggcgta gtggctgttt tgatgatcgc tagaagagaa cctgccacat taccactgat 3900
tgtagcattg ctagcaataa gaacatcagg attcctactg cccgctagca ttgatgtaac 3960
tgtagcagta gtattaattg tacttttgtt ggctagctac ataacagact actttagata 4020
taaaaagtgg cttcaactct tatttagtct gatagctggt atctttatta taaggagctt 4080
aaaacatatc aaccagatgg aggtaccaga aatatctatg ccaagttgga gacctctagc 4140
tctggtcctt ttctatataa catctacagc aataaccact aattgggaca ttgacttagc 4200
aggcttcctg ctgcaatggg cgccagcagt gatcatgatg gctaccatgt gggcagactt 4260
tttgactctg atcatagtcc tgcccagtta cgagttatct aagctttact tcctaaagaa 4320
cgtcaggaca gacgtggaaa agaactggct cggcaaagtg aaatacagac agatcagttc 4380
agtttatgac atctgtgaca gtgaggaagc agtgtaccta tttccatcaa ggcataagag 4440
tggaagcagg ccagatttca tattaccttt tttgaaagcc gtgttaataa gctgcatcag 4500
cagccaatgg caagtggttt acatttctta cctaatactg gaaattacat actatatgca 4560
caggaaaatc atagatgagg tgtcaggagg agcaaatttt ctatcaagac tcatagcagc 4620
catcatagaa ttaaattggg ccatagatga tgaggaatgt aaaggactga agaaactgta 4680
tctcttgtca gggagagcga agaatttgat agttaaacat aaggtaagaa atgaagccgt 4740
ccacagatgg tttggtgagg aggaaatata cggggcaccc aaggtgatca ctatcataaa 4800
agctagtacc ctaagtaaaa acaggcactg cataatctgc acgatctgtg aagggaaaga 4860
atggaatgga gccaactgcc caaagtgtgg aagacaagga aagcccataa catgtggaat 4920
gacactcgca gactttgagg agaaacatta caaaaagata tttataagag aagaatcttc 4980
ttgtcctgtg ccttttgatc cttcttgcca ttgtaattat tttcgccacg atgggccttt 5040
caggaaagag tataagggtt acgtccaata cacagccaga ggacaactct ttctgaggaa 5100
cctaccaatt ctagcgacga agatgaagct attaatggtg ggaaacctcg gcgcagaaat 5160
tggcgacctg gaacatctag gatgggtact gagagggcca gccgtgtgca aaaaaattac 5220
caaccatgag aagtgccacg taaacatcat ggataagcta actgcatttt ttggaatcat 5280
gcctagaggc acgaccccta gggcacctgt gaggttcccc acagcactac taaaagtgag 5340
aagggggcta gagacgggat gggcttacac gcaccaagga gggatcagct cggtagacca 5400
tgtcacagcc ggaaaggatt tactagtgtg tgacagtatg ggcaggacca gggttgtctg 5460
tcatagtaac aataagatga ctgatgagac tgagtatggc atcaagaccg actcagggtg 5520
tcccgaaggt gcgaggtgtt acgtgctaaa cccagaagct gttaacattt ctggcacaaa 5580
aggagctatg gtacacctcc agaaaacggg gggggagttc acatgtgtca ctgcctcagg 5640
gaccccggct ttcttcgatc tgaaaaatct aaaaggctgg tccgggctac caatttttga 5700
agcatccagt ggcagggtgg ttggtagggt gaaagtcggc aagaatgagg attccaagcc 5760
caccaaacta atgagcggaa tccagacagt gtctaagaac cagacagacc tagcggacat 5820
cgtaaaaaaa ttgactagta tgaacagagg agagttcaaa cagataacat tagccactgg 5880

ggcaggaaaa actacggaac tgccaaggtc cgtcatagag gagataggga ggcacaaaag 5940
ggtcttagtc ctgataccat tgagagcagc agcagagtca gtgtatcagt atatgagagt 6000
gaagtaccca agtatatctt tcaatttgag aataggagat atgaaggaag gtgacatggc 6060
cactggtatc acctacgcct catatgggta cttttgtcag cttcctcagc ccaaactgag 6120
agctgccatg gtagagtact catatatatt cttagatgag taccactgtg ctacacccga 6180
gcaattagca ataattggaa agatacacag gtttgctgaa aatcttagag tggtagcaat 6240
gacagcaacc ccagctggaa cggtcacaac gactggtcag aaacacccta tagaggagtt 6300
catagcccca gaggtgatga aaggtgaaga tctaggtagt gaatacttgg atattgcagg 6360
gttgaagata ccgactgaag agatgaaagg caacatgctc gtgttcgcgc caactaggaa 6420
catggcagta gaaacagcta agaaattgaa ggctaaggga tacaactctg gatactatta 6480
cagtggggaa aacccagaga acttgagggt ggtaacctcg caatccccgt atgtggtagt 6540
agccaccaat gccatagagt caggtgtgac attaccagac ttagacacag ttgtagacac 6600
tggactaaag tgtgagaaga gggtgaggat ttcttcaaaa atgcccttca ttgtaacagg 6660
acttaagaga atggcagtca caatcggaga gcaagcccag cgcaggggta gagtaggaag 6720
agtcaagcca ggtaggtact ataggagtca agaaacagct tcagggtcaa aagattacca 6780
ttacgaccta ctgcaagccc agaggtacgg aatagaagat ggaattaatg taacaaagtc 6840
attcagggag atgaactatg attggagcct ttacgaagag gacagcttga tgataactca 6900
actcgaggtc cttaacaacc tccttatatc agaagacctg cctgccgcag tgaagaacat 6960
catggcccgg accgatcacc cagaacccat acaactggcc tataacagtt atgaaaacca 7020
aattccagtg ctgttcccaa agatcaaaaa tggtgaggtg acagacagtt atgagaatta 7080
cacatatctc aatgcaagaa aattaggaga ggacgtgccg gcatatgtgt acgccacaga 7140
ggatgaggat ctagcagtgg atcttctggg tatggattgg ccggacccag gcaaccaaca 7200
ggtggtagag acagggaggg cattaaaaca agtaactggc ttatccacag cagaaaacgc 7260
cctcttgata gccctattcg gctacgtcgg gtaccagaca ctttcaaaaa ggcacatacc 7320
catgattact gacatctata cacttgaaga ccacaggctt gaggacacaa cccacctcca 7380
gtttgcccca aacgctataa ggaccgacgg caaggactca gagttgaagg aattagctgt 7440
gggagacctt gataaatatg tggacgcact ggtagactac tccaaacaag ggatgaaatt 7500
catcaaagtc caagctgaaa aggtcagaga ctcccagtct acgaaggaag gcttgcaaac 7560
cattaaggag tatgtggata agtttataca atcactaaca gagaataagg aggagatcat 7620
caggtatgga ctatggggag ttcacacggc actctacaaa agcttggcag cgagactggg 7680
gcatgaaaca gcttttgcaa ctttagtggt aaaatggttg gcttttgggg gcgaaacggt 7740
atctgctcac atcaagcaag tagcagttga tctagtagta tattatatca tcaacaaacc 7800
atcttttcct ggagatacag agacccaaca agaggggagg aagtttgtgg ctagtctttt 7860
tatatctgca ctagcaacat acacatataa aacctggaat tacaacaatc tgcaacgggt 7920
tgtcgaacct gccttagctt acctcccata tgctacaagt gccttgaagt tgttcacacc 7980
cacaagatta gagagtgtgg tcatactcag ttctacaatt tacaagacat acctctctat 8040
aaggaagggt aagagtgacg gcttgttagg tacaggcata agtgcagcca tggagatctt 8100
aaaccaaaac ccaatctcag taggtatatc tgtgatgctg ggggtaggtg ccatcgccgc 8160
ccataatgca atagaatcta gtgaacagaa aagaactttg ctgatgaagg tctttgtaaa 8220
aaacttctta gaccaagcag caacagatga gctagtcaaa gagaaccctg aaaaaataat 8280
catggctcta tttgaagcag tccagaccat aggaaacccc ctaagactca tctaccatct 8340
gtacggggtg tactataagg ggtgggaagc aaaagaactc gcagagaaaa ctgctggccg 8400
caacttattc acattgatca tgtttgaggc ctttgagctt ttaggtatgg actcagaagg 8460
aaagataaga aacttgtcag gcaactacat actggactta atcttcaact tgcataataa 8520
attaaacaag ggctcaaaa aactagtcct tgggtggct cctgcacctt tgagctgtga 8580
ttggacacca agtgatgaga gaataagcct acctcataac aactacttaa gggtagaaac 8640
caggtgtcct tgtggctatg agatgaaggc aataaaaaat gttgctggta aattgacaaa 8700
agttgaagaa aaggggtcct tcctatgcag gaatagatta gggagaggac ctccaaactt 8760
caaagtaaca aagttctatg atgataactt gatagaagtc aagccagtag ctaggctaga 8820
aggccaggtg gacctctatt acaagggagt aacagctaag ttagactaca acaatgggaa 8880
agtactgtta gctaccaaca agtgggaggt ggaccacgct ttcctgacca gactagtaaa 8940
gaagcacaca gggataggtt ttaaaggtgc atatttgggt gaccgaccag accatcaaga 9000
tcttgtcgat agagattgtg caactataac gaagaactca gtacagttcc taaaaatgaa 9060
gaagggttgc gctttcacat atgacctaac aatctctaac cttgtcaggc ttattgaact 9120
agtccataag aataatttac aagaaagaga gatccctacc gtgacagtaa ctacttggct 9180
tgcatattct tttgtcaatg aagacctggg gactatcaag cctgtattgg gggagaaagt 9240
catcccagaa ccccccgagg agttgagtct ccaacccacc gtgagactag tcaccactga 9300

```
aacagcaata accataacag gggaggctga agtgatgacg acagggatca caccagtggt 9360
agagatgaaa gaagaacctc agctggacca ccagtcaact accctaaagg tagggttgaa 9420
ggaaggggaa tatccagggc caggagttaa ccctaaccat ttagcagagg tgatagatga 9480
gaaagatgac aggccttttg tcctaatcat cggtaacaaa ggttctacct cgaacagagc 9540
aagaacggcc aagaatatac ggctgtacaa aggaaacaac ccaagagaga tcagggatct 9600
gatgagccaa ggaagaatat tgacggttgc tctaaaagag ttggacccgg aattaaaaga 9660
attagtagat tacaagggga cctttctcaa tagggaagct ttagaagccc taagcttagg 9720
taagccaatc aagaggaaaa ccacaacagc aatgatcagg aggttaatag agccagaggt 9780
tgaggaggaa ctaccagatt ggttccaagc ggaagaaccc ctatttttgg aagcaaaaat 9840
acagaatgac ttataccacc taattggcag tgtagatagt ataaaaagca aagcaaagga 9900
attaggggcc acagataaca caaagatagt gaaggaagtt ggggctagga cctatacgat 9960
gaaattgagc agctggagca cacaagttac aaaaaaacag atgagtctag cccctctctt 10020
tgaagagctg ttattaaagt gccctccatg tagtaaaatt tcaaagggac atatggtgtc 10080
agcataccaa ctggctcaag gaaactggga acccctcggg tgtggggtct atatgggaac 10140
cataccagct aggcgtctca agatccaccc ttatgaggct taccttaaac tcaaagagct 10200
ggtggaagtt gaatcttcga gggccactgc aaaagaatcc atcataagag aacataacac 10260
ctggatcctg cggaaggtga gacatgaagg gaacctaaga accaaatcaa tgatcaaccc 10320
tgggaaaata tcagatcagc tatgcagaga tggacacaaa agaaacatat ataataagat 10380
cataggctca acaatggcct ctgctggtat taggctggag aaactgccag tagtccgagc 10440
ccaaactgac acaaccagtt tccaccaagc cataagagaa aaaattgata aaacagaaaa 10500
caagcagacc cctgaattgc atgaagaact aatgaaggtc ttcgactgct taaagatccc 10560
agagctgaag gaatcgtatg atgaagtttc atgggaacaa ttagaagccg ggataaaccg 10620
taagggtgca gcaggctatc tagagagcaa gaacataggg gaagtcctag acacagagaa 10680
acacatagta gagcagctga tcaaggatct gaggaagggg aagaagatta ggtactatga 10740
aacagccatc cccaagaatg agaagagaga cgtcagcgac gactgggaag ccggagagtt 10800
cgttgatgaa aagaaaccaa gagtaatcca gtacccggac gccaaggtga gactggccat 10860
tacaaaagtg atgtacaaat gggtaaagca aaaaccagtg gtgatacccg gctatgaagg 10920
taaaacacct ctatttgaca tattcaacaa agtgaagaag gaatgggatt cattccagga 10980
ccccgtagca gtgagctttg acaccaaagc gtgggataca caagtcacca gtagagacct 11040
aatgttgata aaggatatcc agaaatatta tttcaagaga agtatacaca aatttttaga 11100
tacaataaca gaacacatgg tggaggtacc tgtcattaca gcagacggtg aagtttacat 11160
aaggaatggt cagaggggta gtggccaacc cgacacaagt gctggtaata gtatgttgaa 11220
tgtcctaacc atgatatatg ctttctgtaa aagtacaggc ataccttaca ggggattcag 11280
cagagtggca agaatccatg tgtgtggtga tgatggcttt ttgataacag agagaggact 11340
gggactgaaa ttctctgaga agggtatgca gatattacat gaggccggga agccccagaa 11400
aataactgaa ggggacaaaa tgaaagtggc atacagattc gaggacatag agttttgttc 11460
ccatactccc gtgccagtca gatgggcaga taacaccagt agttacatgg cagggaggag 11520
cacagccact atactagcta agatggcaac caggctggat tccagcggag agaggggtag 11580
cacagcttat gagaaggccg tagccttcag cttccttttg atgtactcat ggaatcccgt 11640
agttagaagg atctgcttac tggtgttgtc acagtttcca gaaatatccc catccaaaaa 11700
cacaatatac tactaccaag gggatcccat agctgcgtac agagaagtga tagggaaaca 11760
gctgtgtgaa ctgaaaagaa caggatttga gaagctggct ggtctgaatt tgagtatgac 11820
cactctaggc atctggacaa aacatactag taaaagacta atccaagcct gtgtagaaat 11880
aggtaagaga gaaggtacct ggttagttaa tgctgacaga ctgattgcag gaaagactgg 11940
gaagttttac atcccaagca ctggtgtcac tctgttggga aaacactatg aggaaattaa 12000
cttaaagcaa aaggcggcac aaccgccgat agaggggtt gacagatata agttgggccc 12060
catagttaat gttatcttga gaaggctgag ggtgatgctg atgacagttg ccagcggaag 12120
ctggtgaatc cgtccggagc gtcgtgccct cactcaaggt ttttaattgt aaatattgta 12180
aatagacagc taagatattt attgtagttg gatagtaatg cagtgatagt aaatacccca 12240
atttaacact acctccaatg cactaagcac tttagctgtg tgaggttaac tcgacgtcca 12300
cggttggact agggaagacc tctaacagcc cc                               12332
```

<210> 2

<211> 11840

<212> DNA <213> Artificial Sequence: Mutated BVDV: XIKE- A-NdN

<400> 2

gtatacgaga ttagctaaag aactcgtata tggattggac gtcaacaaat ttttaattgg 60
caacgtaggg aaccttcccc tcagcgaagg ccgaaaagag gctagccatg cccttagtag 120
gactagcaaa agtaggggac tagcggtagc agtgagttcg ttggatggcc gaacccctga 180
gtacagggga gtcgtcaatg gttcgacact ccattagtcg aggagtctcg agatgccatg 240
tggacgaggg catgcccacg gcacatctta acccatgcgg gggttgcatg ggtgaaagcg 300
ctattcgtgg cgttatggac acagcctgat agggtgtagc agagacctgc tattccgcta 360
gtaaaaactc tgctgtacat ggcacatgga gttgttttcc gatgaaggga gcaagggtgc 420
tacaagtaag aagcagccta agccagatag gatagaaaaa ggtaagatga aaatagcccc 480
aaaagagaca gaaaaagatt gcaaaaccag acccccgac gcgactatag tagtagaagg 540
ggttaagtac caggtgaaga aaaaaggaaa ggtaagggga aaaaatactc aagatgggtt 600
atatcacaac aagaataagc cccctgaatc aagaaaaaaa ttggaaaagg cactgctggc 660
ttgggccatc ttagcagcgg tcctgcttca gctggtaaca ggagagaata tcacccagtg 720
gaacttgatg gacaacggca ccgagggaat acagcaagcg atgttcctaa gaggggtgaa 780
caggagtcta catggaattt ggccagagaa aatttgcacc ggagtaccaa ctcacttagc 840
aacagactat gagcttaaag agatagtggg gatgatggac gcgagtgaga agaccaacta 900
cacgtgttgc aggttgcaaa gacatgagtg gaataaacat ggttggtgta actggtttca 960
tatagaaccg tggatatggt tgatgaacaa aacccaaaac aacctgacag aagggcaacc 1020
gcttagggag tgtgctgtga cttgtaggta tgacaaggaa acagaattga acatcgtgac 1080
acaggctagg gacagaccta caactctgac aggttgcaag aaaggcaaga atttctcttt 1140
cgcaggtgtt atactggatg ggccctgtaa ctttaaagta tcggttgaag atgtgctgtt 1200
caaggagcac gattgcggca acatgctgca agagaccgcg atacagctac tcgatggggc 1260
aaccaacacc attgagggag caagggtagg gacggccaag ttgacaacct ggttagggaa 1320
gcaattaggg atccttggta agaagttgga gaacaaaagc aaagcatggt ttggtgcaca 1380
tgcagcaagt ccatactgcg gagtggagag gaagatcggt tacgtatggt atacaaaaaa 1440
ctgcactcca gcttgccttc caagaaacac tagaataata ggccccggga aatttgatac 1500
caacgccgaa gatggaaaaa tactccatga gatgggggg cacctctcag aatttgtcct 1560
attgtccttg gtggttctgt ctgactttgc cccggaaacc gcgagcgtca tctacttggt 1620
tctacatttt gcgatcccgc aaagccacgt tgatgtagac acatgcgaca agaaccagct 1680
gaatttaacg gtagcaacca cagtagcaga ggtcatacca gggacagtgt ggaacctagg 1740
gaagtatgtc tgcataagac cagactggtg gccatatgag acgacgacag tcttcgtcat 1800
agaggaagca gggcaagtaa tcaaattgat gctaagggcc atcagagact taactaggat 1860
atggaatgct gccactacca cagctttctt aatcttttta gtaaaagcac tgaggggaca 1920
actaatccaa gggctattgt ggctgatgct aataacagga gcacagggct tccctgaatg 1980
caaagagggc ttccaatatg ccatatctaa agacaggaaa atggggttat tggggccaga 2040
gagcttaact acaacatggc acctccccac caaaaaaata gtggattcca tggtgcatgt 2100
atggtgtgaa ggaaaagact tgaaaatatt aaaaatgtgc acaaaggaag agaggtatct 2160
agtggctgtg cacgagagag ccttatcaac cagtgccgag tttatgcaga tcagtgatgg 2220
gacaataggc ccagacgtga tagatatgcc tgatgacttt gagtttggac tctgcccttg 2280
tgactcaaaa ccagtgataa agggcaaatt taatgccagc ttactgaatg gaccagcttt 2340
ccagatggta tgcccacagg ggtggactgg tacaatagaa tgcaccctag cgaaccaaga 2400
caccttggac acaactgtca ttaggacata tagaagaact accccatttc agcggagaaa 2460
atggtgtacc tatgaaaaaa taatagggga agatatctat gaatgcattc taggtggaaa 2520
ctggacatgc ataaccggtg accatagcag gttgaaagac ggacctatca agaagtgtaa 2580
gtggtgtggc catgacttcg tcaactcaga ggggctacca cactacccaa taggcaagtg 2640
catgctcatc aacgagagtg ggtacaggta tgtagatgac acctcttgcg ataggggtgg 2700
tgtagccata gttccatctg gcaccgtaaa gtgtagaata ggtaacgtca cggtgcaagt 2760
tatcgctact aacaatgatc tgggacccat gccttgcagc ccagctgaag tgatagcaag 2820
tgaaggacca gtggaaaaga ctgcatgcac attcaactat tcaaggactc tacctaataa 2880
gtattatgag ccaagggacc ggtacttcca acaatacatg ttaaaagggg agtggcaata 2940
ttggttcgac ctggattctg tagaccacca caaagactac ttctcagagt tcataatcat 3000
agcagtggtc gccttgttgg gtggtaagta cgtactgtgg ctcttgataa catacacaat 3060
actgtctgag cagatggcta tgggtgctgg agtgaatact gaagagatag tcatgatagg 3120
caatttgctg acagacagtg atattgaggt tgtggtttat ttccttcttc tgtacttaat 3180
agttaaagag gaactggcga ggaaatggat tatactggta taccacatcc ttgtagccaa 3240

ccctatgaaa acaattgggg tcgtcttact aatgctaggg ggagtggtga aggccagcag 3300
aatcaatgct gatgaccaaa gtgctatgga cccatgcttt cttctcgtga caggcgtagt 3360
ggctgttttg atgatcgcta gaagagaacc tgccacatta ccactgattg tagcattgct 3420
agcaataaga acatcaggat tcctactgcc cgctagcatt gatgtaactg tagcagtagt 3480
attaattgta cttttgttgg ctagctacat aacagactac tttagatata aaaagtggct 3540
tcaactctta tttagtctga tagctggtat ctttattata aggagcttaa aacatatcaa 3600
ccagatggag gtaccagaaa tatctatgcc aagttggaga cctctagctc tggtcctttt 3660
ctatataaca tctacagcaa taaccactaa ttgggacatt gacttagcag gcttcctgct 3720
gcaatgggcg ccagcagtga tcatgatggc taccatgtgg gcagactttt tgactctgat 3780
catagtcctg cccagttacg agttatctaa gctttacttc ctaaagaacg tcaggacaga 3840
cgtggaaaag aactggctcg gcaaagtgaa atacagacag atcagttcag tttatgacat 3900
ctgtgacagt gaggaagcag tgtacctatt tccatcaagg cataagagtg gaagcaggcc 3960
agatttcata ttaccttttt tgaaagccgt gttaataagc tgcatcagca gccaatggca 4020
agtggtttac atttcttacc taatactgga aattacatac tatatgcaca ggaaaatcat 4080
agatgaggtg tcaggaggag caaattttct atcaagactc atagcagcca tcatagaatt 4140
aaattgggcc atagatgatg aggaatgtaa aggactgaag aaactgtatc tcttgtcagg 4200
gagagcgaag aatttgatag ttaaacataa ggtaagaaat gaagccgtcc acagatggtt 4260
tggtgaggag gaaatatacg gggcacccaa ggtgatcact atcataaaag ctagtaccct 4320
aagtaaaaac aggcactgca taatctgcac gatctgtgaa gggaaagaat ggaatggagc 4380
caactgccca aagtgtggaa gacaaggaaa gcccataaca tgtggaatga cactcgcaga 4440
ctttgaggag aaacattaca aaaagatatt tataagagaa gaatcttctt gtcctgtgcc 4500
ttttgatcct tcttgccatt gtaattattt tcgccacgat gggcctttca ggaaagagta 4560
taagggttac gtccaataca cagccagagg acaactcttt ctgaggaacc taccaattct 4620
agcgacgaag atgaagctat taatggtggg aaacctcggc gcagaaattg gcgacctgga 4680
acatctagga tgggtactga gagggccagc cgtgtgcaaa aaaattacca accatgagaa 4740
gtgccacgta aacatcatgg ataagctaac tgcatttttt ggaatcatgc ctagaggcac 4800
gacccctagg gcacctgtga ggttccccac agcactacta aaagtgagaa gggggctaga 4860
gacgggatgg gcttacacgc accaaggagg gatcagctcg gtagaccatg tcacagccgg 4920
aaaggattta ctagtgtgtg acagtatggg caggaccagg gttgtctgtc atagtaacaa 4980
taagatgact gatgagactg agtatggcat caagaccgac tcagggtgtc ccgaaggtgc 5040
gaggtgttac gtgctaaacc cagaagctgt taacatttct ggcacaaaag gagctatggt 5100
acacctccag aaaacggggg gggagttcac atgtgtcact gcctcaggga ccccggcttt 5160
cttcgatctg aaaaatctaa aaggctggtc cgggctacca atttttgaag catccagtgg 5220
cagggtggtt ggtagggtga aagtcggcaa gaatgaggat tccaagccca ccaaactaat 5280
gagcggaatc cagacagtgt ctaagaacca gacagaccta gcggacatcg taaaaaaatt 5340
gactagtatg aacagaggag agttcaaaca gataacatta gccactgggg caggaaaaac 5400
tacggaactg ccaaggtccg tcatagagga gatagggagg cacaaaaggg tcttagtcct 5460
gataccattg agagcagcag cagagtcagt gtatcagtat atgagagtga agtacccaag 5520
tatatctttc aatttgagaa taggagatat gaaggaaggt gacatggcca ctggtatcac 5580
ctacgcctca tatgggtact tttgtcagct tcctcagccc aaactgagag ctgccatggt 5640
agagtactca tatatattct tagatgagta ccactgtgct acacccgagc aattagcaat 5700
aattggaaag atacacaggt ttgctgaaaa tcttagagtg gtagcaatga cagcaacccc 5760
agctggaacg gtcacaacga ctggtcagaa acaccctata gaggagttca tagccccaga 5820
ggtgatgaaa ggtgaagatc taggtagtga atacttggat attgcagggt tgaagatacc 5880
gactgaagag atgaaaggca acatgctcgt gttcgcgcca actaggaaca tggcagtaga 5940
aacagctaag aaattgaagg ctaagggata caactctgga tactattaca gtggggaaaa 6000
cccagagaac ttgagggtgg taacctcgca atccccgtat gtggtagtag ccaccaatgc 6060
catagagtca ggtgtgacat taccagactt agacacagtt gtagacactg gactaaagtg 6120
tgagaagagg gtgaggattt cttcaaaaat gcccttcatt gtaacaggac ttaagagaat 6180
ggcagtcaca atcggagagc aagcccagcg caggggtaga gtaggaagag tcaagccagg 6240
taggtactat aggagtcaag aaacagcttc agggtcaaaa gattaccatt acgacctact 6300
gcaagcccag aggtacggaa tagaagatgg aattaatgta acaaagtcat tcagggagat 6360
gaactatgat tggagccttt acgaagagga cagcttgatg ataactcaac tcgaggtcct 6420
taacaacctc cttatatcag aagacctgcc tgccgcagtg aagaacatca tggcccggac 6480
cgatcaccca gaacccatac aactggccta taacagttat gaaaaccaaa ttccagtgct 6540
gttcccaaag atcaaaaatg gtgaggtgac agacagttat gagaattaca catatctcaa 6600
tgcaagaaaa ttaggagagg acgtgccggc atatgtgtac gccacagagg atgaggatct 6660

agcagtggat cttctgggta tggattggcc ggacccaggc aaccaacagg tggtagagac 6720
agggagggca ttaaaacaag taactggctt atccacagca gaaaacgccc tcttgatagc 6780
cctattcggc tacgtcgggt accagacact ttcaaaaagg cacataccca tgattactga 6840
catctataca cttgaagacc acaggcttga ggacacaacc cacctccagt ttgccccaaa 6900
cgctataagg accgacggca aggactcaga gttgaaggaa ttagctgtgg gagaccttga 6960
taaatatgtg gacgcactgg tagactactc caaacaaggg atgaaattca tcaaagtcca 7020
agctgaaaag gtcagagact cccagtctac gaaggaaggc ttgcaaacca ttaaggagta 7080
tgtggataag tttatacaat cactaacaga gaataaggag gagatcatca ggtatggact 7140
atggggagtt cacacggcac tctacaaaag cttggcagcg agactggggc atgaaacagc 7200
ttttgcaact ttagtggtaa aatggttggc ttttgggggc gaaacggtat ctgctcacat 7260
caagcaagta gcagttgatc tagtagtata ttatatcatc aacaaaccat cttttcctgg 7320
agatacagag acccaacaag aggggaggaa gtttgtggct agtcttttta tatctgcact 7380
agcaacatac acatataaaa cctggaatta caacaatctg caacgggttg tcgaacctgc 7440
cttagcttac ctcccatatg ctacaagtgc cttgaagttg ttcacaccca caagattaga 7500
gagtgtggtc atactcagtt ctacaattta caagacatac ctctctataa ggaagggtaa 7560
gagtgacggc ttgttaggta caggcataag tgcagccatg gagatcttaa accaaaaccc 7620
aatctcagta ggtatatctg tgatgctggg ggtaggtgcc atcgccgccc ataatgcaat 7680
agaatctagt gaacagaaaa gaactttgct gatgaaggtc tttgtaaaaa acttcttaga 7740
ccaagcagca acagatgagc tagtcaaaga gaaccctgaa aaaataatca tggctctatt 7800
tgaagcagtc cagaccatag gaaaccccct aagactcatc taccatctgt acggggtgta 7860
ctataagggg tgggaagcaa aagaactcgc agagaaaact gctggccgca acttattcac 7920
attgatcatg tttgaggcct ttgagctttt aggtatggac tcagaaggaa agataagaaa 7980
cttgtcaggc aactacatac tggacttaat cttcaacttg cataataaat taaacaaggg 8040
gctcaaaaaa ctagtccttg ggtgggctcc tgcacctttg agctgtgatt ggacaccaag 8100
tgatgagaga ataagcctac ctcataacaa ctacttaagg gtagaaacca ggtgtccttg 8160
tggctatgag atgaaggcaa taaaaaatgt tgctggtaaa ttgacaaaag ttgaagaaaa 8220
ggggtccttc ctatgcagga atagattagg gagaggacct ccaaacttca aagtaacaaa 8280
gttctatgat gataacttga tagaagtcaa gccagtagct aggctagaag gccaggtgga 8340
cctctattac aagggagtaa cagctaagtt agactacaac aatgggaaag tactgttagc 8400
taccaacaag tgggaggtgg accacgcttt cctgaccaga ctagtaaaga agcacacagg 8460
gataggtttt aaaggtgcat atttgggtga ccgaccagac catcaagatc ttgtcgatag 8520
agattgtgca actataacga agaactcagt acagttccta aaaatgaaga agggttgcgc 8580
tttcacatat gacctaacaa tctctaacct tgtcaggctt attgaactag tccataagaa 8640
taatttacaa gaaagagaga tccctaccgt gacagtaact acttggcttg catattcttt 8700
tgtcaatgaa gacctgggga ctatcaagcc tgtattgggg gagaaagtca tcccagaacc 8760
ccccgaggag ttgagtctcc aacccaccgt gagactagtc accactgaaa cagcaataac 8820
cataacaggg gaggctgaag tgatgacgac agggatcaca ccagtggtag agatgaaaga 8880
agaacctcag ctggaccacc agtcaactac cctaaaggta gggttgaagg aaggggaata 8940
tccagggcca ggagttaacc ctaaccattt agcagaggtg atagatgaga aagatgacag 9000
gccttttgtc ctaatcatcg gtaacaaagg ttctacctcg aacagagcaa gaacggccaa 9060
gaatatacgg ctgtacaaag gaaacaaccc aagagagatc agggatctga tgagccaagg 9120
aagaatattg acggttgctc taaaagagtt ggacccggaa ttaaaagaat tagtagatta 9180
caaggggacc tttctcaata gggaagcttt agaagcccta agcttaggta agccaatcaa 9240
gaggaaaacc acaacagcaa tgatcaggag gttaatagag ccagaggttg aggaggaact 9300
accagattgg ttccaagcgg aagaacccct atttttggaa gcaaaaatac agaatgactt 9360
ataccaccta attggcagtg tagatagtat aaaaagcaaa gcaaaggaat taggggccac 9420
agataacaca aagatagtga aggaagttgg ggctaggacc tatacgatga aattgagcag 9480
ctggagcaca caagttacaa aaaaacagat gagtctagcc cctctctttg aagagctgtt 9540
attaaagtgc cctccatgta gtaaaatttc aaagggacat atggtgtcag cataccaact 9600
ggctcaagga aactgggaac ccctcgggtg tggggtctat atgggaacca taccagctag 9660
gcgtctcaag atccacccctt atgaggctta ccttaaactc aaagagctgg tggaagttga 9720
atcttcgagg gccactgcaa aagaatccat cataagagaa cataacacct ggatcctgcg 9780
gaaggtgaga catgaaggga acctaagaac caaatcaatg atcaaccctg ggaaaatatc 9840
agatcagcta tgcagagatg gacacaaaag aaacatatat aataagatca taggctcaac 9900
aatggcctct gctggtatta ggctggagaa actgccagta gtccgagccc aaactgacac 9960
aaccagtttc caccaagcca taagagaaaa aattgataaa acagaaaaca agcagacccc 10020
tgaattgcat gaagaactaa tgaaggtctt cgactgctta aagatcccag agctgaagga 10080

atcgtatgat gaagtttcat gggaacaatt agaagccggg ataaaccgta agggtgcagc 10140
aggctatcta gagagcaaga acatagggga agtcctagac acagagaaac acatagtaga 10200
gcagctgatc aaggatctga ggaaggggaa gaagattagg tactatgaaa cagccatccc 10260
caagaatgag aagagagacg tcagcgacga ctgggaagcc ggagagttcg ttgatgaaaa 10320
gaaaccaaga gtaatccagt acccggacgc caaggtgaga ctggccatta caaaagtgat 10380
gtacaaatgg gtaaagcaaa aaccagtggt gatacccggc tatgaaggta aaacacctct 10440
atttgacata ttcaacaaag tgaagaagga atgggattca ttccaggacc ccgtagcagt 10500
gagctttgac accaaagcgt gggatacaca agtcaccagt agagacctaa tgttgataaa 10560
ggatatccag aaatattatt tcaagagaag tatacacaaa tttttagata caataacaga 10620
acacatggtg gaggtacctg tcattacagc agacggtgaa gtttacataa ggaatggtca 10680
gaggggtagt ggccaacccg acacaagtgc tggtaatagt atgttgaatg tcctaaccat 10740
gatatatgct ttctgtaaaa gtacaggcat accttacagg ggattcagca gagtggcaag 10800
aatccatgtg tgtggtgatg atggcttttt gataacagag agaggactgg gactgaaatt 10860
ctctgagaag ggtatgcaga tattacatga ggccgggaag ccccagaaaa taactgaagg 10920
ggacaaaatg aaagtggcat acagattcga ggacatagag ttttgttccc atactcccgt 10980
gccagtcaga tgggcagata acaccagtag ttacatggca gggaggagca cagccactat 11040
actagctaag atggcaacca ggctggattc cagcggagag aggggtagca cagcttatga 11100
gaaggccgta gccttcagct tcctttgat gtactcatgg aatcccgtag ttagaaggat 11160
ctgcttactg gtgttgtcac agtttccaga aatatcccca tccaaaaaca caatatacta 11220
ctaccaaggg gatcccatag ctgcgtacag agaagtgata gggaaacagc tgtgtgaact 11280
gaaaagaaca ggatttgaga agctggctgg tctgaatttg agtatgacca ctctaggcat 11340
ctggacaaaa catactagta aaagactaat ccaagcctgt gtagaaatag gtaagagaga 11400
aggtacctgg ttagttaatg ctgacagact gattgcagga aagactggga agttttacat 11460
cccaagcact ggtgtcactc tgttgggaaa acactatgag gaaattaact taaagcaaaa 11520
ggcggcacaa ccgccgatag aggggttga cagatataag ttgggcccca tagttaatgt 11580
tatcttgaga aggctgaggg tgatgctgat gacagttgcc agcggaagct ggtgaatccg 11640
tccggagcgt cgtgccctca ctcaaggttt ttaattgtaa atattgtaaa tagacagcta 11700
agatatttat tgtagttgga tagtaatgca gtgatagtaa ataccccaat ttaacactac 11760
ctccaatgca ctaagcactt tagctgtgtg aggttaactc gacgtccacg gttggactag 11820
ggaagacctc taacagcccc 11840

<210> 3
<211> 12329
<212> DNA
<213> Artificial Sequence: Mutated BVDV: XIKE-B

<400> 3

```
gtatacgaga ttagctaaag aactcgtata tggattggac gtcaacaaat ttttaattgg   60
caacgtaggg aaccttcccc tcagcgaagg ccgaaaagag gctagccatg cccttagtag  120
gactagcaaa agtaggggac tagcggtagc agtgagttcg ttggatggcc gaacccctga  180
gtacagggga gtcgtcaatg gttcgacact ccattagtcg aggagtctcg agatgccatg  240
tggacgaggg catgcccacg gcacatctta acccatgcgg gggttgcatg ggtgaaagcg  300
ctattcgtgg cgttatggac acagcctgat agggtgtagc agagacctgc tattccgcta  360
gtaaaaactc tgctgtacat ggcacatgga gttgttttca aatgaacttt tatacaaaac  420
atataaacaa aaaccagcag gcgtcgtgga acctgtttac gacgtcaacg ggcgcccact  480
gtttggagag agcagtgact tgcacccgca gtcaacacta aaactaccac accaacgagg  540
cagcgccaac atcctgacca atgctaggtc cctaccgcgg aaaggtgact gccggagagg  600
taatgtgtat ggaccggtga gtggcatcta tatcaaacca ggaccgatct actaccagga  660
ttatgtgggc cccgtctatc atagagcccc actggaacta tgtagggagg caagtatgtg  720
cgaaacaact aggagagttg gcagagtgac cggtagtgat gggaaattat atcatatcta  780
catctgcata gatgggtgta tcctcctgaa gagggcgact aggaaccaac cagaagtcct  840
gaaatgggta tacaacagat taaattgtcc tttatgggtc accagctgct ccgatgaagg  900
gagcaagggt gctacaagta agaagcagcc taagccagat aggatagaaa aaggtaagat  960
gaaaatagcc ccaaaagaga cagaaaaaga ttgcaaaacc agacccccccg acgcgactat 1020
agtagtagaa ggggttaagt accaggtgaa gaaaaaagga aaggtaaggg gaaaaaatac 1080
tcaagatggg ttatatcaca acaagaataa gccccctgaa tcaagaaaaa aattggaaaa 1140
```

```
ggcactgctg gcttgggcca tcttagcagc ggtcctgctt cagctggtaa caggagagaa 1200
tatcacccag tggaacttga tggacaacgg caccgaggga atacagcaag cgatgttcct 1260
aagaggggtg aacaggagtc tacatggaat ttggccagag aaaatttgca ccggagtacc 1320
aactcactta gcaacagact atgagcttaa agagatagtg gggatgatgg acgcgagtga 1380
gaagaccaac tacacgtgtt gcaggttgca aagacatgag tggaataaag gttggtgtaa 1440
ctggtttcat atagaaccgt ggatatggtt gatgaacaaa acccaaaaca acctgacaga 1500
agggcaaccg cttagggagt gtgctgtgac ttgtaggtat gacaaggaaa cagaattgaa 1560
catcgtgaca caggctaggg acagacctac aactctgaca ggttgcaaga aaggcaagaa 1620
tttctctttc gcaggtgtta tactggatgg gccctgtaac tttaaagtat cggttgaaga 1680
tgtgctgttc aaggagcacg attgcggcaa catgctgcaa gagaccgcga tacagctact 1740
cgatggggca accaacacca ttgagggagc aagggtaggg acggccaagt tgacaacctg 1800
gttagggaag caattaggga tccttggtaa gaagttggag aacaaaagca aagcatggtt 1860
tggtgcacat gcagcaagtc catactgcgg agtggagagg aagatcggtt acgtatggta 1920
tacaaaaaac tgcactccag cttgccttcc aagaaacact agaataatag gccccgggaa 1980
atttgatacc aacgccgaag atggaaaaat actccatgag atggggggc acctctcaga 2040
atttgtccta ttgtccttgg tggttctgtc tgactttgcc ccggaaaccg cgagcgtcat 2100
ctacttggtt ctacattttg cgatcccgca aagccacgtt gatgtagaca catgcgacaa 2160
gaaccagctg aatttaacgg tagcaaccac agtagcagag gtcataccag ggacagtgtg 2220
gaacctaggg aagtatgtct gcataagacc agactggtgg ccatatgaga cgacgacagt 2280
cttcgtcata gaggaagcag ggcaagtaat caaattgatg ctaagggcca tcagagactt 2340
aactaggata tggaatgctg ccactaccac agctttctta atctttttag taaaagcact 2400
gaggggacaa ctaatccaag ggctattgtg gctgatgcta ataacaggag cacagggctt 2460
ccctgaatgc aaagagggct tccaatatgc catatctaaa gacaggaaaa tggggttatt 2520
ggggccagag agcttaacta caacatggca cctccccacc aaaaaaatag tggattccat 2580
ggtgcatgta tggtgtgaag gaaaagactt gaaaatatta aaaatgtgca caaaggaaga 2640
gaggtatcta gtggctgtgc acgagagagc cttatcaacc agtgccgagt ttatgcagat 2700
cagtgatggg acaataggcc cagacgtgat agatatgcct gatgactttg agtttggact 2760
ctgcccttgt gactcaaaac cagtgataaa gggcaaattt aatgccagct tactgaatgg 2820
accagctttc cagatggtat gcccacaggg gtggactggt acaatagaat gcaccctagc 2880
gaaccaagac accttggaca caactgtcat taggacatat agaagaacta ccccatttca 2940
gcggagaaaa tggtgtacct atgaaaaaat aataggggaa gatatctatg aatgcattct 3000
aggtggaaac tggacatgca taaccggtga ccatagcagg ttgaaagacg gacctatcaa 3060
gaagtgtaag tggtgtggcc atgacttcgt caactcagag ggctaccac actacccaat 3120
aggcaagtgc atgctcatca acgagagtgg gtacaggtat gtagatgaca cctcttgcga 3180
taggggtggt gtagccatag ttccatctgg caccgtaaag tgtagaatag gtaacgtcac 3240
ggtgcaagtt atcgctacta acaatgatct gggacccatg ccttgcagcc cagctgaagt 3300
gatagcaagt gaaggaccag tggaaaagac tgcatgcaca ttcaactatt caaggactct 3360
acctaataag tattatgagc caagggaccg gtacttccaa caatacatgt taaaagggga 3420
gtggcaatat tggttcgacc tggattctgt agaccaccac aaagactact tctcagagtt 3480
cataatcata gcagtggtcg ccttgttggg tggtaagtac gtactgtggc tcttgataac 3540
atacacaata ctgtctgagc agatggctat gggtgctgga gtgaatactg aagagatagt 3600
catgataggc aatttgctga cagacagtga tattgaggtt gtggtttatt tccttcttct 3660
gtacttaata gttaaagagg aactggcgag gaaatggatt atactggtat accacatcct 3720
tgtagccaac cctatgaaaa caattggggt cgtcttacta atgctagggg gagtggtgaa 3780
ggccagcaga atcaatgctg atgaccaaag tgctatggac ccatgctttc ttctcgtgac 3840
aggcgtagtg gctgttttga tgatcgctag aagagaacct gccacattac cactgattgt 3900
agcattgcta gcaataagaa catcaggatt cctactgccc gctagcattg atgtaactgt 3960
agcagtagta ttaattgtac ttttgttggc tagctacata acagactact ttagatataa 4020
aaagtggctt caactcttat ttagtctgat agctggtatc tttattataa ggagcttaaa 4080
acatatcaac cagatggagg taccagaaat atctatgcca agttggagac ctctagctct 4140
ggtccttttc tatataacat ctacagcaat aaccactaat tgggacattg acttagcagg 4200
cttcctgctg caatgggcgc cagcagtgat catgatggct accatgtggg cagacttttt 4260
gactctgatc atagtcctgc ccagttacga gttatctaag ctttacttcc taaagaacgt 4320
caggacagac gtggaaaaga actggctcgg caaagtgaaa tacagacaga tcagttcagt 4380
ttatgacatc tgtgacagtg aggaagcagt gtacctattt ccatcaaggc ataagagtgg 4440
aagcaggcca gatttcatat taccttttt gaaagccgtg ttaataagct gcatcagcag 4500
ccaatggcaa gtggtttaca tttcttacct aatactggaa attacatact atatgcacag 4560
```

gaaaatcata gatgaggtgt caggaggagc aaattttcta tcaagactca tagcagccat 4620
catagaatta aattgggcca tagatgatga ggaatgtaaa ggactgaaga aactgtatct 4680
cttgtcaggg agagcgaaga atttgatagt taaacataag gtaagaaatg aagccgtcca 4740
cagatggttt ggtgaggagg aaatatacgg ggcacccaag gtgatcacta tcataaaagc 4800
tagtacccta agtaaaaaca ggcactgcat aatctgcacg atctgtgaag ggaaagaatg 4860
gaatggagcc aactgcccaa agtgtggaag acaaggaaag cccataacat gtggaatgac 4920
actcgcagac tttgaggaga aacattacaa aaagatattt ataagagaag aatcttcttg 4980
tcctgtgcct tttgatcctt cttgccattg taattatttt cgccacgatg ggcctttcag 5040
gaaagagtat aagggttacg tccaatacac agccagagga caactctttc tgaggaacct 5100
accaattcta gcgacgaaga tgaagctatt aatggtggga aacctcggcg cagaaattgg 5160
cgacctggaa catctaggat gggtactgag agggccagcc gtgtgcaaaa aaattaccaa 5220
ccatgagaag tgccacgtaa acatcatgga taagctaact gcattttttg gaatcatgcc 5280
tagaggcacg acccctaggg cacctgtgag gttccccaca gcactactaa aagtgagaag 5340
ggggctagag acgggatggg cttacacgca ccaaggaggg atcagctcgg tagaccatgt 5400
cacagccgga aaggatttac tagtgtgtga cagtatgggc aggaccaggg ttgtctgtca 5460
tagtaacaat aagatgactg atgagactga gtatggcatc aagaccgact cagggtgtcc 5520
cgaaggtgcg aggtgttacg tgctaaaccc agaagctgtt aacatttctg gcacaaaagg 5580
agctatggta cacctccaga aaacgggggg ggagttcaca tgtgtcactg cctcagggac 5640
cccggctttc ttcgatctga aaaatctaaa ggctggtccg ggctaccaa ttttgaagc 5700
atccagtggc agggtggttg gtagggtgaa agtcggcaag aatgaggatt ccaagcccac 5760
caaactaatg agcggaatcc agacagtgtc taagaaccag acagacctag cggacatcgt 5820
aaaaaaattg actagtatga acagaggaga gttcaaacag ataacattag ccactggggc 5880
aggaaaaact acggaactgc caaggtccgt catagaggag atagggaggc acaaaagggt 5940
cttagtcctg ataccattga gagcagcagc agagtcagtg tatcagtata tgagagtgaa 6000
gtacccaagt atatctttca atttgagaat aggagatatg aaggaaggtg acatggccac 6060
tggtatcacc tacgcctcat atgggtactt ttgtcagctt cctcagccca aactgagagc 6120
tgccatggta gagtactcat atatattctt agatgagtac cactgtgcta cacccgagca 6180
attagcaata attggaaaga tacacaggtt tgctgaaaat cttagagtgg tagcaatgac 6240
agcaacccca gctggaacgg tcacaacgac tggtcagaaa caccctatag aggagttcat 6300
agccccagag gtgatgaaag gtgaagatct aggtagtgaa tacttggata ttgcagggtt 6360
gaagataccg actgaagaga tgaaaggcaa catgctcgtg ttcgcgccaa ctaggaacat 6420
ggcagtagaa acagctaaga aattgaaggc taagggatac aactctggat actattacag 6480
tggggaaaac ccagagaact tgagggtggt aacctcgcaa tccccgtatg tggtagtagc 6540
caccaatgcc atagagtcag gtgtgacatt accagactta gacacagttg tagacactgg 6600
actaaagtgt gagaagaggg tgaggatttc ttcaaaaatg cccttcattg taacaggact 6660
taagagaatg gcagtcacaa tcggagagca agcccagcgc aggggtagag taggaagagt 6720
caagccaggt aggtactata ggagtcaaga aacagcttca gggtcaaaag attaccatta 6780
cgacctactg caagcccaga ggtacggaat agaagatgga attaatgtaa caaagtcatt 6840
cagggagatg aactatgatt ggagccttta cgaagaggac agcttgatga taactcaact 6900
cgaggtcctt aacaacctcc ttatatcaga agacctgcct gccgcagtga agaacatcat 6960
ggcccggacc gatcacccag aacccataca actggcctat aacagttatg aaaaccaaat 7020
tccagtgctg ttcccaaaga tcaaaaatgg tgaggtgaca gacagttatg agaattacac 7080
atatctcaat gcaagaaaat taggagagga cgtgccggca tatgtgtacg ccacagagga 7140
tgaggatcta gcagtggatc ttctgggtat ggattggccg gacccaggca accaacaggt 7200
ggtagagaca gggagggcat taaaacaagt aactggctta tccacagcag aaaacgccct 7260
cttgatagcc ctattcggct acgtcgggta ccagacactt tcaaaaaggc acatacccat 7320
gattactgac atctatacac ttgaagacca caggcttgag gacacaaccc acctccagtt 7380
tgccccaaac gctataagga ccgacggcaa ggactcagag ttgaaggaat tagctgtggg 7440
agaccttgat aaatatgtgg acgcactggt agactactcc aaacaaggga tgaaattcat 7500
caaagtccaa gctgaaaagg tcagagactc ccagtctacg aaggaaggct tgcaaaccat 7560
taaggagtat gtggataagt ttatacaatc actaacagag aataaggagg agatcatcag 7620
gtatggacta tggggagttc acacggcact ctacaaaagc ttggcagcga gactggggca 7680
tgaaacagct tttgcaactt tagtggtaaa atggttggct tttgggggcg aaacggtatc 7740
tgctcacatc aagcaagtag cagttgatct agtagtatat tatatcatca acaaaccatc 7800
ttttcctgga gatacagaga cccaacaaga ggggaggaag tttgtggcta gtctttttat 7860
atctgcacta gcaacataca catataaaac ctggaattac aacaatctgc aacgggttgt 7920
cgaacctgcc ttagcttacc tcccatatgc tacaagtgcc ttgaagttgt tcacacccac 7980

aagattagag agtgtggtca tactcagttc tacaatttac aagacatacc tctctataag 8040
gaagggtaag agtgacggct tgttaggtac aggcataagt gcagccatgg agatcttaaa 8100
ccaaaaccca atctcagtag gtatatctgt gatgctgggg gtaggtgcca tcgccgccca 8160
taatgcaata gaatctagtg aacagaaaag aactttgctg atgaaggtct ttgtaaaaaa 8220
cttcttagac caagcagcaa cagatgagct agtcaaagag aaccctgaaa aaataatcat 8280
ggctctattt gaagcagtcc agaccatagg aaacccccta agactcatct accatctgta 8340
cggggtgtac tataaggggt gggaagcaaa agaactcgca gagaaaactg ctggccgcaa 8400
cttattcaca ttgatcatgt ttgaggcctt tgagctttta ggtatggact cagaaggaaa 8460
gataagaaac ttgtcaggca actacatact ggacttaatc ttcaacttgc ataataaatt 8520
aaacaagggg ctcaaaaaac tagtccttgg gtgggctcct gcacctttga gctgtgattg 8580
gacaccaagt gatgagagaa taagcctacc tcataacaac tacttaaggg tagaaaccag 8640
gtgtccttgt ggctatgaga tgaaggcaat aaaaaatgtt gctggtaaat tgacaaaagt 8700
tgaagaaaag gggtccttcc tatgcaggaa tagattaggg agaggacctc caaacttcaa 8760
agtaacaaag ttctatgatg ataacttgat agaagtcaag ccagtagcta ggctagaagg 8820
ccaggtggac ctctattaca agggagtaac agctaagtta gactacaaca atgggaaagt 8880
actgttagct accaacaagt gggaggtgga ccacgctttc ctgaccagac tagtaaagaa 8940
gcacacaggg ataggtttta aaggtgcata tttgggtgac cgaccagacc atcaagatct 9000
tgtcgataga gattgtgcaa ctataacgaa gaactcagta cagttcctaa aaatgaagaa 9060
gggttgcgct ttcacatatg acctaacaat ctctaacctt gtcaggctta ttgaactagt 9120
ccataagaat aatttacaag aaagagagat ccctaccgtg acagtaacta cttggcttgc 9180
atattctttt gtcaatgaag acctggggac tatcaagcct gtattggggg agaaagtcat 9240
cccagaaccc cccgaggagt tgagtctcca acccaccgtg agactagtca ccactgaaac 9300
agcaataacc ataacagggg aggctgaagt gatgacgaca gggatcacac cagtggtaga 9360
gatgaaagaa gaacctcagc tggaccacca gtcaactacc ctaaaggtag ggttgaagga 9420
aggggaatat ccagggccag gagttaaccc taaccattta gcagaggtga tagatgagaa 9480
agatgacagg cctttttgtcc taatcatcgg taacaaaggt tctacctcga acagagcaag 9540
aacggccaag aatatacggc tgtacaaagg aaacaaccca agagagatca gggatctgat 9600
gagccaagga agaatattga cggttgctct aaaagagttg gacccggaat taaaagaatt 9660
agtagattac aaggggacct ttctcaatag ggaagcttta gaagccctaa gcttaggtaa 9720
gccaatcaag aggaaaacca caacagcaat gatcaggagg ttaatagagc cagaggttga 9780
ggaggaacta ccagattggt tccaagcgga agaacccccta tttttggaag caaaaataca 9840
gaatgactta taccacctaa ttggcagtgt agatagtata aaaagcaaag caaaggaatt 9900
aggggccaca gataacacaa agatagtgaa ggaagttggg gctaggacct atacgatgaa 9960
attgagcagc tggagcacac aagttacaaa aaaacagatg agtctagccc ctctctttga 10020
agagctgtta ttaaagtgcc ctccatgtag taaaatttca aagggacata tggtgtcagc 10080
ataccaactg gctcaaggaa actgggaacc cctcgggtgt ggggtctata tgggaaccat 10140
accagctagg cgtctcaaga tccaccctta tgaggcttac cttaaactca aagagctggt 10200
ggaagttgaa tcttcgaggg ccactgcaaa agaatccatc ataagagaac ataacacctg 10260
gatcctgcgg aaggtgagac atgaagggaa cctaagaacc aaatcaatga tcaaccctgg 10320
gaaaatatca gatcagctat gcagagatgg acacaaaaga aacatatata ataagatcat 10380
aggctcaaca atggcctctg ctggtattag gctggagaaa ctgccagtag tccgagccca 10440
aactgacaca accagtttcc accaagccat aagagaaaaa attgataaaa cagaaaacaa 10500
gcagacccct gaattgcatg aagaactaat gaaggtcttc gactgcttaa agatcccaga 10560
gctgaaggaa tcgtatgatg aagtttcatg ggaacaatta gaagccggga taaaccgtaa 10620
gggtgcagca ggctatctag agagcaagaa catagggggaa gtcctagaca cagagaaaca 10680
catagtagag cagctgatca aggatctgag gaaggggaag aagattaggt actatgaaac 10740
agccatcccc aagaatgaga agagagacgt cagcgacgac tgggaagccg gagagttcgt 10800
tgatgaaaag aaaccaagag taatccagta cccggacgcc aaggtgagac tggccattac 10860
aaaagtgatg tacaaatggg taaagcaaaa accagtggtg atacccggct atgaaggtaa 10920
aacacctcta tttgacatat tcaacaaagt gaagaaggaa tgggattcat tccaggaccc 10980
cgtagcagtg agctttgaca ccaaagcgtg ggatacacaa gtcaccagta gagacctaat 11040
gttgataaag gatatccaga aatattattt caagagaagt atacacaaat ttttagatac 11100
aataacagaa cacatggtgg aggtacctgt cattacagca gacggtgaag tttacataag 11160
gaatggtcag aggggtagtg gccaacccga cacaagtgct ggtaatagta tgttgaatgt 11220
cctaaccatg atatatgctt tctgtaaaag tacaggcata ccttacaggg gattcagcag 11280
agtggcaaga atccatgtgt gtggtgatga tggctttttg ataacagaga gaggactggg 11340
actgaaattc tctgagaagg gtatgcagat attacatgag gccgggaagc cccagaaaat 11400

```
aactgaaggg gacaaaatga aagtggcata cagattcgag gacatagagt tttgttccca 11460
tactcccgtg ccagtcagat gggcagataa caccagtagt tacatggcag ggaggagcac 11520
agccactata ctagctaaga tggcaaccag gctggattcc agcggagaga ggggtagcac 11580
agcttatgag aaggccgtag ccttcagctt ccttttgatg tactcatgga atcccgtagt 11640
tagaaggatc tgcttactgg tgttgtcaca gtttccagaa atatccccat ccaaaaacac 11700
aatatactac taccaagggg atcccatagc tgcgtacaga gaagtgatag ggaaacagct 11760
gtgtgaactg aaaagaacag gatttgagaa gctggctggt ctgaatttga gtatgaccac 11820
tctaggcatc tggacaaaac atactagtaa aagactaatc caagcctgtg tagaaatagg 11880
taagagagaa ggtacctggt tagttaatgc tgacagactg attgcaggaa agactgggaa 11940
gttttacatc ccaagcactg gtgtcactct gttgggaaaa cactatgagg aaattaactt 12000
aaagcaaaag gcggcacaac cgccgataga ggggggttgac agatataagt tgggccccat 12060
agttaatgtt atcttgagaa ggctgagggt gatgctgatg acagttgcca gcggaagctg 12120
gtgaatccgt ccggagcgtc gtgccctcac tcaaggtttt taattgtaaa tattgtaaat 12180
agacagctaa gatatttatt gtagttggat agtaatgcag tgatagtaaa taccccaatt 12240
taacactacc tccaatgcac taagcacttt agctgtgtga ggttaactcg acgtccacgg 12300
ttggactagg gaagacctct aacagcccc                                   12329
```

<210> 4
<211> 11837
<212> DNA
<213> Artificial Sequence: Mutated BVDV: XIKE-B-NdN

<400> 4

```
gtatacgaga ttagctaaag aactcgtata tggattggac gtcaacaaat ttttaattgg     60
caacgtaggg aaccttcccc tcagcgaagg ccgaaaagag gctagccatg cccttagtag    120
gactagcaaa agtaggggac tagcggtagc agtgagttcg ttggatggcc gaacccctga    180
gtacagggga gtcgtcaatg gttcgacact ccattagtcg aggagtctcg agatgccatg    240
tggacgaggg catgcccacg gcacatctta acccatgcgg gggttgcatg ggtgaaagcg    300
ctattcgtgg cgttatggac acagcctgat agggtgtagc agagacctgc tattccgcta    360
gtaaaaactc tgctgtacat ggcacatgga gttgttttcc gatgaaggga gcaagggtgc    420
tacaagtaag aagcagccta agccagatag gatagaaaaa ggtaagatga aaatagcccc    480
aaaagagaca gaaaaagatt gcaaaaccag acccccgac gcgactatag tagtagaagg     540
ggttaagtac caggtgaaga aaaaaggaaa ggtaagggga aaaaatactc aagatgggtt    600
atatcacaac aagaataagc cccctgaatc aagaaaaaaa ttggaaaagg cactgctggc    660
ttgggccatc ttagcagcgg tcctgcttca gctggtaaca ggagagaata tcacccagtg    720
gaacttgatg gacaacggca ccgagggaat acagcaagcg atgttcctaa gaggggtgaa    780
caggagtcta catggaattt ggccagagaa aatttgcacc ggagtaccaa ctcacttagc    840
aacagactat gagcttaaag agatagtggg gatgatggac gcgagtgaga agaccaacta    900
cacgtgttgc aggttgcaaa gacatgagtg gaataaaggt tggtgtaact ggtttcatat    960
agaaccgtgg atatggttga tgaacaaaac ccaaaacaac ctgacagaag ggcaaccgct   1020
tagggagtgt gctgtgactt gtaggtatga caaggaaaca gaattgaaca tcgtgacaca   1080
ggctagggac agacctacaa ctctgacagg ttgcaagaaa ggcaagaatt tctctttcgc   1140
aggtgttata ctggatgggc cctgtaactt taaagtatcg gttgaagatg tgctgttcaa   1200
ggagcacgat tgcggcaaca tgctgcaaga gaccgcgata cagctactcg atggggcaac   1260
caacaccatt gagggagcaa gggtagggac ggccaagttg acaacctggt tagggaagca   1320
attagggatc cttggtaaga agttggagaa caaaagcaaa gcatggtttg gtgcacatgc   1380
agcaagtcca tactgcggag tggagaggaa gatcggttac gtatggtata caaaaaactg   1440
cactccagct tgccttccaa gaaacactag aataataggc cccgggaaat ttgataccaa   1500
cgccgaagat ggaaaaatac tccatgagat ggggggcac ctctcagaat ttgtcctatt    1560
gtccttggtg gttctgtctg actttgcccc ggaaaccgcg agcgtcatct acttggttct   1620
acattttgcg atcccgcaaa gccacgttga tgtagacaca tgcgacaaga accagctgaa   1680
tttaacggta gcaaccacag tagcagaggt cataccaggg acagtgtgga acctagggaa   1740
gtatgtctgc ataagaccag actggtggcc atatgagacg acgacagtct tcgtcataga   1800
ggaagcaggg caagtaatca aattgatgct aagggccatc agagacttaa ctaggatatg   1860
gaatgctgcc actaccacag ctttcttaat ctttttagta aaagcactga ggggacaact   1920
aatccaaggg ctattgtggc tgatgctaat aacaggagca cagggcttcc ctgaatgcaa   1980
```

agagggcttc caatatgcca tatctaaaga caggaaaatg gggttattgg ggccagagag 2040
cttaactaca acatggcacc tccccaccaa aaaaatagtg gattccatgg tgcatgtatg 2100
gtgtgaagga aaagacttga aaatattaaa aatgtgcaca aaggaagaga ggtatctagt 2160
ggctgtgcac gagagagcct tatcaaccag tgccgagttt atgcagatca gtgatgggac 2220
aataggccca gacgtgatag atatgcctga tgactttgag tttggactct gcccttgtga 2280
ctcaaaacca gtgataaagg gcaaatttaa tgccagctta ctgaatggac cagctttcca 2340
gatggtatgc ccacaggggt ggactggtac aatagaatgc accctagcga accaagacac 2400
cttggacaca actgtcatta ggacatatag aagaactacc ccatttcagc ggagaaaatg 2460
gtgtacctat gaaaaaataa taggggaaga tatctatgaa tgcattctag gtggaaactg 2520
gacatgcata accggtgacc atagcaggtt gaaagacgga cctatcaaga agtgtaagtg 2580
gtgtggccat gacttcgtca actcagaggg gctaccacac tacccaatag gcaagtgcat 2640
gctcatcaac gagagtgggt acaggtatgt agatgacacc tcttgcgata ggggtggtgt 2700
agccatagtt ccatctggca ccgtaaagtg tagaataggt aacgtcacgg tgcaagttat 2760
cgctactaac aatgatctgg gacccatgcc ttgcagccca gctgaagtga tagcaagtga 2820
aggaccagtg gaaaagactg catgcacatt caactattca aggactctac ctaataagta 2880
ttatgagcca agggaccggt acttccaaca atacatgtta aaaggggagt ggcaatattg 2940
gttcgacctg gattctgtag accaccacaa agactacttc tcagagttca taatcatagc 3000
agtggtcgcc ttgttgggtg gtaagtacgt actgtggctc ttgataacat acacaatact 3060
gtctgagcag atggctatgg gtgctggagt gaatactgaa gagatagtca tgataggcaa 3120
tttgctgaca gacagtgata ttgaggttgt ggtttatttc cttcttctgt acttaatagt 3180
taaagaggaa ctggcgagga aatggattat actggtatac cacatccttg tagccaaccc 3240
tatgaaaaca attggggtcg tcttactaat gctaggggga gtggtgaagg ccagcagaat 3300
caatgctgat gaccaaagtg ctatggaccc atgctttctt ctcgtgacag gcgtagtggc 3360
tgttttgatg atcgctagaa gagaacctgc cacattacca ctgattgtag cattgctagc 3420
aataagaaca tcaggattcc tactgcccgc tagcattgat gtaactgtag cagtagtatt 3480
aattgtactt ttgttggcta gctacataac agactacttt agatataaaa agtggcttca 3540
actcttattt agtctgatag ctggtatctt tattataagg agcttaaaac atatcaacca 3600
gatggaggta ccagaaatat ctatgccaag ttggagacct ctagctctgg tccttttcta 3660
tataacatct acagcaataa ccactaattg ggacattgac ttagcaggct tcctgctgca 3720
atgggcgcca gcagtgatca tgatggctac catgtgggca gactttttga ctctgatcat 3780
agtcctgccc agttacgagt tatctaagct ttacttccta aagaacgtca ggacagacgt 3840
ggaaaagaac tggctcggca aagtgaaata cagacagatc agttcagttt atgacatctg 3900
tgacagtgag gaagcagtgt acctatttcc atcaaggcat aagagtggaa gcaggccaga 3960
tttcatatta ccttttttga aagccgtgtt aataagctgc atcagcagcc aatggcaagt 4020
ggtttacatt tcttacctaa tactggaaat tacatactat atgcacagga aaatcataga 4080
tgaggtgtca ggaggagcaa attttctatc aagactcata gcagccatca tagaattaaa 4140
ttgggccata gatgatgagg aatgtaaagg actgaagaaa ctgtatctct tgtcagggag 4200
agcgaagaat ttgatagtta aacataaggt aagaaatgaa gccgtccaca gatggtttgg 4260
tgaggaggaa atatacgggg cacccaaggt gatcactatc ataaaagcta gtaccctaag 4320
taaaaacagg cactgcataa tctgcacgat ctgtgaaggg aaagaatgga atggagccaa 4380
ctgcccaaag tgtggaagac aaggaaagcc cataacatgt ggaatgacac tcgcagactt 4440
tgaggagaaa cattacaaaa agatatttat aagagaagaa tcttcttgtc ctgtgccttt 4500
tgatccttct tgccattgta attattttcg ccacgatggg cctttcagga aagagtataa 4560
gggttacgtc caatacacag ccagaggaca actctttctg aggaacctac caattctagc 4620
gacgaagatg aagctattaa tggtgggaaa cctcggcgca gaaattggcg acctggaaca 4680
tctaggatgg gtactgagag ggccagccgt gtgcaaaaaa attaccaacc atgagaagtg 4740
ccacgtaaac atcatggata agctaactgc atttttggga atcatgccta gaggcacgac 4800
ccctagggca cctgtgaggt tccccacagc actactaaaa gtgagaaggg ggctagagac 4860
gggatgggct tacacgcacc aaggagggat cagctcggta gaccatgtca cagccggaaa 4920
ggatttacta gtgtgtgaca gtatgggcag gaccagggtt gtctgtcata gtaacaataa 4980
gatgactgat gagactgagt atggcatcaa gaccgactca gggtgtcccg aaggtgcgag 5040
gtgttacgtg ctaaacccag aagctgttaa catttctggc acaaaaggag ctatggtaca 5100
cctccagaaa acgggggggg agttcacatg tgtcactgcc tcagggaccc cggctttctt 5160
cgatctgaaa aatctaaaag gctggtccgg gctaccaatt tttgaagcat ccagtggcag 5220
ggtggttggt agggtgaaag tcggcaagaa tgaggattcc aagcccacca aactaatgag 5280
cggaatccag acagtgtcta agaaccagac agacctagcg gacatcgtaa aaaaattgac 5340
tagtatgaac agaggagagt tcaaacagat aacattagcc actggggcag gaaaaactac 5400

ggaactgcca aggtccgtca tagaggagat agggaggcac aaaagggtct tagtcctgat 5460
accattgaga gcagcagcag agtcagtgta tcagtatatg agagtgaagt acccaagtat 5520
atctttcaat ttgagaatag gagatatgaa ggaaggtgac atggccactg gtatcaccta 5580
cgcctcatat gggtactttt gtcagcttcc tcagcccaaa ctgagagctg ccatggtaga 5640
gtactcatat atattcttag atgagtacca ctgtgctaca cccgagcaat tagcaataat 5700
tggaaagata cacaggtttg ctgaaaatct tagagtggta gcaatgacag caaccccagc 5760
tggaacggtc acaacgactg gtcagaaaca ccctatagag gagttcatag ccccagaggt 5820
gatgaaaggt gaagatctag gtagtgaata cttggatatt gcagggttga agataccgac 5880
tgaagagatg aaaggcaaca tgctcgtgtt cgcgccaact aggaacatgg cagtagaaac 5940
agctaagaaa ttgaaggcta agggatacaa ctctggatac tattacagtg gggaaaaccc 6000
agagaacttg agggtggtaa cctcgcaatc cccgtatgtg gtagtagcca ccaatgccat 6060
agagtcaggt gtgacattac cagacttaga cacagttgta gacactggac taaagtgtga 6120
gaagagggtg aggatttctt caaaaatgcc cttcattgta acaggactta agagaatggc 6180
agtcacaatc ggagagcaag cccagcgcag ggtagagta ggaagagtca agccaggtag 6240
gtactatagg agtcaagaaa cagcttcagg gtcaaaagat taccattacg acctactgca 6300
agcccagagg tacggaatag aagatggaat taatgtaaca aagtcattca gggagatgaa 6360
ctatgattgg agcctttacg aagaggacag cttgatgata actcaactcg aggtccttaa 6420
caacctcctt atatcagaag acctgcctgc cgcagtgaag aacatcatgg cccggaccga 6480
tcacccagaa cccatacaac tggcctataa cagttatgaa aaccaaattc cagtgctgtt 6540
cccaaagatc aaaaatggtg aggtgacaga cagttatgag aattacacat atctcaatgc 6600
aagaaaatta ggagaggacg tgccggcata tgtgtacgcc acagaggatg aggatctagc 6660
agtggatctt ctgggtatgg attggccgga cccaggcaac caacaggtgg tagagacagg 6720
gagggcatta aaacaagtaa ctggcttatc cacagcagaa aacgccctct tgatagccct 6780
attcggctac gtcgggtacc agacactttc aaaaaggcac atacccatga ttactgacat 6840
ctatacactt gaagaccaca ggcttgagga cacaacccac ctccagtttg ccccaaacgc 6900
tataaggacc gacggcaagg actcagagtt gaaggaatta gctgtgggag accttgataa 6960
atatgtggac gcactggtag actactccaa acaagggatg aaattcatca aagtccaagc 7020
tgaaaaggtc agagactccc agtctacgaa ggaaggcttg caaaccatta aggagtatgt 7080
ggataagttt atacaatcac taacagagaa taaggaggag atcatcaggt atggactatg 7140
gggagttcac acggcactct acaaaagctt ggcagcgaga ctggggcatg aaacagcttt 7200
tgcaacttta gtggtaaaat ggttggcttt tggggggcgaa acggtatctg ctcacatcaa 7260
gcaagtagca gttgatctag tagtatatta tatcatcaac aaaccatctt ttcctggaga 7320
tacagagacc caacaagagg ggaggaagtt tgtggctagt ctttttatat ctgcactagc 7380
aacatacaca tataaaacct ggaattacaa caatctgcaa cgggttgtcg aacctgcctt 7440
agcttacctc ccatatgcta caagtgcctt gaagttgttc acacccacaa gattagagag 7500
tgtggtcata ctcagttcta caatttacaa gacatacctc tctataagga agggtaagag 7560
tgacggcttg ttaggtacag gcataagtgc agccatggag atcttaaacc aaaacccaat 7620
ctcagtaggt atatctgtga tgctgggggt aggtgccatc gccgcccata atgcaataga 7680
atctagtgaa cagaaaagaa ctttgctgat gaaggtcttt gtaaaaaact tcttagacca 7740
agcagcaaca gatgagctag tcaaagagaa ccctgaaaaa ataatcatgg ctctatttga 7800
agcagtccag accataggaa acccccctaag actcatctac catctgtacg ggtgtacta 7860
taaggggtgg gaagcaaaag aactcgcaga gaaaactgct ggccgcaact tattcacatt 7920
gatcatgttt gaggcctttg agcttttagg tatggactca gaaggaaaga taagaaactt 7980
gtcaggcaac tacatactgg acttaatctt caacttgcat aataaattaa acaaggggct 8040
caaaaaacta gtccttgggt gggctcctgc acctttgagc tgtgattgga caccaagtga 8100
tgagagaata agcctacctc ataacaacta cttaagggta gaaaccaggt gtccttgtgg 8160
ctatgagatg aaggcaataa aaaatgttgc tggtaaattg acaaaagttg aagaaaaggg 8220
gtccttccta tgcaggaata gattagggag aggacctcca aacttcaaag taacaaagtt 8280
ctatgatgat aacttgatag aagtcaagcc agtagctagg ctagaaggcc aggtggacct 8340
ctattacaag ggagtaacag ctaagttaga ctacaacaat gggaaagtac tgttagctac 8400
caacaagtgg gaggtggacc acgctttcct gaccagacta gtaaagaagc acacagggat 8460
aggttttaaa ggtgcatatt tgggtgaccg accagaccat caagatcttg tcgatagaga 8520
ttgtgcaact ataacgaaga actcagtaca gttcctaaaa atgaagaagg gttgcgcttt 8580
cacatatgac ctaacaatct ctaaccttgt caggcttatt gaactagtcc ataagaataa 8640
tttacaagaa agagagatcc ctaccgtgac agtaactact tggcttgcat attcttttgt 8700
caatgaagac ctggggacta tcaagcctgt attgggggag aaagtcatcc cagaaccccc 8760
cgaggagttg agtctccaac ccaccgtgag actagtcacc actgaaacag caataaccat 8820

```
aacaggggag gctgaagtga tgacgacagg gatcacacca gtggtagaga tgaaagaaga   8880
acctcagctg gaccaccagt caactaccct aaaggtaggg ttgaaggaag gggaatatcc   8940
agggccagga gttaacccta accatttagc agaggtgata gatgagaaag atgacaggcc   9000
ttttgtccta atcatcggta acaaaggttc tacctcgaac agagcaagaa cggccaagaa   9060
tatacggctg tacaaaggaa acaacccaag agagatcagg gatctgatga gccaaggaag   9120
aatattgacg gttgctctaa aagagttgga cccggaatta aaagaattag tagattacaa   9180
ggggaccttt ctcaataggg aagctttaga agccctaagc ttaggtaagc caatcaagag   9240
gaaaaccaca acagcaatga tcaggaggtt aatagagcca gaggttgagg aggaactacc   9300
agattggttc caagcggaag aacccctatt tttggaagca aaaatacaga atgacttata   9360
ccacctaatt ggcagtgtag atagtataaa aagcaaagca aaggaattag gggccacaga   9420
taacacaaag atagtgaagg aagttggggc taggacctat acgatgaaat tgagcagctg   9480
gagcacacaa gttacaaaaa aacagatgag tctagcccct ctctttgaag agctgttatt   9540
aaagtgccct ccatgtagta aaatttcaaa gggacatatg gtgtcagcat accaactggc   9600
tcaaggaaac tgggaacccc tcgggtgtgg ggtctatatg ggaaccatac cagctaggcg   9660
tctcaagatc caccettatg aggcttacct taaactcaaa gagctggtgg aagttgaatc   9720
ttcgagggcc actgcaaaag aatccatcat aagagaacat aacacctgga tcctgcggaa   9780
ggtgagacat gaagggaacc taagaaccaa atcaatgatc aaccctggga aaatatcaga   9840
tcagctatgc agagatggac acaaaagaaa catatataat aagatcatag gctcaacaat   9900
ggcctctgct ggtattaggc tggagaaact gccagtagtc cgagcccaaa ctgacacaac   9960
cagtttccac caagccataa gagaaaaaat tgataaaaca gaaaacaagc agacccctga  10020
attgcatgaa gaactaatga aggtcttcga ctgcttaaag atcccagagc tgaaggaatc  10080
gtatgatgaa gtttcatggg aacaattaga agccgggata aaccgtaagg gtgcagcagg  10140
ctatctagag agcaagaaca taggggaagt cctagacaca gagaaacaca tagtagagca  10200
gctgatcaag gatctgagga aggggaagaa gattaggtac tatgaaacag ccatccccaa  10260
gaatgagaag agagacgtca gcgacgactg ggaagccgga gagttcgttg atgaaaagaa  10320
accaagagta atccagtacc cggacgccaa ggtgagactg gccattacaa aagtgatgta  10380
caaatgggta aagcaaaaac cagtggtgat acccggctat gaaggtaaaa cacctctatt  10440
tgacatattc aacaaagtga agaaggaatg ggattcattc caggaccccg tagcagtgag  10500
ctttgacacc aaagcgtggg atacacaagt caccagtaga gacctaatgt tgataaagga  10560
tatccagaaa tattatttca agagaagtat acacaaattt ttagatacaa taacagaaca  10620
catggtggag gtacctgtca ttacagcaga cggtgaagtt tacataagga atggtcagag  10680
gggtagtggc caacccgaca caagtgctgg taatagtatg ttgaatgtcc taaccatgat  10740
atatgctttc tgtaaaagta caggcatacc ttacagggga ttcagcagag tggcaagaat  10800
ccatgtgtgt ggtgatgatg gctttttgat aacagagaga ggactgggac tgaaattctc  10860
tgagaagggt atgcagatat tacatgaggc cgggaagccc cagaaaataa ctgaagggga  10920
caaaatgaaa gtggcataca gattcgagga catagagttt tgttcccata ctcccgtgcc  10980
agtcagatgg gcagataaca ccagtagtta catggcaggg aggagcacag ccactatact  11040
agctaagatg gcaaccaggc tggattccag cggagagagg ggtagcacag cttatgagaa  11100
ggccgtagcc ttcagcttcc ttttgatgta ctcatggaat cccgtagtta gaaggatctg  11160
cttactggtg ttgtcacagt ttccagaaat atccccatcc aaaaacacaa tatactacta  11220
ccaaggggat cccatagctg cgtacagaga agtgataggg aaacagctgt gtgaactgaa  11280
aagaacagga tttgagaagc tggctggtct gaatttgagt atgaccactc taggcatctg  11340
gacaaaacat actagtaaaa gactaatcca agcctgtgta gaaataggta agagagaagg  11400
tacctggtta gttaatgctg acagactgat tgcaggaaag actgggaagt tttacatccc  11460
aagcactggt gtcactctgt tgggaaaaca ctatgaggaa attaacttaa agcaaaaggc  11520
ggcacaaccg ccgatagagg gggttgacag atataagttg ggccccatag ttaatgttat  11580
cttgagaagg ctgagggtga tgctgatgac agttgccagc ggaagctggt gaatccgtcc  11640
ggagcgtcgt gccctcactc aaggttttta attgtaaata ttgtaaatag acagctaaga  11700
tatttattgt agttggatag taatgcagtg atagtaaata ccccaattta acactacctc  11760
caatgcacta agcactttag ctgtgtgagg ttaactcgac gtccacggtt ggactaggga  11820
agacctctaa cagcccc                                                  11837
```

<210> 5
<211> 3913
<212> PRT
<213> Wildtyp BVDV: XIKE-A

<400> 5

```
Met Glu Leu Phe Ser Asn Glu Leu Leu Tyr Lys Thr Tyr Lys Gln Lys
1               5                   10                  15

Pro Ala Gly Val Val Glu Pro Val Tyr Asp Val Asn Gly Arg Pro Leu
            20                  25                  30

Phe Gly Glu Ser Ser Asp Leu His Pro Gln Ser Thr Leu Lys Leu Pro
        35                  40                  45

His Gln Arg Gly Ser Ala Asn Ile Leu Thr Asn Ala Arg Ser Leu Pro
    50                  55                  60

Arg Lys Gly Asp Cys Arg Arg Gly Asn Val Tyr Gly Pro Val Ser Gly
65                  70                  75                  80

Ile Tyr Ile Lys Pro Gly Pro Ile Tyr Tyr Gln Asp Tyr Val Gly Pro
                85                  90                  95

Val Tyr His Arg Ala Pro Leu Glu Leu Cys Arg Glu Ala Ser Met Cys
            100                 105                 110

Glu Thr Thr Arg Arg Val Gly Arg Val Thr Gly Ser Asp Gly Lys Leu
        115                 120                 125

Tyr His Ile Tyr Ile Cys Ile Asp Gly Cys Ile Leu Leu Lys Arg Ala
    130                 135                 140

Thr Arg Asn Gln Pro Glu Val Leu Lys Trp Val Tyr Asn Arg Leu Asn
145                 150                 155                 160

Cys Pro Leu Trp Val Thr Ser Cys Ser Asp Glu Gly Ser Lys Gly Ala
                165                 170                 175

Thr Ser Lys Lys Gln Pro Lys Pro Asp Arg Ile Glu Lys Gly Lys Met
            180                 185                 190

Lys Ile Ala Pro Lys Glu Thr Glu Lys Asp Cys Lys Thr Arg Pro Pro
        195                 200                 205

Asp Ala Thr Ile Val Val Glu Gly Val Lys Tyr Gln Val Lys Lys Lys
    210                 215                 220

Gly Lys Val Arg Gly Lys Asn Thr Gln Asp Gly Leu Tyr His Asn Lys
225                 230                 235                 240

Asn Lys Pro Pro Glu Ser Arg Lys Lys Leu Glu Lys Ala Leu Leu Ala
                245                 250                 255

Trp Ala Ile Leu Ala Ala Val Leu Leu Gln Leu Val Thr Gly Glu Asn
            260                 265                 270

Ile Thr Gln Trp Asn Leu Met Asp Asn Gly Thr Glu Gly Ile Gln Gln
        275                 280                 285
```

Ala Met Phe Leu Arg Gly Val Asn Arg Ser Leu His Gly Ile Trp Pro
290 295 300

Glu Lys Ile Cys Thr Gly Val Pro Thr His Leu Ala Thr Asp Tyr Glu
305 310 315 320

Leu Lys Glu Ile Val Gly Met Met Asp Ala Ser Glu Lys Thr Asn Tyr
325 330 335

Thr Cys Cys Arg Leu Gln Arg His Glu Trp Asn Lys His Gly Trp Cys
340 345 350

Asn Trp Phe His Ile Glu Pro Trp Ile Trp Leu Met Asn Lys Thr Gln
355 360 365

Asn Asn Leu Thr Glu Gly Gln Pro Leu Arg Glu Cys Ala Val Thr Cys
370 375 380

Arg Tyr Asp Lys Glu Thr Glu Leu Asn Ile Val Thr Gln Ala Arg Asp
385 390 395 400

Arg Pro Thr Thr Leu Thr Gly Cys Lys Lys Gly Lys Asn Phe Ser Phe
405 410 415

Ala Gly Val Ile Leu Asp Gly Pro Cys Asn Phe Lys Val Ser Val Glu
420 425 430

Asp Val Leu Phe Lys Glu His Asp Cys Gly Asn Met Leu Gln Glu Thr
435 440 445

Ala Ile Gln Leu Leu Asp Gly Ala Thr Asn Thr Ile Glu Gly Ala Arg
450 455 460

Val Gly Thr Ala Lys Leu Thr Thr Trp Leu Gly Lys Gln Leu Gly Ile
465 470 475 480

Leu Gly Lys Lys Leu Glu Asn Lys Ser Lys Ala Trp Phe Gly Ala His
485 490 495

Ala Ala Ser Pro Tyr Cys Gly Val Glu Arg Lys Ile Gly Tyr Val Trp
500 505 510

Tyr Thr Lys Asn Cys Thr Pro Ala Cys Leu Pro Arg Asn Thr Arg Ile
515 520 525

Ile Gly Pro Gly Lys Phe Asp Thr Asn Ala Glu Asp Gly Lys Ile Leu
530 535 540

His Glu Met Gly Gly His Leu Ser Glu Phe Val Leu Leu Ser Leu Val
545 550 555 560

Val Leu Ser Asp Phe Ala Pro Glu Thr Ala Ser Val Ile Tyr Leu Val
565 570 575

Leu His Phe Ala Ile Pro Gln Ser His Val Asp Val Asp Thr Cys Asp
580 585 590

Lys Asn Gln Leu Asn Leu Thr Val Ala Thr Thr Val Ala Glu Val Ile
595 600 605

Pro Gly Thr Val Trp Asn Leu Gly Lys Tyr Val Cys Ile Arg Pro Asp
610 615 620

Trp Trp Pro Tyr Glu Thr Thr Thr Val Phe Val Ile Glu Glu Ala Gly
625 630 635 640

Gln Val Ile Lys Leu Met Leu Arg Ala Ile Arg Asp Leu Thr Arg Ile
645 650 655

Trp Asn Ala Ala Thr Thr Thr Ala Phe Leu Ile Phe Leu Val Lys Ala
660 665 670

Leu Arg Gly Gln Leu Ile Gln Gly Leu Leu Trp Leu Met Leu Ile Thr
675 680 685

Gly Ala Gln Gly Phe Pro Glu Cys Lys Glu Gly Phe Gln Tyr Ala Ile
690 695 700

Ser Lys Asp Arg Lys Met Gly Leu Leu Gly Pro Glu Ser Leu Thr Thr
705 710 715 720

Thr Trp His Leu Pro Thr Lys Lys Ile Val Asp Ser Met Val His Val
725 730 735

Trp Cys Glu Gly Lys Asp Leu Lys Ile Leu Lys Met Cys Thr Lys Glu
740 745 750

Glu Arg Tyr Leu Val Ala Val His Glu Arg Ala Leu Ser Thr Ser Ala
755 760 765

Glu Phe Met Gln Ile Ser Asp Gly Thr Ile Gly Pro Asp Val Ile Asp
770 775 780

Met Pro Asp Asp Phe Glu Phe Gly Leu Cys Pro Cys Asp Ser Lys Pro
785 790 795 800

Val Ile Lys Gly Lys Phe Asn Ala Ser Leu Leu Asn Gly Pro Ala Phe
805 810 815

Gln Met Val Cys Pro Gln Gly Trp Thr Gly Thr Ile Glu Cys Thr Leu
820 825 830

Ala Asn Gln Asp Thr Leu Asp Thr Thr Val Ile Arg Thr Tyr Arg Arg
835 840 845

Thr Thr Pro Phe Gln Arg Arg Lys Trp Cys Thr Tyr Glu Lys Ile Ile
850 855 860

Gly Glu Asp Ile Tyr Glu Cys Ile Leu Gly Gly Asn Trp Thr Cys Ile
865 870 875 880

Thr Gly Asp His Ser Arg Leu Lys Asp Gly Pro Ile Lys Lys Cys Lys
885 890 895

Trp Cys Gly His Asp Phe Val Asn Ser Glu Gly Leu Pro His Tyr Pro
900 905 910

Ile Gly Lys Cys Met Leu Ile Asn Glu Ser Gly Tyr Arg Tyr Val Asp
915 920 925

Asp Thr Ser Cys Asp Arg Gly Gly Val Ala Ile Val Pro Ser Gly Thr
930 935 940

Val Lys Cys Arg Ile Gly Asn Val Thr Val Gln Val Ile Ala Thr Asn
945 950 955 960

Asn Asp Leu Gly Pro Met Pro Cys Ser Pro Ala Glu Val Ile Ala Ser
965 970 975

Glu Gly Pro Val Glu Lys Thr Ala Cys Thr Phe Asn Tyr Ser Arg Thr
980 985 990

Leu Pro Asn Lys Tyr Tyr Glu Pro Arg Asp Arg Tyr Phe Gln Gln Tyr
995 1000 1005

Met Leu Lys Gly Glu Trp Gln Tyr Trp Phe Asp Leu Asp Ser Val
1010 1015 1020

Asp His His Lys Asp Tyr Phe Ser Glu Phe Ile Ile Ile Ala Val
1025 1030 1035

Val Ala Leu Leu Gly Gly Lys Tyr Val Leu Trp Leu Leu Ile Thr
1040 1045 1050

Tyr Thr Ile Leu Ser Glu Gln Met Ala Met Gly Ala Gly Val Asn
1055 1060 1065

Thr Glu Glu Ile Val Met Ile Gly Asn Leu Leu Thr Asp Ser Asp
1070 1075 1080

Ile Glu Val Val Val Tyr Phe Leu Leu Leu Tyr Leu Ile Val Lys
1085 1090 1095

Glu Glu Leu Ala Arg Lys Trp Ile Ile Leu Val Tyr His Ile Leu
1100 1105 1110

Val Ala Asn Pro Met Lys Thr Ile Gly Val Val Leu Leu Met Leu
1115 1120 1125

Gly Gly Val Val Lys Ala Ser Arg Ile Asn Ala Asp Asp Gln Ser
1130 1135 1140

Ala Met Asp Pro Cys Phe Leu Leu Val Thr Gly Val Val Ala Val
1145 1150 1155

Leu Met Ile Ala Arg Arg Glu Pro Ala Thr Leu Pro Leu Ile Val
1160 1165 1170

Ala Leu Leu Ala Ile Arg Thr Ser Gly Phe Leu Leu Pro Ala Ser
1175 1180 1185

Ile Asp Val Thr Val Ala Val Val Leu Ile Val Leu Leu Leu Ala
1190 1195 1200

Ser Tyr Ile Thr Asp Tyr Phe Arg Tyr Lys Lys Trp Leu Gln Leu
1205 1210 1215

Leu Phe Ser Leu Ile Ala Gly Ile Phe Ile Ile Arg Ser Leu Lys
1220 1225 1230

His Ile Asn Gln Met Glu Val Pro Glu Ile Ser Met Pro Ser Trp
1235 1240 1245

Arg Pro Leu Ala Leu Val Leu Phe Tyr Ile Thr Ser Thr Ala Ile
1250 1255 1260

Thr Thr Asn Trp Asp Ile Asp Leu Ala Gly Phe Leu Leu Gln Trp
1265 1270 1275

Ala Pro Ala Val Ile Met Met Ala Thr Met Trp Ala Asp Phe Leu
1280 1285 1290

Thr Leu Ile Ile Val Leu Pro Ser Tyr Glu Leu Ser Lys Leu Tyr
1295 1300 1305

Phe Leu Lys Asn Val Arg Thr Asp Val Glu Lys Asn Trp Leu Gly
1310 1315 1320

Lys Val Lys Tyr Arg Gln Ile Ser Ser Val Tyr Asp Ile Cys Asp
1325 1330 1335

Ser Glu Glu Ala Val Tyr Leu Phe Pro Ser Arg His Lys Ser Gly
1340 1345 1350

Ser Arg Pro Asp Phe Ile Leu Pro Phe Leu Lys Ala Val Leu Ile
1355 1360 1365

Ser Cys Ile Ser Ser Gln Trp Gln Val Val Tyr Ile Ser Tyr Leu
1370 1375 1380

Ile Leu Glu Ile Thr Tyr Tyr Met His Arg Lys Ile Ile Asp Glu
1385 1390 1395

Val Ser Gly Gly Ala Asn Phe Leu Ser Arg Leu Ile Ala Ala Ile
1400 1405 1410

Ile Glu Leu Asn Trp Ala Ile Asp Asp Glu Glu Cys Lys Gly Leu
1415 1420 1425

Lys Lys Leu Tyr Leu Leu Ser Gly Arg Ala Lys Asn Leu Ile Val
1430 1435 1440

Lys His Lys Val Arg Asn Glu Ala Val His Arg Trp Phe Gly Glu
1445 1450 1455

Glu Glu Ile Tyr Gly Ala Pro Lys Val Ile Thr Ile Ile Lys Ala
1460 1465 1470

Ser Thr Leu Ser Lys Asn Arg His Cys Ile Ile Cys Thr Ile Cys
1475 1480 1485

Glu Gly Lys Glu Trp Asn Gly Ala Asn Cys Pro Lys Cys Gly Arg
1490 1495 1500

Gln Gly Lys Pro Ile Thr Cys Gly Met Thr Leu Ala Asp Phe Glu
1505 1510 1515

Glu Lys His Tyr Lys Lys Ile Phe Ile Arg Glu Glu Ser Ser Cys
1520 1525 1530

Pro Val Pro Phe Asp Pro Ser Cys His Cys Asn Tyr Phe Arg His
1535 1540 1545

Asp Gly Pro Phe Arg Lys Glu Tyr Lys Gly Tyr Val Gln Tyr Thr
1550 1555 1560

Ala Arg Gly Gln Leu Phe Leu Arg Asn Leu Pro Ile Leu Ala Thr
1565 1570 1575

Lys Met Lys Leu Leu Met Val Gly Asn Leu Gly Ala Glu Ile Gly
1580 1585 1590

Asp Leu Glu His Leu Gly Trp Val Leu Arg Gly Pro Ala Val Cys
1595 1600 1605

Lys Lys Ile Thr Asn His Glu Lys Cys His Val Asn Ile Met Asp
1610 1615 1620

Lys Leu Thr Ala Phe Phe Gly Ile Met Pro Arg Gly Thr Thr Pro
1625 1630 1635

Arg Ala Pro Val Arg Phe Pro Thr Ala Leu Leu Lys Val Arg Arg
1640 1645 1650

Gly Leu Glu Thr Gly Trp Ala Tyr Thr His Gln Gly Gly Ile Ser
1655 1660 1665

Ser Val Asp His Val Thr Ala Gly Lys Asp Leu Leu Val Cys Asp
1670 1675 1680

Ser Met Gly Arg Thr Arg Val Val Cys His Ser Asn Asn Lys Met
1685 1690 1695

Thr Asp Glu Thr Glu Tyr Gly Ile Lys Thr Asp Ser Gly Cys Pro
1700 1705 1710

Glu Gly Ala Arg Cys Tyr Val Leu Asn Pro Glu Ala Val Asn Ile
1715 1720 1725

Ser Gly Thr Lys Gly Ala Met Val His Leu Gln Lys Thr Gly Gly
1730 1735 1740

Glu Phe Thr Cys Val Thr Ala Ser Gly Thr Pro Ala Phe Phe Asp
1745 1750 1755

Leu Lys Asn Leu Lys Gly Trp Ser Gly Leu Pro Ile Phe Glu Ala
1760 1765 1770

Ser Ser Gly Arg Val Val Gly Arg Val Lys Val Gly Lys Asn Glu
1775 1780 1785

Asp Ser Lys Pro Thr Lys Leu Met Ser Gly Ile Gln Thr Val Ser
1790 1795 1800

Lys Asn Gln Thr Asp Leu Ala Asp Ile Val Lys Lys Leu Thr Ser
1805 1810 1815

Met Asn Arg Gly Glu Phe Lys Gln Ile Thr Leu Ala Thr Gly Ala
1820 1825 1830

Gly Lys Thr Thr Glu Leu Pro Arg Ser Val Ile Glu Glu Ile Gly
1835 1840 1845

Arg His Lys Arg Val Leu Val Leu Ile Pro Leu Arg Ala Ala Ala
1850 1855 1860

Glu Ser Val Tyr Gln Tyr Met Arg Val Lys Tyr Pro Ser Ile Ser
1865 1870 1875

Phe Asn Leu Arg Ile Gly Asp Met Lys Glu Gly Asp Met Ala Thr
1880 1885 1890

Gly Ile Thr Tyr Ala Ser Tyr Gly Tyr Phe Cys Gln Leu Pro Gln
1895 1900 1905

Pro Lys Leu Arg Ala Ala Met Val Glu Tyr Ser Tyr Ile Phe Leu
1910 1915 1920

Asp Glu Tyr His Cys Ala Thr Pro Glu Gln Leu Ala Ile Ile Gly
1925 1930 1935

Lys Ile His Arg Phe Ala Glu Asn Leu Arg Val Val Ala Met Thr
1940 1945 1950

Ala Thr Pro Ala Gly Thr Val Thr Thr Thr Gly Gln Lys His Pro
1955 1960 1965

Ile Glu Glu Phe Ile Ala Pro Glu Val Met Lys Gly Glu Asp Leu
1970 1975 1980

Gly Ser Glu Tyr Leu Asp Ile Ala Gly Leu Lys Ile Pro Thr Glu
1985 1990 1995

Glu Met Lys Gly Asn Met Leu Val Phe Ala Pro Thr Arg Asn Met
2000 2005 2010

Ala Val Glu Thr Ala Lys Lys Leu Lys Ala Lys Gly Tyr Asn Ser
2015 2020 2025

Gly Tyr Tyr Tyr Ser Gly Glu Asn Pro Glu Asn Leu Arg Val Val
2030 2035 2040

Thr Ser Gln Ser Pro Tyr Val Val Val Ala Thr Asn Ala Ile Glu
2045 2050 2055

Ser Gly Val Thr Leu Pro Asp Leu Asp Thr Val Val Asp Thr Gly
2060 2065 2070

Leu Lys Cys Glu Lys Arg Val Arg Ile Ser Ser Lys Met Pro Phe
2075 2080 2085

Ile Val Thr Gly Leu Lys Arg Met Ala Val Thr Ile Gly Glu Gln
2090 2095 2100

Ala Gln Arg Arg Gly Arg Val Gly Arg Val Lys Pro Gly Arg Tyr
2105 2110 2115

Tyr Arg Ser Gln Glu Thr Ala Ser Gly Ser Lys Asp Tyr His Tyr
2120 2125 2130

Asp Leu Leu Gln Ala Gln Arg Tyr Gly Ile Glu Asp Gly Ile Asn
2135 2140 2145

Val Thr Lys Ser Phe Arg Glu Met Asn Tyr Asp Trp Ser Leu Tyr
2150 2155 2160

Glu Glu Asp Ser Leu Met Ile Thr Gln Leu Glu Val Leu Asn Asn
2165 2170 2175

Leu Leu Ile Ser Glu Asp Leu Pro Ala Ala Val Lys Asn Ile Met
2180 2185 2190

Ala Arg Thr Asp His Pro Glu Pro Ile Gln Leu Ala Tyr Asn Ser
2195 2200 2205

Tyr Glu Asn Gln Ile Pro Val Leu Phe Pro Lys Ile Lys Asn Gly
2210 2215 2220

Glu Val Thr Asp Ser Tyr Glu Asn Tyr Thr Tyr Leu Asn Ala Arg
2225 2230 2235

Lys Leu Gly Glu Asp Val Pro Ala Tyr Val Tyr Ala Thr Glu Asp
2240 2245 2250

Glu Asp Leu Ala Val Asp Leu Leu Gly Met Asp Trp Pro Asp Pro
2255 2260 2265

Gly Asn Gln Gln Val Val Glu Thr Gly Arg Ala Leu Lys Gln Val
2270 2275 2280

Thr Gly Leu Ser Thr Ala Glu Asn Ala Leu Leu Ile Ala Leu Phe
2285 2290 2295

Gly Tyr Val Gly Tyr Gln Thr Leu Ser Lys Arg His Ile Pro Met
2300 2305 2310

Ile Thr Asp Ile Tyr Thr Leu Glu Asp His Arg Leu Glu Asp Thr
2315 2320 2325

Thr His Leu Gln Phe Ala Pro Asn Ala Ile Arg Thr Asp Gly Lys
2330 2335 2340

Asp Ser Glu Leu Lys Glu Leu Ala Val Gly Asp Leu Asp Lys Tyr
2345 2350 2355

Val Asp Ala Leu Val Asp Tyr Ser Lys Gln Gly Met Lys Phe Ile
2360 2365 2370

Lys Val Gln Ala Glu Lys Val Arg Asp Ser Gln Ser Thr Lys Glu
2375 2380 2385

Gly Leu Gln Thr Ile Lys Glu Tyr Val Asp Lys Phe Ile Gln Ser
2390 2395 2400

Leu Thr Glu Asn Lys Glu Glu Ile Ile Arg Tyr Gly Leu Trp Gly
2405 2410 2415

Val His Thr Ala Leu Tyr Lys Ser Leu Ala Ala Arg Leu Gly His
2420 2425 2430

Glu Thr Ala Phe Ala Thr Leu Val Val Lys Trp Leu Ala Phe Gly
2435 2440 2445

Gly Glu Thr Val Ser Ala His Ile Lys Gln Val Ala Val Asp Leu
2450 2455 2460

Val Val Tyr Tyr Ile Ile Asn Lys Pro Ser Phe Pro Gly Asp Thr
2465 2470 2475

Glu Thr Gln Gln Glu Gly Arg Arg Phe Val Ala Ser Leu Phe Ile
2480 2485 2490

Ser Ala Leu Ala Thr Tyr Thr Tyr Lys Thr Trp Asn Tyr Asn Asn
2495 2500 2505

Leu Gln Arg Val Val Glu Pro Ala Leu Ala Tyr Leu Pro Tyr Ala
2510 2515 2520

Thr Ser Ala Leu Lys Leu Phe Thr Pro Thr Arg Leu Glu Ser Val
2525 2530 2535

Val Ile Leu Ser Ser Thr Ile Tyr Lys Thr Tyr Leu Ser Ile Arg
2540 2545 2550

Lys Gly Lys Ser Asp Gly Leu Leu Gly Thr Gly Ile Ser Ala Ala
2555 2560 2565

Met Glu Ile Leu Asn Gln Asn Pro Ile Ser Val Gly Ile Ser Val
2570 2575 2580

Met Leu Gly Val Gly Ala Ile Ala Ala His Asn Ala Ile Glu Ser
2585 2590 2595

Ser Glu Gln Lys Arg Thr Leu Leu Met Lys Val Phe Val Lys Asn
2600 2605 2610

Phe Leu Asp Gln Ala Ala Thr Asp Glu Leu Val Lys Glu Asn Pro
2615 2620 2625

Glu Lys Ile Ile Met Ala Leu Phe Glu Ala Val Gln Thr Ile Gly
2630 2635 2640

Asn Pro Leu Arg Leu Ile Tyr His Leu Tyr Gly Val Tyr Tyr Lys
2645 2650 2655

Gly Trp Glu Ala Lys Glu Leu Ala Glu Lys Thr Ala Gly Arg Asn
2660 2665 2670

Leu Phe Thr Leu Ile Met Phe Glu Ala Phe Glu Leu Leu Gly Met
2675 2680 2685

Asp Ser Glu Gly Lys Ile Arg Asn Leu Ser Gly Asn Tyr Ile Leu
2690 2695 2700

Asp Leu Ile Phe Asn Leu His Asn Lys Leu Asn Lys Gly Leu Lys
2705 2710 2715

Lys Leu Val Leu Gly Trp Ala Pro Ala Pro Leu Ser Cys Asp Trp
2720 2725 2730

Thr Pro Ser Asp Glu Arg Ile Ser Leu Pro His Asn Asn Tyr Leu
2735 2740 2745

Arg Val Glu Thr Arg Cys Pro Cys Gly Tyr Glu Met Lys Ala Ile
2750 2755 2760

Lys Asn Val Ala Gly Lys Leu Thr Lys Val Glu Glu Lys Gly Ser
2765 2770 2775

Phe Leu Cys Arg Asn Arg Leu Gly Arg Gly Pro Pro Asn Phe Lys
2780 2785 2790

Val Thr Lys Phe Tyr Asp Asp Asn Leu Ile Glu Val Lys Pro Val
2795 2800 2805

Ala Arg Leu Glu Gly Gln Val Asp Leu Tyr Tyr Lys Gly Val Thr
2810 2815 2820

Ala Lys Leu Asp Tyr Asn Asn Gly Lys Val Leu Leu Ala Thr Asn
2825 2830 2835

Lys Trp Glu Val Asp His Ala Phe Leu Thr Arg Leu Val Lys Lys
2840 2845 2850

His Thr Gly Ile Gly Phe Lys Gly Ala Tyr Leu Gly Asp Arg Pro
2855 2860 2865

Asp His Gln Asp Leu Val Asp Arg Asp Cys Ala Thr Ile Thr Lys
2870 2875 2880

Asn Ser Val Gln Phe Leu Lys Met Lys Lys Gly Cys Ala Phe Thr
2885 2890 2895

Tyr Asp Leu Thr Ile Ser Asn Leu Val Arg Leu Ile Glu Leu Val
2900 2905 2910

His Lys Asn Asn Leu Gln Glu Arg Glu Ile Pro Thr Val Thr Val
2915 2920 2925

Thr Thr Trp Leu Ala Tyr Ser Phe Val Asn Glu Asp Leu Gly Thr
2930 2935 2940

Ile Lys Pro Val Leu Gly Glu Lys Val Ile Pro Glu Pro Pro Glu
2945 2950 2955

Glu Leu Ser Leu Gln Pro Thr Val Arg Leu Val Thr Thr Glu Thr
2960 2965 2970

Ala Ile Thr Ile Thr Gly Glu Ala Glu Val Met Thr Thr Gly Ile
2975 2980 2985

Thr Pro Val Val Glu Met Lys Glu Glu Pro Gln Leu Asp His Gln
2990 2995 3000

Ser Thr Thr Leu Lys Val Gly Leu Lys Glu Gly Glu Tyr Pro Gly
3005 3010 3015

Pro Gly Val Asn Pro Asn His Leu Ala Glu Val Ile Asp Glu Lys
3020 3025 3030

Asp Asp Arg Pro Phe Val Leu Ile Ile Gly Asn Lys Gly Ser Thr
3035 3040 3045

Ser Asn Arg Ala Arg Thr Ala Lys Asn Ile Arg Leu Tyr Lys Gly
3050 3055 3060

Asn Asn Pro Arg Glu Ile Arg Asp Leu Met Ser Gln Gly Arg Ile
3065 3070 3075

Leu Thr Val Ala Leu Lys Glu Leu Asp Pro Glu Leu Lys Glu Leu
3080 3085 3090

Val Asp Tyr Lys Gly Thr Phe Leu Asn Arg Glu Ala Leu Glu Ala
3095 3100 3105

Leu Ser Leu Gly Lys Pro Ile Lys Arg Lys Thr Thr Thr Ala Met
3110 3115 3120

Ile Arg Arg Leu Ile Glu Pro Glu Val Glu Glu Glu Leu Pro Asp
3125 3130 3135

Trp Phe Gln Ala Glu Glu Pro Leu Phe Leu Glu Ala Lys Ile Gln
3140 3145 3150

Asn Asp Leu Tyr His Leu Ile Gly Ser Val Asp Ser Ile Lys Ser
3155 3160 3165

Lys Ala Lys Glu Leu Gly Ala Thr Asp Asn Thr Lys Ile Val Lys
3170 3175 3180

Glu Val Gly Ala Arg Thr Tyr Thr Met Lys Leu Ser Ser Trp Ser
3185 3190 3195

Thr Gln Val Thr Lys Lys Gln Met Ser Leu Ala Pro Leu Phe Glu
3200 3205 3210

Glu Leu Leu Leu Lys Cys Pro Pro Cys Ser Lys Ile Ser Lys Gly
3215 3220 3225

His Met Val Ser Ala Tyr Gln Leu Ala Gln Gly Asn Trp Glu Pro
3230 3235 3240

Leu Gly Cys Gly Val Tyr Met Gly Thr Ile Pro Ala Arg Arg Leu
3245 3250 3255

Lys Ile His Pro Tyr Glu Ala Tyr Leu Lys Leu Lys Glu Leu Val
3260 3265 3270

Glu Val Glu Ser Ser Arg Ala Thr Ala Lys Glu Ser Ile Ile Arg
3275 3280 3285

Glu His Asn Thr Trp Ile Leu Arg Lys Val Arg His Glu Gly Asn
3290 3295 3300

Leu Arg Thr Lys Ser Met Ile Asn Pro Gly Lys Ile Ser Asp Gln
3305 3310 3315

Leu Cys Arg Asp Gly His Lys Arg Asn Ile Tyr Asn Lys Ile Ile
3320 3325 3330

Gly Ser Thr Met Ala Ser Ala Gly Ile Arg Leu Glu Lys Leu Pro
3335 3340 3345

Val Val Arg Ala Gln Thr Asp Thr Thr Ser Phe His Gln Ala Ile
3350 3355 3360

Arg Glu Lys Ile Asp Lys Thr Glu Asn Lys Gln Thr Pro Glu Leu
3365 3370 3375

His Glu Glu Leu Met Lys Val Phe Asp Cys Leu Lys Ile Pro Glu
3380 3385 3390

Leu Lys Glu Ser Tyr Asp Glu Val Ser Trp Glu Gln Leu Glu Ala
3395 3400 3405

Gly Ile Asn Arg Lys Gly Ala Ala Gly Tyr Leu Glu Ser Lys Asn
3410 3415 3420

Ile Gly Glu Val Leu Asp Thr Glu Lys His Ile Val Glu Gln Leu
3425 3430 3435

Ile Lys Asp Leu Arg Lys Gly Lys Lys Ile Arg Tyr Tyr Glu Thr
3440 3445 3450

Ala Ile Pro Lys Asn Glu Lys Arg Asp Val Ser Asp Asp Trp Glu
3455 3460 3465

Ala Gly Glu Phe Val Asp Glu Lys Lys Pro Arg Val Ile Gln Tyr
3470 3475 3480

Pro Asp Ala Lys Val Arg Leu Ala Ile Thr Lys Val Met Tyr Lys
3485 3490 3495

Trp Val Lys Gln Lys Pro Val Val Ile Pro Gly Tyr Glu Gly Lys
3500 3505 3510

Thr Pro Leu Phe Asp Ile Phe Asn Lys Val Lys Lys Glu Trp Asp
3515 3520 3525

Ser Phe Gln Asp Pro Val Ala Val Ser Phe Asp Thr Lys Ala Trp
3530 3535 3540

Asp Thr Gln Val Thr Ser Arg Asp Leu Met Leu Ile Lys Asp Ile
3545 3550 3555

Gln Lys Tyr Tyr Phe Lys Arg Ser Ile His Lys Phe Leu Asp Thr
3560 3565 3570

Ile Thr Glu His Met Val Glu Val Pro Val Ile Thr Ala Asp Gly
3575 3580 3585

Glu Val Tyr Ile Arg Asn Gly Gln Arg Gly Ser Gly Gln Pro Asp
3590 3595 3600

Thr Ser Ala Gly Asn Ser Met Leu Asn Val Leu Thr Met Ile Tyr
3605 3610 3615

Ala Phe Cys Lys Ser Thr Gly Ile Pro Tyr Arg Gly Phe Ser Arg
3620 3625 3630

Val Ala Arg Ile His Val Cys Gly Asp Asp Gly Phe Leu Ile Thr
3635 3640 3645

Glu Arg Gly Leu Gly Leu Lys Phe Ser Glu Lys Gly Met Gln Ile
3650 3655 3660

Leu His Glu Ala Gly Lys Pro Gln Lys Ile Thr Glu Gly Asp Lys
3665 3670 3675

Met Lys Val Ala Tyr Arg Phe Glu Asp Ile Glu Phe Cys Ser His
3680 3685 3690

Thr Pro Val Pro Val Arg Trp Ala Asp Asn Thr Ser Ser Tyr Met
3695 3700 3705

Ala Gly Arg Ser Thr Ala Thr Ile Leu Ala Lys Met Ala Thr Arg
3710 3715 3720

Leu Asp Ser Ser Gly Glu Arg Gly Ser Thr Ala Tyr Glu Lys Ala
3725 3730 3735

Val Ala Phe Ser Phe Leu Leu Met Tyr Ser Trp Asn Pro Val Val
3740 3745 3750

Arg Arg Ile Cys Leu Leu Val Leu Ser Gln Phe Pro Glu Ile Ser
3755 3760 3765

Pro Ser Lys Asn Thr Ile Tyr Tyr Tyr Gln Gly Asp Pro Ile Ala
3770 3775 3780

Ala Tyr Arg Glu Val Ile Gly Lys Gln Leu Cys Glu Leu Lys Arg
3785 3790 3795

Thr Gly Phe Glu Lys Leu Ala Gly Leu Asn Leu Ser Met Thr Thr
3800 3805 3810

Leu Gly Ile Trp Thr Lys His Thr Ser Lys Arg Leu Ile Gln Ala
3815 3820 3825

Cys Val Glu Ile Gly Lys Arg Glu Gly Thr Trp Leu Val Asn Ala
3830 3835 3840

Asp Arg Leu Ile Ala Gly Lys Thr Gly Lys Phe Tyr Ile Pro Ser
3845 3850 3855

Thr Gly Val Thr Leu Leu Gly Lys His Tyr Glu Glu Ile Asn Leu
3860 3865 3870

Lys Gln Lys Ala Ala Gln Pro Pro Ile Glu Gly Val Asp Arg Tyr
3875 3880 3885

Lys Leu Gly Pro Ile Val Asn Val Ile Leu Arg Arg Leu Arg Val
3890 3895 3900

Met Leu Met Thr Val Ala Ser Gly Ser Trp
3905 3910

<210> 6
<211> 3749
<212> PRT
<213> Artificial Sequence: Mutated BVDV: XIKE-A-NdN

<400> 6

```
Met Glu Leu Phe Ser Asp Glu Gly Ser Lys Gly Ala Thr Ser Lys Lys
1               5                   10                  15

Gln Pro Lys Pro Asp Arg Ile Glu Lys Gly Lys Met Lys Ile Ala Pro
            20                  25                  30

Lys Glu Thr Glu Lys Asp Cys Lys Thr Arg Pro Pro Asp Ala Thr Ile
        35                  40                  45

Val Val Glu Gly Val Lys Tyr Gln Val Lys Lys Lys Gly Lys Val Arg
    50                  55                  60

Gly Lys Asn Thr Gln Asp Gly Leu Tyr His Asn Lys Asn Lys Pro Pro
65                  70                  75                  80
```

Glu Ser Arg Lys Lys Leu Glu Lys Ala Leu Leu Ala Trp Ala Ile Leu
85 90 95

Ala Ala Val Leu Leu Gln Leu Val Thr Gly Glu Asn Ile Thr Gln Trp
100 105 110

Asn Leu Met Asp Asn Gly Thr Glu Gly Ile Gln Gln Ala Met Phe Leu
115 120 125

Arg Gly Val Asn Arg Ser Leu His Gly Ile Trp Pro Glu Lys Ile Cys
130 135 140

Thr Gly Val Pro Thr His Leu Ala Thr Asp Tyr Glu Leu Lys Glu Ile
145 150 155 160

Val Gly Met Met Asp Ala Ser Glu Lys Thr Asn Tyr Thr Cys Cys Arg
165 170 175

Leu Gln Arg His Glu Trp Asn Lys His Gly Trp Cys Asn Trp Phe His
180 185 190

Ile Glu Pro Trp Ile Trp Leu Met Asn Lys Thr Gln Asn Asn Leu Thr
195 200 205

Glu Gly Gln Pro Leu Arg Glu Cys Ala Val Thr Cys Arg Tyr Asp Lys
210 215 220

Glu Thr Glu Leu Asn Ile Val Thr Gln Ala Arg Asp Arg Pro Thr Thr
225 230 235 240

Leu Thr Gly Cys Lys Lys Gly Lys Asn Phe Ser Phe Ala Gly Val Ile
245 250 255

Leu Asp Gly Pro Cys Asn Phe Lys Val Ser Val Glu Asp Val Leu Phe
260 265 270

Lys Glu His Asp Cys Gly Asn Met Leu Gln Glu Thr Ala Ile Gln Leu
275 280 285

Leu Asp Gly Ala Thr Asn Thr Ile Glu Gly Ala Arg Val Gly Thr Ala
290 295 300

Lys Leu Thr Thr Trp Leu Gly Lys Gln Leu Gly Ile Leu Gly Lys Lys
305 310 315 320

Leu Glu Asn Lys Ser Lys Ala Trp Phe Gly Ala His Ala Ala Ser Pro
325 330 335

Tyr Cys Gly Val Glu Arg Lys Ile Gly Tyr Val Trp Tyr Thr Lys Asn
340 345 350

Cys Thr Pro Ala Cys Leu Pro Arg Asn Thr Arg Ile Ile Gly Pro Gly
355 360 365

Lys Phe Asp Thr Asn Ala Glu Asp Gly Lys Ile Leu His Glu Met Gly
370 375 380

Gly His Leu Ser Glu Phe Val Leu Leu Ser Leu Val Val Leu Ser Asp
385 390 395 400

Phe Ala Pro Glu Thr Ala Ser Val Ile Tyr Leu Val Leu His Phe Ala
405 410 415

Ile Pro Gln Ser His Val Asp Val Asp Thr Cys Asp Lys Asn Gln Leu
420 425 430

Asn Leu Thr Val Ala Thr Thr Val Ala Glu Val Ile Pro Gly Thr Val
435 440 445

Trp Asn Leu Gly Lys Tyr Val Cys Ile Arg Pro Asp Trp Trp Pro Tyr
450 455 460

Glu Thr Thr Thr Val Phe Val Ile Glu Glu Ala Gly Gln Val Ile Lys
465 470 475 480

Leu Met Leu Arg Ala Ile Arg Asp Leu Thr Arg Ile Trp Asn Ala Ala
485 490 495

Thr Thr Thr Ala Phe Leu Ile Phe Leu Val Lys Ala Leu Arg Gly Gln
500 505 510

Leu Ile Gln Gly Leu Leu Trp Leu Met Leu Ile Thr Gly Ala Gln Gly
515 520 525

Phe Pro Glu Cys Lys Glu Gly Phe Gln Tyr Ala Ile Ser Lys Asp Arg
530 535 540

Lys Met Gly Leu Leu Gly Pro Glu Ser Leu Thr Thr Thr Trp His Leu
545 550 555 560

Pro Thr Lys Lys Ile Val Asp Ser Met Val His Val Trp Cys Glu Gly
565 570 575

Lys Asp Leu Lys Ile Leu Lys Met Cys Thr Lys Glu Glu Arg Tyr Leu
580 585 590

Val Ala Val His Glu Arg Ala Leu Ser Thr Ser Ala Glu Phe Met Gln
595 600 605

Ile Ser Asp Gly Thr Ile Gly Pro Asp Val Ile Asp Met Pro Asp Asp
610 615 620

Phe Glu Phe Gly Leu Cys Pro Cys Asp Ser Lys Pro Val Ile Lys Gly
625 630 635 640

Lys Phe Asn Ala Ser Leu Leu Asn Gly Pro Ala Phe Gln Met Val Cys
645 650 655

Pro Gln Gly Trp Thr Gly Thr Ile Glu Cys Thr Leu Ala Asn Gln Asp
660 665 670

Thr Leu Asp Thr Thr Val Ile Arg Thr Tyr Arg Arg Thr Thr Pro Phe
675 680 685

Gln Arg Arg Lys Trp Cys Thr Tyr Glu Lys Ile Ile Gly Glu Asp Ile
    690                 695                 700

Tyr Glu Cys Ile Leu Gly Gly Asn Trp Thr Cys Ile Thr Gly Asp His
705                 710                 715                 720

Ser Arg Leu Lys Asp Gly Pro Ile Lys Lys Cys Lys Trp Cys Gly His
                725                 730                 735

Asp Phe Val Asn Ser Glu Gly Leu Pro His Tyr Pro Ile Gly Lys Cys
            740                 745                 750

Met Leu Ile Asn Glu Ser Gly Tyr Arg Tyr Val Asp Asp Thr Ser Cys
        755                 760                 765

Asp Arg Gly Gly Val Ala Ile Val Pro Ser Gly Thr Val Lys Cys Arg
    770                 775                 780

Ile Gly Asn Val Thr Val Gln Val Ile Ala Thr Asn Asn Asp Leu Gly
785                 790                 795                 800

Pro Met Pro Cys Ser Pro Ala Glu Val Ile Ala Ser Glu Gly Pro Val
                805                 810                 815

Glu Lys Thr Ala Cys Thr Phe Asn Tyr Ser Arg Thr Leu Pro Asn Lys
            820                 825                 830

Tyr Tyr Glu Pro Arg Asp Arg Tyr Phe Gln Gln Tyr Met Leu Lys Gly
        835                 840                 845

Glu Trp Gln Tyr Trp Phe Asp Leu Asp Ser Val Asp His His Lys Asp
    850                 855                 860

Tyr Phe Ser Glu Phe Ile Ile Ile Ala Val Val Ala Leu Leu Gly Gly
865                 870                 875                 880

Lys Tyr Val Leu Trp Leu Leu Ile Thr Tyr Thr Ile Leu Ser Glu Gln
                885                 890                 895

Met Ala Met Gly Ala Gly Val Asn Thr Glu Glu Ile Val Met Ile Gly
            900                 905                 910

Asn Leu Leu Thr Asp Ser Asp Ile Glu Val Val Val Tyr Phe Leu Leu
        915                 920                 925

Leu Tyr Leu Ile Val Lys Glu Glu Leu Ala Arg Lys Trp Ile Ile Leu
    930                 935                 940

Val Tyr His Ile Leu Val Ala Asn Pro Met Lys Thr Ile Gly Val Val
945                 950                 955                 960

Leu Leu Met Leu Gly Gly Val Val Lys Ala Ser Arg Ile Asn Ala Asp
                965                 970                 975

Asp Gln Ser Ala Met Asp Pro Cys Phe Leu Leu Val Thr Gly Val Val
            980                 985                 990

Ala Val Leu Met Ile Ala Arg Arg Glu Pro Ala Thr Leu Pro Leu Ile
995 1000 1005

Val Ala Leu Leu Ala Ile Arg Thr Ser Gly Phe Leu Leu Pro Ala
1010 1015 1020

Ser Ile Asp Val Thr Val Ala Val Val Leu Ile Val Leu Leu Leu
1025 1030 1035

Ala Ser Tyr Ile Thr Asp Tyr Phe Arg Tyr Lys Lys Trp Leu Gln
1040 1045 1050

Leu Leu Phe Ser Leu Ile Ala Gly Ile Phe Ile Ile Arg Ser Leu
1055 1060 1065

Lys His Ile Asn Gln Met Glu Val Pro Glu Ile Ser Met Pro Ser
1070 1075 1080

Trp Arg Pro Leu Ala Leu Val Leu Phe Tyr Ile Thr Ser Thr Ala
1085 1090 1095

Ile Thr Thr Asn Trp Asp Ile Asp Leu Ala Gly Phe Leu Leu Gln
1100 1105 1110

Trp Ala Pro Ala Val Ile Met Met Ala Thr Met Trp Ala Asp Phe
1115 1120 1125

Leu Thr Leu Ile Ile Val Leu Pro Ser Tyr Glu Leu Ser Lys Leu
1130 1135 1140

Tyr Phe Leu Lys Asn Val Arg Thr Asp Val Glu Lys Asn Trp Leu
1145 1150 1155

Gly Lys Val Lys Tyr Arg Gln Ile Ser Ser Val Tyr Asp Ile Cys
1160 1165 1170

Asp Ser Glu Glu Ala Val Tyr Leu Phe Pro Ser Arg His Lys Ser
1175 1180 1185

Gly Ser Arg Pro Asp Phe Ile Leu Pro Phe Leu Lys Ala Val Leu
1190 1195 1200

Ile Ser Cys Ile Ser Ser Gln Trp Gln Val Val Tyr Ile Ser Tyr
1205 1210 1215

Leu Ile Leu Glu Ile Thr Tyr Tyr Met His Arg Lys Ile Ile Asp
1220 1225 1230

Glu Val Ser Gly Gly Ala Asn Phe Leu Ser Arg Leu Ile Ala Ala
1235 1240 1245

Ile Ile Glu Leu Asn Trp Ala Ile Asp Asp Glu Glu Cys Lys Gly
1250 1255 1260

Leu Lys Lys Leu Tyr Leu Leu Ser Gly Arg Ala Lys Asn Leu Ile
1265 1270 1275

Val Lys His Lys Val Arg Asn Glu Ala Val His Arg Trp Phe Gly
1280 1285 1290

Glu Glu Glu Ile Tyr Gly Ala Pro Lys Val Ile Thr Ile Ile Lys
1295 1300 1305

Ala Ser Thr Leu Ser Lys Asn Arg His Cys Ile Ile Cys Thr Ile
1310 1315 1320

Cys Glu Gly Lys Glu Trp Asn Gly Ala Asn Cys Pro Lys Cys Gly
1325 1330 1335

Arg Gln Gly Lys Pro Ile Thr Cys Gly Met Thr Leu Ala Asp Phe
1340 1345 1350

Glu Glu Lys His Tyr Lys Lys Ile Phe Ile Arg Glu Glu Ser Ser
1355 1360 1365

Cys Pro Val Pro Phe Asp Pro Ser Cys His Cys Asn Tyr Phe Arg
1370 1375 1380

His Asp Gly Pro Phe Arg Lys Glu Tyr Lys Gly Tyr Val Gln Tyr
1385 1390 1395

Thr Ala Arg Gly Gln Leu Phe Leu Arg Asn Leu Pro Ile Leu Ala
1400 1405 1410

Thr Lys Met Lys Leu Leu Met Val Gly Asn Leu Gly Ala Glu Ile
1415 1420 1425

Gly Asp Leu Glu His Leu Gly Trp Val Leu Arg Gly Pro Ala Val
1430 1435 1440

Cys Lys Lys Ile Thr Asn His Glu Lys Cys His Val Asn Ile Met
1445 1450 1455

Asp Lys Leu Thr Ala Phe Phe Gly Ile Met Pro Arg Gly Thr Thr
1460 1465 1470

Pro Arg Ala Pro Val Arg Phe Pro Thr Ala Leu Leu Lys Val Arg
1475 1480 1485

Arg Gly Leu Glu Thr Gly Trp Ala Tyr Thr His Gln Gly Gly Ile
1490 1495 1500

Ser Ser Val Asp His Val Thr Ala Gly Lys Asp Leu Leu Val Cys
1505 1510 1515

Asp Ser Met Gly Arg Thr Arg Val Val Cys His Ser Asn Asn Lys
1520 1525 1530

Met Thr Asp Glu Thr Glu Tyr Gly Ile Lys Thr Asp Ser Gly Cys
1535 1540 1545

Pro Glu Gly Ala Arg Cys Tyr Val Leu Asn Pro Glu Ala Val Asn
1550 1555 1560

Ile Ser Gly Thr Lys Gly Ala Met Val His Leu Gln Lys Thr Gly
1565 1570 1575

Gly Glu Phe Thr Cys Val Thr Ala Ser Gly Thr Pro Ala Phe Phe
1580 1585 1590

Asp Leu Lys Asn Leu Lys Gly Trp Ser Gly Leu Pro Ile Phe Glu
1595 1600 1605

Ala Ser Ser Gly Arg Val Val Gly Arg Val Lys Val Gly Lys Asn
1610 1615 1620

Glu Asp Ser Lys Pro Thr Lys Leu Met Ser Gly Ile Gln Thr Val
1625 1630 1635

Ser Lys Asn Gln Thr Asp Leu Ala Asp Ile Val Lys Lys Leu Thr
1640 1645 1650

Ser Met Asn Arg Gly Glu Phe Lys Gln Ile Thr Leu Ala Thr Gly
1655 1660 1665

Ala Gly Lys Thr Thr Glu Leu Pro Arg Ser Val Ile Glu Glu Ile
1670 1675 1680

Gly Arg His Lys Arg Val Leu Val Leu Ile Pro Leu Arg Ala Ala
1685 1690 1695

Ala Glu Ser Val Tyr Gln Tyr Met Arg Val Lys Tyr Pro Ser Ile
1700 1705 1710

Ser Phe Asn Leu Arg Ile Gly Asp Met Lys Glu Gly Asp Met Ala
1715 1720 1725

Thr Gly Ile Thr Tyr Ala Ser Tyr Gly Tyr Phe Cys Gln Leu Pro
1730 1735 1740

Gln Pro Lys Leu Arg Ala Ala Met Val Glu Tyr Ser Tyr Ile Phe
1745 1750 1755

Leu Asp Glu Tyr His Cys Ala Thr Pro Glu Gln Leu Ala Ile Ile
1760 1765 1770

Gly Lys Ile His Arg Phe Ala Glu Asn Leu Arg Val Val Ala Met
1775 1780 1785

Thr Ala Thr Pro Ala Gly Thr Val Thr Thr Thr Gly Gln Lys His
1790 1795 1800

Pro Ile Glu Glu Phe Ile Ala Pro Glu Val Met Lys Gly Glu Asp
1805 1810 1815

Leu Gly Ser Glu Tyr Leu Asp Ile Ala Gly Leu Lys Ile Pro Thr
1820 1825 1830

Glu Glu Met Lys Gly Asn Met Leu Val Phe Ala Pro Thr Arg Asn
1835 1840 1845

Met Ala Val Glu Thr Ala Lys Lys Leu Lys Ala Lys Gly Tyr Asn
1850 1855 1860

Ser Gly Tyr Tyr Tyr Ser Gly Glu Asn Pro Glu Asn Leu Arg Val
1865 1870 1875

Val Thr Ser Gln Ser Pro Tyr Val Val Val Ala Thr Asn Ala Ile
1880 1885 1890

Glu Ser Gly Val Thr Leu Pro Asp Leu Asp Thr Val Val Asp Thr
1895 1900 1905

Gly Leu Lys Cys Glu Lys Arg Val Arg Ile Ser Ser Lys Met Pro
1910 1915 1920

Phe Ile Val Thr Gly Leu Lys Arg Met Ala Val Thr Ile Gly Glu
1925 1930 1935

Gln Ala Gln Arg Arg Gly Arg Val Gly Arg Val Lys Pro Gly Arg
1940 1945 1950

Tyr Tyr Arg Ser Gln Glu Thr Ala Ser Gly Ser Lys Asp Tyr His
1955 1960 1965

Tyr Asp Leu Leu Gln Ala Gln Arg Tyr Gly Ile Glu Asp Gly Ile
1970 1975 1980

Asn Val Thr Lys Ser Phe Arg Glu Met Asn Tyr Asp Trp Ser Leu
1985 1990 1995

Tyr Glu Glu Asp Ser Leu Met Ile Thr Gln Leu Glu Val Leu Asn
2000 2005 2010

Asn Leu Leu Ile Ser Glu Asp Leu Pro Ala Ala Val Lys Asn Ile
2015 2020 2025

Met Ala Arg Thr Asp His Pro Glu Pro Ile Gln Leu Ala Tyr Asn
2030 2035 2040

Ser Tyr Glu Asn Gln Ile Pro Val Leu Phe Pro Lys Ile Lys Asn
2045 2050 2055

Gly Glu Val Thr Asp Ser Tyr Glu Asn Tyr Thr Tyr Leu Asn Ala
2060 2065 2070

Arg Lys Leu Gly Glu Asp Val Pro Ala Tyr Val Tyr Ala Thr Glu
2075 2080 2085

Asp Glu Asp Leu Ala Val Asp Leu Leu Gly Met Asp Trp Pro Asp
2090 2095 2100

Pro Gly Asn Gln Gln Val Val Glu Thr Gly Arg Ala Leu Lys Gln
2105 2110 2115

Val Thr Gly Leu Ser Thr Ala Glu Asn Ala Leu Leu Ile Ala Leu
2120 2125 2130

Phe Gly Tyr Val Gly Tyr Gln Thr Leu Ser Lys Arg His Ile Pro
2135 2140 2145

Met Ile Thr Asp Ile Tyr Thr Leu Glu Asp His Arg Leu Glu Asp
2150 2155 2160

Thr Thr His Leu Gln Phe Ala Pro Asn Ala Ile Arg Thr Asp Gly
2165 2170 2175

Lys Asp Ser Glu Leu Lys Glu Leu Ala Val Gly Asp Leu Asp Lys
2180 2185 2190

Tyr Val Asp Ala Leu Val Asp Tyr Ser Lys Gln Gly Met Lys Phe
2195 2200 2205

Ile Lys Val Gln Ala Glu Lys Val Arg Asp Ser Gln Ser Thr Lys
2210 2215 2220

Glu Gly Leu Gln Thr Ile Lys Glu Tyr Val Asp Lys Phe Ile Gln
2225 2230 2235

Ser Leu Thr Glu Asn Lys Glu Glu Ile Ile Arg Tyr Gly Leu Trp
2240 2245 2250

Gly Val His Thr Ala Leu Tyr Lys Ser Leu Ala Ala Arg Leu Gly
2255 2260 2265

His Glu Thr Ala Phe Ala Thr Leu Val Val Lys Trp Leu Ala Phe
2270 2275 2280

Gly Gly Glu Thr Val Ser Ala His Ile Lys Gln Val Ala Val Asp
2285 2290 2295

Leu Val Val Tyr Tyr Ile Ile Asn Lys Pro Ser Phe Pro Gly Asp
2300 2305 2310

Thr Glu Thr Gln Gln Glu Gly Arg Arg Phe Val Ala Ser Leu Phe
2315 2320 2325

Ile Ser Ala Leu Ala Thr Tyr Thr Tyr Lys Thr Trp Asn Tyr Asn
2330 2335 2340

Asn Leu Gln Arg Val Val Glu Pro Ala Leu Ala Tyr Leu Pro Tyr
2345 2350 2355

Ala Thr Ser Ala Leu Lys Leu Phe Thr Pro Thr Arg Leu Glu Ser
2360 2365 2370

Val Val Ile Leu Ser Ser Thr Ile Tyr Lys Thr Tyr Leu Ser Ile
2375 2380 2385

Arg Lys Gly Lys Ser Asp Gly Leu Leu Gly Thr Gly Ile Ser Ala
2390 2395 2400

Ala Met Glu Ile Leu Asn Gln Asn Pro Ile Ser Val Gly Ile Ser
2405 2410 2415

Val Met Leu Gly Val Gly Ala Ile Ala Ala His Asn Ala Ile Glu
2420 2425 2430

Ser Ser Glu Gln Lys Arg Thr Leu Leu Met Lys Val Phe Val Lys
2435 2440 2445

Asn Phe Leu Asp Gln Ala Ala Thr Asp Glu Leu Val Lys Glu Asn
2450 2455 2460

Pro Glu Lys Ile Ile Met Ala Leu Phe Glu Ala Val Gln Thr Ile
2465 2470 2475

Gly Asn Pro Leu Arg Leu Ile Tyr His Leu Tyr Gly Val Tyr Tyr
2480 2485 2490

Lys Gly Trp Glu Ala Lys Glu Leu Ala Glu Lys Thr Ala Gly Arg
2495 2500 2505

Asn Leu Phe Thr Leu Ile Met Phe Glu Ala Phe Glu Leu Leu Gly
2510 2515 2520

Met Asp Ser Glu Gly Lys Ile Arg Asn Leu Ser Gly Asn Tyr Ile
2525 2530 2535

Leu Asp Leu Ile Phe Asn Leu His Asn Lys Leu Asn Lys Gly Leu
2540 2545 2550

Lys Lys Leu Val Leu Gly Trp Ala Pro Ala Pro Leu Ser Cys Asp
2555 2560 2565

Trp Thr Pro Ser Asp Glu Arg Ile Ser Leu Pro His Asn Asn Tyr
2570 2575 2580

Leu Arg Val Glu Thr Arg Cys Pro Cys Gly Tyr Glu Met Lys Ala
2585 2590 2595

Ile Lys Asn Val Ala Gly Lys Leu Thr Lys Val Glu Glu Lys Gly
2600 2605 2610

Ser Phe Leu Cys Arg Asn Arg Leu Gly Arg Gly Pro Pro Asn Phe
2615 2620 2625

Lys Val Thr Lys Phe Tyr Asp Asp Asn Leu Ile Glu Val Lys Pro
2630 2635 2640

Val Ala Arg Leu Glu Gly Gln Val Asp Leu Tyr Tyr Lys Gly Val
2645 2650 2655

Thr Ala Lys Leu Asp Tyr Asn Asn Gly Lys Val Leu Leu Ala Thr
2660 2665 2670

Asn Lys Trp Glu Val Asp His Ala Phe Leu Thr Arg Leu Val Lys
2675 2680 2685

Lys His Thr Gly Ile Gly Phe Lys Gly Ala Tyr Leu Gly Asp Arg
2690 2695 2700

Pro Asp His Gln Asp Leu Val Asp Arg Asp Cys Ala Thr Ile Thr
2705 2710 2715

Lys Asn Ser Val Gln Phe Leu Lys Met Lys Lys Gly Cys Ala Phe
2720 2725 2730

Thr Tyr Asp Leu Thr Ile Ser Asn Leu Val Arg Leu Ile Glu Leu
2735 2740 2745

Val His Lys Asn Asn Leu Gln Glu Arg Glu Ile Pro Thr Val Thr
2750 2755 2760

Val Thr Thr Trp Leu Ala Tyr Ser Phe Val Asn Glu Asp Leu Gly
2765 2770 2775

Thr Ile Lys Pro Val Leu Gly Glu Lys Val Ile Pro Glu Pro Pro
2780 2785 2790

Glu Glu Leu Ser Leu Gln Pro Thr Val Arg Leu Val Thr Thr Glu
2795 2800 2805

Thr Ala Ile Thr Ile Thr Gly Glu Ala Glu Val Met Thr Thr Gly
2810 2815 2820

Ile Thr Pro Val Val Glu Met Lys Glu Glu Pro Gln Leu Asp His
2825 2830 2835

Gln Ser Thr Thr Leu Lys Val Gly Leu Lys Glu Gly Glu Tyr Pro
2840 2845 2850

Gly Pro Gly Val Asn Pro Asn His Leu Ala Glu Val Ile Asp Glu
2855 2860 2865

Lys Asp Asp Arg Pro Phe Val Leu Ile Ile Gly Asn Lys Gly Ser
2870 2875 2880

Thr Ser Asn Arg Ala Arg Thr Ala Lys Asn Ile Arg Leu Tyr Lys
2885 2890 2895

Gly Asn Asn Pro Arg Glu Ile Arg Asp Leu Met Ser Gln Gly Arg
2900 2905 2910

Ile Leu Thr Val Ala Leu Lys Glu Leu Asp Pro Glu Leu Lys Glu
2915 2920 2925

Leu Val Asp Tyr Lys Gly Thr Phe Leu Asn Arg Glu Ala Leu Glu
2930 2935 2940

Ala Leu Ser Leu Gly Lys Pro Ile Lys Arg Lys Thr Thr Thr Ala
2945 2950 2955

Met Ile Arg Arg Leu Ile Glu Pro Glu Val Glu Glu Glu Leu Pro
2960 2965 2970

Asp Trp Phe Gln Ala Glu Glu Pro Leu Phe Leu Glu Ala Lys Ile
2975 2980 2985

Gln Asn Asp Leu Tyr His Leu Ile Gly Ser Val Asp Ser Ile Lys
2990 2995 3000

Ser Lys Ala Lys Glu Leu Gly Ala Thr Asp Asn Thr Lys Ile Val
3005 3010 3015

Lys Glu Val Gly Ala Arg Thr Tyr Thr Met Lys Leu Ser Ser Trp
3020 3025 3030

Ser Thr Gln Val Thr Lys Lys Gln Met Ser Leu Ala Pro Leu Phe
3035 3040 3045

Glu Glu Leu Leu Leu Lys Cys Pro Pro Cys Ser Lys Ile Ser Lys
3050 3055 3060

Gly His Met Val Ser Ala Tyr Gln Leu Ala Gln Gly Asn Trp Glu
3065 3070 3075

Pro Leu Gly Cys Gly Val Tyr Met Gly Thr Ile Pro Ala Arg Arg
3080 3085 3090

Leu Lys Ile His Pro Tyr Glu Ala Tyr Leu Lys Leu Lys Glu Leu
3095 3100 3105

Val Glu Val Glu Ser Ser Arg Ala Thr Ala Lys Glu Ser Ile Ile
3110 3115 3120

Arg Glu His Asn Thr Trp Ile Leu Arg Lys Val Arg His Glu Gly
3125 3130 3135

Asn Leu Arg Thr Lys Ser Met Ile Asn Pro Gly Lys Ile Ser Asp
3140 3145 3150

Gln Leu Cys Arg Asp Gly His Lys Arg Asn Ile Tyr Asn Lys Ile
3155 3160 3165

Ile Gly Ser Thr Met Ala Ser Ala Gly Ile Arg Leu Glu Lys Leu
3170 3175 3180

Pro Val Val Arg Ala Gln Thr Asp Thr Thr Ser Phe His Gln Ala
3185 3190 3195

Ile Arg Glu Lys Ile Asp Lys Thr Glu Asn Lys Gln Thr Pro Glu
3200 3205 3210

Leu His Glu Glu Leu Met Lys Val Phe Asp Cys Leu Lys Ile Pro
3215 3220 3225

Glu Leu Lys Glu Ser Tyr Asp Glu Val Ser Trp Glu Gln Leu Glu
3230 3235 3240

Ala Gly Ile Asn Arg Lys Gly Ala Ala Gly Tyr Leu Glu Ser Lys
3245 3250 3255

Asn Ile Gly Glu Val Leu Asp Thr Glu Lys His Ile Val Glu Gln
3260 3265 3270

Leu Ile Lys Asp Leu Arg Lys Gly Lys Lys Ile Arg Tyr Tyr Glu
3275 3280 3285

Thr Ala Ile Pro Lys Asn Glu Lys Arg Asp Val Ser Asp Asp Trp
3290 3295 3300

Glu Ala Gly Glu Phe Val Asp Glu Lys Lys Pro Arg Val Ile Gln
3305 3310 3315

Tyr Pro Asp Ala Lys Val Arg Leu Ala Ile Thr Lys Val Met Tyr
3320 3325 3330

Lys Trp Val Lys Gln Lys Pro Val Val Ile Pro Gly Tyr Glu Gly
3335 3340 3345

Lys Thr Pro Leu Phe Asp Ile Phe Asn Lys Val Lys Lys Glu Trp
3350 3355 3360

Asp Ser Phe Gln Asp Pro Val Ala Val Ser Phe Asp Thr Lys Ala
3365 3370 3375

Trp Asp Thr Gln Val Thr Ser Arg Asp Leu Met Leu Ile Lys Asp
3380 3385 3390

Ile Gln Lys Tyr Tyr Phe Lys Arg Ser Ile His Lys Phe Leu Asp
3395 3400 3405

Thr Ile Thr Glu His Met Val Glu Val Pro Val Ile Thr Ala Asp
3410 3415 3420

Gly Glu Val Tyr Ile Arg Asn Gly Gln Arg Gly Ser Gly Gln Pro
3425 3430 3435

Asp Thr Ser Ala Gly Asn Ser Met Leu Asn Val Leu Thr Met Ile
3440 3445 3450

Tyr Ala Phe Cys Lys Ser Thr Gly Ile Pro Tyr Arg Gly Phe Ser
3455 3460 3465

Arg Val Ala Arg Ile His Val Cys Gly Asp Asp Gly Phe Leu Ile
3470 3475 3480

Thr Glu Arg Gly Leu Gly Leu Lys Phe Ser Glu Lys Gly Met Gln
3485 3490 3495

Ile Leu His Glu Ala Gly Lys Pro Gln Lys Ile Thr Glu Gly Asp
3500 3505 3510

Lys Met Lys Val Ala Tyr Arg Phe Glu Asp Ile Glu Phe Cys Ser
3515 3520 3525

His Thr Pro Val Pro Val Arg Trp Ala Asp Asn Thr Ser Ser Tyr
3530 3535 3540

Met Ala Gly Arg Ser Thr Ala Thr Ile Leu Ala Lys Met Ala Thr
3545 3550 3555

Arg Leu Asp Ser Ser Gly Glu Arg Gly Ser Thr Ala Tyr Glu Lys
3560 3565 3570

Ala Val Ala Phe Ser Phe Leu Leu Met Tyr Ser Trp Asn Pro Val
3575 3580 3585

Val Arg Arg Ile Cys Leu Leu Val Leu Ser Gln Phe Pro Glu Ile
3590 3595 3600

Ser Pro Ser Lys Asn Thr Ile Tyr Tyr Tyr Gln Gly Asp Pro Ile
3605 3610 3615

Ala Ala Tyr Arg Glu Val Ile Gly Lys Gln Leu Cys Glu Leu Lys
3620 3625 3630

Arg Thr Gly Phe Glu Lys Leu Ala Gly Leu Asn Leu Ser Met Thr
3635 3640 3645

Thr Leu Gly Ile Trp Thr Lys His Thr Ser Lys Arg Leu Ile Gln
3650 3655 3660

Ala Cys Val Glu Ile Gly Lys Arg Glu Gly Thr Trp Leu Val Asn
3665 3670 3675

Ala Asp Arg Leu Ile Ala Gly Lys Thr Gly Lys Phe Tyr Ile Pro
3680 3685 3690

Ser Thr Gly Val Thr Leu Leu Gly Lys His Tyr Glu Glu Ile Asn
3695 3700 3705

Leu Lys Gln Lys Ala Ala Gln Pro Pro Ile Glu Gly Val Asp Arg
3710 3715 3720

Tyr Lys Leu Gly Pro Ile Val Asn Val Ile Leu Arg Arg Leu Arg
3725 3730 3735

Val Met Leu Met Thr Val Ala Ser Gly Ser Trp
3740 3745

<210> 7
<211> 3912
<212> PRT
<213> Artificial Sequence: Mutated BVDV: XIKE-B

<400> 7

Met Glu Leu Phe Ser Asn Glu Leu Leu Tyr Lys Thr Tyr Lys Gln Lys
1               5                   10                  15

Pro Ala Gly Val Val Glu Pro Val Tyr Asp Val Asn Gly Arg Pro Leu
            20                  25                  30

Phe Gly Glu Ser Ser Asp Leu His Pro Gln Ser Thr Leu Lys Leu Pro
        35                  40                  45

His Gln Arg Gly Ser Ala Asn Ile Leu Thr Asn Ala Arg Ser Leu Pro

```
        50                           55                           60

Arg Lys Gly Asp Cys Arg Arg Gly Asn Val Tyr Gly Pro Val Ser Gly
65                  70                  75                  80

Ile Tyr Ile Lys Pro Gly Pro Ile Tyr Tyr Gln Asp Tyr Val Gly Pro
                85                  90                  95

Val Tyr His Arg Ala Pro Leu Glu Leu Cys Arg Glu Ala Ser Met Cys
            100                 105                 110

Glu Thr Thr Arg Arg Val Gly Arg Val Thr Gly Ser Asp Gly Lys Leu
        115                 120                 125

Tyr His Ile Tyr Ile Cys Ile Asp Gly Cys Ile Leu Leu Lys Arg Ala
    130                 135                 140

Thr Arg Asn Gln Pro Glu Val Leu Lys Trp Val Tyr Asn Arg Leu Asn
145                 150                 155                 160

Cys Pro Leu Trp Val Thr Ser Cys Ser Asp Glu Gly Ser Lys Gly Ala
                165                 170                 175

Thr Ser Lys Lys Gln Pro Lys Pro Asp Arg Ile Glu Lys Gly Lys Met
            180                 185                 190

Lys Ile Ala Pro Lys Glu Thr Glu Lys Asp Cys Lys Thr Arg Pro Pro
        195                 200                 205

Asp Ala Thr Ile Val Val Glu Gly Val Lys Tyr Gln Val Lys Lys Lys
    210                 215                 220

Gly Lys Val Arg Gly Lys Asn Thr Gln Asp Gly Leu Tyr His Asn Lys
225                 230                 235                 240

Asn Lys Pro Pro Glu Ser Arg Lys Lys Leu Glu Lys Ala Leu Leu Ala
                245                 250                 255

Trp Ala Ile Leu Ala Ala Val Leu Leu Gln Leu Val Thr Gly Glu Asn
            260                 265                 270

Ile Thr Gln Trp Asn Leu Met Asp Asn Gly Thr Glu Gly Ile Gln Gln
        275                 280                 285

Ala Met Phe Leu Arg Gly Val Asn Arg Ser Leu His Gly Ile Trp Pro
    290                 295                 300

Glu Lys Ile Cys Thr Gly Val Pro Thr His Leu Ala Thr Asp Tyr Glu
305                 310                 315                 320

Leu Lys Glu Ile Val Gly Met Met Asp Ala Ser Glu Lys Thr Asn Tyr
                325                 330                 335

Thr Cys Cys Arg Leu Gln Arg His Glu Trp Asn Lys Gly Trp Cys Asn
            340                 345                 350

Trp Phe His Ile Glu Pro Trp Ile Trp Leu Met Asn Lys Thr Gln Asn
```

355 360 365

Asn Leu Thr Glu Gly Gln Pro Leu Arg Glu Cys Ala Val Thr Cys Arg
370 375 380

Tyr Asp Lys Glu Thr Glu Leu Asn Ile Val Thr Gln Ala Arg Asp Arg
385 390 395 400

Pro Thr Thr Leu Thr Gly Cys Lys Lys Gly Lys Asn Phe Ser Phe Ala
405 410 415

Gly Val Ile Leu Asp Gly Pro Cys Asn Phe Lys Val Ser Val Glu Asp
420 425 430

Val Leu Phe Lys Glu His Asp Cys Gly Asn Met Leu Gln Glu Thr Ala
435 440 445

Ile Gln Leu Leu Asp Gly Ala Thr Asn Thr Ile Glu Gly Ala Arg Val
450 455 460

Gly Thr Ala Lys Leu Thr Thr Trp Leu Gly Lys Gln Leu Gly Ile Leu
465 470 475 480

Gly Lys Lys Leu Glu Asn Lys Ser Lys Ala Trp Phe Gly Ala His Ala
485 490 495

Ala Ser Pro Tyr Cys Gly Val Glu Arg Lys Ile Gly Tyr Val Trp Tyr
500 505 510

Thr Lys Asn Cys Thr Pro Ala Cys Leu Pro Arg Asn Thr Arg Ile Ile
515 520 525

Gly Pro Gly Lys Phe Asp Thr Asn Ala Glu Asp Gly Lys Ile Leu His
530 535 540

Glu Met Gly Gly His Leu Ser Glu Phe Val Leu Leu Ser Leu Val Val
545 550 555 560

Leu Ser Asp Phe Ala Pro Glu Thr Ala Ser Val Ile Tyr Leu Val Leu
565 570 575

His Phe Ala Ile Pro Gln Ser His Val Asp Val Asp Thr Cys Asp Lys
580 585 590

Asn Gln Leu Asn Leu Thr Val Ala Thr Thr Val Ala Glu Val Ile Pro
595 600 605

Gly Thr Val Trp Asn Leu Gly Lys Tyr Val Cys Ile Arg Pro Asp Trp
610 615 620

Trp Pro Tyr Glu Thr Thr Thr Val Phe Val Ile Glu Glu Ala Gly Gln
625 630 635 640

Val Ile Lys Leu Met Leu Arg Ala Ile Arg Asp Leu Thr Arg Ile Trp
645 650 655

Asn Ala Ala Thr Thr Thr Ala Phe Leu Ile Phe Leu Val Lys Ala Leu

660 665 670

Arg Gly Gln Leu Ile Gln Gly Leu Leu Trp Leu Met Leu Ile Thr Gly
675 680 685

Ala Gln Gly Phe Pro Glu Cys Lys Glu Gly Phe Gln Tyr Ala Ile Ser
690 695 700

Lys Asp Arg Lys Met Gly Leu Leu Gly Pro Glu Ser Leu Thr Thr Thr
705 710 715 720

Trp His Leu Pro Thr Lys Lys Ile Val Asp Ser Met Val His Val Trp
725 730 735

Cys Glu Gly Lys Asp Leu Lys Ile Leu Lys Met Cys Thr Lys Glu Glu
740 745 750

Arg Tyr Leu Val Ala Val His Glu Arg Ala Leu Ser Thr Ser Ala Glu
755 760 765

Phe Met Gln Ile Ser Asp Gly Thr Ile Gly Pro Asp Val Ile Asp Met
770 775 780

Pro Asp Asp Phe Glu Phe Gly Leu Cys Pro Cys Asp Ser Lys Pro Val
785 790 795 800

Ile Lys Gly Lys Phe Asn Ala Ser Leu Leu Asn Gly Pro Ala Phe Gln
805 810 815

Met Val Cys Pro Gln Gly Trp Thr Gly Thr Ile Glu Cys Thr Leu Ala
820 825 830

Asn Gln Asp Thr Leu Asp Thr Thr Val Ile Arg Thr Tyr Arg Arg Thr
835 840 845

Thr Pro Phe Gln Arg Arg Lys Trp Cys Thr Tyr Glu Lys Ile Ile Gly
850 855 860

Glu Asp Ile Tyr Glu Cys Ile Leu Gly Gly Asn Trp Thr Cys Ile Thr
865 870 875 880

Gly Asp His Ser Arg Leu Lys Asp Gly Pro Ile Lys Lys Cys Lys Trp
885 890 895

Cys Gly His Asp Phe Val Asn Ser Glu Gly Leu Pro His Tyr Pro Ile
900 905 910

Gly Lys Cys Met Leu Ile Asn Glu Ser Gly Tyr Arg Tyr Val Asp Asp
915 920 925

Thr Ser Cys Asp Arg Gly Gly Val Ala Ile Val Pro Ser Gly Thr Val
930 935 940

Lys Cys Arg Ile Gly Asn Val Thr Val Gln Val Ile Ala Thr Asn Asn
945 950 955 960

Asp Leu Gly Pro Met Pro Cys Ser Pro Ala Glu Val Ile Ala Ser Glu

965 970 975

Gly Pro Val Glu Lys Thr Ala Cys Thr Phe Asn Tyr Ser Arg Thr Leu
980 985 990

Pro Asn Lys Tyr Tyr Glu Pro Arg Asp Arg Tyr Phe Gln Gln Tyr Met
995 1000 1005

Leu Lys Gly Glu Trp Gln Tyr Trp Phe Asp Leu Asp Ser Val Asp
1010 1015 1020

His His Lys Asp Tyr Phe Ser Glu Phe Ile Ile Ile Ala Val Val
1025 1030 1035

Ala Leu Leu Gly Gly Lys Tyr Val Leu Trp Leu Leu Ile Thr Tyr
1040 1045 1050

Thr Ile Leu Ser Glu Gln Met Ala Met Gly Ala Gly Val Asn Thr
1055 1060 1065

Glu Glu Ile Val Met Ile Gly Asn Leu Leu Thr Asp Ser Asp Ile
1070 1075 1080

Glu Val Val Val Tyr Phe Leu Leu Leu Tyr Leu Ile Val Lys Glu
1085 1090 1095

Glu Leu Ala Arg Lys Trp Ile Ile Leu Val Tyr His Ile Leu Val
1100 1105 1110

Ala Asn Pro Met Lys Thr Ile Gly Val Val Leu Leu Met Leu Gly
1115 1120 1125

Gly Val Val Lys Ala Ser Arg Ile Asn Ala Asp Asp Gln Ser Ala
1130 1135 1140

Met Asp Pro Cys Phe Leu Leu Val Thr Gly Val Val Ala Val Leu
1145 1150 1155

Met Ile Ala Arg Arg Glu Pro Ala Thr Leu Pro Leu Ile Val Ala
1160 1165 1170

Leu Leu Ala Ile Arg Thr Ser Gly Phe Leu Leu Pro Ala Ser Ile
1175 1180 1185

Asp Val Thr Val Ala Val Val Leu Ile Val Leu Leu Leu Ala Ser
1190 1195 1200

Tyr Ile Thr Asp Tyr Phe Arg Tyr Lys Lys Trp Leu Gln Leu Leu
1205 1210 1215

Phe Ser Leu Ile Ala Gly Ile Phe Ile Ile Arg Ser Leu Lys His
1220 1225 1230

Ile Asn Gln Met Glu Val Pro Glu Ile Ser Met Pro Ser Trp Arg
1235 1240 1245

Pro Leu Ala Leu Val Leu Phe Tyr Ile Thr Ser Thr Ala Ile Thr

1250 1255 1260

Thr Asn Trp Asp Ile Asp Leu Ala Gly Phe Leu Leu Gln Trp Ala
1265 1270 1275

Pro Ala Val Ile Met Met Ala Thr Met Trp Ala Asp Phe Leu Thr
1280 1285 1290

Leu Ile Ile Val Leu Pro Ser Tyr Glu Leu Ser Lys Leu Tyr Phe
1295 1300 1305

Leu Lys Asn Val Arg Thr Asp Val Glu Lys Asn Trp Leu Gly Lys
1310 1315 1320

Val Lys Tyr Arg Gln Ile Ser Ser Val Tyr Asp Ile Cys Asp Ser
1325 1330 1335

Glu Glu Ala Val Tyr Leu Phe Pro Ser Arg His Lys Ser Gly Ser
1340 1345 1350

Arg Pro Asp Phe Ile Leu Pro Phe Leu Lys Ala Val Leu Ile Ser
1355 1360 1365

Cys Ile Ser Ser Gln Trp Gln Val Val Tyr Ile Ser Tyr Leu Ile
1370 1375 1380

Leu Glu Ile Thr Tyr Tyr Met His Arg Lys Ile Ile Asp Glu Val
1385 1390 1395

Ser Gly Gly Ala Asn Phe Leu Ser Arg Leu Ile Ala Ala Ile Ile
1400 1405 1410

Glu Leu Asn Trp Ala Ile Asp Asp Glu Glu Cys Lys Gly Leu Lys
1415 1420 1425

Lys Leu Tyr Leu Leu Ser Gly Arg Ala Lys Asn Leu Ile Val Lys
1430 1435 1440

His Lys Val Arg Asn Glu Ala Val His Arg Trp Phe Gly Glu Glu
1445 1450 1455

Glu Ile Tyr Gly Ala Pro Lys Val Ile Thr Ile Ile Lys Ala Ser
1460 1465 1470

Thr Leu Ser Lys Asn Arg His Cys Ile Ile Cys Thr Ile Cys Glu
1475 1480 1485

Gly Lys Glu Trp Asn Gly Ala Asn Cys Pro Lys Cys Gly Arg Gln
1490 1495 1500

Gly Lys Pro Ile Thr Cys Gly Met Thr Leu Ala Asp Phe Glu Glu
1505 1510 1515

Lys His Tyr Lys Lys Ile Phe Ile Arg Glu Glu Ser Ser Cys Pro
1520 1525 1530

Val Pro Phe Asp Pro Ser Cys His Cys Asn Tyr Phe Arg His Asp

1535 1540 1545

Gly Pro Phe Arg Lys Glu Tyr Lys Gly Tyr Val Gln Tyr Thr Ala
1550 1555 1560

Arg Gly Gln Leu Phe Leu Arg Asn Leu Pro Ile Leu Ala Thr Lys
1565 1570 1575

Met Lys Leu Leu Met Val Gly Asn Leu Gly Ala Glu Ile Gly Asp
1580 1585 1590

Leu Glu His Leu Gly Trp Val Leu Arg Gly Pro Ala Val Cys Lys
1595 1600 1605

Lys Ile Thr Asn His Glu Lys Cys His Val Asn Ile Met Asp Lys
1610 1615 1620

Leu Thr Ala Phe Phe Gly Ile Met Pro Arg Gly Thr Thr Pro Arg
1625 1630 1635

Ala Pro Val Arg Phe Pro Thr Ala Leu Leu Lys Val Arg Arg Gly
1640 1645 1650

Leu Glu Thr Gly Trp Ala Tyr Thr His Gln Gly Gly Ile Ser Ser
1655 1660 1665

Val Asp His Val Thr Ala Gly Lys Asp Leu Leu Val Cys Asp Ser
1670 1675 1680

Met Gly Arg Thr Arg Val Val Cys His Ser Asn Asn Lys Met Thr
1685 1690 1695

Asp Glu Thr Glu Tyr Gly Ile Lys Thr Asp Ser Gly Cys Pro Glu
1700 1705 1710

Gly Ala Arg Cys Tyr Val Leu Asn Pro Glu Ala Val Asn Ile Ser
1715 1720 1725

Gly Thr Lys Gly Ala Met Val His Leu Gln Lys Thr Gly Gly Glu
1730 1735 1740

Phe Thr Cys Val Thr Ala Ser Gly Thr Pro Ala Phe Phe Asp Leu
1745 1750 1755

Lys Asn Leu Lys Gly Trp Ser Gly Leu Pro Ile Phe Glu Ala Ser
1760 1765 1770

Ser Gly Arg Val Val Gly Arg Val Lys Val Gly Lys Asn Glu Asp
1775 1780 1785

Ser Lys Pro Thr Lys Leu Met Ser Gly Ile Gln Thr Val Ser Lys
1790 1795 1800

Asn Gln Thr Asp Leu Ala Asp Ile Val Lys Lys Leu Thr Ser Met
1805 1810 1815

Asn Arg Gly Glu Phe Lys Gln Ile Thr Leu Ala Thr Gly Ala Gly

1820 1825 1830

Lys Thr Thr Glu Leu Pro Arg Ser Val Ile Glu Glu Ile Gly Arg
1835 1840 1845

His Lys Arg Val Leu Val Leu Ile Pro Leu Arg Ala Ala Ala Glu
1850 1855 1860

Ser Val Tyr Gln Tyr Met Arg Val Lys Tyr Pro Ser Ile Ser Phe
1865 1870 1875

Asn Leu Arg Ile Gly Asp Met Lys Glu Gly Asp Met Ala Thr Gly
1880 1885 1890

Ile Thr Tyr Ala Ser Tyr Gly Tyr Phe Cys Gln Leu Pro Gln Pro
1895 1900 1905

Lys Leu Arg Ala Ala Met Val Glu Tyr Ser Tyr Ile Phe Leu Asp
1910 1915 1920

Glu Tyr His Cys Ala Thr Pro Glu Gln Leu Ala Ile Ile Gly Lys
1925 1930 1935

Ile His Arg Phe Ala Glu Asn Leu Arg Val Val Ala Met Thr Ala
1940 1945 1950

Thr Pro Ala Gly Thr Val Thr Thr Thr Gly Gln Lys His Pro Ile
1955 1960 1965

Glu Glu Phe Ile Ala Pro Glu Val Met Lys Gly Glu Asp Leu Gly
1970 1975 1980

Ser Glu Tyr Leu Asp Ile Ala Gly Leu Lys Ile Pro Thr Glu Glu
1985 1990 1995

Met Lys Gly Asn Met Leu Val Phe Ala Pro Thr Arg Asn Met Ala
2000 2005 2010

Val Glu Thr Ala Lys Lys Leu Lys Ala Lys Gly Tyr Asn Ser Gly
2015 2020 2025

Tyr Tyr Tyr Ser Gly Glu Asn Pro Glu Asn Leu Arg Val Val Thr
2030 2035 2040

Ser Gln Ser Pro Tyr Val Val Val Ala Thr Asn Ala Ile Glu Ser
2045 2050 2055

Gly Val Thr Leu Pro Asp Leu Asp Thr Val Val Asp Thr Gly Leu
2060 2065 2070

Lys Cys Glu Lys Arg Val Arg Ile Ser Ser Lys Met Pro Phe Ile
2075 2080 2085

Val Thr Gly Leu Lys Arg Met Ala Val Thr Ile Gly Glu Gln Ala
2090 2095 2100

Gln Arg Arg Gly Arg Val Gly Arg Val Lys Pro Gly Arg Tyr Tyr

2105 2110 2115

Arg Ser Gln Glu Thr Ala Ser Gly Ser Lys Asp Tyr His Tyr Asp
2120 2125 2130

Leu Leu Gln Ala Gln Arg Tyr Gly Ile Glu Asp Gly Ile Asn Val
2135 2140 2145

Thr Lys Ser Phe Arg Glu Met Asn Tyr Asp Trp Ser Leu Tyr Glu
2150 2155 2160

Glu Asp Ser Leu Met Ile Thr Gln Leu Glu Val Leu Asn Asn Leu
2165 2170 2175

Leu Ile Ser Glu Asp Leu Pro Ala Ala Val Lys Asn Ile Met Ala
2180 2185 2190

Arg Thr Asp His Pro Glu Pro Ile Gln Leu Ala Tyr Asn Ser Tyr
2195 2200 2205

Glu Asn Gln Ile Pro Val Leu Phe Pro Lys Ile Lys Asn Gly Glu
2210 2215 2220

Val Thr Asp Ser Tyr Glu Asn Tyr Thr Tyr Leu Asn Ala Arg Lys
2225 2230 2235

Leu Gly Glu Asp Val Pro Ala Tyr Val Tyr Ala Thr Glu Asp Glu
2240 2245 2250

Asp Leu Ala Val Asp Leu Leu Gly Met Asp Trp Pro Asp Pro Gly
2255 2260 2265

Asn Gln Gln Val Val Glu Thr Gly Arg Ala Leu Lys Gln Val Thr
2270 2275 2280

Gly Leu Ser Thr Ala Glu Asn Ala Leu Leu Ile Ala Leu Phe Gly
2285 2290 2295

Tyr Val Gly Tyr Gln Thr Leu Ser Lys Arg His Ile Pro Met Ile
2300 2305 2310

Thr Asp Ile Tyr Thr Leu Glu Asp His Arg Leu Glu Asp Thr Thr
2315 2320 2325

His Leu Gln Phe Ala Pro Asn Ala Ile Arg Thr Asp Gly Lys Asp
2330 2335 2340

Ser Glu Leu Lys Glu Leu Ala Val Gly Asp Leu Asp Lys Tyr Val
2345 2350 2355

Asp Ala Leu Val Asp Tyr Ser Lys Gln Gly Met Lys Phe Ile Lys
2360 2365 2370

Val Gln Ala Glu Lys Val Arg Asp Ser Gln Ser Thr Lys Glu Gly
2375 2380 2385

Leu Gln Thr Ile Lys Glu Tyr Val Asp Lys Phe Ile Gln Ser Leu

2390 2395 2400

Thr Glu Asn Lys Glu Glu Ile Ile Arg Tyr Gly Leu Trp Gly Val
2405 2410 2415

His Thr Ala Leu Tyr Lys Ser Leu Ala Ala Arg Leu Gly His Glu
2420 2425 2430

Thr Ala Phe Ala Thr Leu Val Val Lys Trp Leu Ala Phe Gly Gly
2435 2440 2445

Glu Thr Val Ser Ala His Ile Lys Gln Val Ala Val Asp Leu Val
2450 2455 2460

Val Tyr Tyr Ile Ile Asn Lys Pro Ser Phe Pro Gly Asp Thr Glu
2465 2470 2475

Thr Gln Gln Glu Gly Arg Arg Phe Val Ala Ser Leu Phe Ile Ser
2480 2485 2490

Ala Leu Ala Thr Tyr Thr Tyr Lys Thr Trp Asn Tyr Asn Asn Leu
2495 2500 2505

Gln Arg Val Val Glu Pro Ala Leu Ala Tyr Leu Pro Tyr Ala Thr
2510 2515 2520

Ser Ala Leu Lys Leu Phe Thr Pro Thr Arg Leu Glu Ser Val Val
2525 2530 2535

Ile Leu Ser Ser Thr Ile Tyr Lys Thr Tyr Leu Ser Ile Arg Lys
2540 2545 2550

Gly Lys Ser Asp Gly Leu Leu Gly Thr Gly Ile Ser Ala Ala Met
2555 2560 2565

Glu Ile Leu Asn Gln Asn Pro Ile Ser Val Gly Ile Ser Val Met
2570 2575 2580

Leu Gly Val Gly Ala Ile Ala Ala His Asn Ala Ile Glu Ser Ser
2585 2590 2595

Glu Gln Lys Arg Thr Leu Leu Met Lys Val Phe Val Lys Asn Phe
2600 2605 2610

Leu Asp Gln Ala Ala Thr Asp Glu Leu Val Lys Glu Asn Pro Glu
2615 2620 2625

Lys Ile Ile Met Ala Leu Phe Glu Ala Val Gln Thr Ile Gly Asn
2630 2635 2640

Pro Leu Arg Leu Ile Tyr His Leu Tyr Gly Val Tyr Tyr Lys Gly
2645 2650 2655

Trp Glu Ala Lys Glu Leu Ala Glu Lys Thr Ala Gly Arg Asn Leu
2660 2665 2670

Phe Thr Leu Ile Met Phe Glu Ala Phe Glu Leu Leu Gly Met Asp

2675 2680 2685

Ser Glu Gly Lys Ile Arg Asn Leu Ser Gly Asn Tyr Ile Leu Asp
2690 2695 2700

Leu Ile Phe Asn Leu His Asn Lys Leu Asn Lys Gly Leu Lys Lys
2705 2710 2715

Leu Val Leu Gly Trp Ala Pro Ala Pro Leu Ser Cys Asp Trp Thr
2720 2725 2730

Pro Ser Asp Glu Arg Ile Ser Leu Pro His Asn Asn Tyr Leu Arg
2735 2740 2745

Val Glu Thr Arg Cys Pro Cys Gly Tyr Glu Met Lys Ala Ile Lys
2750 2755 2760

Asn Val Ala Gly Lys Leu Thr Lys Val Glu Glu Lys Gly Ser Phe
2765 2770 2775

Leu Cys Arg Asn Arg Leu Gly Arg Gly Pro Pro Asn Phe Lys Val
2780 2785 2790

Thr Lys Phe Tyr Asp Asp Asn Leu Ile Glu Val Lys Pro Val Ala
2795 2800 2805

Arg Leu Glu Gly Gln Val Asp Leu Tyr Tyr Lys Gly Val Thr Ala
2810 2815 2820

Lys Leu Asp Tyr Asn Asn Gly Lys Val Leu Leu Ala Thr Asn Lys
2825 2830 2835

Trp Glu Val Asp His Ala Phe Leu Thr Arg Leu Val Lys Lys His
2840 2845 2850

Thr Gly Ile Gly Phe Lys Gly Ala Tyr Leu Gly Asp Arg Pro Asp
2855 2860 2865

His Gln Asp Leu Val Asp Arg Asp Cys Ala Thr Ile Thr Lys Asn
2870 2875 2880

Ser Val Gln Phe Leu Lys Met Lys Lys Gly Cys Ala Phe Thr Tyr
2885 2890 2895

Asp Leu Thr Ile Ser Asn Leu Val Arg Leu Ile Glu Leu Val His
2900 2905 2910

Lys Asn Asn Leu Gln Glu Arg Glu Ile Pro Thr Val Thr Val Thr
2915 2920 2925

Thr Trp Leu Ala Tyr Ser Phe Val Asn Glu Asp Leu Gly Thr Ile
2930 2935 2940

Lys Pro Val Leu Gly Glu Lys Val Ile Pro Glu Pro Pro Glu Glu
2945 2950 2955

Leu Ser Leu Gln Pro Thr Val Arg Leu Val Thr Thr Glu Thr Ala

2960 2965 2970

Ile Thr Ile Thr Gly Glu Ala Glu Val Met Thr Thr Gly Ile Thr
2975 2980 2985

Pro Val Val Glu Met Lys Glu Glu Pro Gln Leu Asp His Gln Ser
2990 2995 3000

Thr Thr Leu Lys Val Gly Leu Lys Glu Gly Glu Tyr Pro Gly Pro
3005 3010 3015

Gly Val Asn Pro Asn His Leu Ala Glu Val Ile Asp Glu Lys Asp
3020 3025 3030

Asp Arg Pro Phe Val Leu Ile Ile Gly Asn Lys Gly Ser Thr Ser
3035 3040 3045

Asn Arg Ala Arg Thr Ala Lys Asn Ile Arg Leu Tyr Lys Gly Asn
3050 3055 3060

Asn Pro Arg Glu Ile Arg Asp Leu Met Ser Gln Gly Arg Ile Leu
3065 3070 3075

Thr Val Ala Leu Lys Glu Leu Asp Pro Glu Leu Lys Glu Leu Val
3080 3085 3090

Asp Tyr Lys Gly Thr Phe Leu Asn Arg Glu Ala Leu Glu Ala Leu
3095 3100 3105

Ser Leu Gly Lys Pro Ile Lys Arg Lys Thr Thr Thr Ala Met Ile
3110 3115 3120

Arg Arg Leu Ile Glu Pro Glu Val Glu Glu Glu Leu Pro Asp Trp
3125 3130 3135

Phe Gln Ala Glu Glu Pro Leu Phe Leu Glu Ala Lys Ile Gln Asn
3140 3145 3150

Asp Leu Tyr His Leu Ile Gly Ser Val Asp Ser Ile Lys Ser Lys
3155 3160 3165

Ala Lys Glu Leu Gly Ala Thr Asp Asn Thr Lys Ile Val Lys Glu
3170 3175 3180

Val Gly Ala Arg Thr Tyr Thr Met Lys Leu Ser Ser Trp Ser Thr
3185 3190 3195

Gln Val Thr Lys Lys Gln Met Ser Leu Ala Pro Leu Phe Glu Glu
3200 3205 3210

Leu Leu Leu Lys Cys Pro Pro Cys Ser Lys Ile Ser Lys Gly His
3215 3220 3225

Met Val Ser Ala Tyr Gln Leu Ala Gln Gly Asn Trp Glu Pro Leu
3230 3235 3240

Gly Cys Gly Val Tyr Met Gly Thr Ile Pro Ala Arg Arg Leu Lys

3245 3250 3255

Ile His Pro Tyr Glu Ala Tyr Leu Lys Leu Lys Glu Leu Val Glu
3260 3265 3270

Val Glu Ser Ser Arg Ala Thr Ala Lys Glu Ser Ile Ile Arg Glu
3275 3280 3285

His Asn Thr Trp Ile Leu Arg Lys Val Arg His Glu Gly Asn Leu
3290 3295 3300

Arg Thr Lys Ser Met Ile Asn Pro Gly Lys Ile Ser Asp Gln Leu
3305 3310 3315

Cys Arg Asp Gly His Lys Arg Asn Ile Tyr Asn Lys Ile Ile Gly
3320 3325 3330

Ser Thr Met Ala Ser Ala Gly Ile Arg Leu Glu Lys Leu Pro Val
3335 3340 3345

Val Arg Ala Gln Thr Asp Thr Thr Ser Phe His Gln Ala Ile Arg
3350 3355 3360

Glu Lys Ile Asp Lys Thr Glu Asn Lys Gln Thr Pro Glu Leu His
3365 3370 3375

Glu Glu Leu Met Lys Val Phe Asp Cys Leu Lys Ile Pro Glu Leu
3380 3385 3390

Lys Glu Ser Tyr Asp Glu Val Ser Trp Glu Gln Leu Glu Ala Gly
3395 3400 3405

Ile Asn Arg Lys Gly Ala Ala Gly Tyr Leu Glu Ser Lys Asn Ile
3410 3415 3420

Gly Glu Val Leu Asp Thr Glu Lys His Ile Val Glu Gln Leu Ile
3425 3430 3435

Lys Asp Leu Arg Lys Gly Lys Lys Ile Arg Tyr Tyr Glu Thr Ala
3440 3445 3450

Ile Pro Lys Asn Glu Lys Arg Asp Val Ser Asp Asp Trp Glu Ala
3455 3460 3465

Gly Glu Phe Val Asp Glu Lys Lys Pro Arg Val Ile Gln Tyr Pro
3470 3475 3480

Asp Ala Lys Val Arg Leu Ala Ile Thr Lys Val Met Tyr Lys Trp
3485 3490 3495

Val Lys Gln Lys Pro Val Val Ile Pro Gly Tyr Glu Gly Lys Thr
3500 3505 3510

Pro Leu Phe Asp Ile Phe Asn Lys Val Lys Lys Glu Trp Asp Ser
3515 3520 3525

Phe Gln Asp Pro Val Ala Val Ser Phe Asp Thr Lys Ala Trp Asp

3530 3535 3540

Thr Gln Val Thr Ser Arg Asp Leu Met Leu Ile Lys Asp Ile Gln
3545 3550 3555

Lys Tyr Tyr Phe Lys Arg Ser Ile His Lys Phe Leu Asp Thr Ile
3560 3565 3570

Thr Glu His Met Val Glu Val Pro Val Ile Thr Ala Asp Gly Glu
3575 3580 3585

Val Tyr Ile Arg Asn Gly Gln Arg Gly Ser Gly Gln Pro Asp Thr
3590 3595 3600

Ser Ala Gly Asn Ser Met Leu Asn Val Leu Thr Met Ile Tyr Ala
3605 3610 3615

Phe Cys Lys Ser Thr Gly Ile Pro Tyr Arg Gly Phe Ser Arg Val
3620 3625 3630

Ala Arg Ile His Val Cys Gly Asp Asp Gly Phe Leu Ile Thr Glu
3635 3640 3645

Arg Gly Leu Gly Leu Lys Phe Ser Glu Lys Gly Met Gln Ile Leu
3650 3655 3660

His Glu Ala Gly Lys Pro Gln Lys Ile Thr Glu Gly Asp Lys Met
3665 3670 3675

Lys Val Ala Tyr Arg Phe Glu Asp Ile Glu Phe Cys Ser His Thr
3680 3685 3690

Pro Val Pro Val Arg Trp Ala Asp Asn Thr Ser Ser Tyr Met Ala
3695 3700 3705

Gly Arg Ser Thr Ala Thr Ile Leu Ala Lys Met Ala Thr Arg Leu
3710 3715 3720

Asp Ser Ser Gly Glu Arg Gly Ser Thr Ala Tyr Glu Lys Ala Val
3725 3730 3735

Ala Phe Ser Phe Leu Leu Met Tyr Ser Trp Asn Pro Val Val Arg
3740 3745 3750

Arg Ile Cys Leu Leu Val Leu Ser Gln Phe Pro Glu Ile Ser Pro
3755 3760 3765

Ser Lys Asn Thr Ile Tyr Tyr Tyr Gln Gly Asp Pro Ile Ala Ala
3770 3775 3780

Tyr Arg Glu Val Ile Gly Lys Gln Leu Cys Glu Leu Lys Arg Thr
3785 3790 3795

Gly Phe Glu Lys Leu Ala Gly Leu Asn Leu Ser Met Thr Thr Leu
3800 3805 3810

Gly Ile Trp Thr Lys His Thr Ser Lys Arg Leu Ile Gln Ala Cys

3815 3820 3825

Val Glu Ile Gly Lys Arg Glu Gly Thr Trp Leu Val Asn Ala Asp
3830 3835 3840

Arg Leu Ile Ala Gly Lys Thr Gly Lys Phe Tyr Ile Pro Ser Thr
3845 3850 3855

Gly Val Thr Leu Leu Gly Lys His Tyr Glu Glu Ile Asn Leu Lys
3860 3865 3870

Gln Lys Ala Ala Gln Pro Pro Ile Glu Gly Val Asp Arg Tyr Lys
3875 3880 3885

Leu Gly Pro Ile Val Asn Val Ile Leu Arg Arg Leu Arg Val Met
3890 3895 3900

Leu Met Thr Val Ala Ser Gly Ser Trp
3905 3910

<210> 8
<211> 3748
<212> PRT
<213> Artificial Sequence: Mutated BVDV: XIKE-B-NdN

<400> 8

Met Glu Leu Phe Ser Asp Glu Gly Ser Lys Gly Ala Thr Ser Lys Lys
1 5 10 15

Gln Pro Lys Pro Asp Arg Ile Glu Lys Gly Lys Met Lys Ile Ala Pro
20 25 30

Lys Glu Thr Glu Lys Asp Cys Lys Thr Arg Pro Pro Asp Ala Thr Ile
35 40 45

Val Val Glu Gly Val Lys Tyr Gln Val Lys Lys Lys Gly Lys Val Arg
50 55 60

Gly Lys Asn Thr Gln Asp Gly Leu Tyr His Asn Lys Asn Lys Pro Pro
65 70 75 80

Glu Ser Arg Lys Lys Leu Glu Lys Ala Leu Leu Ala Trp Ala Ile Leu
85 90 95

Ala Ala Val Leu Leu Gln Leu Val Thr Gly Glu Asn Ile Thr Gln Trp
100 105 110

Asn Leu Met Asp Asn Gly Thr Glu Gly Ile Gln Gln Ala Met Phe Leu
115 120 125

Arg Gly Val Asn Arg Ser Leu His Gly Ile Trp Pro Glu Lys Ile Cys
130 135 140

Thr Gly Val Pro Thr His Leu Ala Thr Asp Tyr Glu Leu Lys Glu Ile
145 150 155 160

Val Gly Met Met Asp Ala Ser Glu Lys Thr Asn Tyr Thr Cys Cys Arg
165 170 175

Leu Gln Arg His Glu Trp Asn Lys Gly Trp Cys Asn Trp Phe His Ile
180 185 190

Glu Pro Trp Ile Trp Leu Met Asn Lys Thr Gln Asn Asn Leu Thr Glu
195 200 205

Gly Gln Pro Leu Arg Glu Cys Ala Val Thr Cys Arg Tyr Asp Lys Glu
210 215 220

Thr Glu Leu Asn Ile Val Thr Gln Ala Arg Asp Arg Pro Thr Thr Leu
225 230 235 240

Thr Gly Cys Lys Lys Gly Lys Asn Phe Ser Phe Ala Gly Val Ile Leu
245 250 255

Asp Gly Pro Cys Asn Phe Lys Val Ser Val Glu Asp Val Leu Phe Lys
260 265 270

Glu His Asp Cys Gly Asn Met Leu Gln Glu Thr Ala Ile Gln Leu Leu
275 280 285

Asp Gly Ala Thr Asn Thr Ile Glu Gly Ala Arg Val Gly Thr Ala Lys
290 295 300

Leu Thr Thr Trp Leu Gly Lys Gln Leu Gly Ile Leu Gly Lys Lys Leu
305 310 315 320

Glu Asn Lys Ser Lys Ala Trp Phe Gly Ala His Ala Ala Ser Pro Tyr
325 330 335

Cys Gly Val Glu Arg Lys Ile Gly Tyr Val Trp Tyr Thr Lys Asn Cys
340 345 350

Thr Pro Ala Cys Leu Pro Arg Asn Thr Arg Ile Ile Gly Pro Gly Lys
355 360 365

Phe Asp Thr Asn Ala Glu Asp Gly Lys Ile Leu His Glu Met Gly Gly
370 375 380

His Leu Ser Glu Phe Val Leu Leu Ser Leu Val Val Leu Ser Asp Phe
385 390 395 400

Ala Pro Glu Thr Ala Ser Val Ile Tyr Leu Val Leu His Phe Ala Ile
405 410 415

Pro Gln Ser His Val Asp Val Asp Thr Cys Asp Lys Asn Gln Leu Asn
420 425 430

Leu Thr Val Ala Thr Thr Val Ala Glu Val Ile Pro Gly Thr Val Trp
435 440 445

Asn Leu Gly Lys Tyr Val Cys Ile Arg Pro Asp Trp Trp Pro Tyr Glu
450 455 460

Thr Thr Thr Val Phe Val Ile Glu Glu Ala Gly Gln Val Ile Lys Leu
465 470 475 480

Met Leu Arg Ala Ile Arg Asp Leu Thr Arg Ile Trp Asn Ala Ala Thr
485 490 495

Thr Thr Ala Phe Leu Ile Phe Leu Val Lys Ala Leu Arg Gly Gln Leu
500 505 510

Ile Gln Gly Leu Leu Trp Leu Met Leu Ile Thr Gly Ala Gln Gly Phe
515 520 525

Pro Glu Cys Lys Glu Gly Phe Gln Tyr Ala Ile Ser Lys Asp Arg Lys
530 535 540

Met Gly Leu Leu Gly Pro Glu Ser Leu Thr Thr Thr Trp His Leu Pro
545 550 555 560

Thr Lys Lys Ile Val Asp Ser Met Val His Val Trp Cys Glu Gly Lys
565 570 575

Asp Leu Lys Ile Leu Lys Met Cys Thr Lys Glu Glu Arg Tyr Leu Val
580 585 590

Ala Val His Glu Arg Ala Leu Ser Thr Ser Ala Glu Phe Met Gln Ile
595 600 605

Ser Asp Gly Thr Ile Gly Pro Asp Val Ile Asp Met Pro Asp Asp Phe
610 615 620

Glu Phe Gly Leu Cys Pro Cys Asp Ser Lys Pro Val Ile Lys Gly Lys
625 630 635 640

Phe Asn Ala Ser Leu Leu Asn Gly Pro Ala Phe Gln Met Val Cys Pro
645 650 655

Gln Gly Trp Thr Gly Thr Ile Glu Cys Thr Leu Ala Asn Gln Asp Thr
660 665 670

Leu Asp Thr Thr Val Ile Arg Thr Tyr Arg Arg Thr Thr Pro Phe Gln
675 680 685

Arg Arg Lys Trp Cys Thr Tyr Glu Lys Ile Ile Gly Glu Asp Ile Tyr
690 695 700

Glu Cys Ile Leu Gly Gly Asn Trp Thr Cys Ile Thr Gly Asp His Ser
705 710 715 720

Arg Leu Lys Asp Gly Pro Ile Lys Lys Cys Lys Trp Cys Gly His Asp
725 730 735

Phe Val Asn Ser Glu Gly Leu Pro His Tyr Pro Ile Gly Lys Cys Met
740 745 750

Leu Ile Asn Glu Ser Gly Tyr Arg Tyr Val Asp Asp Thr Ser Cys Asp
755 760 765

```
Arg Gly Gly Val Ala Ile Val Pro Ser Gly Thr Val Lys Cys Arg Ile
    770                 775                 780

Gly Asn Val Thr Val Gln Val Ile Ala Thr Asn Asn Asp Leu Gly Pro
785                 790                 795                 800

Met Pro Cys Ser Pro Ala Glu Val Ile Ala Ser Glu Gly Pro Val Glu
                805                 810                 815

Lys Thr Ala Cys Thr Phe Asn Tyr Ser Arg Thr Leu Pro Asn Lys Tyr
            820                 825                 830

Tyr Glu Pro Arg Asp Arg Tyr Phe Gln Gln Tyr Met Leu Lys Gly Glu
        835                 840                 845

Trp Gln Tyr Trp Phe Asp Leu Asp Ser Val Asp His His Lys Asp Tyr
    850                 855                 860

Phe Ser Glu Phe Ile Ile Ile Ala Val Val Ala Leu Leu Gly Gly Lys
865                 870                 875                 880

Tyr Val Leu Trp Leu Leu Ile Thr Tyr Thr Ile Leu Ser Glu Gln Met
                885                 890                 895

Ala Met Gly Ala Gly Val Asn Thr Glu Glu Ile Val Met Ile Gly Asn
            900                 905                 910

Leu Leu Thr Asp Ser Asp Ile Glu Val Val Val Tyr Phe Leu Leu Leu
        915                 920                 925

Tyr Leu Ile Val Lys Glu Glu Leu Ala Arg Lys Trp Ile Ile Leu Val
    930                 935                 940

Tyr His Ile Leu Val Ala Asn Pro Met Lys Thr Ile Gly Val Val Leu
945                 950                 955                 960

Leu Met Leu Gly Gly Val Val Lys Ala Ser Arg Ile Asn Ala Asp Asp
                965                 970                 975

Gln Ser Ala Met Asp Pro Cys Phe Leu Leu Val Thr Gly Val Val Ala
            980                 985                 990

Val Leu Met Ile Ala Arg Arg Glu  Pro Ala Thr Leu Pro  Leu Ile Val
        995                 1000                1005

Ala Leu  Leu Ala Ile Arg Thr  Ser Gly Phe Leu Leu  Pro Ala Ser
    1010                 1015                1020

Ile Asp  Val Thr Val Ala Val  Val Leu Ile Val Leu  Leu Leu Ala
    1025                 1030                1035

Ser Tyr  Ile Thr Asp Tyr Phe  Arg Tyr Lys Lys Trp  Leu Gln Leu
    1040                 1045                1050

Leu Phe  Ser Leu Ile Ala Gly  Ile Phe Ile Ile Arg  Ser Leu Lys
    1055                 1060                1065
```

His Ile Asn Gln Met Glu Val Pro Glu Ile Ser Met Pro Ser Trp
1070 1075 1080

Arg Pro Leu Ala Leu Val Leu Phe Tyr Ile Thr Ser Thr Ala Ile
1085 1090 1095

Thr Thr Asn Trp Asp Ile Asp Leu Ala Gly Phe Leu Leu Gln Trp
1100 1105 1110

Ala Pro Ala Val Ile Met Met Ala Thr Met Trp Ala Asp Phe Leu
1115 1120 1125

Thr Leu Ile Ile Val Leu Pro Ser Tyr Glu Leu Ser Lys Leu Tyr
1130 1135 1140

Phe Leu Lys Asn Val Arg Thr Asp Val Glu Lys Asn Trp Leu Gly
1145 1150 1155

Lys Val Lys Tyr Arg Gln Ile Ser Ser Val Tyr Asp Ile Cys Asp
1160 1165 1170

Ser Glu Glu Ala Val Tyr Leu Phe Pro Ser Arg His Lys Ser Gly
1175 1180 1185

Ser Arg Pro Asp Phe Ile Leu Pro Phe Leu Lys Ala Val Leu Ile
1190 1195 1200

Ser Cys Ile Ser Ser Gln Trp Gln Val Val Tyr Ile Ser Tyr Leu
1205 1210 1215

Ile Leu Glu Ile Thr Tyr Tyr Met His Arg Lys Ile Ile Asp Glu
1220 1225 1230

Val Ser Gly Gly Ala Asn Phe Leu Ser Arg Leu Ile Ala Ala Ile
1235 1240 1245

Ile Glu Leu Asn Trp Ala Ile Asp Asp Glu Glu Cys Lys Gly Leu
1250 1255 1260

Lys Lys Leu Tyr Leu Leu Ser Gly Arg Ala Lys Asn Leu Ile Val
1265 1270 1275

Lys His Lys Val Arg Asn Glu Ala Val His Arg Trp Phe Gly Glu
1280 1285 1290

Glu Glu Ile Tyr Gly Ala Pro Lys Val Ile Thr Ile Ile Lys Ala
1295 1300 1305

Ser Thr Leu Ser Lys Asn Arg His Cys Ile Ile Cys Thr Ile Cys
1310 1315 1320

Glu Gly Lys Glu Trp Asn Gly Ala Asn Cys Pro Lys Cys Gly Arg
1325 1330 1335

Gln Gly Lys Pro Ile Thr Cys Gly Met Thr Leu Ala Asp Phe Glu
1340 1345 1350

Glu Lys His Tyr Lys Lys Ile Phe Ile Arg Glu Glu Ser Ser Cys
1355 1360 1365

Pro Val Pro Phe Asp Pro Ser Cys His Cys Asn Tyr Phe Arg His
1370 1375 1380

Asp Gly Pro Phe Arg Lys Glu Tyr Lys Gly Tyr Val Gln Tyr Thr
1385 1390 1395

Ala Arg Gly Gln Leu Phe Leu Arg Asn Leu Pro Ile Leu Ala Thr
1400 1405 1410

Lys Met Lys Leu Leu Met Val Gly Asn Leu Gly Ala Glu Ile Gly
1415 1420 1425

Asp Leu Glu His Leu Gly Trp Val Leu Arg Gly Pro Ala Val Cys
1430 1435 1440

Lys Lys Ile Thr Asn His Glu Lys Cys His Val Asn Ile Met Asp
1445 1450 1455

Lys Leu Thr Ala Phe Phe Gly Ile Met Pro Arg Gly Thr Thr Pro
1460 1465 1470

Arg Ala Pro Val Arg Phe Pro Thr Ala Leu Leu Lys Val Arg Arg
1475 1480 1485

Gly Leu Glu Thr Gly Trp Ala Tyr Thr His Gln Gly Gly Ile Ser
1490 1495 1500

Ser Val Asp His Val Thr Ala Gly Lys Asp Leu Leu Val Cys Asp
1505 1510 1515

Ser Met Gly Arg Thr Arg Val Val Cys His Ser Asn Asn Lys Met
1520 1525 1530

Thr Asp Glu Thr Glu Tyr Gly Ile Lys Thr Asp Ser Gly Cys Pro
1535 1540 1545

Glu Gly Ala Arg Cys Tyr Val Leu Asn Pro Glu Ala Val Asn Ile
1550 1555 1560

Ser Gly Thr Lys Gly Ala Met Val His Leu Gln Lys Thr Gly Gly
1565 1570 1575

Glu Phe Thr Cys Val Thr Ala Ser Gly Thr Pro Ala Phe Phe Asp
1580 1585 1590

Leu Lys Asn Leu Lys Gly Trp Ser Gly Leu Pro Ile Phe Glu Ala
1595 1600 1605

Ser Ser Gly Arg Val Val Gly Arg Val Lys Val Gly Lys Asn Glu
1610 1615 1620

Asp Ser Lys Pro Thr Lys Leu Met Ser Gly Ile Gln Thr Val Ser
1625 1630 1635

Lys Asn Gln Thr Asp Leu Ala Asp Ile Val Lys Lys Leu Thr Ser
1640 1645 1650

Met Asn Arg Gly Glu Phe Lys Gln Ile Thr Leu Ala Thr Gly Ala
1655 1660 1665

Gly Lys Thr Thr Glu Leu Pro Arg Ser Val Ile Glu Glu Ile Gly
1670 1675 1680

Arg His Lys Arg Val Leu Val Leu Ile Pro Leu Arg Ala Ala Ala
1685 1690 1695

Glu Ser Val Tyr Gln Tyr Met Arg Val Lys Tyr Pro Ser Ile Ser
1700 1705 1710

Phe Asn Leu Arg Ile Gly Asp Met Lys Glu Gly Asp Met Ala Thr
1715 1720 1725

Gly Ile Thr Tyr Ala Ser Tyr Gly Tyr Phe Cys Gln Leu Pro Gln
1730 1735 1740

Pro Lys Leu Arg Ala Ala Met Val Glu Tyr Ser Tyr Ile Phe Leu
1745 1750 1755

Asp Glu Tyr His Cys Ala Thr Pro Glu Gln Leu Ala Ile Ile Gly
1760 1765 1770

Lys Ile His Arg Phe Ala Glu Asn Leu Arg Val Val Ala Met Thr
1775 1780 1785

Ala Thr Pro Ala Gly Thr Val Thr Thr Thr Gly Gln Lys His Pro
1790 1795 1800

Ile Glu Glu Phe Ile Ala Pro Glu Val Met Lys Gly Glu Asp Leu
1805 1810 1815

Gly Ser Glu Tyr Leu Asp Ile Ala Gly Leu Lys Ile Pro Thr Glu
1820 1825 1830

Glu Met Lys Gly Asn Met Leu Val Phe Ala Pro Thr Arg Asn Met
1835 1840 1845

Ala Val Glu Thr Ala Lys Lys Leu Lys Ala Lys Gly Tyr Asn Ser
1850 1855 1860

Gly Tyr Tyr Tyr Ser Gly Glu Asn Pro Glu Asn Leu Arg Val Val
1865 1870 1875

Thr Ser Gln Ser Pro Tyr Val Val Val Ala Thr Asn Ala Ile Glu
1880 1885 1890

Ser Gly Val Thr Leu Pro Asp Leu Asp Thr Val Val Asp Thr Gly
1895 1900 1905

Leu Lys Cys Glu Lys Arg Val Arg Ile Ser Ser Lys Met Pro Phe
1910 1915 1920

Ile Val Thr Gly Leu Lys Arg Met Ala Val Thr Ile Gly Glu Gln
1925 1930 1935

Ala Gln Arg Arg Gly Arg Val Gly Arg Val Lys Pro Gly Arg Tyr
1940 1945 1950

Tyr Arg Ser Gln Glu Thr Ala Ser Gly Ser Lys Asp Tyr His Tyr
1955 1960 1965

Asp Leu Leu Gln Ala Gln Arg Tyr Gly Ile Glu Asp Gly Ile Asn
1970 1975 1980

Val Thr Lys Ser Phe Arg Glu Met Asn Tyr Asp Trp Ser Leu Tyr
1985 1990 1995

Glu Glu Asp Ser Leu Met Ile Thr Gln Leu Glu Val Leu Asn Asn
2000 2005 2010

Leu Leu Ile Ser Glu Asp Leu Pro Ala Ala Val Lys Asn Ile Met
2015 2020 2025

Ala Arg Thr Asp His Pro Glu Pro Ile Gln Leu Ala Tyr Asn Ser
2030 2035 2040

Tyr Glu Asn Gln Ile Pro Val Leu Phe Pro Lys Ile Lys Asn Gly
2045 2050 2055

Glu Val Thr Asp Ser Tyr Glu Asn Tyr Thr Tyr Leu Asn Ala Arg
2060 2065 2070

Lys Leu Gly Glu Asp Val Pro Ala Tyr Val Tyr Ala Thr Glu Asp
2075 2080 2085

Glu Asp Leu Ala Val Asp Leu Leu Gly Met Asp Trp Pro Asp Pro
2090 2095 2100

Gly Asn Gln Gln Val Val Glu Thr Gly Arg Ala Leu Lys Gln Val
2105 2110 2115

Thr Gly Leu Ser Thr Ala Glu Asn Ala Leu Leu Ile Ala Leu Phe
2120 2125 2130

Gly Tyr Val Gly Tyr Gln Thr Leu Ser Lys Arg His Ile Pro Met
2135 2140 2145

Ile Thr Asp Ile Tyr Thr Leu Glu Asp His Arg Leu Glu Asp Thr
2150 2155 2160

Thr His Leu Gln Phe Ala Pro Asn Ala Ile Arg Thr Asp Gly Lys
2165 2170 2175

Asp Ser Glu Leu Lys Glu Leu Ala Val Gly Asp Leu Asp Lys Tyr
2180 2185 2190

Val Asp Ala Leu Val Asp Tyr Ser Lys Gln Gly Met Lys Phe Ile
2195 2200 2205

Lys Val Gln Ala Glu Lys Val Arg Asp Ser Gln Ser Thr Lys Glu
2210 2215 2220

Gly Leu Gln Thr Ile Lys Glu Tyr Val Asp Lys Phe Ile Gln Ser
2225 2230 2235

Leu Thr Glu Asn Lys Glu Glu Ile Ile Arg Tyr Gly Leu Trp Gly
2240 2245 2250

Val His Thr Ala Leu Tyr Lys Ser Leu Ala Ala Arg Leu Gly His
2255 2260 2265

Glu Thr Ala Phe Ala Thr Leu Val Val Lys Trp Leu Ala Phe Gly
2270 2275 2280

Gly Glu Thr Val Ser Ala His Ile Lys Gln Val Ala Val Asp Leu
2285 2290 2295

Val Val Tyr Tyr Ile Ile Asn Lys Pro Ser Phe Pro Gly Asp Thr
2300 2305 2310

Glu Thr Gln Gln Glu Gly Arg Arg Phe Val Ala Ser Leu Phe Ile
2315 2320 2325

Ser Ala Leu Ala Thr Tyr Thr Tyr Lys Thr Trp Asn Tyr Asn Asn
2330 2335 2340

Leu Gln Arg Val Val Glu Pro Ala Leu Ala Tyr Leu Pro Tyr Ala
2345 2350 2355

Thr Ser Ala Leu Lys Leu Phe Thr Pro Thr Arg Leu Glu Ser Val
2360 2365 2370

Val Ile Leu Ser Ser Thr Ile Tyr Lys Thr Tyr Leu Ser Ile Arg
2375 2380 2385

Lys Gly Lys Ser Asp Gly Leu Leu Gly Thr Gly Ile Ser Ala Ala
2390 2395 2400

Met Glu Ile Leu Asn Gln Asn Pro Ile Ser Val Gly Ile Ser Val
2405 2410 2415

Met Leu Gly Val Gly Ala Ile Ala Ala His Asn Ala Ile Glu Ser
2420 2425 2430

Ser Glu Gln Lys Arg Thr Leu Leu Met Lys Val Phe Val Lys Asn
2435 2440 2445

Phe Leu Asp Gln Ala Ala Thr Asp Glu Leu Val Lys Glu Asn Pro
2450 2455 2460

Glu Lys Ile Ile Met Ala Leu Phe Glu Ala Val Gln Thr Ile Gly
2465 2470 2475

Asn Pro Leu Arg Leu Ile Tyr His Leu Tyr Gly Val Tyr Tyr Lys
2480 2485 2490

Gly Trp Glu Ala Lys Glu Leu Ala Glu Lys Thr Ala Gly Arg Asn
2495 2500 2505

Leu Phe Thr Leu Ile Met Phe Glu Ala Phe Glu Leu Leu Gly Met
2510 2515 2520

Asp Ser Glu Gly Lys Ile Arg Asn Leu Ser Gly Asn Tyr Ile Leu
2525 2530 2535

Asp Leu Ile Phe Asn Leu His Asn Lys Leu Asn Lys Gly Leu Lys
2540 2545 2550

Lys Leu Val Leu Gly Trp Ala Pro Ala Pro Leu Ser Cys Asp Trp
2555 2560 2565

Thr Pro Ser Asp Glu Arg Ile Ser Leu Pro His Asn Asn Tyr Leu
2570 2575 2580

Arg Val Glu Thr Arg Cys Pro Cys Gly Tyr Glu Met Lys Ala Ile
2585 2590 2595

Lys Asn Val Ala Gly Lys Leu Thr Lys Val Glu Glu Lys Gly Ser
2600 2605 2610

Phe Leu Cys Arg Asn Arg Leu Gly Arg Gly Pro Pro Asn Phe Lys
2615 2620 2625

Val Thr Lys Phe Tyr Asp Asp Asn Leu Ile Glu Val Lys Pro Val
2630 2635 2640

Ala Arg Leu Glu Gly Gln Val Asp Leu Tyr Tyr Lys Gly Val Thr
2645 2650 2655

Ala Lys Leu Asp Tyr Asn Asn Gly Lys Val Leu Leu Ala Thr Asn
2660 2665 2670

Lys Trp Glu Val Asp His Ala Phe Leu Thr Arg Leu Val Lys Lys
2675 2680 2685

His Thr Gly Ile Gly Phe Lys Gly Ala Tyr Leu Gly Asp Arg Pro
2690 2695 2700

Asp His Gln Asp Leu Val Asp Arg Asp Cys Ala Thr Ile Thr Lys
2705 2710 2715

Asn Ser Val Gln Phe Leu Lys Met Lys Lys Gly Cys Ala Phe Thr
2720 2725 2730

Tyr Asp Leu Thr Ile Ser Asn Leu Val Arg Leu Ile Glu Leu Val
2735 2740 2745

His Lys Asn Asn Leu Gln Glu Arg Glu Ile Pro Thr Val Thr Val
2750 2755 2760

Thr Thr Trp Leu Ala Tyr Ser Phe Val Asn Glu Asp Leu Gly Thr
2765 2770 2775

Ile Lys Pro Val Leu Gly Glu Lys Val Ile Pro Glu Pro Pro Glu
2780 2785 2790

Glu Leu Ser Leu Gln Pro Thr Val Arg Leu Val Thr Thr Glu Thr
2795 2800 2805

Ala Ile Thr Ile Thr Gly Glu Ala Glu Val Met Thr Thr Gly Ile
2810 2815 2820

Thr Pro Val Val Glu Met Lys Glu Glu Pro Gln Leu Asp His Gln
2825 2830 2835

Ser Thr Thr Leu Lys Val Gly Leu Lys Glu Gly Glu Tyr Pro Gly
2840 2845 2850

Pro Gly Val Asn Pro Asn His Leu Ala Glu Val Ile Asp Glu Lys
2855 2860 2865

Asp Asp Arg Pro Phe Val Leu Ile Ile Gly Asn Lys Gly Ser Thr
2870 2875 2880

Ser Asn Arg Ala Arg Thr Ala Lys Asn Ile Arg Leu Tyr Lys Gly
2885 2890 2895

Asn Asn Pro Arg Glu Ile Arg Asp Leu Met Ser Gln Gly Arg Ile
2900 2905 2910

Leu Thr Val Ala Leu Lys Glu Leu Asp Pro Glu Leu Lys Glu Leu
2915 2920 2925

Val Asp Tyr Lys Gly Thr Phe Leu Asn Arg Glu Ala Leu Glu Ala
2930 2935 2940

Leu Ser Leu Gly Lys Pro Ile Lys Arg Lys Thr Thr Thr Ala Met
2945 2950 2955

Ile Arg Arg Leu Ile Glu Pro Glu Val Glu Glu Glu Leu Pro Asp
2960 2965 2970

Trp Phe Gln Ala Glu Glu Pro Leu Phe Leu Glu Ala Lys Ile Gln
2975 2980 2985

Asn Asp Leu Tyr His Leu Ile Gly Ser Val Asp Ser Ile Lys Ser
2990 2995 3000

Lys Ala Lys Glu Leu Gly Ala Thr Asp Asn Thr Lys Ile Val Lys
3005 3010 3015

Glu Val Gly Ala Arg Thr Tyr Thr Met Lys Leu Ser Ser Trp Ser
3020 3025 3030

Thr Gln Val Thr Lys Lys Gln Met Ser Leu Ala Pro Leu Phe Glu
3035 3040 3045

Glu Leu Leu Leu Lys Cys Pro Pro Cys Ser Lys Ile Ser Lys Gly
3050 3055 3060

His Met Val Ser Ala Tyr Gln Leu Ala Gln Gly Asn Trp Glu Pro
3065 3070 3075

Leu Gly Cys Gly Val Tyr Met Gly Thr Ile Pro Ala Arg Arg Leu
3080 3085 3090

Lys Ile His Pro Tyr Glu Ala Tyr Leu Lys Leu Lys Glu Leu Val
3095 3100 3105

Glu Val Glu Ser Ser Arg Ala Thr Ala Lys Glu Ser Ile Ile Arg
3110 3115 3120

Glu His Asn Thr Trp Ile Leu Arg Lys Val Arg His Glu Gly Asn
3125 3130 3135

Leu Arg Thr Lys Ser Met Ile Asn Pro Gly Lys Ile Ser Asp Gln
3140 3145 3150

Leu Cys Arg Asp Gly His Lys Arg Asn Ile Tyr Asn Lys Ile Ile
3155 3160 3165

Gly Ser Thr Met Ala Ser Ala Gly Ile Arg Leu Glu Lys Leu Pro
3170 3175 3180

Val Val Arg Ala Gln Thr Asp Thr Thr Ser Phe His Gln Ala Ile
3185 3190 3195

Arg Glu Lys Ile Asp Lys Thr Glu Asn Lys Gln Thr Pro Glu Leu
3200 3205 3210

His Glu Glu Leu Met Lys Val Phe Asp Cys Leu Lys Ile Pro Glu
3215 3220 3225

Leu Lys Glu Ser Tyr Asp Glu Val Ser Trp Glu Gln Leu Glu Ala
3230 3235 3240

Gly Ile Asn Arg Lys Gly Ala Ala Gly Tyr Leu Glu Ser Lys Asn
3245 3250 3255

Ile Gly Glu Val Leu Asp Thr Glu Lys His Ile Val Glu Gln Leu
3260 3265 3270

Ile Lys Asp Leu Arg Lys Gly Lys Lys Ile Arg Tyr Tyr Glu Thr
3275 3280 3285

Ala Ile Pro Lys Asn Glu Lys Arg Asp Val Ser Asp Asp Trp Glu
3290 3295 3300

Ala Gly Glu Phe Val Asp Glu Lys Lys Pro Arg Val Ile Gln Tyr
3305 3310 3315

Pro Asp Ala Lys Val Arg Leu Ala Ile Thr Lys Val Met Tyr Lys
3320 3325 3330

Trp Val Lys Gln Lys Pro Val Val Ile Pro Gly Tyr Glu Gly Lys
3335 3340 3345

Thr Pro Leu Phe Asp Ile Phe Asn Lys Val Lys Lys Glu Trp Asp
3350 3355 3360

Ser Phe Gln Asp Pro Val Ala Val Ser Phe Asp Thr Lys Ala Trp
3365 3370 3375

Asp Thr Gln Val Thr Ser Arg Asp Leu Met Leu Ile Lys Asp Ile
3380 3385 3390

Gln Lys Tyr Tyr Phe Lys Arg Ser Ile His Lys Phe Leu Asp Thr
3395 3400 3405

Ile Thr Glu His Met Val Glu Val Pro Val Ile Thr Ala Asp Gly
3410 3415 3420

Glu Val Tyr Ile Arg Asn Gly Gln Arg Gly Ser Gly Gln Pro Asp
3425 3430 3435

Thr Ser Ala Gly Asn Ser Met Leu Asn Val Leu Thr Met Ile Tyr
3440 3445 3450

Ala Phe Cys Lys Ser Thr Gly Ile Pro Tyr Arg Gly Phe Ser Arg
3455 3460 3465

Val Ala Arg Ile His Val Cys Gly Asp Asp Gly Phe Leu Ile Thr
3470 3475 3480

Glu Arg Gly Leu Gly Leu Lys Phe Ser Glu Lys Gly Met Gln Ile
3485 3490 3495

Leu His Glu Ala Gly Lys Pro Gln Lys Ile Thr Glu Gly Asp Lys
3500 3505 3510

Met Lys Val Ala Tyr Arg Phe Glu Asp Ile Glu Phe Cys Ser His
3515 3520 3525

Thr Pro Val Pro Val Arg Trp Ala Asp Asn Thr Ser Ser Tyr Met
3530 3535 3540

Ala Gly Arg Ser Thr Ala Thr Ile Leu Ala Lys Met Ala Thr Arg
3545 3550 3555

Leu Asp Ser Ser Gly Glu Arg Gly Ser Thr Ala Tyr Glu Lys Ala
3560 3565 3570

Val Ala Phe Ser Phe Leu Leu Met Tyr Ser Trp Asn Pro Val Val
3575 3580 3585

Arg Arg Ile Cys Leu Leu Val Leu Ser Gln Phe Pro Glu Ile Ser
3590 3595 3600

Pro Ser Lys Asn Thr Ile Tyr Tyr Tyr Gln Gly Asp Pro Ile Ala
3605 3610 3615

Ala Tyr Arg Glu Val Ile Gly Lys Gln Leu Cys Glu Leu Lys Arg
3620 3625 3630

Thr Gly Phe Glu Lys Leu Ala Gly Leu Asn Leu Ser Met Thr Thr
3635 3640 3645

Leu Gly Ile Trp Thr Lys His Thr Ser Lys Arg Leu Ile Gln Ala
3650 3655 3660

Cys Val Glu Ile Gly Lys Arg Glu Gly Thr Trp Leu Val Asn Ala
3665 3670 3675

Asp Arg Leu Ile Ala Gly Lys Thr Gly Lys Phe Tyr Ile Pro Ser
3680 3685 3690

Thr Gly Val Thr Leu Leu Gly Lys His Tyr Glu Glu Ile Asn Leu
3695 3700 3705

Lys Gln Lys Ala Ala Gln Pro Pro Ile Glu Gly Val Asp Arg Tyr
3710 3715 3720

Lys Leu Gly Pro Ile Val Asn Val Ile Leu Arg Arg Leu Arg Val
3725 3730 3735

Met Leu Met Thr Val Ala Ser Gly Ser Trp
3740 3745

<210> 9
<211> 3913
<212> PRT
<213> Artificial sequence:XIKE-C BVDV-Sequence

<400> 9

```
Met Glu Leu Phe Ser Asn Glu Leu Leu Tyr Lys Thr Tyr Lys Gln Lys
1               5                   10                  15

Pro Ala Gly Val Val Glu Pro Val Tyr Asp Val Asn Gly Arg Pro Leu
            20                  25                  30

Phe Gly Glu Ser Ser Asp Leu His Pro Gln Ser Thr Leu Lys Leu Pro
        35                  40                  45

His Gln Arg Gly Ser Ala Asn Ile Leu Thr Asn Ala Arg Ser Leu Pro
    50                  55                  60

Arg Lys Gly Asp Cys Arg Arg Gly Asn Val Tyr Gly Pro Val Ser Gly
65                  70                  75                  80

Ile Tyr Ile Lys Pro Gly Pro Ile Tyr Tyr Gln Asp Tyr Val Gly Pro
                85                  90                  95

Val Tyr His Arg Ala Pro Leu Glu Leu Cys Arg Glu Ala Ser Met Cys
            100                 105                 110

Glu Thr Thr Arg Arg Val Gly Arg Val Thr Gly Ser Asp Gly Lys Leu
        115                 120                 125
```

Tyr His Ile Tyr Ile Cys Ile Asp Gly Cys Ile Leu Leu Lys Arg Ala
130 135 140

Thr Arg Asn Gln Pro Glu Val Leu Lys Trp Val Tyr Asn Arg Leu Asn
145 150 155 160

Cys Pro Leu Trp Val Thr Ser Cys Ser Asp Glu Gly Ser Lys Gly Ala
165 170 175

Thr Ser Lys Lys Gln Pro Lys Pro Asp Arg Ile Glu Lys Gly Lys Met
180 185 190

Lys Ile Ala Pro Lys Glu Thr Glu Lys Asp Cys Lys Thr Arg Pro Pro
195 200 205

Asp Ala Thr Ile Val Val Glu Gly Val Lys Tyr Gln Val Lys Lys Lys
210 215 220

Gly Lys Val Arg Gly Lys Asn Thr Gln Asp Gly Leu Tyr His Asn Lys
225 230 235 240

Asn Lys Pro Pro Glu Ser Arg Lys Lys Leu Glu Lys Ala Leu Leu Ala
245 250 255

Trp Ala Ile Leu Ala Ala Val Leu Leu Gln Leu Val Thr Gly Glu Asn
260 265 270

Ile Thr Gln Trp Asn Leu Met Asp Asn Gly Thr Glu Gly Ile Gln Gln
275 280 285

Ala Met Phe Leu Arg Gly Val Asn Arg Ser Leu Leu Gly Ile Trp Pro
290 295 300

Glu Lys Ile Cys Thr Gly Val Pro Thr His Leu Ala Thr Asp Tyr Glu
305 310 315 320

Leu Lys Glu Ile Val Gly Met Met Asp Ala Ser Glu Lys Thr Asn Tyr
325 330 335

Thr Cys Cys Arg Leu Gln Arg His Glu Trp Asn Lys His Gly Trp Cys
340 345 350

Asn Trp Phe His Ile Glu Pro Trp Ile Trp Leu Met Asn Lys Thr Gln
355 360 365

Asn Asn Leu Thr Glu Gly Gln Pro Leu Arg Glu Cys Ala Val Thr Cys
370 375 380

Arg Tyr Asp Lys Glu Thr Glu Leu Asn Ile Val Thr Gln Ala Arg Asp
385 390 395 400

Arg Pro Thr Thr Leu Thr Gly Cys Lys Lys Gly Lys Asn Phe Ser Phe
405 410 415

Ala Gly Val Ile Leu Asp Gly Pro Cys Asn Phe Lys Val Ser Val Glu
420 425 430

Asp Val Leu Phe Lys Glu His Asp Cys Gly Asn Met Leu Gln Glu Thr
435 440 445

Ala Ile Gln Leu Leu Asp Gly Ala Thr Asn Thr Ile Glu Gly Ala Arg
450 455 460

Val Gly Thr Ala Lys Leu Thr Thr Trp Leu Gly Lys Gln Leu Gly Ile
465 470 475 480

Leu Gly Lys Lys Leu Glu Asn Lys Ser Lys Ala Trp Phe Gly Ala His
485 490 495

Ala Ala Ser Pro Tyr Cys Gly Val Glu Arg Lys Ile Gly Tyr Val Trp
500 505 510

Tyr Thr Lys Asn Cys Thr Pro Ala Cys Leu Pro Arg Asn Thr Arg Ile
515 520 525

Ile Gly Pro Gly Lys Phe Asp Thr Asn Ala Glu Asp Gly Lys Ile Leu
530 535 540

His Glu Met Gly Gly His Leu Ser Glu Phe Val Leu Leu Ser Leu Val
545 550 555 560

Val Leu Ser Asp Phe Ala Pro Glu Thr Ala Ser Val Ile Tyr Leu Val
565 570 575

Leu His Phe Ala Ile Pro Gln Ser His Val Asp Val Asp Thr Cys Asp
580 585 590

Lys Asn Gln Leu Asn Leu Thr Val Ala Thr Thr Val Ala Glu Val Ile
595 600 605

Pro Gly Thr Val Trp Asn Leu Gly Lys Tyr Val Cys Ile Arg Pro Asp
610 615 620

Trp Trp Pro Tyr Glu Thr Thr Thr Val Phe Val Ile Glu Glu Ala Gly
625 630 635 640

Gln Val Ile Lys Leu Met Leu Arg Ala Ile Arg Asp Leu Thr Arg Ile
645 650 655

Trp Asn Ala Ala Thr Thr Thr Ala Phe Leu Ile Phe Leu Val Lys Ala
660 665 670

Leu Arg Gly Gln Leu Ile Gln Gly Leu Leu Trp Leu Met Leu Ile Thr
675 680 685

Gly Ala Gln Gly Phe Pro Glu Cys Lys Glu Gly Phe Gln Tyr Ala Ile
690 695 700

Ser Lys Asp Arg Lys Met Gly Leu Leu Gly Pro Glu Ser Leu Thr Thr
705 710 715 720

Thr Trp His Leu Pro Thr Lys Lys Ile Val Asp Ser Met Val His Val
725 730 735

Trp Cys Glu Gly Lys Asp Leu Lys Ile Leu Lys Met Cys Thr Lys Glu
740 745 750

Glu Arg Tyr Leu Val Ala Val His Glu Arg Ala Leu Ser Thr Ser Ala
755 760 765

Glu Phe Met Gln Ile Ser Asp Gly Thr Ile Gly Pro Asp Val Ile Asp
770 775 780

Met Pro Asp Asp Phe Glu Phe Gly Leu Cys Pro Cys Asp Ser Lys Pro
785 790 795 800

Val Ile Lys Gly Lys Phe Asn Ala Ser Leu Leu Asn Gly Pro Ala Phe
805 810 815

Gln Met Val Cys Pro Gln Gly Trp Thr Gly Thr Ile Glu Cys Thr Leu
820 825 830

Ala Asn Gln Asp Thr Leu Asp Thr Thr Val Ile Arg Thr Tyr Arg Arg
835 840 845

Thr Thr Pro Phe Gln Arg Arg Lys Trp Cys Thr Tyr Glu Lys Ile Ile
850 855 860

Gly Glu Asp Ile Tyr Glu Cys Ile Leu Gly Gly Asn Trp Thr Cys Ile
865 870 875 880

Thr Gly Asp His Ser Arg Leu Lys Asp Gly Pro Ile Lys Lys Cys Lys
885 890 895

Trp Cys Gly His Asp Phe Val Asn Ser Glu Gly Leu Pro His Tyr Pro
900 905 910

Ile Gly Lys Cys Met Leu Ile Asn Glu Ser Gly Tyr Arg Tyr Val Asp
915 920 925

Asp Thr Ser Cys Asp Arg Gly Gly Val Ala Ile Val Pro Ser Gly Thr
930 935 940

Val Lys Cys Arg Ile Gly Asn Val Thr Val Gln Val Ile Ala Thr Asn
945 950 955 960

Asn Asp Leu Gly Pro Met Pro Cys Ser Pro Ala Glu Val Ile Ala Ser
965 970 975

Glu Gly Pro Val Glu Lys Thr Ala Cys Thr Phe Asn Tyr Ser Arg Thr
980 985 990

Leu Pro Asn Lys Tyr Tyr Glu Pro Arg Asp Arg Tyr Phe Gln Gln Tyr
995 1000 1005

Met Leu Lys Gly Glu Trp Gln Tyr Trp Phe Asp Leu Asp Ser Val
1010 1015 1020

Asp His His Lys Asp Tyr Phe Ser Glu Phe Ile Ile Ile Ala Val
1025 1030 1035

Val Ala Leu Leu Gly Gly Lys Tyr Val Leu Trp Leu Leu Ile Thr
1040 1045 1050

Tyr Thr Ile Leu Ser Glu Gln Met Ala Met Gly Ala Gly Val Asn
1055 1060 1065

Thr Glu Glu Ile Val Met Ile Gly Asn Leu Leu Thr Asp Ser Asp
1070 1075 1080

Ile Glu Val Val Val Tyr Phe Leu Leu Leu Tyr Leu Ile Val Lys
1085 1090 1095

Glu Glu Leu Ala Arg Lys Trp Ile Ile Leu Val Tyr His Ile Leu
1100 1105 1110

Val Ala Asn Pro Met Lys Thr Ile Gly Val Val Leu Leu Met Leu
1115 1120 1125

Gly Gly Val Val Lys Ala Ser Arg Ile Asn Ala Asp Asp Gln Ser
1130 1135 1140

Ala Met Asp Pro Cys Phe Leu Leu Val Thr Gly Val Val Ala Val
1145 1150 1155

Leu Met Ile Ala Arg Arg Glu Pro Ala Thr Leu Pro Leu Ile Val
1160 1165 1170

Ala Leu Leu Ala Ile Arg Thr Ser Gly Phe Leu Leu Pro Ala Ser
1175 1180 1185

Ile Asp Val Thr Val Ala Val Val Leu Ile Val Leu Leu Leu Ala
1190 1195 1200

Ser Tyr Ile Thr Asp Tyr Phe Arg Tyr Lys Lys Trp Leu Gln Leu
1205 1210 1215

Leu Phe Ser Leu Ile Ala Gly Ile Phe Ile Ile Arg Ser Leu Lys
1220 1225 1230

His Ile Asn Gln Met Glu Val Pro Glu Ile Ser Met Pro Ser Trp
1235 1240 1245

Arg Pro Leu Ala Leu Val Leu Phe Tyr Ile Thr Ser Thr Ala Ile
1250 1255 1260

Thr Thr Asn Trp Asp Ile Asp Leu Ala Gly Phe Leu Leu Gln Trp
1265 1270 1275

Ala Pro Ala Val Ile Met Met Ala Thr Met Trp Ala Asp Phe Leu
1280 1285 1290

Thr Leu Ile Ile Val Leu Pro Ser Tyr Glu Leu Ser Lys Leu Tyr
1295 1300 1305

Phe Leu Lys Asn Val Arg Thr Asp Val Glu Lys Asn Trp Leu Gly
1310 1315 1320

```
Lys Val Lys Tyr Arg Gln Ile Ser Ser Val Tyr Asp Ile Cys Asp
    1325                1330                1335

Ser Glu Glu Ala Val Tyr Leu Phe Pro Ser Arg His Lys Ser Gly
    1340                1345                1350

Ser Arg Pro Asp Phe Ile Leu Pro Phe Leu Lys Ala Val Leu Ile
    1355                1360                1365

Ser Cys Ile Ser Ser Gln Trp Gln Val Val Tyr Ile Ser Tyr Leu
    1370                1375                1380

Ile Leu Glu Ile Thr Tyr Tyr Met His Arg Lys Ile Ile Asp Glu
    1385                1390                1395

Val Ser Gly Gly Ala Asn Phe Leu Ser Arg Leu Ile Ala Ala Ile
    1400                1405                1410

Ile Glu Leu Asn Trp Ala Ile Asp Asp Glu Glu Cys Lys Gly Leu
    1415                1420                1425

Lys Lys Leu Tyr Leu Leu Ser Gly Arg Ala Lys Asn Leu Ile Val
    1430                1435                1440

Lys His Lys Val Arg Asn Glu Ala Val His Arg Trp Phe Gly Glu
    1445                1450                1455

Glu Glu Ile Tyr Gly Ala Pro Lys Val Ile Thr Ile Ile Lys Ala
    1460                1465                1470

Ser Thr Leu Ser Lys Asn Arg His Cys Ile Ile Cys Thr Ile Cys
    1475                1480                1485

Glu Gly Lys Glu Trp Asn Gly Ala Asn Cys Pro Lys Cys Gly Arg
    1490                1495                1500

Gln Gly Lys Pro Ile Thr Cys Gly Met Thr Leu Ala Asp Phe Glu
    1505                1510                1515

Glu Lys His Tyr Lys Lys Ile Phe Ile Arg Glu Glu Ser Ser Cys
    1520                1525                1530

Pro Val Pro Phe Asp Pro Ser Cys His Cys Asn Tyr Phe Arg His
    1535                1540                1545

Asp Gly Pro Phe Arg Lys Glu Tyr Lys Gly Tyr Val Gln Tyr Thr
    1550                1555                1560

Ala Arg Gly Gln Leu Phe Leu Arg Asn Leu Pro Ile Leu Ala Thr
    1565                1570                1575

Lys Met Lys Leu Leu Met Val Gly Asn Leu Gly Ala Glu Ile Gly
    1580                1585                1590

Asp Leu Glu His Leu Gly Trp Val Leu Arg Gly Pro Ala Val Cys
    1595                1600                1605
```

Lys Lys Ile Thr Asn His Glu Lys Cys His Val Asn Ile Met Asp
1610 1615 1620

Lys Leu Thr Ala Phe Phe Gly Ile Met Pro Arg Gly Thr Thr Pro
1625 1630 1635

Arg Ala Pro Val Arg Phe Pro Thr Ala Leu Leu Lys Val Arg Arg
1640 1645 1650

Gly Leu Glu Thr Gly Trp Ala Tyr Thr His Gln Gly Gly Ile Ser
1655 1660 1665

Ser Val Asp His Val Thr Ala Gly Lys Asp Leu Leu Val Cys Asp
1670 1675 1680

Ser Met Gly Arg Thr Arg Val Val Cys His Ser Asn Asn Lys Met
1685 1690 1695

Thr Asp Glu Thr Glu Tyr Gly Ile Lys Thr Asp Ser Gly Cys Pro
1700 1705 1710

Glu Gly Ala Arg Cys Tyr Val Leu Asn Pro Glu Ala Val Asn Ile
1715 1720 1725

Ser Gly Thr Lys Gly Ala Met Val His Leu Gln Lys Thr Gly Gly
1730 1735 1740

Glu Phe Thr Cys Val Thr Ala Ser Gly Thr Pro Ala Phe Phe Asp
1745 1750 1755

Leu Lys Asn Leu Lys Gly Trp Ser Gly Leu Pro Ile Phe Glu Ala
1760 1765 1770

Ser Ser Gly Arg Val Val Gly Arg Val Lys Val Gly Lys Asn Glu
1775 1780 1785

Asp Ser Lys Pro Thr Lys Leu Met Ser Gly Ile Gln Thr Val Ser
1790 1795 1800

Lys Asn Gln Thr Asp Leu Ala Asp Ile Val Lys Lys Leu Thr Ser
1805 1810 1815

Met Asn Arg Gly Glu Phe Lys Gln Ile Thr Leu Ala Thr Gly Ala
1820 1825 1830

Gly Lys Thr Thr Glu Leu Pro Arg Ser Val Ile Glu Glu Ile Gly
1835 1840 1845

Arg His Lys Arg Val Leu Val Leu Ile Pro Leu Arg Ala Ala Ala
1850 1855 1860

Glu Ser Val Tyr Gln Tyr Met Arg Val Lys Tyr Pro Ser Ile Ser
1865 1870 1875

Phe Asn Leu Arg Ile Gly Asp Met Lys Glu Gly Asp Met Ala Thr
1880 1885 1890

Gly Ile Thr Tyr Ala Ser Tyr Gly Tyr Phe Cys Gln Leu Pro Gln
1895 1900 1905

Pro Lys Leu Arg Ala Ala Met Val Glu Tyr Ser Tyr Ile Phe Leu
1910 1915 1920

Asp Glu Tyr His Cys Ala Thr Pro Glu Gln Leu Ala Ile Ile Gly
1925 1930 1935

Lys Ile His Arg Phe Ala Glu Asn Leu Arg Val Val Ala Met Thr
1940 1945 1950

Ala Thr Pro Ala Gly Thr Val Thr Thr Thr Gly Gln Lys His Pro
1955 1960 1965

Ile Glu Glu Phe Ile Ala Pro Glu Val Met Lys Gly Glu Asp Leu
1970 1975 1980

Gly Ser Glu Tyr Leu Asp Ile Ala Gly Leu Lys Ile Pro Thr Glu
1985 1990 1995

Glu Met Lys Gly Asn Met Leu Val Phe Ala Pro Thr Arg Asn Met
2000 2005 2010

Ala Val Glu Thr Ala Lys Lys Leu Lys Ala Lys Gly Tyr Asn Ser
2015 2020 2025

Gly Tyr Tyr Tyr Ser Gly Glu Asn Pro Glu Asn Leu Arg Val Val
2030 2035 2040

Thr Ser Gln Ser Pro Tyr Val Val Val Ala Thr Asn Ala Ile Glu
2045 2050 2055

Ser Gly Val Thr Leu Pro Asp Leu Asp Thr Val Val Asp Thr Gly
2060 2065 2070

Leu Lys Cys Glu Lys Arg Val Arg Ile Ser Ser Lys Met Pro Phe
2075 2080 2085

Ile Val Thr Gly Leu Lys Arg Met Ala Val Thr Ile Gly Glu Gln
2090 2095 2100

Ala Gln Arg Arg Gly Arg Val Gly Arg Val Lys Pro Gly Arg Tyr
2105 2110 2115

Tyr Arg Ser Gln Glu Thr Ala Ser Gly Ser Lys Asp Tyr His Tyr
2120 2125 2130

Asp Leu Leu Gln Ala Gln Arg Tyr Gly Ile Glu Asp Gly Ile Asn
2135 2140 2145

Val Thr Lys Ser Phe Arg Glu Met Asn Tyr Asp Trp Ser Leu Tyr
2150 2155 2160

Glu Glu Asp Ser Leu Met Ile Thr Gln Leu Glu Val Leu Asn Asn
2165 2170 2175

Leu Leu Ile Ser Glu Asp Leu Pro Ala Ala Val Lys Asn Ile Met
2180 2185 2190

Ala Arg Thr Asp His Pro Glu Pro Ile Gln Leu Ala Tyr Asn Ser
2195 2200 2205

Tyr Glu Asn Gln Ile Pro Val Leu Phe Pro Lys Ile Lys Asn Gly
2210 2215 2220

Glu Val Thr Asp Ser Tyr Glu Asn Tyr Thr Tyr Leu Asn Ala Arg
2225 2230 2235

Lys Leu Gly Glu Asp Val Pro Ala Tyr Val Tyr Ala Thr Glu Asp
2240 2245 2250

Glu Asp Leu Ala Val Asp Leu Leu Gly Met Asp Trp Pro Asp Pro
2255 2260 2265

Gly Asn Gln Gln Val Val Glu Thr Gly Arg Ala Leu Lys Gln Val
2270 2275 2280

Thr Gly Leu Ser Thr Ala Glu Asn Ala Leu Leu Ile Ala Leu Phe
2285 2290 2295

Gly Tyr Val Gly Tyr Gln Thr Leu Ser Lys Arg His Ile Pro Met
2300 2305 2310

Ile Thr Asp Ile Tyr Thr Leu Glu Asp His Arg Leu Glu Asp Thr
2315 2320 2325

Thr His Leu Gln Phe Ala Pro Asn Ala Ile Arg Thr Asp Gly Lys
2330 2335 2340

Asp Ser Glu Leu Lys Glu Leu Ala Val Gly Asp Leu Asp Lys Tyr
2345 2350 2355

Val Asp Ala Leu Val Asp Tyr Ser Lys Gln Gly Met Lys Phe Ile
2360 2365 2370

Lys Val Gln Ala Glu Lys Val Arg Asp Ser Gln Ser Thr Lys Glu
2375 2380 2385

Gly Leu Gln Thr Ile Lys Glu Tyr Val Asp Lys Phe Ile Gln Ser
2390 2395 2400

Leu Thr Glu Asn Lys Glu Glu Ile Ile Arg Tyr Gly Leu Trp Gly
2405 2410 2415

Val His Thr Ala Leu Tyr Lys Ser Leu Ala Ala Arg Leu Gly His
2420 2425 2430

Glu Thr Ala Phe Ala Thr Leu Val Val Lys Trp Leu Ala Phe Gly
2435 2440 2445

Gly Glu Thr Val Ser Ala His Ile Lys Gln Val Ala Val Asp Leu
2450 2455 2460

Val Val Tyr Tyr Ile Ile Asn Lys Pro Ser Phe Pro Gly Asp Thr
2465 2470 2475

Glu Thr Gln Gln Glu Gly Arg Arg Phe Val Ala Ser Leu Phe Ile
2480 2485 2490

Ser Ala Leu Ala Thr Tyr Thr Tyr Lys Thr Trp Asn Tyr Asn Asn
2495 2500 2505

Leu Gln Arg Val Val Glu Pro Ala Leu Ala Tyr Leu Pro Tyr Ala
2510 2515 2520

Thr Ser Ala Leu Lys Leu Phe Thr Pro Thr Arg Leu Glu Ser Val
2525 2530 2535

Val Ile Leu Ser Ser Thr Ile Tyr Lys Thr Tyr Leu Ser Ile Arg
2540 2545 2550

Lys Gly Lys Ser Asp Gly Leu Leu Gly Thr Gly Ile Ser Ala Ala
2555 2560 2565

Met Glu Ile Leu Asn Gln Asn Pro Ile Ser Val Gly Ile Ser Val
2570 2575 2580

Met Leu Gly Val Gly Ala Ile Ala Ala His Asn Ala Ile Glu Ser
2585 2590 2595

Ser Glu Gln Lys Arg Thr Leu Leu Met Lys Val Phe Val Lys Asn
2600 2605 2610

Phe Leu Asp Gln Ala Ala Thr Asp Glu Leu Val Lys Glu Asn Pro
2615 2620 2625

Glu Lys Ile Ile Met Ala Leu Phe Glu Ala Val Gln Thr Ile Gly
2630 2635 2640

Asn Pro Leu Arg Leu Ile Tyr His Leu Tyr Gly Val Tyr Tyr Lys
2645 2650 2655

Gly Trp Glu Ala Lys Glu Leu Ala Glu Lys Thr Ala Gly Arg Asn
2660 2665 2670

Leu Phe Thr Leu Ile Met Phe Glu Ala Phe Glu Leu Leu Gly Met
2675 2680 2685

Asp Ser Glu Gly Lys Ile Arg Asn Leu Ser Gly Asn Tyr Ile Leu
2690 2695 2700

Asp Leu Ile Phe Asn Leu His Asn Lys Leu Asn Lys Gly Leu Lys
2705 2710 2715

Lys Leu Val Leu Gly Trp Ala Pro Ala Pro Leu Ser Cys Asp Trp
2720 2725 2730

Thr Pro Ser Asp Glu Arg Ile Ser Leu Pro His Asn Asn Tyr Leu
2735 2740 2745

Arg Val Glu Thr Arg Cys Pro Cys Gly Tyr Glu Met Lys Ala Ile
2750 2755 2760

Lys Asn Val Ala Gly Lys Leu Thr Lys Val Glu Glu Lys Gly Ser
2765 2770 2775

Phe Leu Cys Arg Asn Arg Leu Gly Arg Gly Pro Pro Asn Phe Lys
2780 2785 2790

Val Thr Lys Phe Tyr Asp Asp Asn Leu Ile Glu Val Lys Pro Val
2795 2800 2805

Ala Arg Leu Glu Gly Gln Val Asp Leu Tyr Tyr Lys Gly Val Thr
2810 2815 2820

Ala Lys Leu Asp Tyr Asn Asn Gly Lys Val Leu Leu Ala Thr Asn
2825 2830 2835

Lys Trp Glu Val Asp His Ala Phe Leu Thr Arg Leu Val Lys Lys
2840 2845 2850

His Thr Gly Ile Gly Phe Lys Gly Ala Tyr Leu Gly Asp Arg Pro
2855 2860 2865

Asp His Gln Asp Leu Val Asp Arg Asp Cys Ala Thr Ile Thr Lys
2870 2875 2880

Asn Ser Val Gln Phe Leu Lys Met Lys Lys Gly Cys Ala Phe Thr
2885 2890 2895

Tyr Asp Leu Thr Ile Ser Asn Leu Val Arg Leu Ile Glu Leu Val
2900 2905 2910

His Lys Asn Asn Leu Gln Glu Arg Glu Ile Pro Thr Val Thr Val
2915 2920 2925

Thr Thr Trp Leu Ala Tyr Ser Phe Val Asn Glu Asp Leu Gly Thr
2930 2935 2940

Ile Lys Pro Val Leu Gly Glu Lys Val Ile Pro Glu Pro Pro Glu
2945 2950 2955

Glu Leu Ser Leu Gln Pro Thr Val Arg Leu Val Thr Thr Glu Thr
2960 2965 2970

Ala Ile Thr Ile Thr Gly Glu Ala Glu Val Met Thr Thr Gly Ile
2975 2980 2985

Thr Pro Val Val Glu Met Lys Glu Glu Pro Gln Leu Asp His Gln
2990 2995 3000

Ser Thr Thr Leu Lys Val Gly Leu Lys Glu Gly Glu Tyr Pro Gly
3005 3010 3015

Pro Gly Val Asn Pro Asn His Leu Ala Glu Val Ile Asp Glu Lys
3020 3025 3030

Asp Asp Arg Pro Phe Val Leu Ile Ile Gly Asn Lys Gly Ser Thr
3035 3040 3045

Ser Asn Arg Ala Arg Thr Ala Lys Asn Ile Arg Leu Tyr Lys Gly
3050 3055 3060

Asn Asn Pro Arg Glu Ile Arg Asp Leu Met Ser Gln Gly Arg Ile
3065 3070 3075

Leu Thr Val Ala Leu Lys Glu Leu Asp Pro Glu Leu Lys Glu Leu
3080 3085 3090

Val Asp Tyr Lys Gly Thr Phe Leu Asn Arg Glu Ala Leu Glu Ala
3095 3100 3105

Leu Ser Leu Gly Lys Pro Ile Lys Arg Lys Thr Thr Thr Ala Met
3110 3115 3120

Ile Arg Arg Leu Ile Glu Pro Glu Val Glu Glu Glu Leu Pro Asp
3125 3130 3135

Trp Phe Gln Ala Glu Glu Pro Leu Phe Leu Glu Ala Lys Ile Gln
3140 3145 3150

Asn Asp Leu Tyr His Leu Ile Gly Ser Val Asp Ser Ile Lys Ser
3155 3160 3165

Lys Ala Lys Glu Leu Gly Ala Thr Asp Asn Thr Lys Ile Val Lys
3170 3175 3180

Glu Val Gly Ala Arg Thr Tyr Thr Met Lys Leu Ser Ser Trp Ser
3185 3190 3195

Thr Gln Val Thr Lys Lys Gln Met Ser Leu Ala Pro Leu Phe Glu
3200 3205 3210

Glu Leu Leu Leu Lys Cys Pro Pro Cys Ser Lys Ile Ser Lys Gly
3215 3220 3225

His Met Val Ser Ala Tyr Gln Leu Ala Gln Gly Asn Trp Glu Pro
3230 3235 3240

Leu Gly Cys Gly Val Tyr Met Gly Thr Ile Pro Ala Arg Arg Leu
3245 3250 3255

Lys Ile His Pro Tyr Glu Ala Tyr Leu Lys Leu Lys Glu Leu Val
3260 3265 3270

Glu Val Glu Ser Ser Arg Ala Thr Ala Lys Glu Ser Ile Ile Arg
3275 3280 3285

Glu His Asn Thr Trp Ile Leu Arg Lys Val Arg His Glu Gly Asn
3290 3295 3300

Leu Arg Thr Lys Ser Met Ile Asn Pro Gly Lys Ile Ser Asp Gln
3305 3310 3315

Leu Cys Arg Asp Gly His Lys Arg Asn Ile Tyr Asn Lys Ile Ile
3320 3325 3330

Gly Ser Thr Met Ala Ser Ala Gly Ile Arg Leu Glu Lys Leu Pro
3335 3340 3345

Val Val Arg Ala Gln Thr Asp Thr Thr Ser Phe His Gln Ala Ile
3350 3355 3360

Arg Glu Lys Ile Asp Lys Thr Glu Asn Lys Gln Thr Pro Glu Leu
3365 3370 3375

His Glu Glu Leu Met Lys Val Phe Asp Cys Leu Lys Ile Pro Glu
3380 3385 3390

Leu Lys Glu Ser Tyr Asp Glu Val Ser Trp Glu Gln Leu Glu Ala
3395 3400 3405

Gly Ile Asn Arg Lys Gly Ala Ala Gly Tyr Leu Glu Ser Lys Asn
3410 3415 3420

Ile Gly Glu Val Leu Asp Thr Glu Lys His Ile Val Glu Gln Leu
3425 3430 3435

Ile Lys Asp Leu Arg Lys Gly Lys Lys Ile Arg Tyr Tyr Glu Thr
3440 3445 3450

Ala Ile Pro Lys Asn Glu Lys Arg Asp Val Ser Asp Asp Trp Glu
3455 3460 3465

Ala Gly Glu Phe Val Asp Glu Lys Lys Pro Arg Val Ile Gln Tyr
3470 3475 3480

Pro Asp Ala Lys Val Arg Leu Ala Ile Thr Lys Val Met Tyr Lys
3485 3490 3495

Trp Val Lys Gln Lys Pro Val Val Ile Pro Gly Tyr Glu Gly Lys
3500 3505 3510

Thr Pro Leu Phe Asp Ile Phe Asn Lys Val Lys Lys Glu Trp Asp
3515 3520 3525

Ser Phe Gln Asp Pro Val Ala Val Ser Phe Asp Thr Lys Ala Trp
3530 3535 3540

Asp Thr Gln Val Thr Ser Arg Asp Leu Met Leu Ile Lys Asp Ile
3545 3550 3555

Gln Lys Tyr Tyr Phe Lys Arg Ser Ile His Lys Phe Leu Asp Thr
3560 3565 3570

Ile Thr Glu His Met Val Glu Val Pro Val Ile Thr Ala Asp Gly
3575 3580 3585

Glu Val Tyr Ile Arg Asn Gly Gln Arg Gly Ser Gly Gln Pro Asp
3590 3595 3600

Thr Ser Ala Gly Asn Ser Met Leu Asn Val Leu Thr Met Ile Tyr
3605 3610 3615

Ala Phe Cys Lys Ser Thr Gly Ile Pro Tyr Arg Gly Phe Ser Arg
3620 3625 3630

Val Ala Arg Ile His Val Cys Gly Asp Asp Gly Phe Leu Ile Thr
3635 3640 3645

Glu Arg Gly Leu Gly Leu Lys Phe Ser Glu Lys Gly Met Gln Ile
3650 3655 3660

Leu His Glu Ala Gly Lys Pro Gln Lys Ile Thr Glu Gly Asp Lys
3665 3670 3675

Met Lys Val Ala Tyr Arg Phe Glu Asp Ile Glu Phe Cys Ser His
3680 3685 3690

Thr Pro Val Pro Val Arg Trp Ala Asp Asn Thr Ser Ser Tyr Met
3695 3700 3705

Ala Gly Arg Ser Thr Ala Thr Ile Leu Ala Lys Met Ala Thr Arg
3710 3715 3720

Leu Asp Ser Ser Gly Glu Arg Gly Ser Thr Ala Tyr Glu Lys Ala
3725 3730 3735

Val Ala Phe Ser Phe Leu Leu Met Tyr Ser Trp Asn Pro Val Val
3740 3745 3750

Arg Arg Ile Cys Leu Leu Val Leu Ser Gln Phe Pro Glu Ile Ser
3755 3760 3765

Pro Ser Lys Asn Thr Ile Tyr Tyr Tyr Gln Gly Asp Pro Ile Ala
3770 3775 3780

Ala Tyr Arg Glu Val Ile Gly Lys Gln Leu Cys Glu Leu Lys Arg
3785 3790 3795

Thr Gly Phe Glu Lys Leu Ala Gly Leu Asn Leu Ser Met Thr Thr
3800 3805 3810

Leu Gly Ile Trp Thr Lys His Thr Ser Lys Arg Leu Ile Gln Ala
3815 3820 3825

Cys Val Glu Ile Gly Lys Arg Glu Gly Thr Trp Leu Val Asn Ala
3830 3835 3840

Asp Arg Leu Ile Ala Gly Lys Thr Gly Lys Phe Tyr Ile Pro Ser
3845 3850 3855

Thr Gly Val Thr Leu Leu Gly Lys His Tyr Glu Glu Ile Asn Leu
3860 3865 3870

Lys Gln Lys Ala Ala Gln Pro Pro Ile Glu Gly Val Asp Arg Tyr
3875 3880 3885

Lys Leu Gly Pro Ile Val Asn Val Ile Leu Arg Arg Leu Arg Val
3890 3895 3900

Met Leu Met Thr Val Ala Ser Gly Ser Trp
3905 3910

<210> 10
<211> 12332
<212> DNA
<213> Artificial-sequence:XIKE-C BVDV-Sequence

<400> 10

```
gtatacgaga ttagctaaag aactcgtata tggattggac gtcaacaaat ttttaattgg    60
caacgtaggg aaccttcccc tcagcgaagg ccgaaaagag gctagccatg cccttagtag   120
gactagcaaa agtaggggac tagcggtagc agtgagttcg ttggatggcc gaacccctga   180
gtacagggga gtcgtcaatg gttcgacact ccattagtcg aggagtctcg agatgccatg   240
tggacgaggg catgcccacg gcacatctta acccatgcgg gggttgcatg ggtgaaagcg   300
ctattcgtgg cgttatggac acagcctgat agggtgtagc agagacctgc tattccgcta   360
gtaaaaactc tgctgtacat ggcacatgga gttgttttca aatgaacttt tatacaaaac   420
atataaacaa aaaccagcag gcgtcgtgga acctgtttac gacgtcaacg ggcgcccact   480
gtttggagag agcagtgact tgcacccgca gtcaacacta aaactaccac accaacgagg   540
cagcgccaac atcctgacca atgctaggtc cctaccgcgg aaaggtgact gccggagagg   600
taatgtgtat ggaccggtga gtggcatcta tatcaaacca ggaccgatct actaccagga   660
ttatgtgggc cccgtctatc atagagcccc actggaacta tgtagggagg caagtatgtg   720
cgaaacaact aggagagttg gcagagtgac cggtagtgat gggaaattat atcatatcta   780
catctgcata gatgggtgta tcctcctgaa gagggcgact aggaaccaac cagaagtcct   840
gaaatgggta tacaacagat taaattgtcc tttatgggtc accagctgct ccgatgaagg   900
gagcaagggt gctacaagta agaagcagcc taagccagat aggatagaaa aaggtaagat   960
gaaaatagcc ccaaaagaga cagaaaaaga ttgcaaaacc agaccccccg acgcgactat  1020
agtagtagaa ggggttaagt accaggtgaa gaaaaaagga aaggtaaggg gaaaaaatac  1080
tcaagatggg ttatatcaca acaagaataa gcccccctgaa tcaagaaaaa aattggaaaa  1140
ggcactgctg gcttgggcca tcttagcagc ggtcctgctt cagctggtaa caggagagaa  1200
tatcacccag tggaacttga tggacaacgg caccgaggga atacagcaag cgatgttcct  1260
aagaggggtg aacaggagtc tattaggaat ttggccagag aaaatttgca ccggagtacc  1320
aactcactta gcaacagact atgagcttaa agagatagtg gggatgatgg acgcgagtga  1380
gaagaccaac tacacgtgtt gcaggttgca aagacatgag tggaataaac atggttggtg  1440
taactggttt catatagaac cgtggatatg gttgatgaac aaaacccaaa acaacctgac  1500
agaagggcaa ccgcttaggg agtgtgctgt gacttgtagg tatgacaagg aaacagaatt  1560
gaacatcgtg acacaggcta gggacagacc tacaactctg acaggttgca agaaaggcaa  1620
gaatttctct ttcgcaggtg ttatactgga tgggccctgt aactttaaag tatcggttga  1680
agatgtgctg ttcaaggagc acgattgcgg caacatgctg caagagaccg cgatacagct  1740
actcgatggg gcaaccaaca ccattgaggg agcaagggta gggacggcca agttgacaac  1800
ctggttaggg aagcaattag ggatccttgg taagaagttg gagaacaaaa gcaaagcatg  1860
gtttggtgca catgcagcaa gtccatactg cggagtggag aggaagatcg gttacgtatg  1920
gtatacaaaa aactgcactc cagcttgcct tccaagaaac actagaataa taggccccgg  1980
gaaatttgat accaacgccg aagatggaaa aatactccat gagatggggg ggcacctctc  2040
agaatttgtc ctattgtcct tggtggttct gtctgacttt gccccggaaa ccgcgagcgt  2100
catctacttg gttctacatt ttgcgatccc gcaaagccac gttgatgtag acacatgcga  2160
caagaaccag ctgaatttaa cggtagcaac cacagtagca gaggtcatac cagggacagt  2220
gtggaaccta gggaagtatg tctgcataag accagactgg tggccatatg agacgacgac  2280
agtcttcgtc atagaggaag cagggcaagt aatcaaattg atgctaaggg ccatcagaga  2340
cttaactagg atatggaatg ctgccactac cacagctttc ttaatctttt tagtaaaagc  2400
actgagggga caactaatcc aagggctatt gtggctgatg ctaataacag gagcacaggg  2460
cttccctgaa tgcaaagagg gcttccaata tgccatatct aaagacagga aaatggggtt  2520
attggggcca gagagcttaa ctacaacatg gcacctcccc accaaaaaaa tagtggattc  2580
catggtgcat gtatggtgtg aaggaaaaga cttgaaaata ttaaaaatgt gcacaaagga  2640
```

agagaggtat ctagtggctg tgcacgagag agccttatca accagtgccg agtttatgca 2700
gatcagtgat gggacaatag gcccagacgt gatagatatg cctgatgact ttgagtttgg 2760
actctgccct tgtgactcaa aaccagtgat aaagggcaaa tttaatgcca gcttactgaa 2820
tggaccagct ttccagatgg tatgcccaca ggggtggact ggtacaatag aatgcaccct 2880
agcgaaccaa gacaccttgg acacaactgt cattaggaca tatagaagaa ctaccccatt 2940
tcagcggaga aaatggtgta cctatgaaaa aataataggg gaagatatct atgaatgcat 3000
tctaggtgga aactggacat gcataaccgg tgaccatagc aggttgaaag acggacctat 3060
caagaagtgt aagtggtgtg gccatgactt cgtcaactca gaggggctac cacactaccc 3120
aataggcaag tgcatgctca tcaacgagag tgggtacagg tatgtagatg acacctcttg 3180
cgataggggt ggtgtagcca tagttccatc tggcaccgta aagtgtagaa taggtaacgt 3240
cacggtgcaa gttatcgcta ctaacaatga tctgggaccc atgccttgca gcccagctga 3300
agtgatagca agtgaaggac cagtggaaaa gactgcatgc acattcaact attcaaggac 3360
tctacctaat aagtattatg agccaaggga ccggtacttc caacaataca tgttaaaagg 3420
ggagtggcaa tattggttcg acctggattc tgtagaccac cacaaagact acttctcaga 3480
gttcataatc atagcagtgg tcgccttgtt gggtggtaag tacgtactgt ggctcttgat 3540
aacatacaca atactgtctg agcagatggc tatgggtgct ggagtgaata ctgaagagat 3600
agtcatgata ggcaatttgc tgacagacag tgatattgag gttgtggttt atttccttct 3660
tctgtactta atagttaaag aggaactggc gaggaaatgg attatactgg tataccacat 3720
ccttgtagcc aaccctatga aaacaattgg ggtcgtctta ctaatgctag ggggagtggt 3780
gaaggccagc agaatcaatg ctgatgacca aagtgctatg gacccatgct ttcttctcgt 3840
gacaggcgta gtggctgttt tgatgatcgc tagaagagaa cctgccacat taccactgat 3900
tgtagcattg ctagcaataa gaacatcagg attcctactg cccgctagca ttgatgtaac 3960
tgtagcagta gtattaattg tacttttgtt ggctagctac ataacagact actttagata 4020
taaaaagtgg cttcaactct tatttagtct gatagctggt atctttatta taaggagctt 4080
aaaacatatc aaccagatgg aggtaccaga aatatctatg ccaagttgga gacctctagc 4140
tctggtcctt ttctatataa catctacagc aataaccact aattgggaca ttgacttagc 4200
aggcttcctg ctgcaatggg cgccagcagt gatcatgatg gctaccatgt gggcagactt 4260
tttgactctg atcatagtcc tgcccagtta cgagttatct aagctttact tcctaaagaa 4320
cgtcaggaca gacgtggaaa agaactggct cggcaaagtg aaatacagac agatcagttc 4380
agtttatgac atctgtgaca gtgaggaagc agtgtaccta tttccatcaa ggcataagag 4440
tggaagcagg ccagatttca tattaccttt tttgaaagcc gtgttaataa gctgcatcag 4500
cagccaatgg caagtggttt acatttctta cctaatactg gaaattacat actatatgca 4560
caggaaaatc atagatgagg tgtcaggagg agcaaatttt ctatcaagac tcatagcagc 4620
catcatagaa ttaaattggg ccatagatga tgaggaatgt aaaggactga agaaactgta 4680
tctcttgtca gggagagcga agaatttgat agttaaacat aaggtaagaa atgaagccgt 4740
ccacagatgg tttggtgagg aggaaatata cggggcaccc aaggtgatca ctatcataaa 4800
agctagtacc ctaagtaaaa acaggcactg cataatctgc acgatctgtg aagggaaaga 4860
atggaatgga gccaactgcc caaagtgtgg aagacaagga aagcccataa catgtggaat 4920
gacactcgca gactttgagg agaaacatta caaaaagata tttataagag aagaatcttc 4980
ttgtcctgtg ccttttgatc cttcttgcca ttgtaattat tttcgccacg atgggccttt 5040
caggaaagag tataagggtt acgtccaata cacagccaga ggacaactct ttctgaggaa 5100
cctaccaatt ctagcgacga agatgaagct attaatggtg ggaaacctcg gcgcagaaat 5160
tggcgacctg gaacatctag gatgggtact gagagggcca gccgtgtgca aaaaaattac 5220
caaccatgag aagtgccacg taaacatcat ggataagcta actgcatttt ttggaatcat 5280
gcctagaggc acgaccccta gggcacctgt gaggttcccc acagcactac taaaagtgag 5340
aagggggcta gagacgggat gggcttacac gcaccaagga gggatcagct cggtagacca 5400
tgtcacagcc ggaaaggatt tactagtgtg tgacagtatg ggcaggacca gggttgtctg 5460
tcatagtaac aataagatga ctgatgagac tgagtatggc atcaagaccg actcagggtg 5520
tcccgaaggt gcgaggtgtt acgtgctaaa cccagaagct gttaacattt ctggcacaaa 5580
aggagctatg gtacacctcc agaaaacggg gggggagttc acatgtgtca ctgcctcagg 5640
gaccccggct ttcttcgatc tgaaaaatct aaaaggctgg tccgggctac caatttttga 5700
agcatccagt ggcagggtgg ttggtagggt gaaagtcggc aagaatgagg attccaagcc 5760
caccaaacta atgagcggaa tccagacagt gtctaagaac cagacagacc tagcggacat 5820
cgtaaaaaaa ttgactagta tgaacagagg agagttcaaa cagataacat tagccactgg 5880
ggcaggaaaa actacggaac tgccaaggtc cgtcatagag gagataggga ggcacaaaag 5940
ggtcttagtc ctgataccat tgagagcagc agcagagtca gtgtatcagt atatgagagt 6000
gaagtaccca agtatatctt tcaatttgag aataggagat atgaaggaag gtgacatggc 6060

cactggtatc acctacgcct catatgggta cttttgtcag cttcctcagc ccaaactgag 6120
agctgccatg gtagagtact catatatatt cttagatgag taccactgtg ctacacccga 6180
gcaattagca ataattggaa agatacacag gtttgctgaa aatcttagag tggtagcaat 6240
gacagcaacc ccagctggaa cggtcacaac gactggtcag aaacacccta tagaggagtt 6300
catagcccca gaggtgatga aaggtgaaga tctaggtagt gaatacttgg atattgcagg 6360
gttgaagata ccgactgaag agatgaaagg caacatgctc gtgttcgcgc caactaggaa 6420
catggcagta gaaacagcta agaaattgaa ggctaaggga tacaactctg gatactatta 6480
cagtggggaa aacccagaga acttgagggt ggtaacctcg caatccccgt atgtggtagt 6540
agccaccaat gccatagagt caggtgtgac attaccagac ttagacacag ttgtagacac 6600
tggactaaag tgtgagaaga gggtgaggat ttcttcaaaa atgcccttca ttgtaacagg 6660
acttaagaga atggcagtca caatcggaga gcaagcccag cgcaggggta gagtaggaag 6720
agtcaagcca ggtaggtact ataggagtca agaaacagct tcagggtcaa aagattacca 6780
ttacgaccta ctgcaagccc agaggtacgg aatagaagat ggaattaatg taacaaagtc 6840
attcagggag atgaactatg attggagcct ttacgaagag gacagcttga tgataactca 6900
actcgaggtc cttaacaacc tccttatatc agaagacctg cctgccgcag tgaagaacat 6960
catggcccgg accgatcacc cagaacccat acaactggcc tataacagtt atgaaaacca 7020
aattccagtg ctgttcccaa agatcaaaaa tggtgaggtg acagacagtt atgagaatta 7080
cacatatctc aatgcaagaa aattaggaga ggacgtgccg gcatatgtgt acgccacaga 7140
ggatgaggat ctagcagtgg atcttctggg tatggattgg ccggacccag gcaaccaaca 7200
ggtggtagag acagggaggg cattaaaaca agtaactggc ttatccacag cagaaaacgc 7260
cctcttgata gccctattcg gctacgtcgg gtaccagaca ctttcaaaaa ggcacatacc 7320
catgattact gacatctata cacttgaaga ccacaggctt gaggacacaa cccacctcca 7380
gtttgcccca aacgctataa ggaccgacgg caaggactca gagttgaagg aattagctgt 7440
gggagacctt gataaatatg tggacgcact ggtagactac tccaaacaag ggatgaaatt 7500
catcaaagtc caagctgaaa aggtcagaga ctcccagtct acgaaggaag gcttgcaaac 7560
cattaaggag tatgtggata agtttataca atcactaaca gagaataagg aggagatcat 7620
caggtatgga ctatggggag ttcacacggc actctacaaa agcttggcag cgagactggg 7680
gcatgaaaca gcttttgcaa ctttagtggt aaaatggttg gcttttgggg gcgaaacggt 7740
atctgctcac atcaagcaag tagcagttga tctagtagta tattatatca tcaacaaacc 7800
atcttttcct ggagatacag agacccaaca agaggggagg aagtttgtgg ctagtctttt 7860
tatatctgca ctagcaacat acacatataa aacctggaat tacaacaatc tgcaacgggt 7920
tgtcgaacct gccttagctt acctcccata tgctacaagt gccttgaagt tgttcacacc 7980
cacaagatta gagagtgtgg tcatactcag ttctacaatt tacaagacat acctctctat 8040
aaggaagggt aagagtgacg gcttgttagg tacaggcata agtgcagcca tggagatctt 8100
aaaccaaaac ccaatctcag taggtatatc tgtgatgctg ggggtaggtg ccatcgccgc 8160
ccataatgca atagaatcta gtgaacagaa aagaactttg ctgatgaagg tctttgtaaa 8220
aaacttctta gaccaagcag caacagatga gctagtcaaa gagaaccctg aaaaaataat 8280
catggctcta tttgaagcag tccagaccat aggaaacccc ctaagactca tctaccatct 8340
gtacggggtg tactataagg ggtgggaagc aaaagaactc gcagagaaaa ctgctggccg 8400
caacttattc acattgatca tgtttgaggc ctttgagctt ttaggtatgg actcagaagg 8460
aaagataaga aacttgtcag gcaactacat actggactta atcttcaact tgcataataa 8520
attaaacaag ggctcaaaa aactagtcct tgggtgggct cctgcacctt tgagctgtga 8580
ttggacacca agtgatgaga gaataagcct acctcataac aactacttaa gggtagaaac 8640
caggtgtcct tgtggctatg agatgaaggc aataaaaaat gttgctggta aattgacaaa 8700
agttgaagaa aaggggtcct tcctatgcag gaatagatta gggagaggac ctccaaactt 8760
caaagtaaca aagttctatg atgataactt gatagaagtc aagccagtag ctaggctaga 8820
aggccaggtg gacctctatt acaagggagt aacagctaag ttagactaca acaatgggaa 8880
agtactgtta gctaccaaca agtgggaggt ggaccacgct ttcctgacca gactagtaaa 8940
gaagcacaca gggataggtt ttaaaggtgc atatttgggt gaccgaccag accatcaaga 9000
tcttgtcgat agagattgtg caactataac gaagaactca gtacagttcc taaaaatgaa 9060
gaagggttgc gctttcacat atgacctaac aatctctaac cttgtcaggc ttattgaact 9120
agtccataag aataatttac aagaaagaga gatccctacc gtgacagtaa ctacttggct 9180
tgcatattct tttgtcaatg aagacctggg gactatcaag cctgtattgg gggagaaagt 9240
catcccagaa ccccccgagg agttgagtct ccaacccacc gtgagactag tcaccactga 9300
aacagcaata accataacag ggaggctga agtgatgacg acagggatca caccagtggt 9360
agagatgaaa gaagaacctc agctggacca ccagtcaact accctaaagg tagggttgaa 9420
ggaaggggaa tatccagggc caggagttaa ccctaaccat ttagcagagg tgatagatga 9480

```
gaaagatgac aggccttttg tcctaatcat cggtaacaaa ggttctacct cgaacagagc   9540
aagaacggcc aagaatatac ggctgtacaa aggaaacaac ccaagagaga tcagggatct   9600
gatgagccaa ggaagaatat tgacggttgc tctaaaagag ttggacccgg aattaaaaga   9660
attagtagat tacaagggga cctttctcaa tagggaagct ttagaagccc taagcttagg   9720
taagccaatc aagaggaaaa ccacaacagc aatgatcagg aggttaatag agccagaggt   9780
tgaggaggaa ctaccagatt ggttccaagc ggaagaaccc ctatttttgg aagcaaaaat   9840
acagaatgac ttataccacc taattggcag tgtagatagt ataaaaagca aagcaaagga   9900
attaggggcc acagataaca caaagatagt gaaggaagtt ggggctagga cctatacgat   9960
gaaattgagc agctggagca cacaagttac aaaaaaacag atgagtctag cccctctctt  10020
tgaagagctg ttattaaagt gccctccatg tagtaaaatt tcaaagggac atatggtgtc  10080
agcataccaa ctggctcaag gaaactggga acccctcggg tgtggggtct atatgggaac  10140
cataccagct aggcgtctca agatccaccc ttatgaggct taccttaaac tcaaagagct  10200
ggtggaagtt gaatcttcga gggccactgc aaaagaatcc atcataagag aacataacac  10260
ctggatcctg cggaaggtga gacatgaagg gaacctaaga accaaatcaa tgatcaaccc  10320
tgggaaaata tcagatcagc tatgcagaga tggacacaaa agaaacatat ataataagat  10380
cataggctca acaatggcct ctgctggtat taggctggag aaactgccag tagtccgagc  10440
ccaaactgac acaaccagtt tccaccaagc cataagagaa aaaattgata aaacagaaaa  10500
caagcagacc cctgaattgc atgaagaact aatgaaggtc ttcgactgct taaagatccc  10560
agagctgaag gaatcgtatg atgaagtttc atgggaacaa ttagaagccg ggataaaccg  10620
taagggtgca gcaggctatc tagagagcaa gaacataggg gaagtcctag acacagagaa  10680
acacatagta gagcagctga tcaaggatct gaggaagggg aagaagatta ggtactatga  10740
aacagccatc cccaagaatg agaagagaga cgtcagcgac gactgggaag ccggagagtt  10800
cgttgatgaa aagaaaccaa gagtaatcca gtacccggac gccaaggtga gactggccat  10860
tacaaaagtg atgtacaaat gggtaaagca aaaaccagtg gtgatacccg gctatgaagg  10920
taaaacacct ctatttgaca tattcaacaa agtgaagaag gaatgggatt cattccagga  10980
ccccgtagca gtgagctttg acaccaaagc gtgggataca caagtcacca gtagagacct  11040
aatgttgata aaggatatcc agaaatatta tttcaagaga agtatacaca aatttttaga  11100
tacaataaca gaacacatgg tggaggtacc tgtcattaca gcagacggtg aagtttacat  11160
aaggaatggt cagaggggta gtggccaacc cgacacaagt gctggtaata gtatgttgaa  11220
tgtcctaacc atgatatatg ctttctgtaa aagtacaggc ataccttaca ggggattcag  11280
cagagtggca agaatccatg tgtgtggtga tgatggcttt ttgataacag agagaggact  11340
gggactgaaa ttctctgaga agggtatgca gatattacat gaggccggga agccccagaa  11400
aataactgaa ggggacaaaa tgaaagtggc atacagattc gaggacatag agttttgttc  11460
ccatactccc gtgccagtca gatgggcaga taacaccagt agttacatgg cagggaggag  11520
cacagccact atactagcta agatggcaac caggctggat tccagcggag agaggggtag  11580
cacagcttat gagaaggccg tagccttcag cttccttttg atgtactcat ggaatcccgt  11640
agttagaagg atctgcttac tggtgttgtc acagtttcca gaaatatccc catccaaaaa  11700
cacaatatac tactaccaag gggatcccat agctgcgtac agagaagtga tagggaaaca  11760
gctgtgtgaa ctgaaaagaa caggatttga gaagctggct ggtctgaatt tgagtatgac  11820
cactctaggc atctggacaa aacatactag taaaagacta atccaagcct gtgtagaaat  11880
aggtaagaga gaaggtacct ggttagttaa tgctgacaga ctgattgcag gaaagactgg  11940
gaagttttac atcccaagca ctggtgtcac tctgttggga aaacactatg aggaaattaa  12000
cttaaagcaa aaggcggcac aaccgccgat agaggggggtt gacagatata agttgggccc  12060
catagttaat gttatcttga gaaggctgag ggtgatgctg atgacagttg ccagcggaag  12120
ctggtgaatc cgtccggagc gtcgtgccct cactcaaggt ttttaattgt aaatattgta  12180
aatagacagc taagatattt attgtagttg gatagtaatg cagtgatagt aaatacccca  12240
atttaacact acctccaatg cactaagcac tttagctgtg tgaggttaac tcgacgtcca  12300
cggttggact agggaagacc tctaacagcc cc                               12332
```

<210> 11

<211> 11840

<212> DNA

<213> Artificial sequence:XIKE-C-NdN BVDV-Sequence

<400> 11

gtatacgaga ttagctaaag aactcgtata tggattggac gtcaacaaat ttttaattgg 60

```
caacgtaggg aaccttcccc tcagcgaagg ccgaaaagag gctagccatg cccttagtag   120
gactagcaaa agtaggggac tagcggtagc agtgagttcg ttggatggcc gaacccctga   180
gtacagggga gtcgtcaatg gttcgacact ccattagtcg aggagtctcg agatgccatg   240
tggacgaggg catgcccacg gcacatctta acccatgcgg gggttgcatg ggtgaaagcg   300
ctattcgtgg cgttatggac acagcctgat agggtgtagc agagacctgc tattccgcta   360
gtaaaaactc tgctgtacat ggcacatgga gttgttttcc gatgaaggga gcaagggtgc   420
tacaagtaag aagcagccta agccagatag gatagaaaaa ggtaagatga aaatagcccc   480
aaaagagaca gaaaaagatt gcaaaaccag accccccgac gcgactatag tagtagaagg   540
ggttaagtac caggtgaaga aaaaaggaaa ggtaagggga aaaaatactc aagatgggtt   600
atatcacaac aagaataagc cccctgaatc aagaaaaaaa ttggaaaagg cactgctggc   660
ttgggccatc ttagcagcgg tcctgcttca gctggtaaca ggagagaata tcacccagtg   720
gaacttgatg gacaacggca ccgagggaat acagcaagcg atgttcctaa gaggggtgaa   780
caggagtcta ttaggaattt ggccagagaa aatttgcacc ggagtaccaa ctcacttagc   840
aacagactat gagcttaaag agatagtggg gatgatggac gcgagtgaga agaccaacta   900
cacgtgttgc aggttgcaaa gacatgagtg gaataaacat ggttggtgta actggtttca   960
tatagaaccg tggatatggt tgatgaacaa aacccaaaac aacctgacag aagggcaacc  1020
gcttagggag tgtgctgtga cttgtaggta tgacaaggaa acagaattga acatcgtgac  1080
acaggctagg gacagaccta caactctgac aggttgcaag aaaggcaaga atttctcttt  1140
cgcaggtgtt atactggatg ggccctgtaa ctttaaagta tcggttgaag atgtgctgtt  1200
caaggagcac gattgcggca acatgctgca agagaccgcg atacagctac tcgatggggc  1260
aaccaacacc attgagggag caagggtagg gacggccaag ttgacaacct ggttagggaa  1320
gcaattaggg atccttggta agaagttgga gaacaaaagc aaagcatggt ttggtgcaca  1380
tgcagcaagt ccatactgcg gagtggagag gaagatcggt tacgtatggt atacaaaaaa  1440
ctgcactcca gcttgccttc caagaaacac tagaataata ggccccggga aatttgatac  1500
caacgccgaa gatggaaaaa tactccatga gatggggggg cacctctcag aatttgtcct  1560
attgtccttg gtggttctgt ctgactttgc ccggaaacc gcgagcgtca tctacttggt   1620
tctacatttt gcgatcccgc aaagccacgt tgatgtagac acatgcgaca agaaccagct  1680
gaatttaacg gtagcaacca cagtagcaga ggtcatacca gggacagtgt ggaacctagg  1740
gaagtatgtc tgcataagac cagactggtg gccatatgag acgacgacag tcttcgtcat  1800
agaggaagca gggcaagtaa tcaaattgat gctaagggcc atcagagact taactaggat  1860
atggaatgct gccactacca cagctttctt aatcttttta gtaaaagcac tgaggggaca  1920
actaatccaa gggctattgt ggctgatgct aataacagga gcacagggct tccctgaatg  1980
caaagagggc ttccaatatg ccatatctaa agacaggaaa atggggttat tggggccaga  2040
gagcttaact acaacatggc acctccccac caaaaaaata gtggattcca tggtgcatgt  2100
atggtgtgaa ggaaaagact tgaaaatatt aaaaatgtgc acaaaggaag agaggtatct  2160
agtggctgtg cacgagagag ccttatcaac cagtgccgag tttatgcaga tcagtgatgg  2220
gacaataggc ccagacgtga tagatatgcc tgatgacttt gagtttggac tctgcccttg  2280
tgactcaaaa ccagtgataa agggcaaatt taatgccagc ttactgaatg gaccagcttt  2340
ccagatggta tgcccacagg ggtggactgg tacaatagaa tgcaccctag cgaaccaaga  2400
caccttggac acaactgtca ttaggacata tagaagaact accccatttc agcggagaaa  2460
atggtgtacc tatgaaaaaa taatagggga agatatctat gaatgcattc taggtggaaa  2520
ctggacatgc ataaccggtg accatagcag gttgaaagac ggacctatca agaagtgtaa  2580
gtggtgtggc catgacttcg tcaactcaga ggggctacca cactacccaa taggcaagtg  2640
catgctcatc aacgagagtg ggtacaggta tgtagatgac acctcttgcg ataggggtgg  2700
tgtagccata gttccatctg gcaccgtaaa gtgtagaata ggtaacgtca cggtgcaagt  2760
tatcgctact aacaatgatc tgggacccat gccttgcagc ccagctgaag tgatagcaag  2820
tgaaggacca gtggaaaaga ctgcatgcac attcaactat tcaaggactc tacctaataa  2880
gtattatgag ccaaggaccc ggtacttcca acaatacatg ttaaaagggg agtggcaata  2940
ttggttcgac ctggattctg tagaccacca caaagactac ttctcagagt tcataatcat  3000
agcagtggtc gccttgttgg gtggtaagta cgtactgtgg ctcttgataa catacacaat  3060
actgtctgag cagatggcta tgggtgctgg agtgaatact gaagagatag tcatgatagg  3120
caatttgctg acagacagtg atattgaggt tgtggtttat ttccttcttc tgtacttaat  3180
agttaaagag gaactggcga ggaaatggat tatactggta taccacatcc ttgtagccaa  3240
ccctatgaaa acaattgggg tcgtcttact aatgctaggg ggagtggtga aggccagcag  3300
aatcaatgct gatgaccaaa gtgctatgga cccatgcttt cttctcgtga caggcgtagt  3360
ggctgttttg atgatcgcta gaagagaacc tgccacatta ccactgattg tagcattgct  3420
agcaataaga acatcaggat tcctactgcc cgctagcatt gatgtaactg tagcagtagt  3480
```

attaattgta cttttgttgg ctagctacat aacagactac tttagatata aaaagtggct 3540
tcaactctta tttagtctga tagctggtat ctttattata aggagcttaa aacatatcaa 3600
ccagatggag gtaccagaaa tatctatgcc aagttggaga cctctagctc tggtcctttt 3660
ctatataaca tctacagcaa taaccactaa ttgggacatt gacttagcag gcttcctgct 3720
gcaatgggcg ccagcagtga tcatgatggc taccatgtgg gcagactttt tgactctgat 3780
catagtcctg cccagttacg agttatctaa gctttacttc ctaaagaacg tcaggacaga 3840
cgtggaaaag aactggctcg gcaaagtgaa atacagacag atcagttcag tttatgacat 3900
ctgtgacagt gaggaagcag tgtacctatt tccatcaagg cataagagtg gaagcaggcc 3960
agatttcata ttaccttttt tgaaagccgt gttaataagc tgcatcagca gccaatggca 4020
agtggtttac atttcttacc taatactgga aattacatac tatatgcaca ggaaaatcat 4080
agatgaggtg tcaggaggag caaattttct atcaagactc atagcagcca tcatagaatt 4140
aaattgggcc atagatgatg aggaatgtaa aggactgaag aaactgtatc tcttgtcagg 4200
gagagcgaag aatttgatag ttaaacataa ggtaagaaat gaagccgtcc acagatggtt 4260
tggtgaggag gaaatatacg gggcacccaa ggtgatcact atcataaaag ctagtaccct 4320
aagtaaaaac aggcactgca taatctgcac gatctgtgaa gggaaagaat ggaatggagc 4380
caactgccca aagtgtggaa gacaaggaaa gcccataaca tgtggaatga cactcgcaga 4440
ctttgaggag aaacattaca aaaagatatt tataagagaa gaatcttctt gtcctgtgcc 4500
ttttgatcct tcttgccatt gtaattattt tcgccacgat gggcctttca ggaaagagta 4560
taagggttac gtccaataca cagccagagg acaactcttt ctgaggaacc taccaattct 4620
agcgacgaag atgaagctat taatggtggg aaacctcggc gcagaaattg gcgacctgga 4680
acatctagga tgggtactga gagggccagc cgtgtgcaaa aaaattacca accatgagaa 4740
gtgccacgta aacatcatgg ataagctaac tgcatttttt ggaatcatgc ctagaggcac 4800
gacccctagg gcacctgtga ggttccccac agcactacta aaagtgagaa gggggctaga 4860
gacgggatgg gcttacacgc accaaggagg gatcagctcg gtagaccatg tcacagccgg 4920
aaaggattta ctagtgtgtg acagtatggg caggaccagg gttgtctgtc atagtaacaa 4980
taagatgact gatgagactg agtatggcat caagaccgac tcagggtgtc ccgaaggtgc 5040
gaggtgttac gtgctaaacc cagaagctgt taacatttct ggcacaaaag gagctatggt 5100
acacctccag aaaacggggg gggagttcac atgtgtcact gcctcaggga ccccggcttt 5160
cttcgatctg aaaaatctaa aaggctggtc cgggctacca atttttgaag catccagtgg 5220
cagggtggtt ggtagggtga aagtcggcaa gaatgaggat tccaagccca ccaaactaat 5280
gagcggaatc cagacagtgt ctaagaacca gacagaccta gcggacatcg taaaaaaatt 5340
gactagtatg aacagaggag agttcaaaca gataacatta gccactgggg caggaaaaac 5400
tacggaactg ccaaggtccg tcatagagga gatagggagg cacaaaaggg tcttagtcct 5460
gataccattg agagcagcag cagagtcagt gtatcagtat atgagagtga agtacccaag 5520
tatatctttc aatttgagaa taggagatat gaaggaaggt gacatggcca ctggtatcac 5580
ctacgcctca tatgggtact tttgtcagct tcctcagccc aaactgagag ctgccatggt 5640
agagtactca tatatattct tagatgagta ccactgtgct acacccgagc aattagcaat 5700
aattggaaag atacacaggt ttgctgaaaa tcttagagtg gtagcaatga cagcaacccc 5760
agctggaacg gtcacaacga ctggtcagaa acaccctata gaggagttca tagccccaga 5820
ggtgatgaaa ggtgaagatc taggtagtga atacttggat attgcagggt tgaagatacc 5880
gactgaagag atgaaaggca acatgctcgt gttcgcgcca actaggaaca tggcagtaga 5940
aacagctaag aaattgaagg ctaagggata caactctgga tactattaca gtggggaaaa 6000
cccagagaac ttgagggtgg taacctcgca atccccgtat gtggtagtag ccaccaatgc 6060
catagagtca ggtgtgacat taccagactt agacacagtt gtagacactg gactaaagtg 6120
tgagaagagg gtgaggattt cttcaaaaat gcccttcatt gtaacaggac ttaagagaat 6180
ggcagtcaca atcggagagc aagcccagcg caggggtaga gtaggaagag tcaagccagg 6240
taggtactat aggagtcaag aaacagcttc agggtcaaaa gattaccatt acgacctact 6300
gcaagcccag aggtacggaa tagaagatgg aattaatgta acaaagtcat tcagggagat 6360
gaactatgat tggagccttt acgaagagga cagcttgatg ataactcaac tcgaggtcct 6420
taacaacctc cttatatcag aagacctgcc tgccgcagtg aagaacatca tggcccggac 6480
cgatcaccca gaacccatac aactggccta taacagttat gaaaaccaaa ttccagtgct 6540
gttcccaaag atcaaaaatg gtgaggtgac agacagttat gagaattaca catatctcaa 6600
tgcaagaaaa ttaggagagg acgtgccggc atatgtgtac gccacagagg atgaggatct 6660
agcagtggat cttctgggta tggattggcc ggacccaggc aaccaacagg tggtagagac 6720
agggagggca ttaaaacaag taactggctt atccacagca gaaaacgccc tcttgatagc 6780
cctattcggc tacgtcgggt accagacact ttcaaaaagg cacataccca tgattactga 6840
catctataca cttgaagacc acaggcttga ggacacaacc cacctccagt ttgccccaaa 6900

```
cgctataagg accgacggca aggactcaga gttgaaggaa ttagctgtgg gagaccttga   6960
taaatatgtg gacgcactgg tagactactc caaacaaggg atgaaattca tcaaagtcca   7020
agctgaaaag gtcagagact cccagtctac gaaggaaggc ttgcaaacca ttaaggagta   7080
tgtggataag tttatacaat cactaacaga gaataaggag gagatcatca ggtatggact   7140
atggggagtt cacacggcac tctacaaaag cttggcagcg agactggggc atgaaacagc   7200
ttttgcaact ttagtggtaa aatggttggc ttttgggggc gaaacggtat ctgctcacat   7260
caagcaagta gcagttgatc tagtagtata ttatatcatc aacaaaccat cttttcctgg   7320
agatacagag acccaacaag aggggaggaa gtttgtggct agtcttttta tatctgcact   7380
agcaacatac acatataaaa cctggaatta caacaatctg caacgggttg tcgaacctgc   7440
cttagcttac ctcccatatg ctacaagtgc cttgaagttg ttcacaccca caagattaga   7500
gagtgtggtc atactcagtt ctacaattta caagacatac ctctctataa ggaagggtaa   7560
gagtgacggc ttgttaggta caggcataag tgcagccatg gagatcttaa accaaaaccc   7620
aatctcagta ggtatatctg tgatgctggg ggtaggtgcc atcgccgccc ataatgcaat   7680
agaatctagt gaacagaaaa gaactttgct gatgaaggtc tttgtaaaaa acttcttaga   7740
ccaagcagca acagatgagc tagtcaaaga gaaccctgaa aaaataatca tggctctatt   7800
tgaagcagtc cagaccatag gaaacccccct aagactcatc taccatctgt acggggtgta   7860
ctataagggg tgggaagcaa aagaactcgc agagaaaact gctggccgca acttattcac   7920
attgatcatg tttgaggcct ttgagctttt aggtatggac tcagaaggaa agataagaaa   7980
cttgtcaggc aactacatac tggacttaat cttcaacttg cataataaat taaacaaggg   8040
gctcaaaaaa ctagtccttg ggtgggctcc tgcacctttg agctgtgatt ggacaccaag   8100
tgatgagaga ataagcctac ctcataacaa ctacttaagg gtagaaacca ggtgtccttg   8160
tggctatgag atgaaggcaa taaaaaatgt tgctggtaaa ttgacaaaag ttgaagaaaa   8220
ggggtccttc ctatgcagga atagattagg gagaggacct ccaaacttca aagtaacaaa   8280
gttctatgat gataacttga tagaagtcaa gccagtagct aggctagaag gccaggtgga   8340
cctctattac aagggagtaa cagctaagtt agactacaac aatgggaaag tactgttagc   8400
taccaacaag tgggaggtgg accacgcttt cctgaccaga ctagtaaaga agcacacagg   8460
gataggtttt aaaggtgcat atttgggtga ccgaccagac catcaagatc ttgtcgatag   8520
agattgtgca actataacga agaactcagt acagttccta aaaatgaaga agggttgcgc   8580
tttcacatat gacctaacaa tctctaacct tgtcaggctt attgaactag tccataagaa   8640
taatttacaa gaaagagaga tccctaccgt gacagtaact acttggcttg catattcttt   8700
tgtcaatgaa gacctgggga ctatcaagcc tgtattgggg gagaaagtca tcccagaacc   8760
ccccgaggag ttgagtctcc aacccaccgt gagactagtc accactgaaa cagcaataac   8820
cataacaggg gaggctgaag tgatgacgac agggatcaca ccagtggtag agatgaaaga   8880
agaacctcag ctggaccacc agtcaactac cctaaaggta gggttgaagg aaggggaata   8940
tccagggcca ggagttaacc ctaaccattt agcagaggtg atagatgaga aagatgacag   9000
gccttttgtc ctaatcatcg gtaacaaagg ttctacctcg aacagagcaa gaacggccaa   9060
gaatatacgg ctgtacaaag gaaacaaccc aagagagatc agggatctga tgagccaagg   9120
aagaatattg acggttgctc taaaagagtt ggacccggaa ttaaaagaat tagtagatta   9180
caaggggacc tttctcaata gggaagcttt agaagcccta agcttaggta agccaatcaa   9240
gaggaaaacc acaacagcaa tgatcaggag gttaatagag ccagaggttg aggaggaact   9300
accagattgg ttccaagcgg aagaacccct atttttggaa gcaaaaatac agaatgactt   9360
ataccaccta attggcagtg tagatagtat aaaaagcaaa gcaaaggaat taggggccac   9420
agataacaca aagatagtga aggaagttgg ggctaggacc tatacgatga aattgagcag   9480
ctggagcaca caagttacaa aaaaacagat gagtctagcc cctctctttg aagagctgtt   9540
attaaagtgc cctccatgta gtaaaatttc aaagggacat atggtgtcag cataccaact   9600
ggctcaagga aactgggaac ccctcgggtg tggggtctat atgggaacca taccagctag   9660
gcgtctcaag atccaccctt atgaggctta ccttaaactc aaagagctgg tggaagttga   9720
atcttcgagg gccactgcaa aagaatccat cataagagaa cataacacct ggatcctgcg   9780
gaaggtgaga catgaaggga acctaagaac caaatcaatg atcaaccctg ggaaaatatc   9840
agatcagcta tgcagagatg gacacaaaag aaacatatat aataagatca taggctcaac   9900
aatggcctct gctggtatta ggctggagaa actgccagta gtccgagccc aaactgacac   9960
aaccagtttc caccaagcca taagagaaaa aattgataaa acagaaaaca agcagacccc   10020
tgaattgcat gaagaactaa tgaaggtctt cgactgctta aagatcccag agctgaagga   10080
atcgtatgat gaagtttcat gggaacaatt agaagccggg ataaaccgta agggtgcagc   10140
aggctatcta gagagcaaga acatagggga agtcctagac acagagaaac acatagtaga   10200
gcagctgatc aaggatctga ggaaggggaa gaagattagg tactatgaaa cagccatccc   10260
caagaatgag aagagagacg tcagcgacga ctgggaagcc ggagagttcg ttgatgaaaa   10320
```

```
gaaaccaaga gtaatccagt acccggacgc caaggtgaga ctggccatta caaaagtgat  10380
gtacaaatgg gtaaagcaaa aaccagtggt gatacccggc tatgaaggta aaacacctct  10440
atttgacata ttcaacaaag tgaagaagga atgggattca ttccaggacc ccgtagcagt  10500
gagctttgac accaaagcgt gggatacaca agtcaccagt agagacctaa tgttgataaa  10560
ggatatccag aaatattatt tcaagagaag tatacacaaa tttttagata caataacaga  10620
acacatggtg gaggtacctg tcattacagc agacggtgaa gtttacataa ggaatggtca  10680
gaggggtagt ggccaacccg acacaagtgc tggtaatagt atgttgaatg tcctaaccat  10740
gatatatgct ttctgtaaaa gtacaggcat accttacagg ggattcagca gagtggcaag  10800
aatccatgtg tgtggtgatg atggcttttt gataacagag agaggactgg gactgaaatt  10860
ctctgagaag ggtatgcaga tattacatga ggccgggaag ccccagaaaa taactgaagg  10920
ggacaaaatg aaagtggcat acagattcga ggacatagag ttttgttccc atactcccgt  10980
gccagtcaga tgggcagata acaccagtag ttacatggca gggaggagca cagccactat  11040
actagctaag atggcaacca ggctggattc cagcggagag aggggtagca cagcttatga  11100
gaaggccgta gccttcagct tccttttgat gtactcatgg aatcccgtag ttagaaggat  11160
ctgcttactg gtgttgtcac agtttccaga aatatcccca tccaaaaaca caatatacta  11220
ctaccaaggg gatcccatag ctgcgtacag agaagtgata gggaaacagc tgtgtgaact  11280
gaaaagaaca ggatttgaga agctggctgg tctgaatttg agtatgacca ctctaggcat  11340
ctggacaaaa catactagta aaagactaat ccaagcctgt gtagaaatag gtaagagaga  11400
aggtacctgg ttagttaatg ctgacagact gattgcagga aagactggga agttttacat  11460
cccaagcact ggtgtcactc tgttgggaaa acactatgag gaaattaact taaagcaaaa  11520
ggcggcacaa ccgccgatag agggggttga cagatataag ttgggcccca tagttaatgt  11580
tatcttgaga aggctgaggg tgatgctgat gacagttgcc agcggaagct ggtgaatccg  11640
tccggagcgt cgtgccctca ctcaaggttt ttaattgtaa atattgtaaa tagacagcta  11700
agatatttat tgtagttgga tagtaatgca gtgatagtaa ataccccaat ttaacactac  11760
ctccaatgca ctaagcactt tagctgtgtg aggttaactc gacgtccacg gttggactag  11820
ggaagacctc taacagcccc                                              11840
```

<210> 12
<211> 3749
<212> PRT
<213> Artificial sequence:XIKE-C-NdN

<400> 12

Met Glu Leu Phe Ser Asp Glu Gly Ser Lys Gly Ala Thr Ser Lys Lys
1               5                   10                  15

Gln Pro Lys Pro Asp Arg Ile Glu Lys Gly Lys Met Lys Ile Ala Pro
            20                  25                  30

Lys Glu Thr Glu Lys Asp Cys Lys Thr Arg Pro Pro Asp Ala Thr Ile
        35                  40                  45

Val Val Glu Gly Val Lys Tyr Gln Val Lys Lys Lys Gly Lys Val Arg
    50                  55                  60

Gly Lys Asn Thr Gln Asp Gly Leu Tyr His Asn Lys Asn Lys Pro Pro
65                  70                  75                  80

Glu Ser Arg Lys Lys Leu Glu Lys Ala Leu Leu Ala Trp Ala Ile Leu
                85                  90                  95

Ala Ala Val Leu Leu Gln Leu Val Thr Gly Glu Asn Ile Thr Gln Trp
            100                 105                 110

Asn Leu Met Asp Asn Gly Thr Glu Gly Ile Gln Gln Ala Met Phe Leu

115 120 125

Arg Gly Val Asn Arg Ser Leu Leu Gly Ile Trp Pro Glu Lys Ile Cys
130 135 140

Thr Gly Val Pro Thr His Leu Ala Thr Asp Tyr Glu Leu Lys Glu Ile
145 150 155 160

Val Gly Met Met Asp Ala Ser Glu Lys Thr Asn Tyr Thr Cys Cys Arg
165 170 175

Leu Gln Arg His Glu Trp Asn Lys His Gly Trp Cys Asn Trp Phe His
180 185 190

Ile Glu Pro Trp Ile Trp Leu Met Asn Lys Thr Gln Asn Asn Leu Thr
195 200 205

Glu Gly Gln Pro Leu Arg Glu Cys Ala Val Thr Cys Arg Tyr Asp Lys
210 215 220

Glu Thr Glu Leu Asn Ile Val Thr Gln Ala Arg Asp Arg Pro Thr Thr
225 230 235 240

Leu Thr Gly Cys Lys Lys Gly Lys Asn Phe Ser Phe Ala Gly Val Ile
245 250 255

Leu Asp Gly Pro Cys Asn Phe Lys Val Ser Val Glu Asp Val Leu Phe
260 265 270

Lys Glu His Asp Cys Gly Asn Met Leu Gln Glu Thr Ala Ile Gln Leu
275 280 285

Leu Asp Gly Ala Thr Asn Thr Ile Glu Gly Ala Arg Val Gly Thr Ala
290 295 300

Lys Leu Thr Thr Trp Leu Gly Lys Gln Leu Gly Ile Leu Gly Lys Lys
305 310 315 320

Leu Glu Asn Lys Ser Lys Ala Trp Phe Gly Ala His Ala Ala Ser Pro
325 330 335

Tyr Cys Gly Val Glu Arg Lys Ile Gly Tyr Val Trp Tyr Thr Lys Asn
340 345 350

Cys Thr Pro Ala Cys Leu Pro Arg Asn Thr Arg Ile Ile Gly Pro Gly
355 360 365

Lys Phe Asp Thr Asn Ala Glu Asp Gly Lys Ile Leu His Glu Met Gly
370 375 380

Gly His Leu Ser Glu Phe Val Leu Leu Ser Leu Val Val Leu Ser Asp
385 390 395 400

Phe Ala Pro Glu Thr Ala Ser Val Ile Tyr Leu Val Leu His Phe Ala
405 410 415

Ile Pro Gln Ser His Val Asp Val Asp Thr Cys Asp Lys Asn Gln Leu

420 425 430

Asn Leu Thr Val Ala Thr Thr Val Ala Glu Val Ile Pro Gly Thr Val
435 440 445

Trp Asn Leu Gly Lys Tyr Val Cys Ile Arg Pro Asp Trp Trp Pro Tyr
450 455 460

Glu Thr Thr Thr Val Phe Val Ile Glu Glu Ala Gly Gln Val Ile Lys
465 470 475 480

Leu Met Leu Arg Ala Ile Arg Asp Leu Thr Arg Ile Trp Asn Ala Ala
485 490 495

Thr Thr Thr Ala Phe Leu Ile Phe Leu Val Lys Ala Leu Arg Gly Gln
500 505 510

Leu Ile Gln Gly Leu Leu Trp Leu Met Leu Ile Thr Gly Ala Gln Gly
515 520 525

Phe Pro Glu Cys Lys Glu Gly Phe Gln Tyr Ala Ile Ser Lys Asp Arg
530 535 540

Lys Met Gly Leu Leu Gly Pro Glu Ser Leu Thr Thr Thr Trp His Leu
545 550 555 560

Pro Thr Lys Lys Ile Val Asp Ser Met Val His Val Trp Cys Glu Gly
565 570 575

Lys Asp Leu Lys Ile Leu Lys Met Cys Thr Lys Glu Glu Arg Tyr Leu
580 585 590

Val Ala Val His Glu Arg Ala Leu Ser Thr Ser Ala Glu Phe Met Gln
595 600 605

Ile Ser Asp Gly Thr Ile Gly Pro Asp Val Ile Asp Met Pro Asp Asp
610 615 620

Phe Glu Phe Gly Leu Cys Pro Cys Asp Ser Lys Pro Val Ile Lys Gly
625 630 635 640

Lys Phe Asn Ala Ser Leu Leu Asn Gly Pro Ala Phe Gln Met Val Cys
645 650 655

Pro Gln Gly Trp Thr Gly Thr Ile Glu Cys Thr Leu Ala Asn Gln Asp
660 665 670

Thr Leu Asp Thr Thr Val Ile Arg Thr Tyr Arg Arg Thr Thr Pro Phe
675 680 685

Gln Arg Arg Lys Trp Cys Thr Tyr Glu Lys Ile Ile Gly Glu Asp Ile
690 695 700

Tyr Glu Cys Ile Leu Gly Gly Asn Trp Thr Cys Ile Thr Gly Asp His
705 710 715 720

Ser Arg Leu Lys Asp Gly Pro Ile Lys Lys Cys Lys Trp Cys Gly His

725 730 735

Asp Phe Val Asn Ser Glu Gly Leu Pro His Tyr Pro Ile Gly Lys Cys
740 745 750

Met Leu Ile Asn Glu Ser Gly Tyr Arg Tyr Val Asp Asp Thr Ser Cys
755 760 765

Asp Arg Gly Gly Val Ala Ile Val Pro Ser Gly Thr Val Lys Cys Arg
770 775 780

Ile Gly Asn Val Thr Val Gln Val Ile Ala Thr Asn Asn Asp Leu Gly
785 790 795 800

Pro Met Pro Cys Ser Pro Ala Glu Val Ile Ala Ser Glu Gly Pro Val
805 810 815

Glu Lys Thr Ala Cys Thr Phe Asn Tyr Ser Arg Thr Leu Pro Asn Lys
820 825 830

Tyr Tyr Glu Pro Arg Asp Arg Tyr Phe Gln Gln Tyr Met Leu Lys Gly
835 840 845

Glu Trp Gln Tyr Trp Phe Asp Leu Asp Ser Val Asp His His Lys Asp
850 855 860

Tyr Phe Ser Glu Phe Ile Ile Ile Ala Val Val Ala Leu Leu Gly Gly
865 870 875 880

Lys Tyr Val Leu Trp Leu Leu Ile Thr Tyr Thr Ile Leu Ser Glu Gln
885 890 895

Met Ala Met Gly Ala Gly Val Asn Thr Glu Glu Ile Val Met Ile Gly
900 905 910

Asn Leu Leu Thr Asp Ser Asp Ile Glu Val Val Val Tyr Phe Leu Leu
915 920 925

Leu Tyr Leu Ile Val Lys Glu Glu Leu Ala Arg Lys Trp Ile Ile Leu
930 935 940

Val Tyr His Ile Leu Val Ala Asn Pro Met Lys Thr Ile Gly Val Val
945 950 955 960

Leu Leu Met Leu Gly Gly Val Val Lys Ala Ser Arg Ile Asn Ala Asp
965 970 975

Asp Gln Ser Ala Met Asp Pro Cys Phe Leu Leu Val Thr Gly Val Val
980 985 990

Ala Val Leu Met Ile Ala Arg Arg Glu Pro Ala Thr Leu Pro Leu Ile
995 1000 1005

Val Ala Leu Leu Ala Ile Arg Thr Ser Gly Phe Leu Leu Pro Ala
1010 1015 1020

Ser Ile Asp Val Thr Val Ala Val Val Leu Ile Val Leu Leu Leu

1025 1030 1035

Ala Ser Tyr Ile Thr Asp Tyr Phe Arg Tyr Lys Lys Trp Leu Gln
1040 1045 1050

Leu Leu Phe Ser Leu Ile Ala Gly Ile Phe Ile Ile Arg Ser Leu
1055 1060 1065

Lys His Ile Asn Gln Met Glu Val Pro Glu Ile Ser Met Pro Ser
1070 1075 1080

Trp Arg Pro Leu Ala Leu Val Leu Phe Tyr Ile Thr Ser Thr Ala
1085 1090 1095

Ile Thr Thr Asn Trp Asp Ile Asp Leu Ala Gly Phe Leu Leu Gln
1100 1105 1110

Trp Ala Pro Ala Val Ile Met Met Ala Thr Met Trp Ala Asp Phe
1115 1120 1125

Leu Thr Leu Ile Ile Val Leu Pro Ser Tyr Glu Leu Ser Lys Leu
1130 1135 1140

Tyr Phe Leu Lys Asn Val Arg Thr Asp Val Glu Lys Asn Trp Leu
1145 1150 1155

Gly Lys Val Lys Tyr Arg Gln Ile Ser Ser Val Tyr Asp Ile Cys
1160 1165 1170

Asp Ser Glu Glu Ala Val Tyr Leu Phe Pro Ser Arg His Lys Ser
1175 1180 1185

Gly Ser Arg Pro Asp Phe Ile Leu Pro Phe Leu Lys Ala Val Leu
1190 1195 1200

Ile Ser Cys Ile Ser Ser Gln Trp Gln Val Val Tyr Ile Ser Tyr
1205 1210 1215

Leu Ile Leu Glu Ile Thr Tyr Tyr Met His Arg Lys Ile Ile Asp
1220 1225 1230

Glu Val Ser Gly Gly Ala Asn Phe Leu Ser Arg Leu Ile Ala Ala
1235 1240 1245

Ile Ile Glu Leu Asn Trp Ala Ile Asp Asp Glu Glu Cys Lys Gly
1250 1255 1260

Leu Lys Lys Leu Tyr Leu Leu Ser Gly Arg Ala Lys Asn Leu Ile
1265 1270 1275

Val Lys His Lys Val Arg Asn Glu Ala Val His Arg Trp Phe Gly
1280 1285 1290

Glu Glu Glu Ile Tyr Gly Ala Pro Lys Val Ile Thr Ile Ile Lys
1295 1300 1305

Ala Ser Thr Leu Ser Lys Asn Arg His Cys Ile Ile Cys Thr Ile

1310 1315 1320

Cys Glu Gly Lys Glu Trp Asn Gly Ala Asn Cys Pro Lys Cys Gly
1325 1330 1335

Arg Gln Gly Lys Pro Ile Thr Cys Gly Met Thr Leu Ala Asp Phe
1340 1345 1350

Glu Glu Lys His Tyr Lys Lys Ile Phe Ile Arg Glu Glu Ser Ser
1355 1360 1365

Cys Pro Val Pro Phe Asp Pro Ser Cys His Cys Asn Tyr Phe Arg
1370 1375 1380

His Asp Gly Pro Phe Arg Lys Glu Tyr Lys Gly Tyr Val Gln Tyr
1385 1390 1395

Thr Ala Arg Gly Gln Leu Phe Leu Arg Asn Leu Pro Ile Leu Ala
1400 1405 1410

Thr Lys Met Lys Leu Leu Met Val Gly Asn Leu Gly Ala Glu Ile
1415 1420 1425

Gly Asp Leu Glu His Leu Gly Trp Val Leu Arg Gly Pro Ala Val
1430 1435 1440

Cys Lys Lys Ile Thr Asn His Glu Lys Cys His Val Asn Ile Met
1445 1450 1455

Asp Lys Leu Thr Ala Phe Phe Gly Ile Met Pro Arg Gly Thr Thr
1460 1465 1470

Pro Arg Ala Pro Val Arg Phe Pro Thr Ala Leu Leu Lys Val Arg
1475 1480 1485

Arg Gly Leu Glu Thr Gly Trp Ala Tyr Thr His Gln Gly Gly Ile
1490 1495 1500

Ser Ser Val Asp His Val Thr Ala Gly Lys Asp Leu Leu Val Cys
1505 1510 1515

Asp Ser Met Gly Arg Thr Arg Val Val Cys His Ser Asn Asn Lys
1520 1525 1530

Met Thr Asp Glu Thr Glu Tyr Gly Ile Lys Thr Asp Ser Gly Cys
1535 1540 1545

Pro Glu Gly Ala Arg Cys Tyr Val Leu Asn Pro Glu Ala Val Asn
1550 1555 1560

Ile Ser Gly Thr Lys Gly Ala Met Val His Leu Gln Lys Thr Gly
1565 1570 1575

Gly Glu Phe Thr Cys Val Thr Ala Ser Gly Thr Pro Ala Phe Phe
1580 1585 1590

Asp Leu Lys Asn Leu Lys Gly Trp Ser Gly Leu Pro Ile Phe Glu

1595 1600 1605

Ala Ser Ser Gly Arg Val Val Gly Arg Val Lys Val Gly Lys Asn
1610 1615 1620

Glu Asp Ser Lys Pro Thr Lys Leu Met Ser Gly Ile Gln Thr Val
1625 1630 1635

Ser Lys Asn Gln Thr Asp Leu Ala Asp Ile Val Lys Lys Leu Thr
1640 1645 1650

Ser Met Asn Arg Gly Glu Phe Lys Gln Ile Thr Leu Ala Thr Gly
1655 1660 1665

Ala Gly Lys Thr Thr Glu Leu Pro Arg Ser Val Ile Glu Glu Ile
1670 1675 1680

Gly Arg His Lys Arg Val Leu Val Leu Ile Pro Leu Arg Ala Ala
1685 1690 1695

Ala Glu Ser Val Tyr Gln Tyr Met Arg Val Lys Tyr Pro Ser Ile
1700 1705 1710

Ser Phe Asn Leu Arg Ile Gly Asp Met Lys Glu Gly Asp Met Ala
1715 1720 1725

Thr Gly Ile Thr Tyr Ala Ser Tyr Gly Tyr Phe Cys Gln Leu Pro
1730 1735 1740

Gln Pro Lys Leu Arg Ala Ala Met Val Glu Tyr Ser Tyr Ile Phe
1745 1750 1755

Leu Asp Glu Tyr His Cys Ala Thr Pro Glu Gln Leu Ala Ile Ile
1760 1765 1770

Gly Lys Ile His Arg Phe Ala Glu Asn Leu Arg Val Val Ala Met
1775 1780 1785

Thr Ala Thr Pro Ala Gly Thr Val Thr Thr Thr Gly Gln Lys His
1790 1795 1800

Pro Ile Glu Glu Phe Ile Ala Pro Glu Val Met Lys Gly Glu Asp
1805 1810 1815

Leu Gly Ser Glu Tyr Leu Asp Ile Ala Gly Leu Lys Ile Pro Thr
1820 1825 1830

Glu Glu Met Lys Gly Asn Met Leu Val Phe Ala Pro Thr Arg Asn
1835 1840 1845

Met Ala Val Glu Thr Ala Lys Lys Leu Lys Ala Lys Gly Tyr Asn
1850 1855 1860

Ser Gly Tyr Tyr Tyr Ser Gly Glu Asn Pro Glu Asn Leu Arg Val
1865 1870 1875

Val Thr Ser Gln Ser Pro Tyr Val Val Val Ala Thr Asn Ala Ile

1880 1885 1890

Glu Ser Gly Val Thr Leu Pro Asp Leu Asp Thr Val Val Asp Thr
1895 1900 1905

Gly Leu Lys Cys Glu Lys Arg Val Arg Ile Ser Ser Lys Met Pro
1910 1915 1920

Phe Ile Val Thr Gly Leu Lys Arg Met Ala Val Thr Ile Gly Glu
1925 1930 1935

Gln Ala Gln Arg Arg Gly Arg Val Gly Arg Val Lys Pro Gly Arg
1940 1945 1950

Tyr Tyr Arg Ser Gln Glu Thr Ala Ser Gly Ser Lys Asp Tyr His
1955 1960 1965

Tyr Asp Leu Leu Gln Ala Gln Arg Tyr Gly Ile Glu Asp Gly Ile
1970 1975 1980

Asn Val Thr Lys Ser Phe Arg Glu Met Asn Tyr Asp Trp Ser Leu
1985 1990 1995

Tyr Glu Glu Asp Ser Leu Met Ile Thr Gln Leu Glu Val Leu Asn
2000 2005 2010

Asn Leu Leu Ile Ser Glu Asp Leu Pro Ala Ala Val Lys Asn Ile
2015 2020 2025

Met Ala Arg Thr Asp His Pro Glu Pro Ile Gln Leu Ala Tyr Asn
2030 2035 2040

Ser Tyr Glu Asn Gln Ile Pro Val Leu Phe Pro Lys Ile Lys Asn
2045 2050 2055

Gly Glu Val Thr Asp Ser Tyr Glu Asn Tyr Thr Tyr Leu Asn Ala
2060 2065 2070

Arg Lys Leu Gly Glu Asp Val Pro Ala Tyr Val Tyr Ala Thr Glu
2075 2080 2085

Asp Glu Asp Leu Ala Val Asp Leu Leu Gly Met Asp Trp Pro Asp
2090 2095 2100

Pro Gly Asn Gln Gln Val Val Glu Thr Gly Arg Ala Leu Lys Gln
2105 2110 2115

Val Thr Gly Leu Ser Thr Ala Glu Asn Ala Leu Leu Ile Ala Leu
2120 2125 2130

Phe Gly Tyr Val Gly Tyr Gln Thr Leu Ser Lys Arg His Ile Pro
2135 2140 2145

Met Ile Thr Asp Ile Tyr Thr Leu Glu Asp His Arg Leu Glu Asp
2150 2155 2160

Thr Thr His Leu Gln Phe Ala Pro Asn Ala Ile Arg Thr Asp Gly

2165 2170 2175

Lys Asp Ser Glu Leu Lys Glu Leu Ala Val Gly Asp Leu Asp Lys
2180 2185 2190

Tyr Val Asp Ala Leu Val Asp Tyr Ser Lys Gln Gly Met Lys Phe
2195 2200 2205

Ile Lys Val Gln Ala Glu Lys Val Arg Asp Ser Gln Ser Thr Lys
2210 2215 2220

Glu Gly Leu Gln Thr Ile Lys Glu Tyr Val Asp Lys Phe Ile Gln
2225 2230 2235

Ser Leu Thr Glu Asn Lys Glu Glu Ile Ile Arg Tyr Gly Leu Trp
2240 2245 2250

Gly Val His Thr Ala Leu Tyr Lys Ser Leu Ala Ala Arg Leu Gly
2255 2260 2265

His Glu Thr Ala Phe Ala Thr Leu Val Val Lys Trp Leu Ala Phe
2270 2275 2280

Gly Gly Glu Thr Val Ser Ala His Ile Lys Gln Val Ala Val Asp
2285 2290 2295

Leu Val Val Tyr Tyr Ile Ile Asn Lys Pro Ser Phe Pro Gly Asp
2300 2305 2310

Thr Glu Thr Gln Gln Glu Gly Arg Arg Phe Val Ala Ser Leu Phe
2315 2320 2325

Ile Ser Ala Leu Ala Thr Tyr Thr Tyr Lys Thr Trp Asn Tyr Asn
2330 2335 2340

Asn Leu Gln Arg Val Val Glu Pro Ala Leu Ala Tyr Leu Pro Tyr
2345 2350 2355

Ala Thr Ser Ala Leu Lys Leu Phe Thr Pro Thr Arg Leu Glu Ser
2360 2365 2370

Val Val Ile Leu Ser Ser Thr Ile Tyr Lys Thr Tyr Leu Ser Ile
2375 2380 2385

Arg Lys Gly Lys Ser Asp Gly Leu Leu Gly Thr Gly Ile Ser Ala
2390 2395 2400

Ala Met Glu Ile Leu Asn Gln Asn Pro Ile Ser Val Gly Ile Ser
2405 2410 2415

Val Met Leu Gly Val Gly Ala Ile Ala Ala His Asn Ala Ile Glu
2420 2425 2430

Ser Ser Glu Gln Lys Arg Thr Leu Leu Met Lys Val Phe Val Lys
2435 2440 2445

Asn Phe Leu Asp Gln Ala Ala Thr Asp Glu Leu Val Lys Glu Asn

2450 2455 2460

Pro Glu Lys Ile Ile Met Ala Leu Phe Glu Ala Val Gln Thr Ile
2465 2470 2475

Gly Asn Pro Leu Arg Leu Ile Tyr His Leu Tyr Gly Val Tyr Tyr
2480 2485 2490

Lys Gly Trp Glu Ala Lys Glu Leu Ala Glu Lys Thr Ala Gly Arg
2495 2500 2505

Asn Leu Phe Thr Leu Ile Met Phe Glu Ala Phe Glu Leu Leu Gly
2510 2515 2520

Met Asp Ser Glu Gly Lys Ile Arg Asn Leu Ser Gly Asn Tyr Ile
2525 2530 2535

Leu Asp Leu Ile Phe Asn Leu His Asn Lys Leu Asn Lys Gly Leu
2540 2545 2550

Lys Lys Leu Val Leu Gly Trp Ala Pro Ala Pro Leu Ser Cys Asp
2555 2560 2565

Trp Thr Pro Ser Asp Glu Arg Ile Ser Leu Pro His Asn Asn Tyr
2570 2575 2580

Leu Arg Val Glu Thr Arg Cys Pro Cys Gly Tyr Glu Met Lys Ala
2585 2590 2595

Ile Lys Asn Val Ala Gly Lys Leu Thr Lys Val Glu Glu Lys Gly
2600 2605 2610

Ser Phe Leu Cys Arg Asn Arg Leu Gly Arg Gly Pro Pro Asn Phe
2615 2620 2625

Lys Val Thr Lys Phe Tyr Asp Asp Asn Leu Ile Glu Val Lys Pro
2630 2635 2640

Val Ala Arg Leu Glu Gly Gln Val Asp Leu Tyr Tyr Lys Gly Val
2645 2650 2655

Thr Ala Lys Leu Asp Tyr Asn Asn Gly Lys Val Leu Leu Ala Thr
2660 2665 2670

Asn Lys Trp Glu Val Asp His Ala Phe Leu Thr Arg Leu Val Lys
2675 2680 2685

Lys His Thr Gly Ile Gly Phe Lys Gly Ala Tyr Leu Gly Asp Arg
2690 2695 2700

Pro Asp His Gln Asp Leu Val Asp Arg Asp Cys Ala Thr Ile Thr
2705 2710 2715

Lys Asn Ser Val Gln Phe Leu Lys Met Lys Lys Gly Cys Ala Phe
2720 2725 2730

Thr Tyr Asp Leu Thr Ile Ser Asn Leu Val Arg Leu Ile Glu Leu

2735 2740 2745

Val His Lys Asn Asn Leu Gln Glu Arg Glu Ile Pro Thr Val Thr
2750 2755 2760

Val Thr Thr Trp Leu Ala Tyr Ser Phe Val Asn Glu Asp Leu Gly
2765 2770 2775

Thr Ile Lys Pro Val Leu Gly Glu Lys Val Ile Pro Glu Pro Pro
2780 2785 2790

Glu Glu Leu Ser Leu Gln Pro Thr Val Arg Leu Val Thr Thr Glu
2795 2800 2805

Thr Ala Ile Thr Ile Thr Gly Glu Ala Glu Val Met Thr Thr Gly
2810 2815 2820

Ile Thr Pro Val Val Glu Met Lys Glu Glu Pro Gln Leu Asp His
2825 2830 2835

Gln Ser Thr Thr Leu Lys Val Gly Leu Lys Glu Gly Glu Tyr Pro
2840 2845 2850

Gly Pro Gly Val Asn Pro Asn His Leu Ala Glu Val Ile Asp Glu
2855 2860 2865

Lys Asp Asp Arg Pro Phe Val Leu Ile Ile Gly Asn Lys Gly Ser
2870 2875 2880

Thr Ser Asn Arg Ala Arg Thr Ala Lys Asn Ile Arg Leu Tyr Lys
2885 2890 2895

Gly Asn Asn Pro Arg Glu Ile Arg Asp Leu Met Ser Gln Gly Arg
2900 2905 2910

Ile Leu Thr Val Ala Leu Lys Glu Leu Asp Pro Glu Leu Lys Glu
2915 2920 2925

Leu Val Asp Tyr Lys Gly Thr Phe Leu Asn Arg Glu Ala Leu Glu
2930 2935 2940

Ala Leu Ser Leu Gly Lys Pro Ile Lys Arg Lys Thr Thr Thr Ala
2945 2950 2955

Met Ile Arg Arg Leu Ile Glu Pro Glu Val Glu Glu Glu Leu Pro
2960 2965 2970

Asp Trp Phe Gln Ala Glu Glu Pro Leu Phe Leu Glu Ala Lys Ile
2975 2980 2985

Gln Asn Asp Leu Tyr His Leu Ile Gly Ser Val Asp Ser Ile Lys
2990 2995 3000

Ser Lys Ala Lys Glu Leu Gly Ala Thr Asp Asn Thr Lys Ile Val
3005 3010 3015

Lys Glu Val Gly Ala Arg Thr Tyr Thr Met Lys Leu Ser Ser Trp

3020 3025 3030

Ser Thr Gln Val Thr Lys Lys Gln Met Ser Leu Ala Pro Leu Phe
3035 3040 3045

Glu Glu Leu Leu Leu Lys Cys Pro Pro Cys Ser Lys Ile Ser Lys
3050 3055 3060

Gly His Met Val Ser Ala Tyr Gln Leu Ala Gln Gly Asn Trp Glu
3065 3070 3075

Pro Leu Gly Cys Gly Val Tyr Met Gly Thr Ile Pro Ala Arg Arg
3080 3085 3090

Leu Lys Ile His Pro Tyr Glu Ala Tyr Leu Lys Leu Lys Glu Leu
3095 3100 3105

Val Glu Val Glu Ser Ser Arg Ala Thr Ala Lys Glu Ser Ile Ile
3110 3115 3120

Arg Glu His Asn Thr Trp Ile Leu Arg Lys Val Arg His Glu Gly
3125 3130 3135

Asn Leu Arg Thr Lys Ser Met Ile Asn Pro Gly Lys Ile Ser Asp
3140 3145 3150

Gln Leu Cys Arg Asp Gly His Lys Arg Asn Ile Tyr Asn Lys Ile
3155 3160 3165

Ile Gly Ser Thr Met Ala Ser Ala Gly Ile Arg Leu Glu Lys Leu
3170 3175 3180

Pro Val Val Arg Ala Gln Thr Asp Thr Thr Ser Phe His Gln Ala
3185 3190 3195

Ile Arg Glu Lys Ile Asp Lys Thr Glu Asn Lys Gln Thr Pro Glu
3200 3205 3210

Leu His Glu Glu Leu Met Lys Val Phe Asp Cys Leu Lys Ile Pro
3215 3220 3225

Glu Leu Lys Glu Ser Tyr Asp Glu Val Ser Trp Glu Gln Leu Glu
3230 3235 3240

Ala Gly Ile Asn Arg Lys Gly Ala Ala Gly Tyr Leu Glu Ser Lys
3245 3250 3255

Asn Ile Gly Glu Val Leu Asp Thr Glu Lys His Ile Val Glu Gln
3260 3265 3270

Leu Ile Lys Asp Leu Arg Lys Gly Lys Lys Ile Arg Tyr Tyr Glu
3275 3280 3285

Thr Ala Ile Pro Lys Asn Glu Lys Arg Asp Val Ser Asp Asp Trp
3290 3295 3300

Glu Ala Gly Glu Phe Val Asp Glu Lys Lys Pro Arg Val Ile Gln

3305 3310 3315

Tyr Pro Asp Ala Lys Val Arg Leu Ala Ile Thr Lys Val Met Tyr
3320 3325 3330

Lys Trp Val Lys Gln Lys Pro Val Val Ile Pro Gly Tyr Glu Gly
3335 3340 3345

Lys Thr Pro Leu Phe Asp Ile Phe Asn Lys Val Lys Lys Glu Trp
3350 3355 3360

Asp Ser Phe Gln Asp Pro Val Ala Val Ser Phe Asp Thr Lys Ala
3365 3370 3375

Trp Asp Thr Gln Val Thr Ser Arg Asp Leu Met Leu Ile Lys Asp
3380 3385 3390

Ile Gln Lys Tyr Tyr Phe Lys Arg Ser Ile His Lys Phe Leu Asp
3395 3400 3405

Thr Ile Thr Glu His Met Val Glu Val Pro Val Ile Thr Ala Asp
3410 3415 3420

Gly Glu Val Tyr Ile Arg Asn Gly Gln Arg Gly Ser Gly Gln Pro
3425 3430 3435

Asp Thr Ser Ala Gly Asn Ser Met Leu Asn Val Leu Thr Met Ile
3440 3445 3450

Tyr Ala Phe Cys Lys Ser Thr Gly Ile Pro Tyr Arg Gly Phe Ser
3455 3460 3465

Arg Val Ala Arg Ile His Val Cys Gly Asp Asp Gly Phe Leu Ile
3470 3475 3480

Thr Glu Arg Gly Leu Gly Leu Lys Phe Ser Glu Lys Gly Met Gln
3485 3490 3495

Ile Leu His Glu Ala Gly Lys Pro Gln Lys Ile Thr Glu Gly Asp
3500 3505 3510

Lys Met Lys Val Ala Tyr Arg Phe Glu Asp Ile Glu Phe Cys Ser
3515 3520 3525

His Thr Pro Val Pro Val Arg Trp Ala Asp Asn Thr Ser Ser Tyr
3530 3535 3540

Met Ala Gly Arg Ser Thr Ala Thr Ile Leu Ala Lys Met Ala Thr
3545 3550 3555

Arg Leu Asp Ser Ser Gly Glu Arg Gly Ser Thr Ala Tyr Glu Lys
3560 3565 3570

Ala Val Ala Phe Ser Phe Leu Leu Met Tyr Ser Trp Asn Pro Val
3575 3580 3585

Val Arg Arg Ile Cys Leu Leu Val Leu Ser Gln Phe Pro Glu Ile

```
                3590                    3595                    3600

Ser Pro  Ser Lys Asn Thr Ile  Tyr Tyr Tyr Gln Gly  Asp Pro Ile
    3605                    3610                    3615

Ala Ala  Tyr Arg Glu Val Ile  Gly Lys Gln Leu Cys  Glu Leu Lys
    3620                    3625                    3630

Arg Thr  Gly Phe Glu Lys Leu  Ala Gly Leu Asn Leu  Ser Met Thr
    3635                    3640                    3645

Thr Leu  Gly Ile Trp Thr Lys  His Thr Ser Lys Arg  Leu Ile Gln
    3650                    3655                    3660

Ala Cys  Val Glu Ile Gly Lys  Arg Glu Gly Thr Trp  Leu Val Asn
    3665                    3670                    3675

Ala Asp  Arg Leu Ile Ala Gly  Lys Thr Gly Lys Phe  Tyr Ile Pro
    3680                    3685                    3690

Ser Thr  Gly Val Thr Leu Leu  Gly Lys His Tyr Glu  Glu Ile Asn
    3695                    3700                    3705

Leu Lys  Gln Lys Ala Ala Gln  Pro Pro Ile Glu Gly  Val Asp Arg
    3710                    3715                    3720

Tyr Lys  Leu Gly Pro Ile Val  Asn Val Ile Leu Arg  Arg Leu Arg
    3725                      3730                     3735

Val Met  Leu Met Thr Val Ala  Ser Gly Ser Trp
    3740                      3745
```